# FASCICULE DE BREVET EUROPEEN

(11) **EP 1 263 720 B1**
(45) Date de publication et mention de la délivrance du brevet: **03.06.2009**
(21) Numéro de dépôt: 01909937.3
(22) Date de dépôt: 01.03.2001
(51) Int. Cl.: C07D 205/04, C07D 205/06, C07D 403/10, C07D 403/04, C07D 409/14, C07D 403/14, C07D 409/06, C07D 401/12, C07D 409/12, C07D 401/06, C07D 403/06, A61K 31/397, A61P 25/00

(54) **DERIVES D'AZETIDINE, LEUR PREPARATION ET LES COMPOSITIONS PHARMACEUTIQUES LES CONTENANT**
AZETIDINDERIVATE, IHRE HERSTELLUNG UND SIE ENTHALTENDE PHARMAZEUTISCHE ZUSAMMENSETZUNGEN
AZETIDINE DERIVATIVES, PREPARATION THEREOF AND PHARMACEUTICAL COMPOSITIONS CONTAINING SAME

(30) Priorité: 03.03.2000 FR 0002775
(43) Date de publication de la demande: 11.12.2002
(73) Titulaire: Aventis Pharma S.A., 92160 Antony (FR)
(72) Inventeur: ACHARD, Daniel, F-94320 Thiais (FR); BOUCHARD, Hervé, F-94320 Thiais (FR); BOUQUEREL, Jean, F-93700 Drancy (FR); FILOCHE, Bruno, F-94000 Créteil (FR); GRISONI, Serge, F-94600 Choisy le Roi (FR); HITTINGER, Augustin, F-91430 Igny (FR); MYERS, Michael, Fishers, Indiana 46038 (US)
(74) Mandataire: Rousseau, Pierrick Edouard
(86) Numéro de dépôt international: PCT/FR2001/000600
(87) Numéro de publication internationale: WO 2001/064632

(56) Documents cités:
- EP-A- 0 406 112
- WO-A-97/01556
- US-A- 4 242 261

## Description

La présente invention concerne des dérivés de formule : 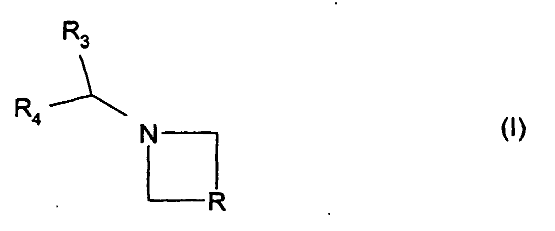 leurs sels, leur préparation et les médicaments les contenant.

Dans la formule (I),
R représente un radical CR₁R₂, C=C(R₅)SO₂R₆ ou C=C(R₇)SO₂alk,
soit R₁ représente un atome d'hydrogène et R₂ représente un radical -C(R₈)(R₉)(R₁₀), -C(R₈)(R₁₁)(R₁₂), -CO-NR₁₃R₁₄, -CH₂-CO-NR₁₃R₁₄, -CH₂-CO-R₆, -CO-R₆, -CO-cycloalkyle, -SO-R₆, -SO₂-R₆, -C(OH)(R₁₂)(R₆), -C(OH)(R₆)(alkyle), -C(=NOalk)R₆, -C(=NO-CH₂-CH=CH₂)R₆. -CH₂CH(R₆)NR₃₁R₃₂, -CH₂-C(=NOalk)R₆, -CH(R₆)NR₃₁R₃₂, -CH(R₆)NHSO₂alk, -CH(R₆)NHCONHalk ou -CH(R₆)NHCOalk,
soit R₁ représente un radical alkyle, NH-R₁₅, cyano, -S-alk-NR₁₆R₁₇, -CH₂-NR₁₈R₁₉, ou -NR₂₀R₂₁ et R₂ représente un radical -C(R₈)(R₁₁)(R₁₂),
R₃ et R₄, identiques ou différents, représentent soit un radical alkyle ou cycloalkyle, soit un aromatique choisi parmi phényle, naphtyle ou indényle, ces aromatiques étant non substitués ou substitués par un ou plusieurs halogène, alkyle, alcoxy, formyle, hydroxy, trifluorométhyle, trifluorométhoxy, -CO-alk, cyano, -COOH, -COOalk, -CONR₂₂R₂₃, -CO-NH-NR₂₄R₂₅, alkylsulfanyle, alkylsulfinyle, alkylsulfonyle, alkysulfanylalkyle, alkylsulfinylalkyle, alkylsulfonylalkyle, hydroxyalkyle ou -alk-NR₂₄R₂₅; soit un hétéroaromatique choisi parmi les cycles benzofuryle, benzothiazolyle, benzothiényle, benzoxazolyle, chromannyle, 2,3-dihydrobenzofuryle, 2,3-dihydrobenzothiényle, furyle, imidazolyle, isochromannyle, isoquinolyle, pyrrolyle, pyridyle, pyrimidinyle, quinolyle, 1,2,3,4-tétrahydroisoquinolyle, thiazolyle, thiényle, ces hétéroaromatiques pouvant être non substitués ou substitués par un ou plusieurs halogène, alkyle, alcoxy, hydroxy, trifluorométhyle, trifluorométhoxy, cyano, -COOH, -COOalk, -CO-NH-NR₂₄R₂₅ -CONR₂₂R₂₃, -alk-NR₂₄R₂₅, alkylsulfanyle, alkylsulfinyle, alkylsulfonyle, alkylsulfanylalkyle, alkylsulfinylalkyle, alkylsulfonylalkyle ou hydroxyalkyle,
R₅ représente un atome d'hydrogène ou un radical alkyle,
R₆ représente un radical Ar ou Het,
R₇ représente un radical cycloalkyle, hétérocycloalkyle ou hétérocyclényle éventuellement substitué par un radical -CSO-phényle,
R₈ représente un atome d'hydrogène ou un radical alkyle,
R₉ représente un radical -CO-NR₂₆R₂₇ -COOH, -COOalk, -CH₂OH, -NH-CO-NH-alk, -CH₂-NHR₂₈ ou -NHCOOalk,
R₁₀ représente un radical Ar ou Het,
R₁₁ représente un radical -SO₂-alk, -SO₂-Ar, -SO₂-Het,
R₁₂ représente un atome d'hydrogène ou un radical Ar ou Het,
R₁₃ représente un atome d'hydrogène ou un radical alkyle,
R₁₄ représente un radical Ar, Het, -alk-Ar ou -alk-Het,
R₁₅ représente un radical alkyle, cycloalkyle ou -alk-NR₂₉R₃₀,
R₁₆ et R₁₇, identiques ou différents, représentent un atome d'hydrogène ou un radical alkyle ou bien R₁₆ et R₁₇ forment ensemble avec l'atome d'azote auquel ils sont rattachés un hétérocycle mono ou bicyclique insaturé ou saturé, ayant 3 à 10 chaînons et contenant éventuellement un ou plusieurs autres hétéroatomes choisis parmi oxygène, soufre et azote et éventuellement substitué par un ou plusieurs radicaux alkyle,
R₁₈ représente un atome d'hydrogène ou un radical alkyle,
R₁₉ représente un atome d'hydrogène ou un radical alkyle, cycloalkyle, cycloalkylalkyle, cycloalkylcarbonyle, -SO₂alk, -CO-NHalk ou -COOalk,
ou bien R₁₆ et R₁₉ forment avec l'atome d'azote auquel ils sont rattachés un hétérocycle mono ou bicyclique insaturé ou saturé, ayant 3 à 10 chaînons et contenant éventuellement un ou plusieurs hétéroatomes choisis parmi oxygène, soufre et azote et éventuellement substitué par un ou plusieurs radicaux alkyle,
-NR₂₀R₂₁ représente un hétérocycle monocyclique saturé ou insaturé ayant 3 à 8 chaînons et contenant éventuellement un autre hétéroatome choisi parmi oxygène, azote et soufre,
R₂₂ et R₂₃, identiques ou différents, représentent un atome d'hydrogène ou un radical alkyle ou bien R₂₂ et R₂₃ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs radicaux alkyle,
R₂₄ et R₂₅, identiques ou différents, représentent un atome d'hydrogène ou un radical alkyle, -COOalk, cycloalkyle, alkylcycloalkyle, -alk-O-alk, hydroxyalkyle ou bien R₂₄ et R₂₅ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs radicaux alkyle, -COalk, -COOalk, -CO-NHalk, -CS-NHalk, oxo, hydroxyalkyle, -alk-O-alk, -CO-NH₂,
R₂₆ et R₂₇, identiques ou différents, représentent un atome d'hydrogène ou un radical alkyle, hydroxyalkyle, cycloalkyle, cycloalkylalkyle, -alk-COOalk, -alk-Ar, -alk-Het, Het, -alk-N(alk)₂₁ R₂₆ et R₂₇ peuvent également former avec l'atome d'azote auquel ils sont rattachés un hétérocycle mono ou bicyclique insaturé ou saturé, ayant 3 à 10 chaînons et contenant éventuellement un ou plusieurs autres hétéroatomes choisis parmi oxygène, soufre et azote et éventuellement substitué par un ou plusieurs radicaux alkyle, alcoxy, halogène,
R₂. représente un radical -CH₂-alk, benzyle, -SO₂alk, -CONHalk, -COalk, cycloalkylalkylcarbonyle, cycloalkylcarbonyle, -CO-(CH₂)ₙOH,
n est égal à 1, 2 ou 3,
R₂₉ et R₃₀, identiques ou différents, représentent un atome d'hydrogène ou un radical alkyle ou bien R₂₉ et R₃₀ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs radicaux alkyle,
R₃₁ et R₃₂, identiques ou différents, représentent un atome d'hydrogène ou un radical alkyle, Ar ou -alk-Ar ou bien R₃₁ et R₃₂ forment ensemble avec l'atome d'azote auquel ils sont rattachés un hétérocycle choisi parmi aziridinyle, azétidinyle, pyrrolidinyle et pipéridinyle, alk représente un radical alkyle ou alkylène,
Ar représente un radical phényle ou naphtyle éventuellement substitué par un ou plusieurs substituants choisis parmi halogène, alkyle, alcoxy, -CO-alk, cyano, -COOH, -COOalk, -CONR₂₂R₂₃, -CO-NH-NR₂₄R₂₅, alkylsulfanyle, alkylsulfinyle, alkylsulfonyle, alkylsulfanylalkyle, alkylsulfinylalkyle, alkylsulfonylalkyle, hydroxyalkyle, -alk-NR₂₄R₂₅, -NR₂₄R₂₅, alkylthioalkyle, formyle, hydroxy, CF₃, OCF₃, Het, -O-alk-NH-cycloalkyle ou SO₂NH₂,
Het représente un hétérocycle mono ou bicyclique insaturé ou saturé, ayant 3 à 10 chaînons et contenant un ou plusieurs hétéroatomes choisis parmi oxygène, soufre et azote et éventuellement substitué par un ou plusieurs halogène, alkyle, alcoxy, alcôxycarbonyle, -CONR₂₂R₂₃, hydroxy, hydroxyalkyle, oxo ou SO₂NH₂.

Dans les définitions précédentes et celles qui suivent, sauf mention contraire, les radicaux et portions alkyle et alkylène et les radicaux et portions alcoxy sont en chaîne droite ou ramifiée et contiennent 1 à 6 atomes de carbone, les radicaux cycloalkyle contiennent 3 à 10 atomes de carbone et les radicaux hétérocycloalkyle et hétérocyclényle contiennent 3 à 10 atomes de carbone.

Parmi les radicaux alkyle on peut citer les radicaux méthyle, éthyle, n-propyle, isopropyle, n-butyle, sec-butyle, iso-butyle, tert-butyle, pentyle, hexyle. Parmi les radicaux alcoxy on peut citer les radicaux méthoxy, éthoxy, n-propoxy, iso-propoxy, n-butoxy, iso-butoxy, sec-butoxy, tert-butoxy, pentyloxy.

Parmi les radicaux cycloalkyle, on peut citer les radicaux cyclopropyle, cyclobutyle, cyclopentyle, cyclohexyle.

Les radicaux hétérocycloalkyle sont des radicaux cycloalkyle dont au moins un des atomes de carbone est remplacé par un hétéroatome choisi parmi azote, soufre et oxygène. Parmi ceux-ci on peut citer les cycles pyrrolidinyle, imidazolidinyle, pyrazolidinyle, pipéridyle, pipérazinyle, morpholinyle.

Les radicaux hétérocyclényle sont des radicaux cycloalkyle dont au moins un atome de carbone est remplacé par un hétéroatome choisi parmi oxygène, soufre et azote et qui contient au moins une double liaison carbone-carbone ou carbone-azote. Parmi les radicaux hétérocyclényle on peut citer les cycles 1,2,3,4-tétrahydrohydropyridinyle, 3,6-dihydropyridyle, 1,2-dihydropyridyle, 1,4-dihydropyridyle, 1,2,3,6-tétrahydropyridinyle, 1,4,5,6-tétrahydropyrimidinyle, 2-pyrrolinyle, 3-pyrrolinyle, 2-imidazolinyle, 2-pyrazolinyle, 3,4-dihydro-2*H*-pyrane, dihydrofuranyle, et fluorodihydrofuranyle. Les préférés sont les cycles 3,6-dihydropyridyle.

Le terme halogène comprend chlore, fluor, brome et iode.

Parmi les hétérocycles représentant Het, on peut citer les hétérocycles suivants : benzofuryle, benzothiazolyle, benzothiényle, benzoxazolyle, chromannyle, 2,3-dihydrobenzofuryle, 2,3-dihydrobenzothiényle, furyle, indolinyle, indolyle, isochromannyle, isoquinolyle, pipéridyle, pyrrolyle, pyridyle, pyrimidinyle, quinolyle, 1,2,3,4-tétrahydroisoquinolyle, 1,2,3,4-tétrahydroquinolyle, thiazolyle, thiényle.

Lorsque R₃ et/ou R₄ représentent indépendamment un phényle substitué celui-ci est de préférence mono, di ou trisubstitué.

Lorsque R₁₆ et R₁₇ forment ensemble avec l'atome d'azote auquel ils sont rattachés un hétérocycle mono ou bicyclique, saturé ou insaturé ayant 3 à 10 chaînons celui-ci est de préférence un cycle azétidinyle, pyrrolidinyle, pipéridinyle, morpholinyle, thiamorpholinyle ou pipérazinyle.

Lorsque R₁₈ et R₁₉ forment ensemble avec l'atome d'azote auquel ils sont rattachés un hétérocycle mono ou bicyclique, saturé ou insaturé ayant 3 à 10 chaînons celui-ci est de préférence un cycle azétidinyle, pyrrolidinyle, pipéridinyle, morpholinyle, thiamorpholinyle ou pipérazinyle.

L'hétérocycle formé par NR₂₀R₂₁ est de préférence azétidinyle, pyrrolidinyle, pipéridinyle, morpholinyle, thiamorpholinyle, pipérazinyle ou imidazolyle.

Lorsque R₂₂ et R₂₃ forment ensemble avec l'atome d'azote auquel ils sont rattachés un hétérocycle mono ou bicyclique, saturé ou insaturé ayant 3 à 10 chaînons celui-ci est de préférence un cycle azétidinyle, pyrrolidinyle, pipéridinyle, morpholinyle, thiamorpholinyle ou pipérazinyle.

Lorsque R₂₄ et R₂₅ forment ensemble avec l'atome d'azote auquel ils sont rattachés un hétérocycle mono ou bicyclique, saturé ou insaturé ayant 3 à 10 chaînons celui-ci est de préférence un cycle azétidinyle, pyrrolidinyle, pipéridinyle, morpholinyle, thiamorpholinyle ou pipérazinyle.

Lorsque R₂₆ et R₂₇ forment ensemble avec l'atome d'azote auquel ils sont rattachés un hétérocycle mono ou bicyclique, saturé ou insaturé ayant 3 à 10 chaînons celui-ci est de préférence un cycle azétidinyle, pyrrolidinyle, pipéridinyle, morpholinyle, thiamorpholinyle ou pipérazinyle.

Lorsque R₂₉ et R₃₀ forment ensemble avec l'atome d'azote auquel ils sont rattachés un hétérocycle mono ou bicyclique, saturé ou insaturé ayant 3 à 10 chaînons celui-ci est de préférence un cycle azétidinyle, pyrrolidinyle, pipéridinyle, morpholinyle, thiamorpholinyle ou pipérazinyle.

Lorsque R₃₁ er R₃₂ forment ensemble avec l'atome d'azote auquel ils sont rattachés un hétérocycle mono ou bicyclique, saturé ou insaturé ayant 3 à 10 chaînons celui-ci est de préférence un cycle azétidinyle, pyrrolidinyle, pipéridinyle, morpholinyle, thiamorpholinyle ou pipérazinyle.

De façon préférentielle,
R représente un radical CR₁R₂,
soit R₁ représente un atome d'hydrogène, et R₂ représente un radical -C(R₈)(R₁₁)(R₁₂) ou C(R₈)(R₉)(R₁₀).
soit R₁ représente un radical alkyle et R₂ représente un radical -C(R₈)(R₁₁)(R₁₂),
R₃ et R₄, identiques ou différents, représentent soit un phényle non substitué ou substitué par un ou plusieurs halogène, alkyle, alcoxy, trifluorométhyle, trifluorométhoxy, cyano, -CONR₂₂R₂₃, hydroxyalkyle, ou -alk-NR₂₄R₂₅; soit un hétéroaromatique choisi parmi les cycles pyridyle, pyrimidinyle, thiazolyle, thiényle, ces hétéroaromatiques pouvant être non substitués ou substitués par un ou plusieurs halogène, alkyle, alcoxy, hydroxy, trifluorométhyle, trifluorométhoxy, cyano, -CONR₂₂R₂₃, -alk-NR₂₄R₂₅ ou hydroxyalkyle,
R₈ représente un atome d'hydrogène,
R₉ représente un radical -CO-NR₂₆R₂₇, -COOalk, -CH₂OH, -NH-CO-NH-alk, -CH₂-NHR₂₈ ou -NHCOOalk,
R₁₀ représente un radical Ar ou Het,
R₁₁ représente un radical -SO₂-alk, -SO₂-Ar, -SO₂-Het,
R₁₂ représente représente un atome d'hydrogène ou un radical Ar ou Het,
R₂₂ et R₂₃, identiques ou différents, représentent un atome d'hydrogène ou un radical alkyle ou bien R₂₂ et R₂₃ forment ensemble avec l'atome d'azote auquel ils sont rattachés un hétérocycle mono ou bicyclique saturé ayant 3 à 10 chaînons, contenant éventuellement un autre hétéroatome choisi parmi soufre et azote et étant éventuellement substitué par un ou plusieurs radicaux alkyle,
R₂₄ et R₂₅, identiques ou différents représentent un atome d'hydrogène ou un radical alkyle, cycloalkyle, alkylcycloalkyle, hydroxyalkyle ou bien R₂₄ et R₂₅ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs radicaux alkyle, -COalk, -COOalk, -CO-NHalk, -CS-NHalk, oxo, -CO-NH₂,
Ar représente un radical phényle ou naphtyle éventuellement substitué par 1 ou 2 substituants choisis parmi halogène alkyle, alcoxy, -CO-alk, cyano, -COOalk, -CONR₂₂R₂₃, alkylsulfonyle, hydroxyalkyle, -alk-NR₂₄R₂₅, -NR₂₄R₂₅, hydroxy, CF₃, OCF₃, -O-alk-NH-cycloalkyle ou SO₂NH₂,

Het représente un cycle benzofuryle, benzothiazolyle, benzothiényle, benzoxazolyle, furyle, isoquinolyle, pyrrolyle, pyridyle, quinolyle, 1,2,3,4-tétrahydroisoquinolyle, 1,2,3,4-tétrahydroquinolyle, thiazolyle ou thiényle.

Les composés de formule (I) peuvent se présenter sous forme d'énantiomères et de diastéréoisomères. Ces isomères optiques et leurs mélanges font partie de l'invention.

Les composés de formule (I) pour lesquels R représente un radical CR₁R₂ dans lequel R₁ représente un atome d'hydrogène et R₂ représente un radical C(R₈)(R₁₁)(R₁₂) dans lequel R₈ représente un atome d'hydrogène, R₁₁ représente un radical -SO₂-Ar, -SO₂-Het ou -SO₂alk et R₁₂ représente un atome d'hydrogène ou un radical Ar ou Het et les composés de formule (I) pour lesquels R représente un radical C=C(R₅)SO₂R₆ ou C=C(R₇)SO₂alk peuvent être préparés selon le schéma réactionnel suivant : 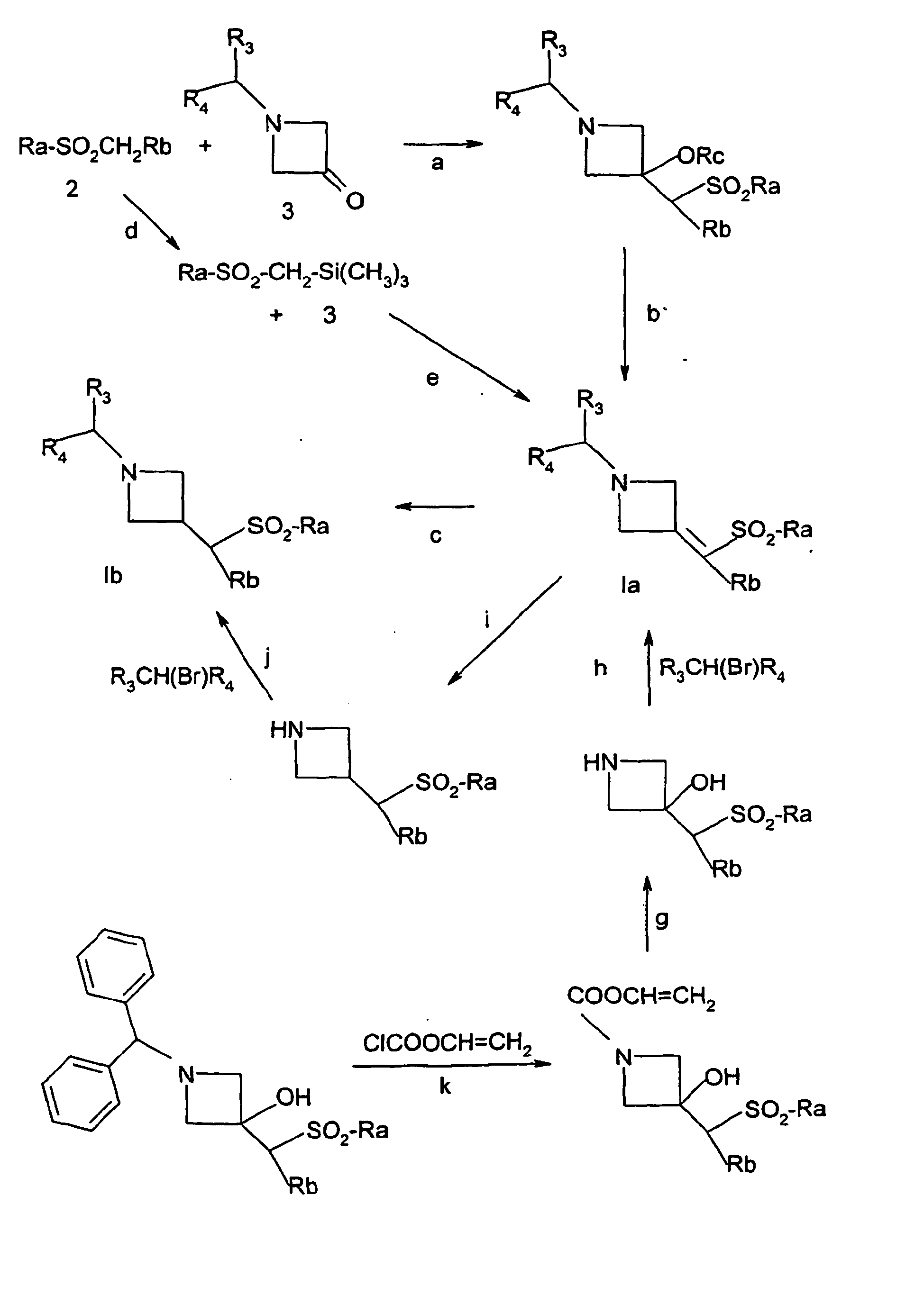 dans ces formules, soit Ra représente un radical alkyle, Het ou Ar et Rb représente un atome d'hydrogène ou un radical Ar ou Het, soit Ra représente un radical Ar ou Het et Rb représente un atome d'hydrogène ou un radical alkyle, soit Ra représente un radical alkyle et Rb représente un radical cycloalkyle, hétérocycloalkyle ou hétérocyclényle éventuellement substitué par un radical -CSO-phényle, Rc représente un atome d'hydrogène ou un radical acétyle, R₃, R₄, Ar et Het ont les mêmes significations que dans la formule (I).

Les réactions d et e ne peuvent être utilisées que lorsque Rb est un atome d'hydrogène.

La réaction a s'effectue généralement au sein d'un solvant inerte tel qu'un éther (tétrahydrofuranne par exemple), en présence d'une base forte telle que le tert-butyllithium, le n-butyllithium, le diisopropylamidure de lithium, le tert-butylate de potassium, à une température comprise entre -70°C et -15°C.

La réaction de déshydratation b s'effectue généralement par toute méthode de déshydratation connue de l'homme de l'art permettant de déshydrater un alcool pour obtenir l'alcène correspondant. De préférence, on prépare le dérivé acétyloxy par action de chlorure d'acétyle, au sein d'un solvant inerte tel que la pyridine, le tétrahydrofuranne, le dioxanne, un solvant chloré (dichlorométhane, chloroforme par exemple), à une température comprise entre 5°C et 20°C puis on traite avec une base telle qu'un hydroxyde de métal alcalin (soude par exemple), un carbonate de métal alcalin (carbonate de sodium ou de potassium par exemple), une amine telle qu'une trialkylamine (triéthylamine par exemple), la 4-diméthylaminopyridine, le diaza-1,8-bicyclo[5.4.0]undécène-7, à une température comprise entre 0°C et la température d'ébullition du milieu réactionnel. L'acétyloxy intermédiaire peut être isolé ou non. L'acétyloxy peut aussi être préparé directement dans le milieu réactionnel de la réaction a.

La réduction c s'effectue généralement au sein d'un solvant inerte tel qu'un alccol aliphatique (1-4C) (méthanol par exemple), un solvant chloré (chloroforme, dichlorométhane par exemple) ou un mélange de ces solvants, en présence de NaBH₄, à une température comprise entre 0°C et la température d'ébullition du milieu réactionnel

La réaction d s'effectue par action de chlorure de triméthylsilyle, au sein d'un solvant inerte tel qu'un éther (tétrahydrofuranne par exemple), en présence de n-butyllithium, à une température de -70°C.

La réaction e s'effectue généralement au sein d'un solvant inerte tel qu'un éther (tétrahydrofuranne par exemple), en présence d'une base forte telle que le tert-butyllithium, le n-butyllithium, le diisopropylamidure de lithium, le tert-butylate de potassium, à une température comprise entre -70°C et -15°C.

La réaction f s'effectue généralement au sein d'un solvant chloré (dichlorométhane, chloroforme par exemple), à une température de 0°C à la température d'ébullition du milieu réactionnel.

L'hydrolyse g s'effectue au sein d'un solvant inerte tel qu'un éther (dioxane par exemple), au moyen d'acide chlorhydrique, à une température voisine de 20°C.

Les réactions h et j s'effectuent de préférence au sein d'un solvant inerte tel que l'acétonitrile, en présence d'une base tel qu'un carbonate de métal alcalin (carbonate de potassium par exemple), à la température d'ébullition du milieu réactionnel.

La réaction i s'effectue sous atmosphère d'hydrogène, en présence d'un catalyseur tel que le palladium ou l'un de ses dérivés, au sein d'un solvant inerte tel que le méthanol ou l'éthanol, à une température comprise entre 15°C et 60°C.

La réaction k s'effectue au sein d'un solvant inerte tel qu'un solvant chloré (dichlorométhane, chloroforme par exemple), à une température comprise entre 0°C et la température d'ébullition du mélange réactionnel.

Les dérivés R₃CH(Br)R₄ sont commercialisés ou peuvent être obtenus par application ou adaptation de la méthode décrite par BACHMANN W.E., J. Am. Chem. Soc., 2135 (1933). Généralement, on brome l'alcool correspondant R₃CHOHR₄ au moyen d'acide bromhydrique, au sein de l'acide acétique, à une température comprise entre 0°C et la température d'ébullition du milieu réactionnel.

Les alcools correspondants R₃CHOHR₄ sont commercialisés ou peuvent être obtenus par application ou adaptation des méthodes décrites par PLASZ A.C. et coll., J. Chem. Soc. Chem. Comm., 527 (1972). ,

Les intermédiaires de formule 2 peuvent être obtenus par application ou adaptation des méthodes décrites dans les exemples. Notamment, on opère selon les schémas réactionnels suivants : 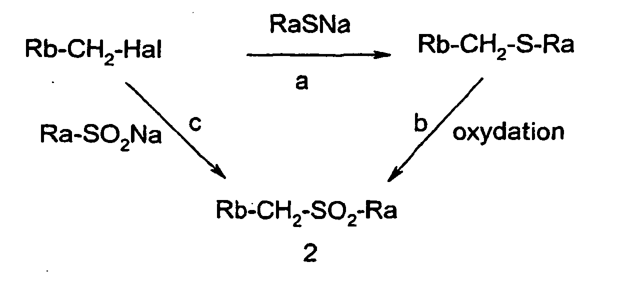 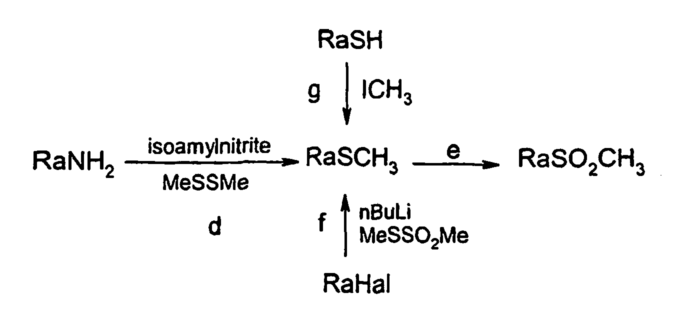 dans ces formules Hal représente un atome d'halogène et, de préférence, chlore, brome ou iode, Ra et Rb ont les mêmes significations que précédemment mentionnées pour le dérivé 2.

La réaction a s'effectue généralement au sein d'un solvant inerte tel que le diméthylformamide ou un alcool aliphatique 1-4C, à une température comprise entre 20°C et 30°C.

Les réactions b et e s'effectuent par toutes méthodes connues permettant d'oxyder un dérivé soufré sans toucher au reste de la molécule comme celles décrites par M. HUDLICKY, Oxidations in Organic Chemistry, ACS Monograph, 186, 252-263 (1990). Par exemple, on opère par action d'un peroxyacide organique ou un sel d'un tel peroxyacide (acides peroxycarboxyliques ou peroxysulfoniques, notamment l'acide peroxybenzoïque, l'acide 3-chloroperoxybenzoïque, l'acide 4-nitroperoxybenzoïque, l'acide peroxyacétique, l'acide trifluoroperoxyacétique, l'acide peroxyformique, l'acide monoperoxyphtalique) ou les peracides minéraux ou un sel d'un tel acide (par exemple l'acide periodique ou persulfurique), au sein d'un solvant inerte tel qu'un solvant chloré (chloroforme, dichlorométhane par exemple), à une température comprise entre 0 et 25°C. On peut utiliser également le peroxyde d'hydrogène, éventuellement en présence d'un oxyde métallique (tungstate de sodium) ou un periodate (periodate de sodium par exemple), au sein d'un solvant inerte tel qu'un alcool aliphatique 1-4C (méthanol, éthanol par exemple), l'acide acétique, l'eau ou un mélange de ces solvants, à une température comprise entre 0 et 60°C. II est également possible d'opérer au moyen de tertiobutylhydroperoxyde en présence de tétraisopropylate de titane au sein d'un alcool aliphatique 1-4C (méthanol, éthanol par exemple) ou un mélange eau-alcool, à une température voisine de 25°C ou au moyen d'oxone^{R} (peroxymonosulfate de potassium), au sein d'un alcool aliphatique 1-4C (méthanol, éthanol par exemple), en présence d'eau, d'acide acétique ou d'acide sulfurique, à une température voisine de 20°C.

La réaction c s'effectue de préférence au sein d'un solvant inerte tel qu'un alcool aliphatique 1-4C (méthanol, éthanol par exemple), à une température comprise entre 20°C et la température d'ébullition du milieu réactionnel.

La réaction d s'effectue sous atmosphère inerte (argon), à une température comprise entre 50°C et la température d'ébullition du milieu réactionnel.

La réaction f s'effectue généralement au sein d'un solvant inerte tel que le tétrahydrofuranne ou un éther aliphatique (éther éthylique par exemple), à une température voisine de -70°C.

La réaction g s'effectue généralement au sein d'un solvant inerte tel que le diméthylformamide, un éther aliphatique (éther éthylique par exemple) ou un alcool aliphatique 1-4C, en présence d'une base (hydrure de sodium par exemple), à une température comprise entre 0°C et 60°.

Les dérivés de formule Rb-CH₂-Hal sont commercialisés ou peuvent être obtenus par application ou adaptation des méthodes décrites dans les exemples. En particulier, on halogène le dérivé méthylé ou l'alcool correspondant, au moyen d'un agent d'halogénation tel que l'acide bromhydrique, au sein de l'acide acétique, à une température voisine de 20°C ou le N-bromo ou N-chlorosuccinimide en présence de peroxyde de benzoyle, au sein d'un solvant inerte tel que le tétrachlorométhane, à la température d'ébullition du milieu réactionnel. Les dérivés méthylés ou les alcools correspondants sont commercialisés ou peuvent être obtenus selon les méthodes décrites par BRINE G. A. et coll., J. Heterocyl. Chem, 26, 677 (1989) et NAGARATHNAM D., Synthesis, 8, 743 (1992) et dans les exemples.

Les azétidinones de formule 3 peuvent être obtenues par application ou adaptation des méthodes décrites par KATRITZKY A.R et coll., J. Heterocycl. Chem., 271 (1994), ou DAVE P.R., J. Org. Chem., 61, 5453 (1996) et dans les exemples. On opère généralement selon le schéma réactionnel suivant : 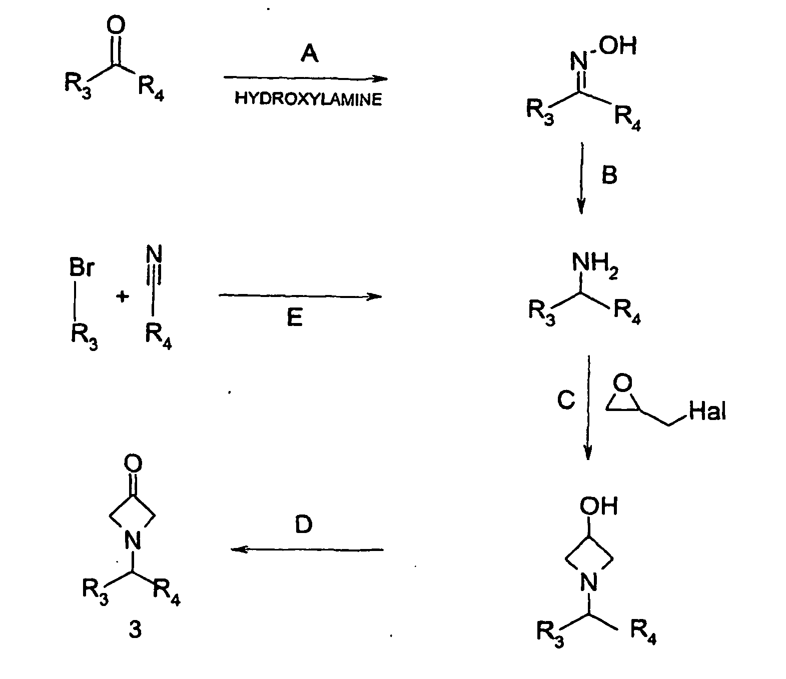 dans ces formules R₃ et R₄ ont les mêmes significations que dans la formule (I) et Hal représente un atome de chlore ou de brome.

Dans l'étape A, on opère de préférence au sein d'un solvant inerte tel qu'un alcool aliphatique 1-4C (éthanol, méthanol par exemple), éventuellement en présence d'un hydroxyde de métal alcalin, à la température d'ébullition du milieu réactionnel.

Dans l'étape B, la réduction s'effectue généralement, au moyen d'hydrure de lithium et d'aluminium, au sein du tétrahydrofuranne à la température d'ébullition du milieu réactionnel.

Dans l'étape C, on opère de préférence au sein d'un solvant inerte tel qu'un alcool aliphatique 1-4C (éthanol, méthanol par exemple), en présence d'hydrogénocarbonate de sodium, à une température comprise entre 20°C et la température d'ébullition du milieu réactionnel.

Dans l'étape D on oxyde de préférence au sein de DMSO, au moyen du complexe trioxyde de soufre-pyridine, à une température voisine de 20°C ou au moyen de diméthylsulfoxyde, en présence de chlorure d'oxalyle et de triéthylamine, à une température comprise entre -70 et -50°C.

Dans l'étape E, on opère selon la méthode décrite par GRISAR M. et coll. dans J. Med. Chem., 885 (1973). On forme le magnésien du dérivé bromé puis on fait réagir le nitrile, au sein d'un éther tel que l'éther éthylique, à une température comprise entre 0°C et la température d'ébullition du milieu réactionnel. Après hydrolyse avec un alcool, l'imine intermédiaire est réduite *in situ* par du borohydrure de sodium à une température comprise entre 0°C et la température d'ébullition du milieu réactionnel.

Les dérivés R₃-CO-R₄ sont commercialisés ou peuvent être obtenus par application ou adaptation des méthodes décrites par KUNDER N.G. et coll. J. Chem. Soc. Perkin Trans 1, 2815 (1997); MORENO-MARRAS M., Eur. J. Med. Chem., 23 (5) 477 (1988); SKINNER et coll., J. Med. Chem., 14 (6) 546 (1971); HURN N.K., Tet. Lett., 36 (52) 9453 (1995); MEDICI A. et coll., Tet. Lett., 24 (28) 2901 (1983); RIECKE R.D. et coll., J. Org. Chem., 62 (20) 6921 (1997); KNABE J. et coll., Arch. Pharm., 306 (9) 648 (1973); CONSONNI R. et coll., J. Chem. Soc. Perkin Trans 1, 1809 (1996); FR-96-2481 et JP-94-261393.

Les dérivés R₃Br sont commercialisés ou peuvent être obtenus par application ou adaptation des méthodes décrites par BRANDSMA L. et coll., Synth. Comm., 20 (11) 1697 et 3153 (1990); LEMAIRE M. et coll., Synth. Comm., 24 (1) 95 (1994); GODA H. et coll., Synthesis, 9 849 (1992); BAEUERLE P. et coll., J. Chem. Soc. Perkin Trans 2, 489 (1993).

Les dérivés R₄CN sont commercialisés ou peuvent être obtenus par application ou adaptation des méthodes décrites par BOUYSSOU P. et coll., J. Het. Chem., 29 (4) 895 (1992); SUZUKI N. et coll., J. Chem. Soc. Chem. Comm., 1523 (1984); MARBURG S. et coll., J. Het. Chem., 17 1333 (1980); PERCEC V. et coll., J. Org. Chem., 60 (21) 6895 (1995).

Les composés de formule (I) pour lesquels R représente un radical CR₁R₂ dans lequel R₁ représente un atome d'hydrogène et R₂ représente un radical C(R₈)(R₉)(R₁₀) dans lequel R₈ représente un atome d'hydrogène, R₉ représente un radical -CO-NR₂₆R₂₇, -COOH, -COOalk, -CH₂OH, -NHCOOalk ou -NH-CO-NH-alk et R₁₀ représente un radical Ar ou Het peuvent être préparés selon le schéma réactionnel suivant : 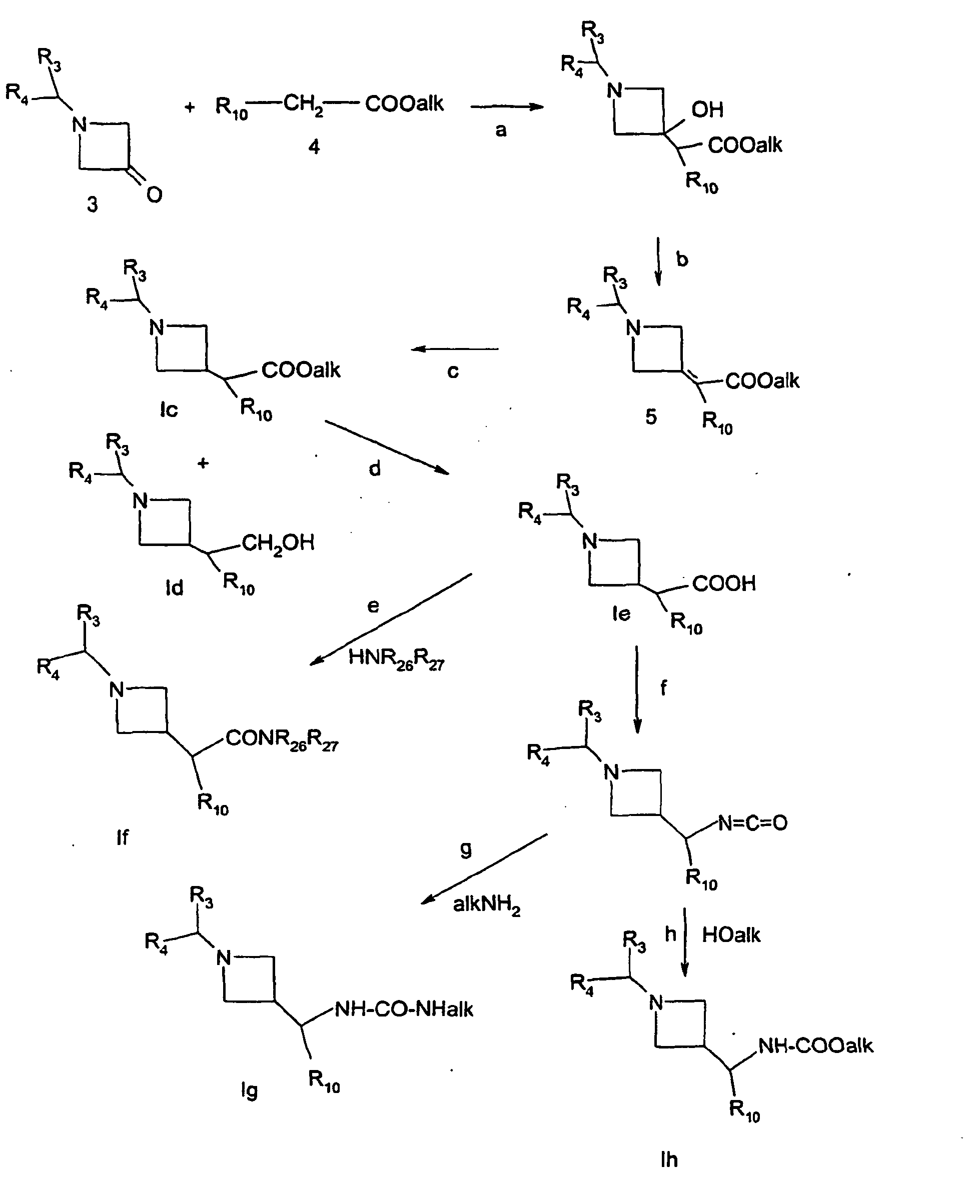 dans ces formules R₃, R₄, R₁₀, R₂₆ et R₂₇ ont les mêmes significations que dans la formule (I) et alk représente un radical alkyle.

Les dérivés de formule 4 sont commercialisés ou peuvent être obtenus par estérification des acides correspondants éventuellement sous une forme activée telle que le chlorure d'acide. Les acides sont commercialisés ou peuvent être obtenus à partir des dérivés méthylés correspondants selon la méthode décrite par JP. HANSEN et coll., J. Heteocycl., 10, 711 (1973).

La réaction a s'effectue généralement au sein d'un solvant inerte tel qu'un éther (tétrahydrofuranne par exemple), en présence d'une base forte telle que le tert-butyllithium, le n-butyllithium, le diisopropylamidure de lithium, le tert-butylate de potassium, à une température comprise entre -70°C et -15°C.

La réaction b s'effectue généralement par toute méthode de déshydratation connue de l'homme de l'art permettant de déshydrater un alcool pour obtenir l'alcène correspondant et notamment les méthodes décrites précédemment.

La réduction c s'effectue généralement au sein d'un solvant inerte tel qu'un alcool aliphatique (1-4C) tel que le méthanol, un solvant chloré tel que le chloroforme, le dichlorométhane ou un mélange de ces solvants, en présence de NaBH₄, à une température comprise entre 0°C et la température d'ébullition du milieu réactionnel.

La réaction d s'effectue par toute méthode connue de l'homme de l'art permettant de passer d'un ester à l'acide correspondant sans toucher au reste de la molécule. On opère de préférence, au sein d'un solvant inerte tel que le dioxanne, en présence d'acide chlorhydrique, à la température d'ébullition du milieu réactionnel.

La réaction e s'effectue par toute méthode connue de l'homme de l'art permettant de passer d'un acide ou un dérivé réactif de cet acide à un carboxamide sans toucher au reste de la molécule. De préférence, lorsque l'on met en oeuvre l'acide, on opère en présence d'un agent de condensation utilisé en chimie peptidique tel qu'un carbodiimide (par exemple le N,N'-dicyclohexytcarbodiimide) ou le N,N'-dümidazole carbonyle, dans un solvant inerte tel qu'un éther (tétrahydrofuranne, dioxanne par exemple), un amide (diméthylformamide) ou un solvant chloré (chlorure de méthylène, dichloro-1,2 éthane, chloroforme par exemple) à une température comprise entre 0°C et la température de reflux du mélange réactionnel. Lorsque l'on met en oeuvre un dérivé réactif de l'acide, il est possible de faire réagir l'anhydride, un anhydride mixte ou un ester (qui peut être choisi parmi les esters activés ou non de l'acide); on opère alors soit en milieu organique, éventuellement en présence d'un accepteur d'acide tel qu'une base organique azotée (trialkylamine, pyridine, diaza-1,8 bicyclo [5.4.0] undécène-7 ou diaza-1,5 bicyclo [4.3:0] nonène-5 par exemple), dans un solvant tel que cité ci-dessus, ou un mélange de ces solvants, à une température comprise entre 0°C et la température de reflux du mélange réactionnel, soit en milieu hydroorganique biphasique en présence d'une base alcaline ou alcalino-terreuse (soude, potasse) ou d'un carbonate ou bicarbonate d'un métal alcalin ou alcalino-terreux à une température comprise entre 0 et 40°C.

La réaction f s'effectue par réarrangement de CURTIUS, en présence de diphénylphosphorazide et de triéthylamine, au sein du toluène, à une température voisine de 50°C.

Pour les réactions g et h, on opère directement au sein du milieu réactionnel de l'étape g à une température voisine de 20°C.

Les composés de formule (I) pour lesquels R représente un radical CR₁R₂ dans lequel R₁ est un atome d'hydrogène et R₂ représente un radical -C(R₈)(R₉)(R₁₀) pour lequel R₈ est un atome d'hydrogène, R₉ est un radical -CH₂-NHR₂₈ et R₁₀ représente un radical Ar ou Het, peuvent être préparés selon le schéma réactionnel suivant :

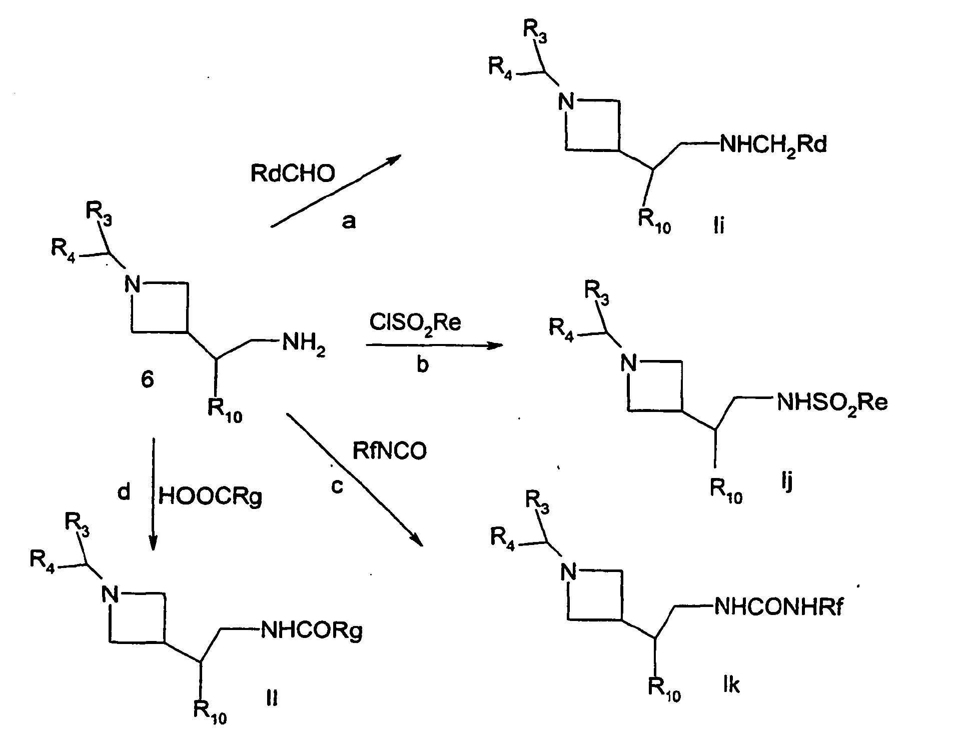

Dans ces formules R₃, R₄ et R₁₀ ont les mêmes significations que dans la formule (I), Rd représente un radical alkyle ou phényle, Re représente un radical alkyle, Rf représente un radical alkyle, Rg représente un radical alkyle, cycloalkylalkyle, cycloalkyle, -(CH₂)ₙOH, n est égal à 1, 2, 3.

L'étape a s'effectue généralement au sein d'un solvant inerte tel qu'un alccol aliphatique (1-4C) (méthanol par exemple), un solvant chloré (dichlorométhane, dichloroéthane par exemple) ou le tétrahydrofuranne, en présence d'une base telle que NaBH(OCOCH₃)₃, à une température voisine de 20°C.

L'étape b s'effectue génralement au sein d'un solvant inerte tel qu'un solvant halogéné (dichlorométhane par exemple), en présence d'une base organique telle que la triéthylamine, la diméthylaminopyridine, à une température comprise entre 0°C et la température d'ébullition du milieu réactionnel.

L'étape c s'effectue généralement au sein d'un solvant inerte tel que le tétrahydrofuranne, le diméthylformamide, un solvant chloré (chloroforme, dichloro-1,2 éthane par exemple), un solvant aromatique (benzène, toluène par exemple), à une température comprise entre 10°C et la température d'ébullition du milieu réactionnel.

L'étape d s'effectue par toute méthode connue de l'homme de l'art permettant de passer d'un acide ou un dérivé réactif de cet acide à un carboxamide sans toucher au reste de la molécule et notamment les méthodes préférées décrites précédemment.

Les dérivés 6 peuvent être obtenus selon le schéma réactionnel suivant :

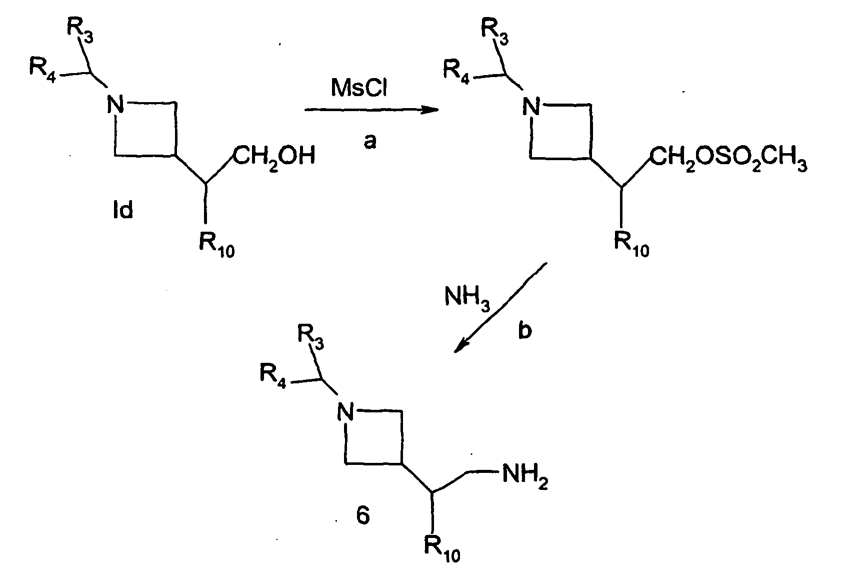

Dans ces formules R₃, R₄ et R₁₀ ont les mêmes significations que dans la formule (I) et Ms est un radical méthylsulfonyloxy.

L'étape a s'effectue généralement au sein d'un solvant inerte tel que le tétrahydrofuranne, en présence de triéthylamine, à une température comprise entre 10 et 20°C.

L'étape b s'effectue généralement avec de l'ammoniaque liquide dans le méthanol, en autoclave, à une température voisine de 60°C.

Les composés de formule (I) dans laquelle R représente un radical CR₁R₂ dans lequel R₁ est un atome d'hydrogène et R₂ est un radical -CONR₁₃R₁₄ peuvent être préparés selon le schéma réactionnel suivant :

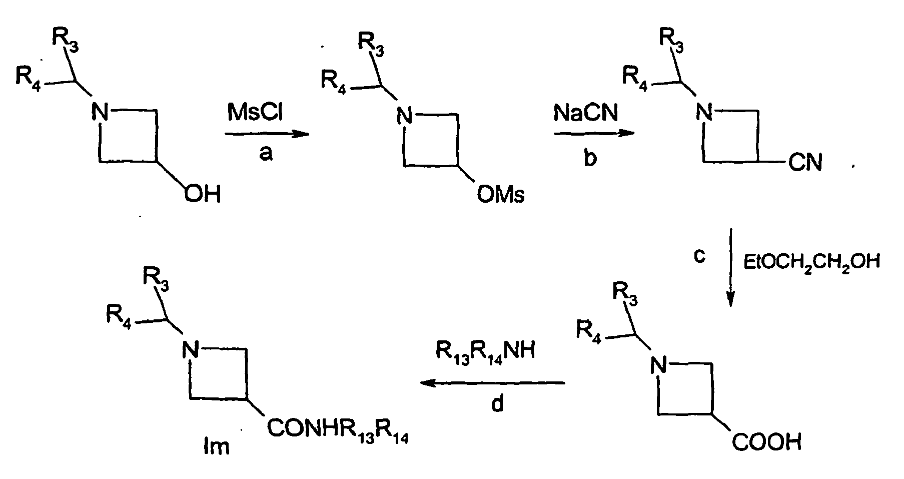

Dans ces formules R₃, R₄, R₁₃ et R₁₄ ont les mêmes significations que dans la formule (I), Ms représente un radical méthylsulfonyloxy et Et représente éthyle.

L'étape a s'effectue en présence de triéthylamine, au sein d'un solvant inerte tel qu'un éther (tétrahydrofuranne par exemple), à une température voisine de 0°C.

L'étape b s'effectue généralement au sein d'un solvant inerte tel qu'un mélange d'eau et de diméthylformamide, à une température comprise entre 30 et 75°C.

L'étape c s'effectue par toute méthode connue de l'homme de l'art permettant de passer d'un cyané à l'acide correspondant sans toucher au reste de la molécule. De préférence, on opère au moyen de potasse au sein d'un alcool aliphatique (1-4C) (éthanol par exemple) ou en milieu aqueux, à la température d'ébullition du milieu réactionnel.

L'étape d s'effectue par toute méthode connue de l'homme de l'art permettant de passer d'un acide ou un dérivé réactif de cet acide à un carboxamide sans toucher au reste de la molécule molécule et notamment les méthodes préférées décrites précédemment.

Les composés de formule (I) pour lesquels R représente un radical CR₁R₂ dans lequel R₁ est un atome d'hydrogène et R₂ est un radical -CH₂-CONR₁₃R₁₄ peuvent être préparés selon le schéma réactionnel suivant :

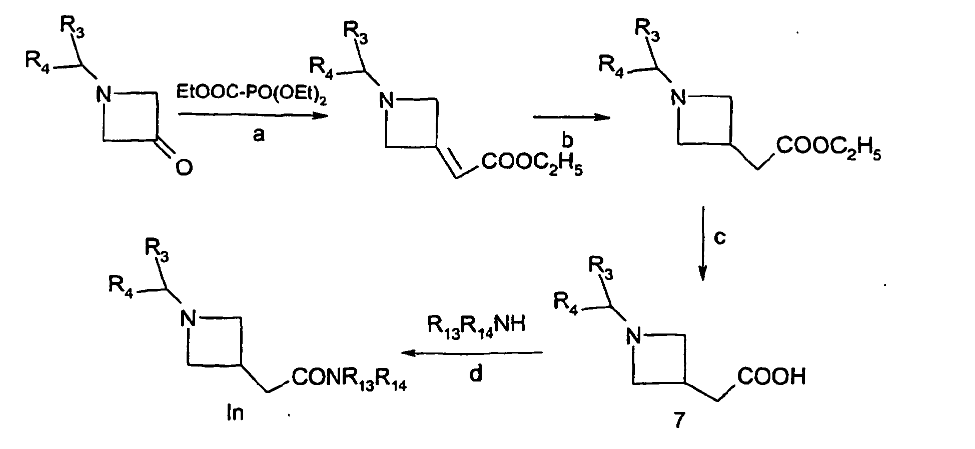

Dans ces formules R₃, R₄, R₁₃ et R₁₄ ont les mêmes significations que dans la formule (I) et Et représente un radical éthyle.

La réaction a s'effectue généralement au sein d'un solvant inerte tel que le tétrahydrofuranne, en présence d'une base telle que l'hydrure de sodium, ou un carbonate de métal alcalin (carbonate de potassium par exemple), à une température comprise entre 20°C et la température d'ébullition du milieu réactionnel.

La réaction b s'effectue généralement au moyen de NaBH₄, au sein de l'éthanol, à une tempéraure voisine de 0°C.

La réaction c s'effectue par toute méthode connue de l'homme de l'art permettant de passer d'un ester à l'acide correspondant sans toucher au reste de la molécule. On opère de préférence, au sein d'un solvant inerte tel que le dioxanne, en présence d'acide chlorhydrique, à la température d'ébullition du milieu réactionnel.

La réaction d s'effectue par toute méthode connue de l'homme de l'art permettant de passer d'un acide ou un dérivé réactif de cet acide à un carboxamide sans toucher au reste de la molécule molécule et notamment les méthodes préférées décrites précédemment.

Les intermédiaires 7 peuvent également être obtenus par synthèse malonique selon le schéma réactionnel suivant :

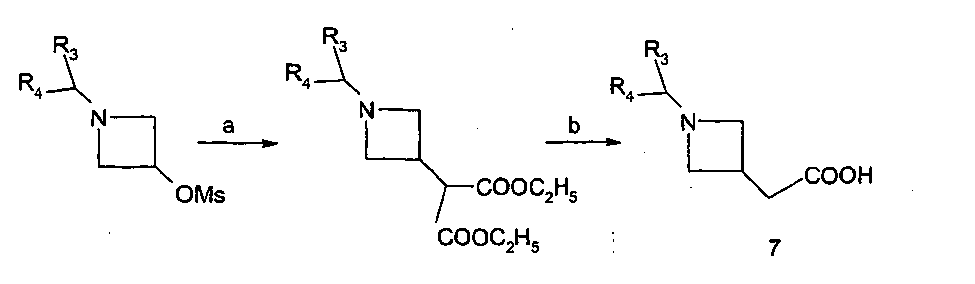

Dans ces formules Ms représente un radical méthylsulfonyloxy, R₃ et R₄ ont les mêmes significations que dans la formule (I).

La réaction a s'effectue généralement par action de diéthylmalonate, au sein d'un solvant inerte tel que le tétrahydrofuranne, en présence d'éthylate de sodium fraîchement préparé, à la température d'ébullition du milieu réactionnel.

La réaction b s'effectue généralement en solution aqueuse d'acide chlorhyrique à la température d'ébullition du milieu réactionnel.

Les composés In peuvent également être obtenus selon le schéma réactionnel suivant : 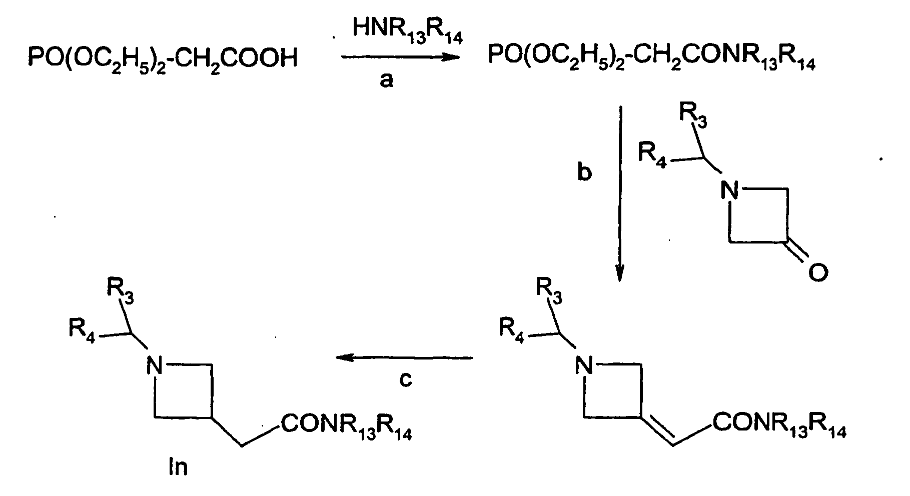 Dans ces formules R₃, R₄, R₁₃ et R₁₄ ont les mêmes significations que dans la formule (I).

L'étape a s'effectue par toute méthode connue de l'homme de l'art permettant de passer d'un acide ou un dérivé réactif de cet acide à un carboxamide sans toucher au reste de la molécule molécule et notamment les méthodes préférées décrites précédemment.

L'étape b s'effectue généralement au sein d'un solvant inerte tel que le tétrahydrofuranne, en présence d'une base telle que l'hydrure de sodium ou le carbonate de potassium à une température comprise entre 20°C et la température d'ébullition du mélange réactionnel.

La réduction de l'étape c s'effectue généralement au moyen de NaBH₄, au sein de méthanol, à une tempéraure de voisine de 20°C.

Les composés de formule (I) pour lesquels R représente un radical CR₁R₂ dans lequel R₁ est un atome d'hydrogène et R₂ représente un radical -SOR₆ ou -SO₂R₆, peuvent être préparés selon le schéma réactionnel suivant :

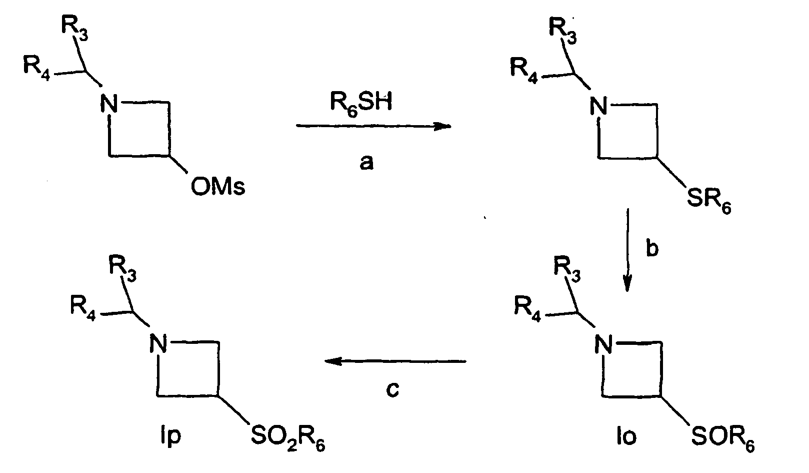

Dans ces formules R₃, R₄ et R₆ ont les mêmes significations que dans la formule (I) et Ms est un radical méthylsulfonyloxy.

L'étape a s'effectue généralement dans un solvant inerte tel que le tétrahydrofuranne en présence d'une base minérale tel que l'hydrure de sodium, à une température comprise entre 0°C et la température d'ébullition du milieu réactionnel.

L'étape b s'effectue généralement par toute méthode de l'homme de l'art d'oxydation d'un dérivé soufré comme celles décrites par M. HUDLICKY, Oxidations in Organic Chemistry, ACS Monograph, 186, 252-263 (1990). Par exemple, on opère par action d'un peroxyacide organique ou un sel d'un tel peroxyacide (acides peroxycarboxyliques ou peroxysulfoniques, notamment l'acide peroxybenzoïque, l'acide 3-chloroperoxybenzoïque, l'acide 4-nitroperoxybenzoïque, l'acide peroxyacétique, l'acide trifluoroperoxyacétique, l'acide peroxyformique, l'acide monoperoxyphtalique) ou les peracides minéraux ou un sel d'un tel acide (par exemple l'acide périodique ou persulfurique), au sein d'un solvant inerte tel qu'un solvant chloré (chloroforme, dichlorométhane par exemple), à une température comprise entre 0 et 25°C ou bien au moyen d'oxone au sein d'un mélange eau-alcool (méthanol, éthanol).

L'étape c s'effectue généralement par toute méthode de l'homme de l'art d'oxydation d'un dérivé sulfinyle. De préférence, on opère par action d'un peroxyacide organique ou un sel d'un tel peroxyacide (acides peroxycarboxyliques ou peroxysulfoniques, notamment l'acide peroxybenzoïque, l'acide 3-chloroperoxybenzoïque, l'acide 4-nitroperoxybenzoïque, l'acide peroxyacétique, l'acide trifluoroperoxyacétique, l'acide peroxyformique, l'acide monoperoxyphtalique) ou bien au moyen d'oxone au sein d'un mélange eau-alcool (méthanol, éthanol).

Les composés de formule (I) pour lesquels R représente un radical CR₁R₂ dans lequel R₁ est un atome d'hydrogène et R₂ représente un radical -COR₆, ou -CO-cycloalkyle, peuvent être préparés selon le schéma réactionnel suivant :

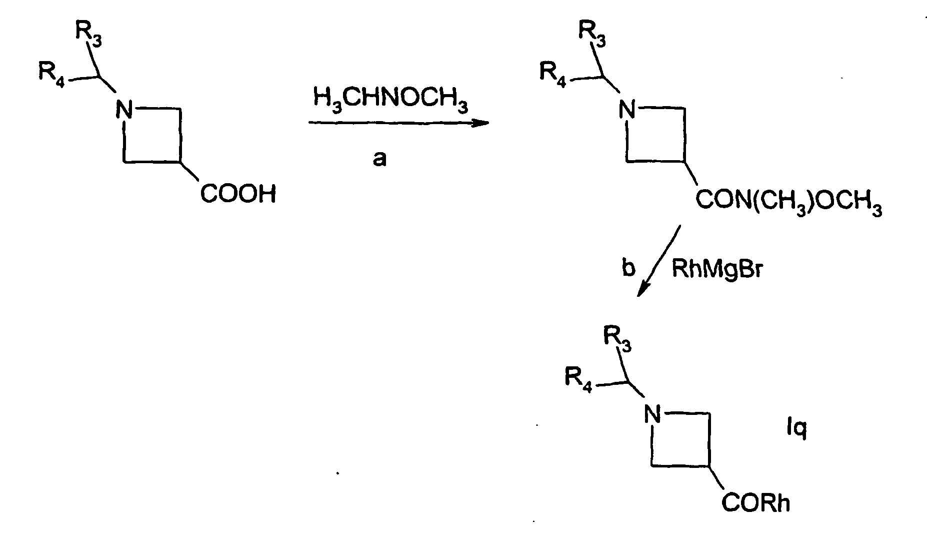

Dans ces formules, R₃ et R₄ ont les mêmes significations que dans la formule (I) et Rh a les mêmes significations que R₆ ou représente un radical cycloalkyle (3 à 10 atomes de carbone).

L'étape a s'effectue par toute méthode connue de l'homme de l'art permettant de passer d'un acide ou un dérivé réactif de cet acide à un carboxamide sans toucher au reste de la molécule molécule et notamment les méthodes préférées décrites précédemment.

L' étape b s'effectue généralement au sein d'un solvant inerte tel qu'un éther comme le tétrahydrofuranne, à une température voisine de 0°C. Les organomagnésiens sont préparés selon les méthodes connues de l'homme de l'art telles que celles décrites dans les exemples.

Les composés de formule (I) pour lesquels R₁ est un atome d'hydrogène et R₂ est un radical -C(OH)(R₆)(R₁₂), -C(OH)(R₆)(alkyle), -C(=NO-CH₂-CH=CH₂)R₆ ou -C(=NOalk)R₆ peuvent être préparés selon le schéma réactionnel suivant :

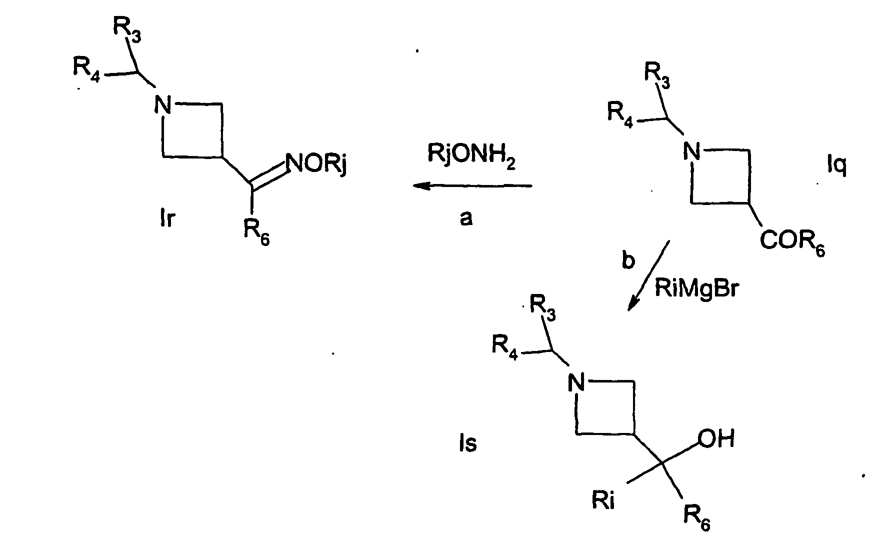

Dans ces formules, R₃, R₄ et R₆ ont les mêmes significations que dans la formule (I), Ri a les mêmes significations que R₁₂ ou représente un radical alkyle (1 à 6 atomes de carbone en chaîne droite ou ramifiée) et Rj représente un radical alkyle (1 à 6 atomes de carbone en chaîne droite ou ramifiée) ou -CH₂-CH=CH₂.

L'étape a s'effectue généralement au sein d'un solvant inerte tel qu'un alcool aliphatique (éthanol par exemple), en présence d'acétate de sodium, à une température comprise entre 20°C et la température d'ébullition du milieu réactionnel.

L' étape b s'effectue généralement au sein d'un solvant inerte tel qu'un éther comme le tétrahydrofuranne, à une température voisine de 0°C. Les organomagnésiens sont préparés selon les méthodes connues de l'homme de l'art telles que celles décrites dans les exemples.

Les composés de formule (I) pour lesquels R représente un radical CR₁R₂ dans lequel R₁ est un atome d'hydrogène et R₂ représente un radical -CH(R₆)NR₃₁R₃₂, dans lequel R₃₁ et R₃₂ sont des atomes d'hydrogène, -CH(R₆)NHSO₂alk, -CH(R₆)NHCONHalk ou -CH(R₆)NHCOR₃₁, peuvent être préparés selon le schéma réactionnel suivant :

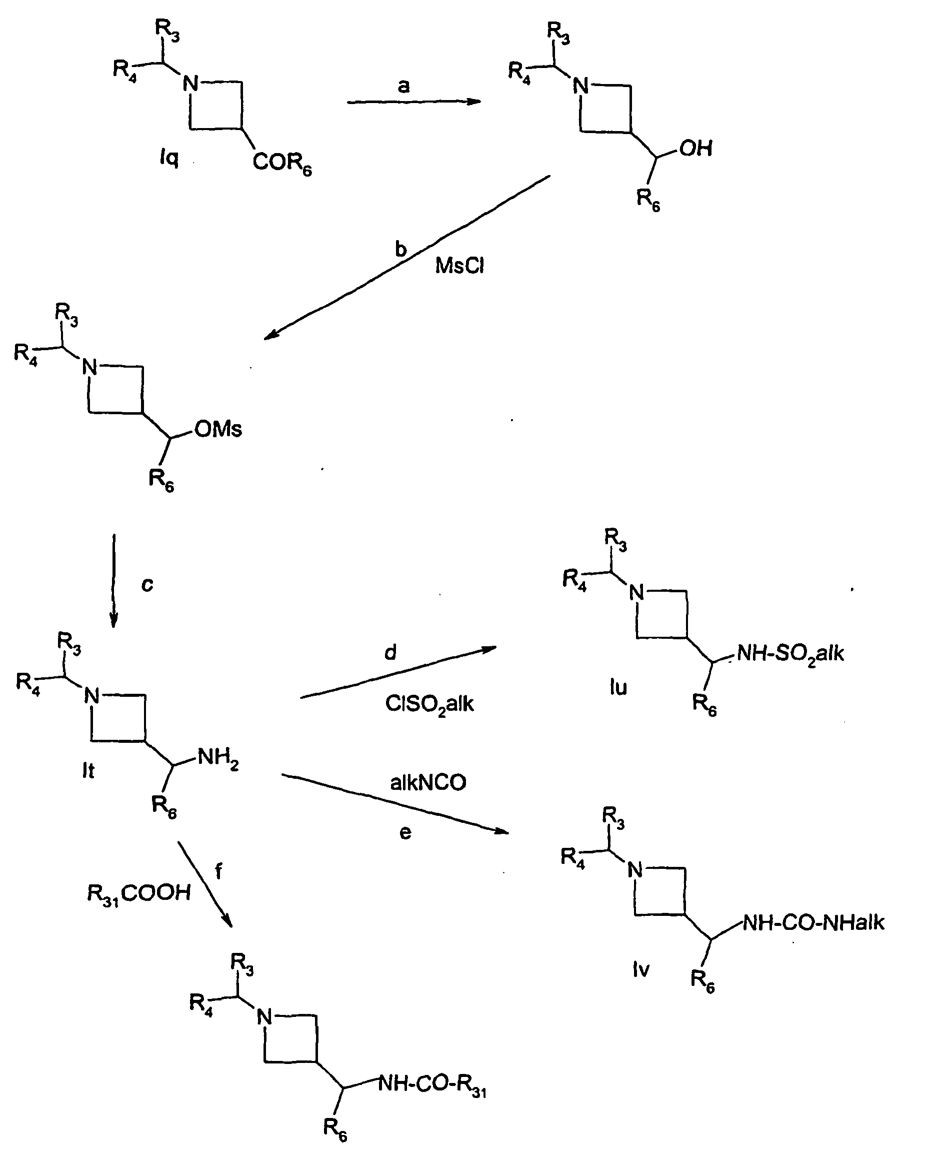

Dans ces formules R₃, R₄, R₆ et R₃₁ ont les mêmes significations que dans la formule (I), Ms représente un radical méthylsulfonyloxy, alk représente un radical alkyle.

La réaction a s'effectue généralement au moyen de NaBH₄, au sein de l'éthanol, à une tempéraure de voisine de 20°C.

L'étape b s'effectue en présence de triéthylamine, au sein d'un solvant inerte tel qu'un éther (tétrahydrofuranne par exemple), à une température voisine de 0°C.

L'étape c s'effectue au moyen d'ammoniaque liquide dans le méthanol, en autoclave à une température voisine de 60°.

L'étape d s'effectue généralement au sein d'un solvant inerte tel qu'un solvant halogéné (dichlorométhane par exemple) ou le tétrahydrofuranne, en présence d'une base organique telle que la triéthylamine, la diméthylaminopyridine, à une température voisine de 20°C.

L'étape e s'effectue par toute méthode connue de l'homme de l'art permettant de passer d'un acide ou un dérivé réactif de cet acide à un carboxamide sans toucher au reste de la molécule et notamment les méthodes préférées décrites précédemment.

L'étape f s'effectue généralement au sein d'un solvant inerte tel que le tétrahydrofuranne, le diméthylformamide, un solvant chloré (chloroforme, dichloroéthane par exemple), un solvant aromatique (benzène, toluène par exemple), à une température comprise entre 10°C et la température d'ébullition du milieu réactionnel.

Les composés de formule (I) pour lesquels R représente un radical CR₁R₂ dans lequel R₁ est un atome d'hydrogène et R₂ représente un radical -CH(R₆)NR₃₁R₃₂, R₃₁ est un atome d'hydrogène et R₃₂ est un radical alkyle, Ar ou -alk-Ar peuvent être préparés par action d'un halogénure HalR₃₁ sur un composé de formule (I) pour lequel R représente un radical CR₁R₂ dans lequel R₁ est un atome d'hydrogène et R₂ représente un radical -CH(R₆)NR₃₁R₃₂, R₃₁ et R₃₂ sont des atomes d'hydrogène.

Cette réaction s'effectue dans un solvant polaire inerte tel que l'acétonitrile, le tétrahydrofuranne ou le diméthylformamide, en présence d'une base organique ou minérale (carbonate de métalalcalin (sodium, potassium par exemple), trialkylamine (triéthylamine, diméthylaminapyridine par exemple)), à une température comprise entre 0°C et la température d'ébullition du solvant, en présence éventuellement de palladium ou de l'un de ses sels ou complexes.

Les composés de formule (I) pour lesquels R représente un radical CR₁R₂ dans lequel R₁ est un atome d'hydrogène et R₂ représente un radical -CH(R₆)NR₃₁R₃₂, R₃₁ est un atome d'hydrogène et R₃₂ est un radical alkyle peuvent également être préparés par action d'un composé de formule (I) correspondant pour lequel R représente un radical CR₁R₂ dans lequel R₁ est un atome d'hydrogène et R₂ représente un radical -CO-R₆ sur une amine HNR₃₁R₃₂ pour laquelle R₃₁ est un atome d'hydrogène et R₃₂ est un radical alkyle.

Cette réaction s'effectue généralement au sein d'un solvant inerte tel qu'un solvant chloré (dichlorométhane, dichloroéthane par exemple), en présence d'un agent réducteur tel que le triacétoxyborohydrure de sodium, à une température comprise entre 0°C et 70°C.

Les composés de formule (I) pour lesquels R représente un radical CR₁R₂ dans lequel R₁ est un atome d'hydrogène et R₂ représente un radical -CH(R₆)NR₃₁R₃₂, R₃₁ et R₃₂ sont des radicaux alkyle, Ar ou -alk-Ar peuvent être préparés par action d'un halogénure HalR₃₂ sur un composé de formule (I) pour lequel R représente un radical CR₁R₂ dans lequel R₁ est un atome d'hydrogène et R₂ représente un radical -CH(R₆)NR₃₁R₃₂ , R₃₁ est un atome d'hydrogène et R₃₂ est un radical alkyle, Ar ou -alk-Ar.

Cette réaction s'effectue dans un solvant polaire inerte tel que l'acétonitrile, le tétrahydrofuranne ou le diméthylformamide, en présence d'une base organique ou minérale (carbonate de métalalcalin (sodium, potassium par exemple), trialkylamine (triéthylamine, diméthylaminapyridine par exemple)), à une température comprise entre 0°C et la température d'ébullition du solvant, en présence éventuellement de palladium ou de l'un de ses sels ou complexes.

Les composés de formule (I) pour lesquels R représente un radical CR₁R₂ dans lequel R₁ est un atome d'hydrogène et R₂ représente un radical -CH(R₆)NR₃₁R₃₂, R₃₁ est un atome d'hydrogène et R₃₂ est un radical alkyle (2-6C) ou -alk(2-6C)-Ar peuvent être préparés par action d'un aldéhyde RaCHO pour lequel Ra est un radical alkyle ou -alk-Ar sur un composé de formule (I) pour lequel R représente un radical CR₁R₂ dans lequel R₁ est un atome d'hydrogène et R₂ représente un radical -CH(R₆)NR₃₁R_{32 ,} R₃₁ et R₃₂ sont des atomes d'hydrogène.

Cette réaction s'effectue dans un solvant inerte, tel que le dichlorométhane, le dichloroéthane, le toluène ou le tétrahydrofuranne, à une température comprise entre 0°C et 50°C, en présence d'un agent réducteur tel que le triacétoxyborohydrure de sodium ou le cyanoborohydrure de sodium.

Les composés de formule (I) pour lesquels R représente un radical CR₁R₂ dans lequel R₁ est un atome d'hydrogène et R₂ représente un radical -CH(R₆)NR₃₁R₃₂ , R₃₁ est un radical alkyle, Ar ou -alk-Ar et R₃₂ est un radical alkyle (2-6C)ou -alk(2-6C)-Ar peuvent être préparés par action d'un aldéhyde RaCHO pour lequel Ra est un radical alkyle ou -alk-Ar sur un composé de formule (I) pour lequel R représente un radical CR₁R₂ dans lequel R₁ est un atome d'hydrogène et R₂ représente un radical -CH(R₆)NR₃₁R₃₂, R₃₁ est un atome d'hydrogène et R₃₂ est un radical alkyle, Ar ou -alk-Ar.

Cette réaction s'effectue dans un solvant inerte, tel que le dichlorométhane, le dichloroéthane, le toluène ou le tétrahydrofuranne, à une température comprise entre 0°C et 50°C, en présence d'un agent réducteur tel que le triacétoxyborohydrure de sodium ou le cyanoborohydrure de sodium.

Les composés de formule (I) pour lesquels R représente un radical CR₁R₂ dans lequel R₁ est un atome d'hydrogène et R₂ représente un radical -CH(R₆)NR₃₁R₃₂, R₃₁ et R₃₂ forment avec l'atome d'azote auquel ils sont rattachés un hétérocycle choisi parmi aziridinyle, azétidinyle, pyrrolidinyle, pipéridinyle peuvent être préparés par action d'un dihalogénure Hal-alk(2-5C)-Hal sur un composé de formule (I) pour lequel R représente un radical CR₁R₂ dans lequel R₁ est un atome d'hydrogène et R₂ représente un radical -CH(R₆)NR₃₁R₃₂, R₃₁ et R₃₂ sont des atomes d'hydrogène.

Cette réaction s'effectue dans un solvant polaire inerte tel que l'acétonitrile, le tétrahydrofuranne ou le diméthylformamide, en présence d'une base organique ou minérale (carbonate de métalalcalin (sodium, potassium par exemple), trialkylamine (triéthylamine, diméthylaminapyridine par exemple)), à une température comprise entre 0°C et la température d'ébullition du solvant, en présence éventuellement de palladium ou de l'un de ses sels ou complexes.

Les composés de formule (I) pour lesquels R représente un radical CR₁R₂ dans lequel R₁ est un atome d'hydrogène et R₂ représente un radical -CH₂-COR₆, -CH₂-CH(R₆)-NR₃₁R₃₂, -CH₂-C(=NOalk)R₆ peuvent être préparés selon le schéma réactionnel suivant :

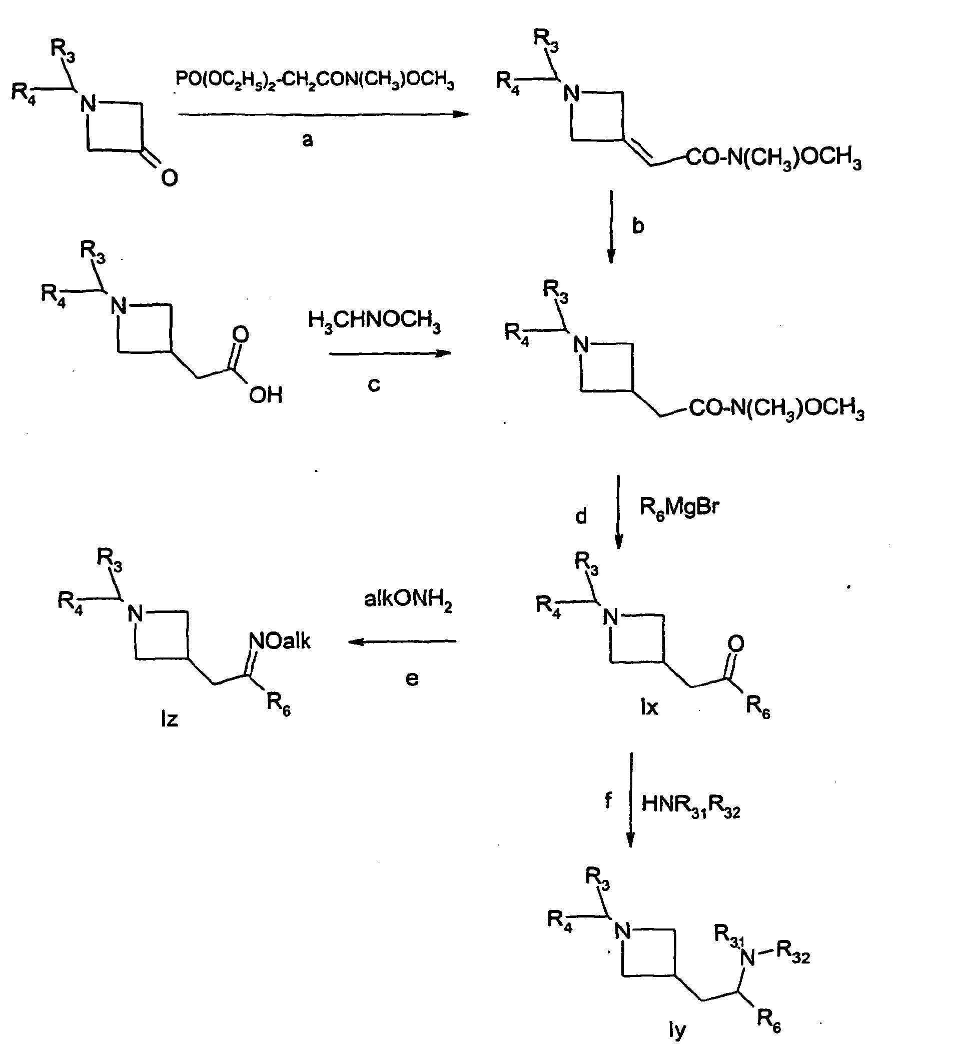

Dans ces formules R₃, R₄, R₆, R₃₁, R₃₂ ont les mêmes significations que dans la formule (I) et alk représente un radical alkyle.

L'étape a s'effectue généralement au sein d'un solvant tel que le tétrahydrofuranne, ,à une température comprise entre 20°C et la température d'ébullition du milieu réactionnel.

L'étape b s'effectue généralement au sein d'un solvant inerte tel qu'un alcool aliphatiqe (méthanol par exemple), un solvant chloré (chloroforme, dichlorométhane) ou un mélange de ces solvants, en présence d'un agent réducteur tel que NaBH₄, à une température comprise entre 0°C et la température d'ébullition du milieu réactionnel.

L'étape c s'effectue par toute méthode connue de l'homme de l'art permettant de passer d'un acide ou un dérivé réactif de cet acide à un carboxamide sans toucher au reste de la molécule et notamment les méthodes préférées décrites précédemment.

L'étape d s'effectue généralement au sein d'un solvant inerte tel qu'un éther comme le tétrahydrofuranne, à une température voisine de 0°C. Les organomagnésiens sont préparés selon les m'yhodes connues de l'homme de l'art telles que celles décrites dans les exemples.

L'étape e s'effectue généralement au sein d'un solvant inerte tel qu'un alcool aliphétique 1-4C tel que le méthanol, en présence d'acétate de sodium, à une température comprise entre 20°C et la température d'ébullition du milieu réactionnel.

L'étape f s'effectue au sein d'un solvant inerte tel qu'un solvant chloré (dichlorométhane, dichloroéthane par exemple), en présence d'un agent réducteur tel que le triacétoxyborohydrure de sodium, à une température comprise entre 0°C et 70°C.

Les composés de formule (I) pour lesquels R représente un radical CR₁R₂ dans lequel R₁ représente un radical cyano, -S-alk-NR₁₆R₁₇, -NHR₁₅, alkyle ou -NR₂₀R₂₁ et R₂ représente un radical -C(R₈)(R₁₁)(R₁₂) dans lequel R₈ est un atome d'hydrogène peuvent être préparés selon le schéma réactionnel suivant :

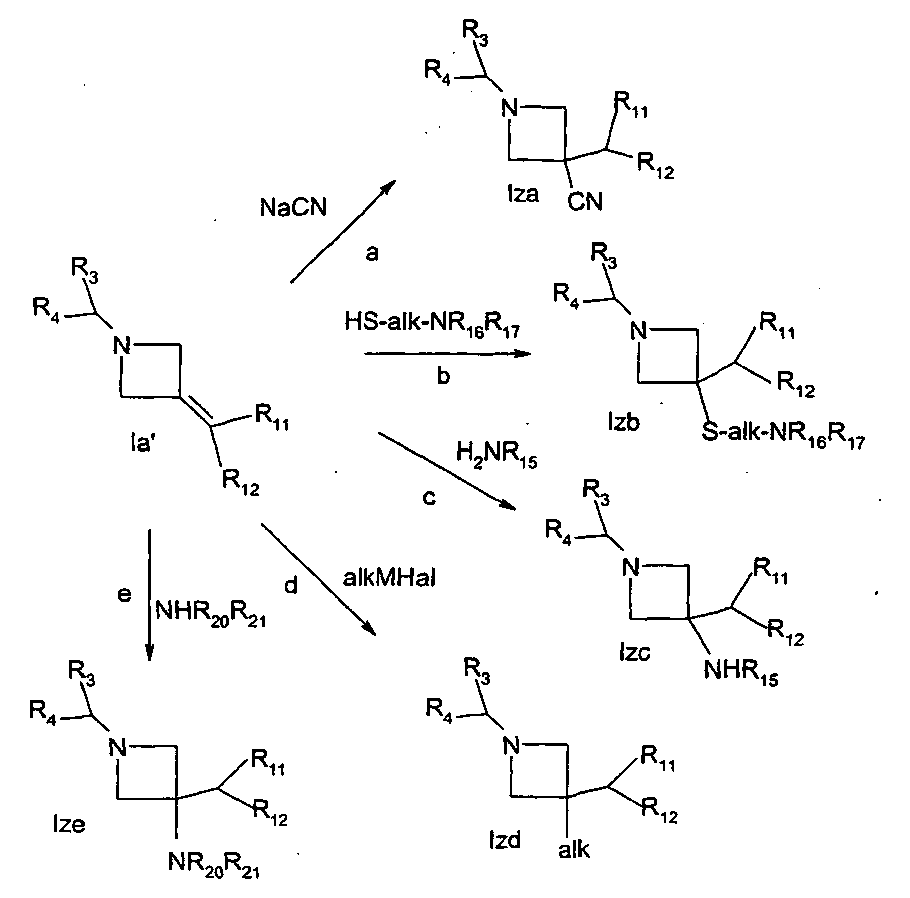

Dans ces formules R₃, R₄, R₁₁, R₁₂, R₁₅, R₁₆ et R₁₇ ont les mêmes significations que dans la formule (I), alk représente un radical alkyle, Hal représente un atome d'halogène et M représente un métal et de préférence le cuivre.

L'étape a s'effectue de préférence au sein d'un solvant polaire tel que le diméthylsulfoxyde, à une température comprise entre 20 et 50°C.

L'étape b s'effectue de préférence au sein d'un solvant inerte tel que le diméthylsulfoxyde, le tétrahydrofuranne, l'acétonitrile, en présence d'une base telle qu'un carbonate de métal alcalin (carbonate de potassium par exemple)

ou l'hydroxyde d'ammonium, à une température comprise entre 20°C et la température d'ébullition du milieu réactionnel.

L'étape c s'effectue de préférence au sein d'un solvant inerte tel que le diméthylsulfoxyde, le tétrahydrofuranne, l'acétonitrile, en présence d'une base telle qu'un carbonate de métal alcalin (carbonate de potassium par exemple) ou l'hydroxyde d'ammonium, à une température comprise entre 20°C et la température d'ébullition du milieu réactionnel.

L"étape d s'effectue de préférence au sein d'un solvant inerte tel qu'un éther (éther éthylique), tétrahydrofuranne à une température comprise entre -78°C et 20°C .

L'étape e s'effectue de préférence au sein d'un solvant inerte tel que le diméthylsulfoxyde, le tétrahydrofuranne, l'acétonitrile, le dichlorométhane; le dichloroéthane, en présence d'une base telle qu'un carbonate de métam alcalin (carbonete de potassium par exemple) ou l'hydroxyde d'ammonium, à une température comprise entre 20°C et la température d'ébullition du milieu réactionnel.

Les composés de formule (I) pour lesquels R représente un radical CR₁R₂ dans lequel R₁ représente un radical -alk-NR₁₈R₁₉, R₁₈ et R₁₉ représentent un atome d'hydrogène peuvent être préparés par réduction du composé de formule (I) correspondant pour lequel R représente un radical CR₁R₂ dans lequel R₁ représente un radical cyano.

Cette réaction s'effectue généralement au sein d'un solvant inerte tel que le tétrahydrofuranne, l'éther éthylique, le toluène, à une température comprise entre 0°C et la température d'ébullition du milieu réactionnel, en présence d'un agent réducteur tel que l'hydrure d'aluminium.

Les composés de formule (I) pour lesquels R représente un radical CR₁R₂ dans lequel R₁ représente un radical -alk-NR₁₈R₁₉, R₁₈ représente un atome d'hydrogène et R₁₉ représente un radical alkyle, cycloalkyle, cycloalkylalkyle, cycloalkylcarbonyle, -SO₂alk, -CO-NHalk ou -COOalk peuvent être préparés par action d'un halogénure HalR₁₉, Hal représente un halogène sur un composé de formule (I) pour lequel R représente un radical CR₁R₂ dans lequel R₁ représente un radical -alk-NR₁₈R₁₉, R₁₈ et R₁₉ représentent un atome d'hydrogène.

Cette réaction s'effectue dans un solvant polaire inerte tel que l'acétonitrile, le tétrahydrofuranne ou le diméthylformamide, en présence d'une base organique ou minérale (carbonate de métalalcalin (sodium, potassium par exemple), trialkylamine (triéthylamine, diméthylaminapyridine par exemple)), à une température comprise entre 0°C et la température d'ébullition du solvant, en présence éventuellement de palladium ou de l'un de ses sels ou complexes.

Les composés de formule (I) pour lesquels R représente un radical CR₁R₂ dans lequel R₁ représente un radical -alk-NR₁₈R₁₉, R₁₈ représente un radical alkyle et R₁₉ représente un radical alkyle, cycloalkyle, cycloalkylalkyle, cycloalkylcarbonyle, -SO₂alk, -CO-NHalk ou -COOalk peuvent être préparés par action d'un halogénure d'alkyle sur un composé de formule (I) pour lequel R représente un radical CR₁R₂ dans lequel R₁ représente un radical -alk-NR₁₈R₁₉, R₁₈ représente un atome d'hydrogène et R₁₉ représente un radical alkyle, cycloalkyle, cycloalkylalkyle, cycloalkylcarbonyle, -SO₂alk, -CO-NHalk ou -COOalk.

Cette réaction s'effectue dans un solvant polaire inerte tel que l'acétonitrile, le tétrahydrofuranne ou le diméthylformamide, en présence d'une base organique ou minérale (carbonate de métalalcalin (sodium, potassium par exemple), trialkylamine (triéthylamine, diméthylaminapyridine par exemple)), à une température comprise entre 0°C et la température d'ébullition du solvant, en présence éventuellement de palladium ou de l'un de ses sels ou complexes.

Les composés de formule (I) pour lesquels R représente un radical CR₁R₂ dans lequel soit R₁ représente un atome d'hydrogène et R₂ représente un radical -C(R₈)(R₉)(R₁₀) ou -C(R₈)(R₁₁)(R₁₂), soit R₁ représente un radical alkyle, NH-R₁₅, cyano, -S-alk-NR₁₆R₁₇, -alk-NR₁₈R₁₉, ou -NR₂₀R₂₁ et R₂ représente un radical -C(R₈)(R₁₁)(R₁₂) et R₈ représente un radical alkyle peuvent être préparés par alkylation d'un composé de formule (I) correspondant pour lequel R₈ est un atome d'hydrogène.

Cette réaction s'effectue de préférence au moyen d'une base telle qu'un hydrure de métal alcalin (hydrure de sodium par exemple), un amidure de métal alcalin (amidure de sodium par exemple) ou un dérivé organométallique, au sein d'un solvant inerte tel qu'un éther aliphétique (éther éthylique) ou le tétrahydrofuranne, à une température comprise entre -78°C et 30°C, au moyen d'un agent d'alkylation tel qu'un halogènure d'alkyle ou un sulfonate d'alkyle.

Les composés de formule (I) pour lesquels R représente un radical C=C(R₇)SO₂alk peuvent également être préparés selon le schéma réactionnel suivant :

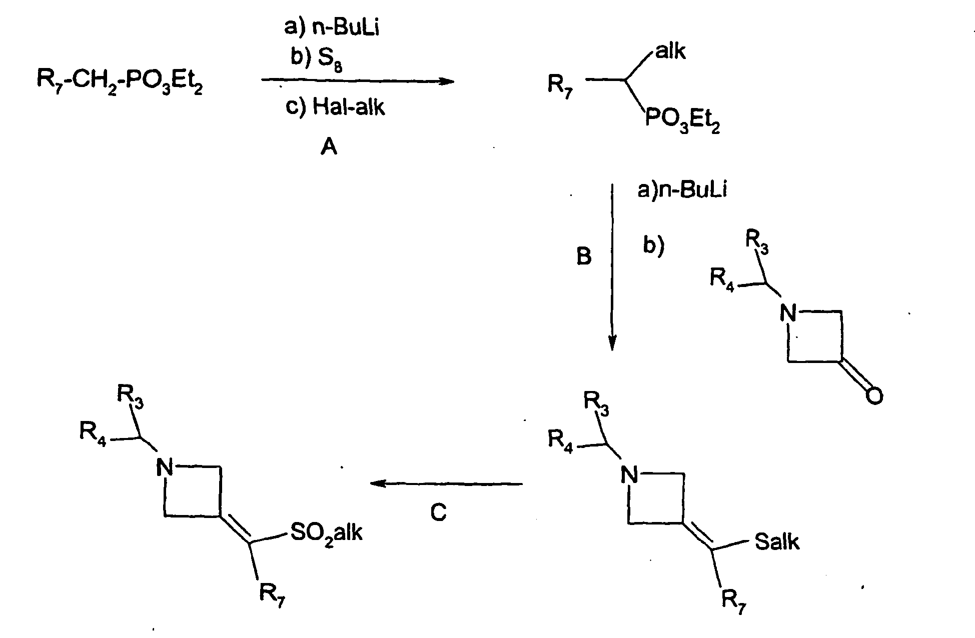

Dans ces formules R₃, R₄ et R₇ ont les mêmes significations que dans la formule (I), alk représente un radical alkyle et Hal représente un atome d'halogène

La réaction A s'effectue généralement au sein d'un solvant inerte tel qu'un éther (éther éthylique par exemple), en présence d'une base forte telle que le tert-butyllithium ou le n-butyllithium, à une température comprise entre -70°C et -50°C, puis addition de soufre puis d'un halogénure d'alkyle (iodure, bromure par exemple).

La réaction B s'effectue généralement au sein d'un solvant inerte tel qu'un éther (tétrahydrofuranne par exemple), en présence d'une base forte telle que le tert-butyllithium ou le n-butyllithium, à une température comprise entre -70°C et -50°C, puis addition de l'azétidin-3-one, retour à température ambiante et hydrolyse.

La réaction C s'effectue par toutes méthodes connues permettant d'oxyder un dérivé soufré sans toucher au reste de la molécule comme celles décrites précédemment.

Il est entendu pour l'homme du métier que, pour la mise en oeuvre des procédés selon l'invention décrits précédemment, il peut être nécessaire d'introduire des groupes protecteurs des fonctions amino, hydroxy et carboxy afin d'éviter des réactions secondaires. Ces groupes sont ceux qui permettent d'être éliminés sans toucher au reste de la molécule. Comme exemples de groupes protecteurs de la fonction amino on peut citer les carbamates de tert-butyle ou de méthyle qui peuvent être régénérées au moyen d'iodotriméthylsilane ou d'allyle au moyen de catalyseurs du palladium. Comme exemples de groupes protecteurs de la fonction hydroxy, on peut citer les triéthylsilyle, tert-butyldiméthylsilyle qui peuvent être régénérés au moyen de fluorure de tétrabutylammonium ou bien les acétals dissymétriques (méthoxyméthyle, tétrahydropyranyle par exemple) avec dégénération au moyen d'acide chlorhydrique. Comme groupes protecteurs des fonctions carboxy, on peut citer les esters (allyle, benzyle par exemple), les oxazoles et les 2-alkyl-1,3-oxazolines. D'autres groupes protecteurs utilisables sont décrits par GREENE T.W. et coll., Protecting Groups in Organic Synthesis, second edition, 1991, Jonh Wiley & Sons.

Les composés de formule (I) peuvent être purifiés par les méthodes connues habituelles, par exemple par cristallisation, chromatographie ou extraction.

Les énantiomères des composés de formule (I) peuvent être obtenus par dédoublement des racémiques par exemple par chromatographie sur colonne chirale selon PIRCKLE W.H. et coll., asymmetric synthesis, vol. 1, Academic Press (1983) ou par formation de sels ou par synthèse à partir des précurseurs chiraux. Les diastéréoisomères peuvent être préparés selon les méthodes classiques connues (cristallisation, chromatographie ou à partir des précurseurs chiraux).

Les composés de formule (I) peuvent être éventuellement transformés en sels d'addition avec un acide minéral ou organique par action d'un tel acide au sein d'un solvant organique tel qu'un alcool, une cétone, un éther ou un solvant chloré. Ces sels font également partie de l'invention.

Comme exemples de sels pharmaceutiquement acceptables, peuvent être cités les sels suivants : benzènesulfonate, bromhydrate, chlorhydrate, citrate, éthanesulfonate, fumarate, gluconate, iodate, iséthionate, maléate, méthanesulfonate, méthylène-bis-β-oxynaphtoate, nitrate, oxalate, pamoate, phosphate, salicylate, succinate, sulfate, tartrate, théophyllinacétate et p-toluènesulfonate.

Les composés de formule (I) présentent des propriétés pharmacologiques intéressantes. Ces composés possèdent une forte affinité pour les récepteurs cannabinoïdes et particulièrement ceux de type CB1. Ce sont des antagonistes du récepteur CB1 et sont donc utiles dans le traitement et la prévention des désordres touchant au système nerveux central, au système immunitaire, au système cardio-vasculaire ou endocrinien, au système respiratoire, à l'appareil gastrointestinal et aux désordres de la reproduction (Hollister, Pharm. Rev.; 38, 1986, 1-20, Reny et Sinha, Prog. Drug Res., 36, 71-114 (1991), Consroe et Sandyk, in Marijuana/Cannabinoids, Neurobiology and Neurophysiology, 459, Murphy L. and Barthe A. Eds, CRC Press, 1992).

C'est ainsi que ces composés peuvent être utilisés pour le traitement ou la prévention des psychoses y compris la schizophrénie, des troubles anxieux, de la dépression, de l'épilepsie, de la neurodégénération, des désordres cérébelleux et spinocérébelleux, des désordres cognitifs, du trauma crânien, des attaques de panique, des neuropathies périphériques, des glaucomes, de la migraine, de la maladie de Parkinson, de la maladie d'Alzheimer, de la chorée de Huntington, du syndrome de Raynaud, des tremblements, du désordre compulso-obsessionnel, de la démence sénile, des désordres thymiques, du syndrome de Tourette, de la dyskinésie tardive, des désordres bipolaires, des cancers, des désordres du mouvement induit par les médicaments, des dystonies, des chocs endotoxémiques, des chocs hémorragiques, de l'hypotension, de l'insomnie, des maladies immunologiques, de la sclérose en plaques, des vomissements, de l'asthme, des troubles de l'appétit (boulimie, anorexie), de l'obésité, des troubles de la mémoire, dans le sevrage aux traitements chroniques et abus d'alcool ou de médicaments (opioides, barbituriques, cannabis, cocaïne, amphétamine, phencyclide, hallucinogènes, benzodiazépines par exemple), comme analgésiques ou potentialisateurs de l'activité analgésique des médicaments narcotiques et non narcotiques. Ils peuvent également être utilisés pour le traitement ou la prévention du transit intestinal.

L'affinité des composés de formule (I) pour les récepteurs du cannabis a été déterminée selon la méthode décrite par KUSTER J.E., STEVENSON J.I., WARD S.J., D'AMBRA T.E., HAYCOCK D.A. dans J. Pharmacol. Exp. Ther., 264 1352-1363 (1993).

Dans ce test, la CI₅₀ des composés de formule (I) est inférieure ou égale à 1000 nM.

Leur activité antagonistique a été montrée au moyen du modèle d'hypothermie induite par un agoniste des récepteurs du cannabis (CP-55940) chez la souris, selon la méthode décrite par Pertwee R.G. dans Marijuana, Harvey D.J. eds, 84 Oxford IRL Press, 263-277 (1985).

Dans ce test, la DE50 des composés de formule (I) est inférieure ou égale à 50 mg/kg.

Les composés de formule (I) présentent une toxicité faible. Leur DL₅₀ est supérieure à 40 mg/kg par voie sous cutanée chez la souris.

Les composés de formule (I) préférés sont les suivants :
(RS)-1-[bis(4-chlorophényl)méthyl)]-3-[(3,5-difluorophényl)(méthylsulfonyl) méthyl] -azétidine,
(R)-1-[bis(4-chlorophényl)méthyl)]-3-[(3,5-difluorophényl)(méthylsulfonyl) méthyl]-azétidine,
(S)-1-[bis(4-chlorophényl)méthyl)]-3-[(3,5-difluorophényl)(méthylsulfonyl) méthyl]-azétidine,
(RS)-1-[bis(4-chlorophényl)méthyl)]-3-[(pyrid-3-yl)(méthylsulfonyl)méthyl]-azétidine,
(R)-1-[bis(4-chlorophényl)méthyl)]-3-[(pyrid-3-yl)(méthylsulfonyl)méthyl]-azétidine,
(S)-1-[bis(4-chlorophényl)méthyl)]-3-[(pyrid-3-yl)(méthylsulfonyl)méthyl]-azétidine,
(RS)-1-[bis-(3-fluorophényl)méthyl]-3-[(3,5-difluorophényl)méthylsu(fonyl-méthyl]azétidine,
(R)-1-[bis-(3-fluorophényl)méthyl]-3-[(3,5-difluorophényl)méthylsulfonylméthyl]azétidine,
(S)-1-[bis-(3-fluorophényl)méthyl]-3-[(3,5-difluorophényl)méthylsulfonylméthyl]azétidine,
1-[bis-(4-chlorophényl)méthyl]-3-(RS)-{[3-azétidin-1-yl-phényl]méthylsulfonylméthyl}azétidine,
1-[bis-(4-chlorophényl)méthyl]-3-(R)-{[3-azétidin-1-yl-phényl]méthylsulfonylméthyl}azétidine,
1-[bis-(4-chlorophényl)méthyl]-3-(S)-{[3-azétidin-1-yl-phényl]méthylsulfonylméthyl}azétidine,
(RS)-1-[3-({1-[bis-(4-chlorophényl)méthyl]azétidin-3-yl}méthylsulfonylméthyl)phényl]pyrrolidine,
(R)-1-[3-({1-[bis-(4-chlorophényl)méthyl]azétidin-3-yl}méthylsulfonylméthyl)phényl]pyrrolidine,
(S)-1-[3-({1-[bis-(4-chlorophényl)méthyl]azétidin-3-yl}méthylsulfonylméthyl)phényl]pyrrolidine,
(RS)-N-[3-({1-[bis-(4-chlorophényl)méthyl]azétidin-3-yl}méthylsulfonyl-méthyl)phényl]-N-méthyl-amine,
(R)-N-[3-({1-[bis-(4-chlorophényl)méthyl]azétidin-3-yl}méthylsulfonylméthyl)phényl]-N-méthyl-amine,
(S)-N-[3-({1-[bis-(4-chlorophényl)méthyl]azétidin-3-yl}méthylsulfonylméthyl)phényl]-N-méthyl-amine,
(RS)-1-[bis-(4-chlorophényl)méthyl]-3-[(3,5-bis-trifluorométhylphényl) méthylsulfonyl-méthyl]azétidine,
(R)-1-[bis-(4-chlorophényl)méthyl]-3-[(3,5-bis-trifluorométhylphényl) méthylsulfonyl-méthyl]azétidine,
(S)-1-[bis-(4-chlorophényl)méthyl]-3-[(3,5-bis-trifluorométhylphényl) méthylsulfonyl-méthyl]azétidine
1-[bis-(4-chlorophényl)méthyl]-3-(phénylsulfonylméthyl)-azétidine,
(RS)-1-[bis-(4-chlorophényl)-méthyl]-3-[(3,5-difluorophényl)-méthylsulfonylméthyl]-3-méthyl-azétidine,
(R)-1-[bis-(4-chlorophényl)-méthyl]-3-[(3,5-difluorophényl)-méthylsulfonylméthyl]-3-méthyl-azétidine,
(S)-1-[bis-(4-chlorophényl)-méthyl]-3-[(3,5-difluorophényl)-méthylsulfonylméthyl]-3-méthyl-azétidine,
(RS)-2-{1-[bis-(4-chlorophényl)méthyl]-azétidin-3-yl}-2-(3,5-difluorophényl)-N-cyclohexylacétamide,
(R)-2-{1-[bis-(4-chlorophényl)méthyl]-azétidin-3-yl}-2-(3,5-difluorophényl)-N-cyclohexylacétamide,
(S)-2-{1-[bis-(4-chlorophényl)méthyl]-azétidin-3-yl}-2-(3,5-difluorophényl)-N-cyclohexylacétamide,
(RS)-2-{1-[bis-(4-chlorophényl)méthyl]-azétidin-3-yl)-2-(3,5-difluorophényl)-N-isobutylacétamide,
(R)-2-{1-[bis-(4-chlorophényl)méthyl]-azétidin-3-yl}-2-(3,5-difluorophényl)-N-isobutylacétamide,
(S)-2-{1-[bis-(4-chlorophényl)méthyl]-azétidin-3-yl}-2-(3,5-difluorophényl)-N-isobutylacétamide,
(RS)-2-{1-[bis-(4-chlorophényl)méthyl]-azétidin-3-yl}-2-(3,5-difluorophényl)-N-cyclopropylméthyl acétamide,
(R)-2-{1-[bis-(4-chlorophényl)méthyl]-azétidin-3-yl}-2-(3,5-difluorophényl)-N-cyclopropylméthyl acétamide,
(S)-2-{1-[bis-(4-chlorophényl)méthyl]-azétidin-3-yl}-2-(3,5-difluorophényl)-N-cyclopropylméthyl acétamide,
(RS)-2-{1-[bis-(4-chlorophényl)méthyl]-azétidin-3-yl}-2-(3,5-difluorophényl)-N-isopropylacétamide,
(R)-2-{1-[bis-(4-chlorophényl)méthyl]-azétidin-3-yl}-2-(3,5-difluorophényl)-N-isopropylacétamide,
(S)-2-{1-[bis-(4-chlorophényl)méthyl]-azétidin-3-yl}-2-(3,5-difluorophényl)-N-isopropylacétamide,
(RS)-1-[bis-(4-chlorophényl)-méthyl]-3-[1-(3,5-difluorophényl)-1-méthylsulfonyl-éthyl]-azétidine,
(R)-1-[bis-(4-chlorophényl)-méthyl]-3-[1-(3,5-difluorophényl)-1-méthylsulfonyl-éthyl]-azétidine,
(S)-1-[bis-(4-chlorophényl)-méthyl]-3-[1-(3,5-difluorophényl)-1-méthylsulfonyl-éthyl]-azétidine,
(RS)-1-[bis-(4-fluoro-phényl)-méthyl]-3-[(3,5-difluorophényl)-méthylsulfonylméthyl]-azétidine,
(R)-1-[bis-(4-fluoro-phényl)-méthyl]-3-[(3,5-difluorophényl)-méthylsulfonylméthyl]-azétidine,
(S)-1-[bis-(4-fluoro-phényl)-méthyl]-3-[(3,5-difluorophényl)-méthylsulfonylméthyl]-azétidine,
(RS)-{1-[(3-pyridyl)-(4-chlorophényl)méthyl]-3-[(3,5-difluorophényl)-méthylsulfonyl-méthyl]-azétidine,
(SS)-{1-[(3-pyridyl)-(4-chlorophényl)méthyl]-3-[(3,5-difluorophényl)-méthylsulfonyl-méthyl]-azétidine,
(RR)-{1-[(3-pyridyl)-(4-chlorophényl)méthyl]-3-[(3,5-difluorophényl)-méthylsulfonyl-méthyl]-azétidine,
(SR)-{1-[(3-pyridyl)-(4-chlorophényl)méthyl]-3-[(3,5-difluorophényl)-méthylsulfonyl-méthyl]-azétidine,
(RS)-{1-[(4-pyridyl)-(4-chlorophényl)méthyl]-3-[(3,5-difluorophényl)-méthylsulfonyl-méthyl]-azétidine,
(SS)-{1-[(4-pyridyl)-(4-chlorophényl)méthyl]-3-[(3,5-difluorophényl)-méthylsulfonyl-méthyl]-azétidine,
(RR)-{1-[(4-pyridyl)-(4-chlorophényl)méthyl]-3-[(3,5-difluorophényl)-méthylsulfonyl-méthyl]-azétidine,
(SR)-{1-[(4-pyridyl)-(4-chlorophényl)méthyl]-3-[(3,5-difluorophényl)-méthylsulfonyl-méthyl]-azétidine,
(RS)-5-((4-chlorophényl)-{3-[(3,5-difluorophényl)-méthylsulfonyl-méthyl]-azétidin-1-yl}-méthyl)-pyrimidine,
(SR)-5-((4-chlorophényl)-{3-[(3,5-difluorophényl)-méthylsulfonyl-méthyl]-azétidin-1-yl}-méthyl)-pyrimidine,
(RR)-5-((4-chlorophényl)-{3-[(3,5-difluorophényl)-méthylsulfonyl-méthyl]-azétidin-1-yl}-méthyl)-pyrimidine,
(SS)-5-((4-chlorophényl)-{3-[(3,5-difluorophényl)-méthylsulfonyl-méthyl]-azétidin-1-yl}-méthyl)-pyrimidine,
(SS)-{1-[(2-chloro-pyrid-5-yl)-(4-chlorophényl)méthyl]-3-[(3,5-difluorophényl)-méthylsulfonyl-méthyl]-azétidine,
(RR)-{1-[(2-chloro-pyrid-5-yl)-(4-chlorophényl)méthyl]-3-[(3,5-difluorophényl)-méthylsulfonyl-méthyl]-azétidine,
(RS)-{1-[(2-chloro-pyrid-5-yl)-(4-chlorophényl)méthyl]-3-[(3,5-difluorophényl)-méthylsulfonyl-méthyl]-azétidine,
(SR)-{1-[(2-chloro-pyrid-5-yl)-(4-chlorophényl)méthyl]-3-[(3,5-difluorophényl)-méthylsulfonyl-méthyl]-azétidine,
leurs isomères optiques et leurs sels pharmaceutiquement acceptables.

Les exemples suivants illustrent l'invention.

### Exemple 1

La (RS)-1-[bis(4-chlorophényl)méthyl)]-3-[(3,5-difluorophényl)(méthylsulfonyl méthyl]-azétidine peut être préparée à partir de 1,0 g de 1-[bis(4-chlorophényl)méthyl)]-3-[(3,5-difluorophényl)(méthylsulfonyl)méthylène]-azétidine dans 40 cm³ de méthanol, on additionne 96 mg d'hydroborure de sodium et agite pendant 3 heures à 20°C. Après addition de 100 cm³ de dichlorométhane, le mélange réactionnel est lavé 2 fois avec 50 cm³ d'eau, séché sur sulfate de magnésium, filtré et concentré à sec sous pression réduite (2,7 kPa). Le produit brut est chromatographié sur une colonne de gel de silice (granulométrie 0,063-0,200 mm, hauteur 6 cm, diamètre 3 cm), en éluant sous une pression de 0,8 bar d'argon avec du dichlorométhane puis le mélange dichlorométhane + 1% méthanol et en recueillant des fractions de 80 cm³. Les fractions 13 à 15 sont réunies et concentrées à sec sous pression réduite (2,7 kPa). On obtient 0,55 g d'un solide blanc qui est repris avec 50 cm³ d'éther isopropylique, filtré et sèché pour donner 0,47 g de (RS)-1-[bis(4-chlorophényl)méthyl)]-3-[(3,5-difluorophényl)(méthylsulfonyl)méthyl]-azétidine sous la forme d'un solide blanc [Spectre de R.M.N ¹H (400 MHz, (CD₃)₂SO d6 avec ajout de quelques gouttes de CD₃COOD d4, à une tempéraure de 353 K, δ en ppm) : 2,46 (t, J = 7,5 Hz : 1 H); 2,77 (s : 3H); 3,15 (mt : 2H); 3,40 (mt : 1 H); 3,49 (t large, J = 7,5 Hz : 1 H); 4,46 (s : 1 H); 4,81 (d, J = 9 Hz : 1 H); de 7,05 à 7,20 (mt : 3H); de 7,15 à 7,45 (mt : 8H)].

La 1-[bis(4-chlorophényl)méthyl)]-3-[(3,5-difluorophényl)(méthylsulfonyl) méthylène]-azétidine peut être préparée selon deux méthodes :

### Méthode 1

A une solution de 2,94 g de 1-[bis(4-chlorophényl)méthyl]-3-[(3,5-difluorophényl)(méthylsulfonyl)méthyl-(RS)]azétidin-3-ol dans 250 cm³ de dichlorométhane à 22°C, on ajoute 0,65 cm³ de chlorure de méthylsulfonyle puis, par petites portions en 15 minutes, 2,42 g de 4-diméthylamino pyridine; la solution orange est agitée 2 heures à température ambiante. Le mélange réactionnel est lavé 3 fois avec 150 cm³ d'eau distillée et une fois avec 150 cm³ d'une solution saturée de chlorure de sodium, puis séchée avec du sulfate de magnésium, filtrée et concentrée à sec sous pression réduite (2,7 kPa). Le résidu obtenu est chromatographié sur colonne de gel de silice (granulométrie 0,04-0,06 mm, diamètre 5,5 cm, hauteur 15 cm), sous une pression de 0,5 bar d'argon avec un mélange d'acétate d'éthyle et cyclohexane (1/9 en volumes) comme éluant et en recueillant des fractions de 70 cm³. Les fractions 15 à 36 sont réunies puis concentrées à sec sous pression réduite (2,7 kPa). On obtient 1,86 g d'une meringue blanche qui est cristallisée dans de l'éther isopropylique pour obtenir un solide fondant à 190°C. Une recristallisation dans 45 cm³ d'éthanol conduit à 1,08 g de 1-[bis(4-chlorophényl)méthyl]-3-[(3,5-difluorophényl)(méthyl-sulfonyl)méthylène] azétidine fondant à 206°C [Spectre RMN dans DMSO-d6, T=300K, δ en ppm (300 MHz) : 3,00 (3H,s, SCH₃), 3,87 (2H, s, NCH₂), 4,20 (2H, s, NCH₂), 4,75 (1H, s, NCH), 7,15 (2H, d, J=8Hz, 2CH arom.), 7,30 (5H, m, 5CH arom.), 7,45 (4H, d, J=7Hz, 4CH arom.)].

A une solution de 6,8 g de bis(4-chlorophényl)bromométhane dans 300 cm³ d'acétonitrile, on ajoute 6,75 g de chlorhydrate de 3-[(3,5-difluorophényl)(méthylsulfonyl)méthyl-(RS)]azétidin-3-ol puis 2,97 g de carbonate de potassium. Le mélange réactionnel est chauffé 1 heure au reflux, refroidi à température ambiante, filtré et concentré à sec sous pression réduite (2,7 kPa). Le résidu obtenu est chromatographié sur colonne de gel de silice (granulométrie 0,04-0,06 mm, diamètre 8,5 cm, hauteur 22 cm), sous une pression de 0,5 bar d'argon avec un mélange d'acétate d'éthyle et cyclohexane (25/75 en volumes) comme éluants et en recueillant des fractions de 250 cm³. Les fractions 11 à 48 sont réunies puis concentrées à sec sous pression réduite (2,7 kPa). On obtient 5,3 g de 1-[bis(4-chlorophényl)méthyl]-3-[(3,5-difluorophényl)(méthylsulfonyl)méthyl-(RS)]azétidin-3-ol [Spectre de R.M.N ¹H (300 MHz, (CD₃)₂SO d6, δ en ppm) : 2,00 (s : 3H); 2,94 (s : 3H); 3,25 (mt : 2H); 3,48 (d, J = 9 Hz : 1 H); 3,80 (d, J = 9 Hz : 1H); 4,54 (s : 1 H); 5,34 (s : 1 H); 7,15 (d, J = 8,5 Hz : 2H); de 7,20 à 7,40 (mt : 8H); 7,50 (t large, J = 9 Hz : 1H)].

Le bis(4-chlorophényl)bromométhane peut être préparé selon le mode opératoire décrit par BACHMANN W.E., J. Am. Chem. Soc., 2135 (1933).

Le chlorhydrate de 3-[(3,5-difluorophényl)(méthylsulfonyl)méthyl-(RS)]azétidin-3-ol peut être obtenu de la manière suivante : à une solution de 37 g de 3-[(3,5-difluorophényl)(méthylsulfonyl)méthyl-(RS)]-1-(vinyloxy-carbonyl)azétidin-3-ol dans 160 cm³ de dioxane on ajoute 160 cm³ d'une solution 6,2N d'acide chlorhydrique dans le dioxane. Après 16 heures à température ambiante, le mélange réactionnel est concentré à sec sous pression réduite (2,7 kPa). Le résidu obtenu est repris avec 320 cm³ d'éthanol, chauffé 1 heure au reflux et refroidi dans un bain d'eau glacée. Le solide apparu est filtré, lavé à l'éther éthylique et séché à 40°C sous pression réduite (2,7 kPa). On obtient 29,85 g de cristaux blancs dont la température de fusion est supérieure à 260°C.

Le 3-[(3,5-difluorophényl)(méthylsulfonyl)méthyl-(RS)]-1-(vinyloxycarbonyl) azétidin-3-ol peut être obtenu de la manière suivante : à une solution de 60,18 g de 1-benzhydryl-3-[(3,5-difluorophényl)(méthylsulfonyl)méthyl-(RS)]azétidin-3-ol dans 1000 cm³ de dichlorométhane on ajoute à 5°C une solution de 14 cm³ : de chloroformiate de vinyle dans 35 cm³ de dichlorométhane. Après 20 heures à température ambiante, le mélange réactionnel est concentré à sec sous pression réduite (2,7 kPa). Le résidu obtenu est chromatographié sur colonne de gel de silice (granulométrie 0,04-0,06 mm, diamètre 11 cm, hauteur 32 cm), sous une pression de 0,5 bar d'argon avec un mélange d'acétate d'éthyle et cyclohexane (3/7 en volumes) comme éluants et en recueillant des fractions de 1000 cm³. Les fractions 8 à 18 sont réunies puis concentrées à sec sous pression réduite (2,7 kPa). On obtient 37 g de 3-[(3,5-difluorophényl)(méthylsulfonyl)méthyl-(RS)]-1-(vinyloxycarbonyl)azétidin-3-ol sous forme de cristaux blancs fondants à 195°C.

Le 1-benzhydryl-3-[(3,5-difluorophényl)(méthylsulfonyl)méthyl-(RS)]azétidin-3-ol peut être obtenu de la manière suivante : à une solution de 6,73 cm³ de diisopropylamine dans 110 cm³ de tétrahydrofuranne, sous atmosphère d'argon, refroidie à -70°C, on ajoute 29,5 cm³ de n-butyllithium 1,6N en solution dans l'hexane. Après 30 minutes, on ajoute ensuite un mélange de 8,7 g de 3,5-difluorobenzyl méthyl sulfone dans 200 cm³ de tétrahydrofuranne et maintient l'agitation 45 minutes à -70°C. On additionne 10 g de 1-benzhydryl azétidin-3-one dissoute dans 60 cm³ de tétrahydrofuranne puis agite 20 minutes en laissant le mélange revenir à température ambiante. Le mélange réactionnel est hydrolysé avec 400 cm³ d'une solution saturée de chlorure d'ammonium, extrait avec du dichlorométhane, lavé 3 fois avec 500 cm³ d'eau puis une solution saturée de chlorure de sodium. La phase organique est sèchée sur sulfate de magnésium, filtrée, puis concentré à sec sous pression réduite (2,7 kPa). Le résidu (19 g) est repris par de l'éther isopropylique dans lequel il cristallise. Après filtration et séchage, on obtient 15,35 g de 1-benzhydryl-3-[(3,5-difluorophényl)(méthylsulfonyl)méthyl-(RS)]azétidin-3-ol sous forme de cristaux blancs.

La 1-benzhydryl azétidin-3-one peut être préparée selon le mode opératoire décrit par KATRITZKY A.R. et coll. dans J. Heterocycl. Chem., 271 (1994).

La 3,5-difluorobenzyl méthyl sulfone peut être préparée de la manière suivante : à partir de 33,46 g de 3,5-difluorobenzyl méthyl sulfure dans 318 cm³ d'eau, 318 cm³ d'acide acétique et 318 cm³ d'éthanol, on ajoute à 5°C 129,9 g d'oxone^{R}. Après 16 heures à température ambiante, le mélange réactionnel est dilué avec du dichlorométhane, lavé à l'eau et avec une solution saturée de chlorure de sodium, sèché, filtré et concentré à sec sous pression réduite (2,7 kPa). On obtient 35,57 g de 3,5-difluorobenzyl méthyl sulfone sous forme de cristaux blancs, PF = 135°C.

Le 3,5-difluorobenzyl méthyl sulfure peut être préparé de la manière suivante : à partir de 40 g de bromure de 3,5-difluorobenzyle et 16,25 g de méthyl thiolate de sodium dans le DMF à 60°C, on obtient après traitement 33,46 g de 3,5-difluorobenzyl méthyl sulfure sous forme d'une huile jaune.

### Méthode 2

A une solution de 2,2 g de 3-acétoxy-1-[bis(4-chlorophényl)méthyl]-3-[(3,5-difluorophényl)(méthylsulfonyl)méthyl-(RS)]azétidine dans 25 cm³ de dioxane à température ambiante, on ajoute 0,80 g de soude broyée. Après 16 heures à température ambiante, on ajoute 50 cm³ d'eau et 100 cm³ d'acétate d'éthyle. Le mélange est décanté, la phase organique relavée avec 100 cm³ d'eau, séchée sur sulfate de magnésium, filtrée et concentrée à sec sous pression réduite (2,7 kPa). On obtient une meringue blanche qui est cristallisée dans de l'éther d'isopropyle pour obtenir 0,85 g d'un solide fondant à 190°C. Une recristallisation dans 20 cm³ d'éthanol conduit à 0,70 g de 1-[bis(4-chlorophényl)méthyl]-3-[(3,5-difluorophényl)(méthylsulfonyl) méthylène]azétidine fondant à 205°C.

A une solution de 4,77 g de (3,5-difluorobenzyl)méthylsulfone dans 70 cm³ de tétrahydrofuranne sous atmosphère d'argon, on ajoute à -70°C, 14 cm³ d'une solution 1,6N de n butyllithium dans l'hexane. Après 1 heure à -70°C, une solution de 6,8 g de 1-[bis(4-chlorophényl)méthyl]azétidin-3-one dans 30 cm³ de tétrahydrofuranne est additionnée puis, 1 heure après, une solution de 2,34 cm³ de chlorure d'acétyle dans 20 cm³ de tétrahydrofuranne et la température du mélange réactionnel est élevée à 20°C pendant 1 heure. On ajoute 50 cm³ d'eau et 200 cm³ d'acétate d'éthyle. Le mélange est décanté, la phase organique lavée avec 100 cm³ d'eau, 100 cm³ d'une solution saturée de chlorure de sodium, séchée sur sulfate de magnésium, filtrée et concentrée à sec sous pression réduite (2,7 kPa). On obtient 14,4 g de 3-acétoxy-1-[bis-(4-chlorophényl)méthyl]-3-[(3,5-difluorophényl)(méthylsulfonyl) méthyl)méthyl sulfonylméthyl-(RS)]azétidine sous forme d'une huile jaune [Spectre de R.M.N ¹H (400 MHz, CDCl₃, δ en ppm) : 2,79 (s : 3H); 3,04 (AB, J = 9 Hz : 2H); 3,27 (d, J = 9 Hz : 1H); 3,45 (s : 1 H); 3,81 (d, J = 9 Hz : 1 H); 4,32 (s : 1 H); 4,49 (s : 1 H); 6,88 (tt, J = 9 et 2,5 Hz : 1 H); de 7,20 à 7,35 (mt : 10H)].

La 1-[bis(4-chlorophényl)méthyl]azétidin-3-one peut être préparée selon le mode opératoire suivant : à une solution de 5,0 cm³ de chlorure d'oxalyle dans 73 cm³ de dichlorométhane refroidie à -78°C, on additionne une solution de 8,1 cm³ de diméthylsulfoxyde dans 17,6 cm³ de dichlorométhane. Après 0,5 heure à -78°C, on coule une solution de 16,0 g de 1-[bis(4-chlorophényl)méthyl]azétidin-3-ol dissous dans 50 cm³ de dichlorométhane. Après 5 heures à -78°C, 26,6 cm³ de triéthylamine sont ajoutés goutte à goutte et on laisse le mélange réactionnel revenir à température ambiante. Après 16 heures, le mélange réactionnel est lavé par 4 fois 200 cm³ d'eau puis par 200 cm³ d'une solution saturée de chlorure de sodium, séché sur sulfate de magnésium, filtré et concentré à sec sous pression réduite (2,7 kPa). Le résidu obtenu est chromatographié sur colonne de gel de silice (granulométrie 0,04-0,06 mm, diamètre 9,2 cm, hauteur 21 cm), sous une pression de 0,5 bar d'argon avec un mélange d'acétate d'éthyle et cyclohexane (40/60 en volumes) comme éluants et en recueillant des fractions de 200 cm³. Les fractions 15 à 25 sont réunies puis concentrées à sec sous pression réduite (2,7 kPa). On obtient 8,9 g de 1-[bis(4-chlorophényl)méthyl]azétidin-3-one sous forme de cristaux jaunes pâle fondants à 111°C.

Le 1-[bis(4-chlorophényl)méthyl]azétidin-3-ol peut être préparée selon le mode opératoire décrit par KATRITZKY A.R. et coll., J. Heterocycl. Chem., (1994), 271 en partant de 35,5 g de chlorhydrate de [bis(4-chlorophényl)méthyl]amine et 11,0 cm³ d'épichlorhydrine. On isole 9,0 g de 1-[bis(4-chlorophényl)méthyl]azétidin-3-ol.

Le chlorhydrate de [bis(4-chlorophényl)méthyl]amine peut être préparé selon la méthode décrite par GRISAR M. et coll., J. Med. Chem., 885 (1973).

### Exemple 2

Les (-)-1-[bis(4-chlorophényl)méthyl)]-3-[(3,5-difluorophényl)(méthylsulfonyl)méthyl]-azétidine et (+)-1-[bis(4-chlorophényl)méthyl)]-3-[(3,5-difluorophényl)(méthylsulfonyl)méthyl]-azétidine peuvent être préparées par séparation CLHP sur colonne chirale CHIRALPAK AS (granulométrie 20µm, hauteur 23 cm, diamètre 6 cm) de 0,52 g du racémate préparé à l'exemple 1. En éluant avec le mélange heptane/éthanol (90/10) avec un débit de 80 cm³/min et après avoir concentrées les fractions collectées à sec sous pression réduite (2,7 kPa), on obtient 110 mg de (-)-1-[bis(4-chlorophényl)méthyl)]-3-[(3,5-difluorophényl)(méthylsulfonyl)méthyl]-azétidine [α_{D}] = -6,3° (C = 0,5M dans méthanol) sous la forme d'un solide blanc fondant à 178°C et 134 mg de (+)-1-[bis(4-chlorophényl)méthyl)]-3-[(3,5-difluorophényl) (méthylsulfonyl)méthyl]-azétidine [α_{D}] = +5,8° (C = 0,5M dans méthanol) sous la forme d'un solide blanc fondant à 178°C.

### Exemple 3

Le mélange des 2 diastéréoisomères formes A 1-[4-[(R*)-(4-chlorophényl)-{3-[(3,5-difluorophényl)-méthylsulfonyl-méthyl-(R)]azétidin-1-yl}méthyl]benzyl] pyrrolidine et 1-[4-[(R*)-(4-chlorophényl)-{3-[(3,5-difluorophényl)-méthylsulfonyl-méthyl-(S)]azétidin-1-yl}méthyl]benzyl]pyrrolidine peut être préparé en opérant de la façon suivante : A une solution de 60 mg de 1-(R*)-[4-(4-chlorophényl)-{3-[(3,5-difluorophényl)méthylsulfonyl-méthylène]azétidin-1-yl}méthyl)benzyl]pyrrolidine, isomère forme A dans 2 cm³ d'éthanol et 2 cm³ de dichlorométhane, on ajoute 20 mg d'hydroborure de sodium. Après 20 heures d'agitation à 20°C, on ajoute 0,25 cm³ d'eau, 20 cm³ de dichlorométhane, agite, et sèche le mélange sur sulfate de magnésium, puis filtre et concentre à sec sous pression réduite (2,7 kPa). Le résidu est chromatographié sur une colonne de gel de silice (granulométrie 0,063-0,200 mm, hauteur 7 cm, diamètre 1 cm), en éluant sous une pression de 0,1 bar d'argon avec du dichlorométhane puis avec un mélange de dichlorométhane et de méthanol (95/5 en volumes) et en recueillant des fractions de 5 cm³. Les fractions 13 à 18 sont réunies et concentrées à sec sous pression réduite (2,7 kPa). On obtient 38 mg du mélange des 2 diastéréoisomères formes A 1-[4-[(R*)-(4-chlorophényl)-{3-[(3,5-difluorophényl)-méthylsulfonyl-méthyl-(R)]azétidin-1-yl}méthyl]benzyl] pyrrolidine, et 1-[4-[(R*)-(4-chlorophényl)-{3-[(3,5-difluorophényl)-méthylsulfonyl-méthyl-(S)]azétidin-1-yl}méthyl]benzyl]pyrrolidine, sous la forme d'une meringue blanche [Spectre de R.M.N ¹H (400 MHz, CDCl₃, δ en ppm) : 1,77 (mt : 4H); de 2,40 à 2,60 (mt : 5H); 2,67 (s : 3H); de 3,10 à 3,25 (mt : 2H); 3,38 (mt : 1 H); de 3,50 à 3,70 (mt : 3H); 4,24 (s : 1 H); 4,25 (d, J = 11 Hz : 1 H); 6,83 (t large, J = 9 Hz : 1 H); 6,94 (mt : 2H); de 7,10 à 7,35 (mt : 8H)].

La 1-(R*)-[4-(4-chlorophényl)-{3-[(3,5-difluorophényl)méthylsulfonyl-méthylène]azétidin-1-yl}méthyl)benzyl]pyrrolidine, isomère forme A, peut être préparée en opérant de la façon suivante : A une solution de 0,32 g de 1-{(R*)-[4-(chlorométhyl)phényl)-(4-chlorophényl)méthyl}-3-[(3,5-difluorophényl)méthylsulfonyl-méthylène]azétidine, isomère forme A et 5 mg d'iodure de sodium dans 10 cm³ de dichlorométhane, on ajoute 50 mm³ de pyrrolidine. Après 20 heures d'agitation à 20°C, on ajoute au mélange 50 mm³ de pyrrolidine, agite pendant 8 heures puis ajoute à nouveau 50 mm³ de pyrrolidine et agite pendant 20 heures à 20°C. On lave le mélange réactionnel par de l'eau puis sèche la phase organique sur sulfate de magnésium, concentre à sec sous vide (2,7 kPa). Le résidu obtenu est chromatographié sur une colonne de gel de silice (granulométrie 0,06-0,200 mm, diamètre 1,2 cm, hauteur 30 cm), sous une pression de 0,1 bar d'argon en éluant avec du dichlorométhane puis avec un mélange dichlorométhane et méthanol (97,5/2,5 en volumes) et en recueillant des fractions de 3 cm³. Les fractions 12 à 40 sont réunies puis concentrées à sec sous pression réduite (2,7 kPa). On obtient 0,18 g de 1-(R*)-[4-(4-chlorophényl)-{3-[(3,5-difluorophényl)méthylsulfonyl-méthylène]azétidin-1-yl}méthyl)benzyl]pyrrolidine, isomère forme A, sous la forme d'une meringue blanche [α]²⁰365nm = -22,5° +/- 0,7 (c = 0,5 %; dichlorométhane) [Spectre de R.M.N. ¹H (300 MHz, CDCl₃, δ en ppm) : 1,78 (mt : 4H); 2,51 (mt : 4H); 2,81 (s : 3H); 3,58 (s : 2H); 3,84 (mt : 2H); 4,33 (mt : 2H); 4,50 (s : 1 H); 6,84 (tt, J = 9 et 2,5 Hz : 1 H); 6,98 (mt : 2H); de 7,20 à 7,40 (mt : 8H)].

La 1-{(R*)-[(4-chlorométhyl)phényl](4-chlorophényl)méthyl}-3-[(3,5-difluorophényl)méthyl-sulfonyl-méthylène]azétidine, isomère forme A, peut être préparée en opérant de la façon suivante : A une solution de 28,0 g du mélange des 2 diastéréoisomères (formes A) 1-{(R*)-[(4-chlorométhyl)phényl]-(4-chlorophényl)méthyl}-3-[(R)-(3,5-difluorophényl) méthylsulfonyl-méthyl)]azétidin-3-ol, et 1-{(R*)-[(4-chlorométhyl)phényl]-(4-chlorophényl)méthyl}-3-[(S)-(3,5-difluorophényl)méthylsulfonyl-méthyl] azétidin-3-ol, de 32 g de 4-diméthylaminopyridine, dans 500 cm³ de dichlorométhane, on ajoute 12,4 cm³ de chlorure de méthylsulfonyle. Après une heure d'agitation à 10°C, puis une heure à 20°C, le mélange réactionnel est lavé par 500 cm³ d'eau, la phase organique séchée sur sulfate de magnésium et concentrée à sec sous pression réduite (2,7 kPa). Le résidu est chromatographié sur une colonne de gel de silice (granulométrie 0,06-0,200 mm, diamètre 6 cm, hauteur 30 cm), sous une pression de 0,2 bar d'argon en éluant avec du dichlorométhane et en recueillant des fractions de 250 cm³. Les fractions 9 à 25 sont réunies puis concentrées à sec sous pression réduite (2,7 kPa). On obtient 6,3 g de 1-{(R*)-[4-(chlorométhyl)phényl]-(4-chlorophényl)méthyl}-3-[(3,5-difluorophényl)méthylsulfonyl-méthylène]azétidine, isomère forme A, sous la forme d'une meringue blanche.

Le mélange des 2 diastéréoisomères (formes A) 1-{(R*)-[4-(chlorométhyl) phényl](4-chlorophényl)méthyl}-3-[(R)-(3,5-difluorophényl)méthylsulfonylméthyl)]azétidin-3-ol, et 1-{(R*)-[4-(chlorométhyl)phényl](4-chlorophényl) méthyl}-3-[(S)-(3,5-difluorophényl)méthy-sulfonyl-méthyl)]azétidin-3-ol, peut être préparé en opérant de la façon suivante : A une solution de 0,20 g du mélange des 2 diastéréoisomères (formes A) 1-{(R*)-(4-chlorophényl)[4-(hydroxyméthyl)phényl]méthyl}-3-[(R)-(3,5-difluorophényl)méthylsulfonyl-méthyl)]azétidin-3-ol, et 1-{(R*)-(4-chlorophényl)[4-(hydroxyméthyl)phényl] méthyl}-3-[(S)-(3,5-difluorophényl)méthylsulfonyl-méthyl)]azétidin-3-ol, dans 10 cm³ de dichlorométhane, on ajoute 6 cm³ de chlorure de thionyle. Après 20 heures d'agitation à 20°C, on ajoute au mélange réactionnel 5 cm³ d'une solution aqueuse saturée d'hydrogénocarbonate de sodium, puis agite pendant 15 minutes. Le mélange est décanté, la phase organique est lavée avec de l'eau, séchée sur sulfate de magnésium, puis concentrée à sec sous pression réduite (2,7 kPa). Le résidu est chromatographié sur une colonne de gel de silice (granulométrie 0,04-0,06 mm, diamètre 1,0 cm, hauteur 20 cm), sous une pression de 0,2 bar d'argon en éluant avec un mélange cyclohexane et acétate d'éthyle (75/25 en volumes) et en recueillant des fractions de 20 cm³. Les fractions 4 à 7 sont réunies puis concentrées à sec sous pression réduite (2,7 kPa). On obtient 0,17 g du mélange des 2 diastéréoisomères (formes A) 1-{(R*)-[4-(chlorométhyl)phényl]-(4-chlorophényl)méthyl}-3-[(R)-(3,5-difluorophényl)méthylsulfonyl-méthyl)]azétidin-3-ol et 1-{(R*)-[4-(chlorométhyl)phényl]-(4-chlorophényl)méthyl}-3-[(S)-(3,5-difluorophényl)méthylsulfonyl-méthyl)]azétidin-3-ol, sous la forme d'une meringue blanche.

Le mélange des 2 diastéréoisomères (formes A) 1-{(R*)-(4-chlorophényl)[4-(hydroxyméthyl)phényl]méthyl}-3-[(R)-(3,5-difluorophényl)méthylsulfonyl-méthyl)]azétidin-3-ol et 1-{(R*)-(4-chlorophényl)[4-(hydroxyméthyl)phényl] méthyl}-3-[(S)-(3,5-difluorophényl)méthylsulfonyl-méthyl)]azétidin-3-ol, peut être préparé en opérant de la façon suivante : A une solution maintenue sous argon et refroidie à -30°C de 0,58 g du mélange des 2 diastéréoisomères (formes A) 3-acétoxy-1-{(R*)-(4-chlorophényl)[4-(méthoxycarbonyl)phényl] méthyl}-3-[(R)-(3,5-difluorophényl) méthylsulfonyl-méthyl)]azétidine, et 3-acétoxy-1-{(R*)-(4-chlorophényl)[4-(méthoxycarbonyl)phényl]méthyl}-3-[(S)-(3,5-difluorophényl)méthylsulfonyl- méthyl)]azétidine, dans 10 cm³ de toluène anhydre, on ajoute 1,6 cm³ d'une solution 1,5M dans le toluène d'hydrure de diisobutylaluminium. Après 15 minutes d'agitation à -30°C, on ajoute à nouveau 1,0 cm³ de cette même solution d'hydrure, puis laisse le mélange revenir à 0°C. Après 30 minutes d'agitation, le mélange agité est additionné de 3 cm³ d'eau et 6 cm³ d'hydroxyde de sodium 1 N puis extrait par 25 cm³ de dichlorométhane. La phase organique est lavée par 5 cm³ d'eau, 5 cm³ de saumure, puis séchée sur sulfate de magnésium et concentrée à sec sous pression réduite (2,7 kPa). Le résidu est chromatographié sur une colonne de gel de silice (granulométrie 0,06-0,200 mm, diamètre 1,2 cm, hauteur 30 cm), sous une pression de 0,1 bar d'argon en éluant avec un mélange cyclohexane et acétate d'éthyle (50/50 en volumes) et en recueillant des fractions de 30 cm³. Les fractions 4 à 12 sont réunies puis concentrées à sec sous pression réduite (2,7 kPa). On obtient 0,42 g du mélange des 2 diastéréoisomères (formes A) 1-{(R*)-(4-chlorophényl)[4-(hydroxyméthyl)phényl]méthyl}-3-[(R)-(3,5-difluorophényl)méthylsulfonyl-méthyl)]azétidin-3-ol, et 1-{(R*)-(4-chlorophényl)[4-(hydroxyméthyl phényl]méthyl}-3-[(S)-(3,5-difluorophényl)méthylsulfonyl-méthyl)]azétidin-3-ol, sous la forme d'une laque blanche.

Le mélange des 2 diastéréoisomères (formes A) 3-acétoxy-1-{(R*)-(4-chlorophényl)[4-(méthoxycarbonyl)phényl]méthyl}-3-[(R)-(3,5-difluorophényl) méthylsulfonyl-méthyl)]azétidine, et 3-acétoxy-1-{(R*)-(4-chlorophényl)[4-(méthoxycarbonyl)phényl]méthyl}-3-[(S)-(3,5-difluorophényl) méthylsulfonylméthyl)]azétidine, peut être préparé en opérant de la façon suivante : A une solution refroidie à -60°C de 1,0 g de (3,5-difluorobenzyl)méthylsulfone dans 30 cm³ de tétrahydrofuranne, on coule sous argon en 5 minutes, 3 cm³ d'une solution 1,6N de n-butyl lithium dans l'hexane. Après 1 heure d'agitation à -60°C puis 30 minutes à -30°C, on ajoute goutte à goutte à ce mélange une solution préalablement refroidie à -60°C de 1,45 g de 1-{(R*)-(4-chlorophényl)[4-(méthoxycarbonyl)phényl]méthyl}azétidin-3-one, isomère forme A dans 15 cm³ de tétrahydrofuranne. Après 30 minutes d'agitation à -60°C, puis 30 minutes à -30°C, on ajoute 0,43 cm³ de chlorure d'acétyle, puis laisse revenir le mélange réactionnel à 0°C. On ajoute ensuite au milieu en agitant 40 cm³ d'eau et 40 cm³ de dichlorométhane, puis laisse revenir à température ordinaire et décante. La phase organique est lavée avec 20 cm³ d'eau, puis est séchée sur sulfate de magnésium, filtrée et concentrée à sec sous pression réduite (2,7 kPa). Le résidu est chromatographié sur une colonne de gel de silice (granulométrie 0,040-0,063 mm, diamètre 3 cm, hauteur 30 cm), en éluant sous une pression de 0,5 bar d'argon avec un mélange cyclohexane et acétate d'éthyle (75/25 en volumes) et en recueillant des fractions de 30 cm³. Les fractions 21 à 35 sont réunies puis concentrées à sec sous pression réduite (2,7 kPa). On obtient 1,28 g du mélange des 2 diastéréoisomères (formes A) 3-acétoxy-1-{(R*)-(4-chlorophényl)[4-(méthoxycarbonyl)phényl]méthyl}-3-[(R)-(3,5-difluorophényl)méthylsulfonylméthyl)]azétidine, et 3-acétoxy-1-{(R*)-(4-chlorophényl)[4-(méthoxycarbonyl) phényl]méthyl}-3-[(S)-(3,5-difluorophényl)méthylsulfonyl-méthyl)]azétidine, sous la forme d'une meringue crème.

Le 1-{(R*)-(4-chlorophényl)[4-(méthoxycarbonyl)phényl]méthyl}azétidin-3-one, isomère forme A, peut être préparé en opérant de la façon suivante : A une solution refroidie à -60°C de 0,55 cm³ de chlorure d'oxalyle, dans 5 cm³ de dichlorométhane, on coule en 10 minutes 0,90 cm³ de diméthylsulfoxyde. Après 30 minutes d'agitation à -60°C, on ajoute en 15 minutes au mélange une solution de 1,75 g de 1-{(R*)-(4-chlorophényl)[4-(méthoxycarbonyl)phényl]méthyl} azétidin-3-ol, isomère forme A dans 20 cm³ de dichlorométhane. Après 3 heures d'agitation à -60°C, on coule 2,70 cm³ de triéthylamine puis laisse le milieu réactionnel revenir à 0°C. On ajoute alors 20 cm³ d'eau, agite puis décante. La phase organique est séchée sur sulfate de magnésium, filtrée, puis concentrée à sec à 50°C sous pression réduite (2,7 kPa). L'huile orangée obtenue est chromatographiée sur une colonne de gel de silice (granulométrie 0,06-0,200 mm, diamètre 2 cm, hauteur 30 cm), sous une pression de 0,5 bar d'argon en éluant avec un mélange cyclohexane et acétate d'éthyle (75/25 en volumes) et en recueillant des fractions de 30 cm³. Les fractions 2 à 15 sont réunies puis concentrées à sec sous pression réduite (2,7 kPa). On obtient 1,45 g de 1-{(R*)-(4-chlorophényl)[4-(méthoxycarbonyl)phényl]méthyl}azétidin-3-one, isomère forme A, sous la forme d'une meringue jaune.

Le 1-{(R*)-(4-chlorophényl)[4-(méthoxycarbonyl)phényl]méthyl}azétidin-3-ol, isomère forme A, peut être préparé en opérant de la façon suivante : A une suspension de 2,0 g de (+)-4-[(R*)-amino-(4-chlorophényl)méthyl]benzoate de méthyle, dans 30 cm³ d'éthanol, on ajoute 0,605 g d'hydrogénocarbonate de sodium, puis 0,60 cm³ d'épibromhydrine. Après 20 heures d'agitation à 60°C, le mélange réactionnel est concentré à sec sous pression réduite (2,7 kPa). Le résidu est chromatographié sur une colonne de gel de silice (granulométrie 0,06-0,200 mm, diamètre 3 cm, hauteur 35 cm), en éluant sous une pression de 0,5 bar d'argon avec un mélange de cyclohexane et acétate d'éthyle (70/30 en volumes pour les fractions 6 à 10, puis 60/40 pour les fractions 18 à 27, puis 50/50) et en recueillant des fractions de 60 cm³. Les fractions 15 à 40 sont réunies puis concentrées à sec sous pression réduite (2,7 kPa). Le résidu est repris avec 30 cm³ d'éthanol, puis est additionné de 0,20 g d'hydrogénocarbonate de sodium et de 0,2 cm³ d'épibromhydrine. Après 48 heures d'agitation à 20°C puis 24 heures à 35°C, le mélange est filtré, et le filtrat est concentré à sec à 60°C sous pression réduite (2,7 kPa). On obtient 1,76 g de 1-{(R*)-(4-chlorophényl)[4-(méthoxycarbonyl) phényl]méthyl}azétidin-3-ol, isomère forme A, sous la forme d'un solide pâteux.

Le (+)-4-[(R*)-amino-(4-chlorophényl)méthyl]benzoate de méthyle peut être préparé en opérant de la façon suivante : A une solution de 9,2 g de 4-[(RS)-amino-(4-chlorophényl)méthyl]benzoate de méthyle dans 10 cm³ de méthanol, on ajoute 2,51 g d'acide D-(-)-tartrique. La solution est concentrée à sec sous pression réduite (2,7 kPa). La meringue crème obtenue est dissoute dans 50 cm³ d'éthanol contenant 5% d'eau et la solution résultante est laissée cristalliser pendant 20 heures à 20°C. Les cristaux sont filtrés, lavés avec l'éthanol à 5% d'eau, essorés, puis séchés sous pression réduite (2,7 kPa). On obtient 3,4 g de cristaux blancs que l'on nomme « cristaux A » [et que l'on conserve pour la préparation ultérieure du deuxième énantiomère (-)-4-[(R*)-amino-(4-chlorophényl)méthyl]benzoate de méthyle)]. Les liqueurs mères sont concentrées à sec, et on obtient une meringue blanche (8,1 g) qui est dissoute dans 100 cm³ d'acétate d'éthyle. La solution obtenue est additionnée de 50 cm³ d'hydroxyde de sodium 1N, agitée, décantée. La phase organique est lavée avec 50 cm³ d'eau, puis séchée sur sulfate de magnésium, et concentrée à sec sous pression réduite (2,7 kPa). On obtient un solide jaune, que l'on dissout dans 100 cm³ de méthanol. La solution obtenue est additionnée de 1,85 g d'acide L-(+)-tartrique et la solution résultante est concentrée à sec sous pression réduite (2,7 kPa). On obtient une meringue crème qui, une fois dissoute dans 27 cm³ d'éthanol à 4% d'eau, est laissée cristalliser pendant 20 heures à 20°C. Les cristaux sont filtrés, lavés avec de l'éthanol à 4% d'eau, essorés, puis séchés sous pression réduite (2,7 kPa). On obtient 3,4 g de cristaux de L-(+)-tartrate de (+)-4-[(R*)-amino-(4-chlorophényl)méthyl]benzoate de méthyle, que l'on recristallise dans 60 cm³ d'éthanol à 5% d'eau. Après essorage, puis séchage, on obtient 2,78 g de cristaux blancs que l'on dissout dans 50 cm³ d'acétate d'éthyle. La solution obtenue est additionnée de 100 cm³ d'hydroxyde de sodium 1N, agitée, décantée. La phase organique est lavée avec 50 cm³ d'eau, puis séchée sur sulfate de magnésium, et concentrée à sec sous pression réduite (2,7 kPa). On obtient 2,1 g de (+)-4-[(R*)-amino-(4-chlorophényl)méthyl]benzoate de méthyle, sous la forme d'un solide blanc.

Le 4-[(RS)-amino-(4-chlorophényl)méthyl]benzoate de méthyle peut être préparé en opérant de la façon suivante : A une suspension de 16,3 g de 4-[(RS)-phtalimido-(4-chlorophényl)méthyl]benzoate de méthyle dans 200 cm³ de méthanol, on ajoute 3,9 cm³ d'hydrate d'hydrazine. Après 5 heures d'agitation à la température du reflux puis 20 heures à 20°C, le mélange réactionnel est filtré, le filtrat est concentré à sec sous pression réduite (2,7 kPa). Le résidu obtenu est repris par un mélange de 200 cm³ d'eau et 200 cm³ d'acétate d'éthyle. Après 15 minutes d'agitation, la suspension résultante est filtrée, le filtrat décanté en ampoule à décanter, et la phase organique est lavée par 50 cm³ d'eau, séchée sur sulfate de magnésium, et concentré à sec sous pression réduite (2,7 kPa). On obtient 8,4 g de 4-[(RS)-amino-(4-chlorophényl)méthyl]benzoate de méthyle, sous la forme d'une huile jaune pâle.

Le 4-[(RS)-phtalimido-(4-chlorophényl)méthyl]benzoate de méthyle, peut être préparé en opérant de la façon suivante : A une solution de 11,6 g de 4-[(RS)-bromo-(4-chlorophényl)méthyl]benzoate de méthyle, dans 70 cm³ de N,N-diméthylformamide, on ajoute 12,6 g de phtalimide de potassium. Après 3 heures d'agitation à la température du reflux, le mélange réactionnel est refroidi à 20°C puis additionné de 300 cm³ d'acétate d'éthyle et 300 cm³ d'eau. Après agitation, le mélange est décanté, la phase aqueuse réextraite par 2 fois 100 cm³ d'acétate d'éthyle, les phases organiques réunies sont lavées par 2 fois 400 cm³ d'eau, puis séchées sur sulfate de magnésium, et concentrées à sec sous pression réduite (2,7 kPa). On obtient 16,3 g de 4-[(RS)-phtalimido-(4-chlorophényl)méthyl]benzoate de méthyle, sous la forme d'un solide jaune pâteux.

Le 4-[(RS)-bromo-(4-chlorophényl)méthyl]benzoate de méthyle, peut être préparé en opérant de la façon suivante : A une solution de 17,4 g de 4-[(RS)-(4-chlorophényl)(hydroxy)méthyl]benzoate de méthyle dans 200 cm³ d'acétonitrile, on ajoute 10,18 g de NN'-carbonyldiimidazole, et 54,3 cm³ de bromure d'allyle. Après 30 minutes d'agitation à 20°C, le mélange réactionnel est porté au reflux pendant 2 heures, agité pendant 20 heures à 20°C et concentré presqu'à sec sous pression réduite (2,7 kPa). Le mélange, repris par du dichlorométhane, est chromatographié sur une colonne de gel de silice (granulométrie 0,06-0,200 mm, diamètre 7 cm, hauteur 30 cm), sous une pression de 0,5 bar d'argon en éluant avec du dichlorométhane, et en recueillant des fractions de 500 cm³. Les fractions 3 à 6 sont réunies puis concentrées à sec sous pression réduite (2,7 kPa). On obtient 11,6 g de 4-[(RS)-bromo-(4-chlorophényl)méthyl]benzoate de méthyle, sous la forme d'une huile qui sera utilisée telle quelle à l'étape suivante.

Le 4-[(RS)-(4-chlorophényl)(hydroxy)méthyl]benzoate de méthyle peut être préparé en opérant de la façon suivante : A une suspension de 2,75 g de 4-(4-chlorobenzoyl)benzoate de méthyle, dans 200 cm³ de méthanol à 20°C, on ajoute lentement par petites fractions (il se produit un échauffement du milieu jusqu'à 50°C), 1,21 g de borohydrure de sodium. Après 20 heures d'agitation à 20°C, le mélange réactionnel, est concentré à volume réduit puis additionné de 150 cm³ de dichlorométhane et en agitant de 100 cm³ d'acide chlorhydrique 0,5N. Après décantation, la phase organique est séchée sur sulfate de magnésium, concentrée à sec sous pression réduite (2,7 kPa). On obtient 2,5 g de 4-[(RS)-(4-chlorophényl)(hydroxy)méthyl]benzoate de méthyle, sous la forme d'une huile incolore qui cristallise lentement à 20°C, et qui sera utilisée telle quelle à l'étape suivante.

Le 4-(4-chlorobenzoyl)benzoate de méthyle, peut être préparé en opérant de la façon suivante : A une solution refroidie à -22°C de 19,3 g de chlorure de l'acide téréphtalique monométhylester dans 200 cm³ de tétrahydrofuranne, on ajoute sous argon 27,4 cm³ de tri-n-butylphosphine. Après 20 minutes d'agitation à -22°C, on coule en maintenant cette température, une solution de bromure de 4-chlorophénylmagnésien (préparée à partir de 19,15 g de bromo-4-chlorobenzène, de 2,43 g de magnésium et un cristal d'iode dans 100 cm³ d'oxyde de diéthyle au reflux). Après 30 minutes d'agitation à -22°C, on ajoute lentement 150 cm³ d'acide chlorhydrique 1N, laisse le mélange revenir à 20°C puis dilue le milieu avec 200 cm³ d'oxyde de diéthyle. La suspension blanche obtenue est filtrée, le solide est lavé par 2 fois 50 cm³ d'eau, puis par 2 fois 50 cm³ de d'oxyde de diéthyle. On obtient après essorage puis séchage sous pression réduite (2,7 kPa), 16,2 g de 4-(4-chlorobenzoyl)benzoate de méthyle, sous la forme d'un solide blanc fondant à 170°C

### Exemple 4

Le mélange des 2 diastéréoisomères formes B 1-[4-[(R*)-(4-chlorophényl)-{3-[(3,5-difluorophényl)-méthylsulfonyl-méthyl-(R)]azétidin-1-yl}méthyl] benzyl]pyrrolidine, et 1-[4-[(R*)-(4-chlorophényl)-{3-[(3,5-difluoro-phényl)-méthylsulfonyl-méthyl-(S)]azétidin-1-yl}méthyl]benzyl pyrrolidine peut être préparé en opérant comme il est décrit dans l'exemple 3, à partir de 50 mg de (+)-1-[4-(R*)-(4-chlorophényl)-{3-[(3,5-difluorophényl)méthyl-sulfonyl-méthylène]azétidin-1-yl}méthyl)benzyl] pyrrolidine, isomère forme B, de 1,5 cm³ d'éthanol, de 1,5 cm³ de dichlorométhane, et de 18 mg d'hydroborure de sodium, en agitant pendant 8 heures à 50°C puis 48 heures à 20°C. On obtient 50 mg du mélange des 2 diastéréoisomères formes B 1-[4-[(R*)-(4-chlorophényl)-{3-[(3,5-difluorophényl)-méthylsulfonyl-méthyl-(R)]azétidin-1-yl}méthyl]benzyl]pyrrolidine, et 1-[4-[(R*)-(4-chlorophényl)-{3-[(3,5-difluorophényl)-méthylsulfonyl-méthyl-(S)]azétidin-1-yl}méthyl]benzyl] pyrrolidine, sous la forme d'une meringue blanche [Spectre de R.M.N. ¹H (300 MHz, CDCl₃, δ en ppm). On observe un mélange de diastéréoisomère 60/40, * 1,79 (mt : 4H); de 2,45 à 2,60 (mt : 5H); 2,67 (s : 3H); de 3,10 à 3,30 (mt: 2H); 3,40 (mt : 1H); 3,57 et 3,60 (2s : 2H en totalité); 3,65 (t large, J = 7,5 Hz : 1H); 4,26 et 4,30 (2s : 2H en totalité); 6,84 (tt, J = 9 et 2 Hz : 1H); 6,96 (mt : 2H); de 7,25 à 7,40 (mt : 8H).

La (+)-1-[4-(R*)-(4-chlorophényl)-{3-[(3,5-difluorophényl)méthylsulfonyl-méthylène]azétidin-1-yl}méthyl)benzyl]pyrrolidine, isomère forme B peut être préparée en opérant comme il est décrit dans l'exemple 3, à partir de à partir de 0,50 g de 1-{(R*)-[4-(chlorométhyl)phényl]-(4-chlorophényl)méthyl}-3-[(3,5-difluorophényl)(méthyl-sulfonyl)méthylène]azétidine, isomère forme B, 5 mg d'iodure de sodium, 15 cm³ de dichlorométhane, et 0,190 g de pyrrolidine. Le produit brut est chromatographié sur une colonne de gel de silice (granulométrie 0,06-0,200 mm, diamètre 1,5 cm, hauteur 20 cm), sous une pression de 0,1 bar d'argon en éluant avec du dichlorométhane puis avec un mélange dichlorométhane et méthanol (95/5 en volumes) et en recueillant des fractions de 25 cm³. Les fractions 20 à 40 sont réunies puis concentrées à sec sous pression réduite (2,7 kPa). On obtient 0,28 g de (+)-1-[4-(R*)-(4-chlorophényl)-{3-[(3,5-difluorophényl)méthylsulfonyl-méthylènelazétidin-1-yl}méthyl)benzyl]pyrrolidine, isomère forme B, sous la forme d'une meringue blanche. [a]²⁰365nm = +26,8° +/- 0,8 (c = 0,5 %; dichlorométhane) [Spectre de R.M.N. ¹H (300 MHz, CDCl₃. δ en ppm) : 1,78 (mt : 4H); 2,50 (mt : 4H); 2,80 (s : 3H); 3,57 (s : 2H); 3,84 (mt : 2H); 4,34 (mt : 2H); 4,50 (s : 1H); 6,84 (tt, J = 9 et 2,5 Hz : 1H); 6,98 (mt : 2H); de 7,20 à 7,40 (mt : 8H)].

La 1-{(R*)-[4-(chlorométhyl)phényl]-(4-chlorophényl)méthyl}-3-[(3,5-difluorophényl)(méthylsulfonyl)méthylène]azétidine, isomère forme B, peut être préparée en opérant comme il est décrit dans l'exemple 3, à partir de 7,3 g du mélange des 2 diastéréoisomères formes B 1-{(R*)-[4-(chlorométhyl)phényl]-(4-chlorophényl)méthyl}-3-[(R)-(3,5-difluorophényl) (méthylsulfonyl)méthyl)]azétidin-3-ol et 1-{(R*)-[4-(chlorométhyl)phényl]-(4-chlorophényl)méthyl}-3-[(S)-(3,5-difluorophényl)(méthylsulfonyl)méthyl)]azétidin-3-ol, de 8,2 g de 4-diméthylaminopyridine, de 150 cm³ de dichlorométhane, et 3,2 cm³ de chlorure de méthylsulfonyle. Le produit brut est chromatographié sur une colonne de gel de silice (granulométrie 0,04-0,06 mm, diamètre 3 cm, hauteur 30 cm), sous une pression de 0,2 bar d'argon en éluant avec du dichlorométhane et en recueillant des fractions de 100 cm³. Les fractions 15 à 30 sont réunies puis concentrées à sec sous pression réduite (2,7 kPa). On obtient 2,50 g de 1-{(R*)-[4-(chlorométhyl)phényl]-(4-chlorophényl)méthyl}-3-[(3,5-difluorophénylméthylsulfonyl)méthylène]azétidine, isomère forme B, sous la forme d'une meringue blanche.

Le mélange des 2 diastéréoisomères formes B 1-{(R*)-[4-(chlorométhyl)phényl]-(4-chloro phényl)méthyl}-3-[(R)-(3,5-difluorophényl) (méthylsulfonyl)méthyl)]azétidin-3-ol, et 1-{(R*)-[4-(chlorométhyl)phényl]-(4-chlorophényl)méthyl}-3-[(S)-(3,5-difluorophényl)(méthylsulfonyl)méthyl)]azétidin-3-ol, peut être préparé en opérant comme il est décrit dans l'exemple 3, à partir de 11,0 g du mélange des 2 diastéréoisomères formes B 1-{(R*)-(4-chlorophényl)[4-(hydroxyméthyl)phényl]méthyl}-3-[(R)-(3,5-difluorophényl)(méthylsulfonyl)méthyl)]azétidin-3-ol et 1-{(R*)-(4-chlorophényl)[4-(hydroxyméthyl)phényl]méthyl}-3-[(S)-(3,5-difluorophényl)(méthylsulfonyl) méthyl)]azétidin-3-ol, de 250 cm³ de dichlorométhane, et 3,1 cm³ de chlorure de thionyle. Le produit brut est chromatographié sur une colonne de gel de silice (granulométrie 0,04-0,06 mm, diamètre 3 cm, hauteur 30 cm), sous une pression de 0,2 bar d'argon en éluant avec un mélange cyclohexane et acétate d'éthyle (70/30 en volumes) et en recueillant des fractions de 50 cm³. Les fractions 9 à 25 sont réunies puis concentrées à sec sous pression réduite (2,7 kPa). On obtient 7,3 g du mélange des 2 diastéréoisomères formes B 1-{(R*)-[4-(chlorométhyl)phényl]-(4-chlorophényl)méthyl}-3-[(R)-(3,5-difluorophényl)(méthylsulfonyl)méthyl)]azétidin-3-ol, et 1-{(R*)-[4-(chlorométhyl)phényl]-(4-chlorophényl)méthyl}-3-[(S)-(3,5-difluorophényl) (méthylsulfonyl)méthyl)]azétidin-3-ol, sous la forme d'une meringue blanche.

Le mélange des 2 diastéréoisomères formes B 1-{(R*)-(4-chlorophényl)[4-(hydroxyméthyl)phényl]méthyl}-3-[(R)-(3,5-difluorophényl)(méthylsulfonyl)méthyl)]azétidin-3-ol et 1-{(R*)-(4-chlorophényl)[4-(hydroxyméthyl)phényl] méthyl}-3-[(R)-(3,5-difluorophényl)(méthylsulfonyl)méthyl)]azétidin-3-ol, peut être préparé en opérant comme il est décrit dans l'exemple 3, à partir de 18,0 g du mélange des 2 diastéréoisomères formes B 3-acétoxy-1-{(R*)-(4-chlorophényl)[4-(méthoxycarbonyl)phényl]méthyl}-3-[(R)-(3,5-difluorophényl)(méthylsulfonyl)méthyl]azétidine, et 3-acétoxy-1-{(R*)-(4-chlorophényl)[4-(méthyloxycarbonyl)phényl]méthyl}-3-[(S)-(3,5-difluorophényl)(méthylsulfonyl)méthyl]azétidine, 150 cm³ de toluène anhydre, et 100 cm³ d'une solution à 20% dans le toluène d'hydrure de diisobutylaluminium. Le produit brut est chromatographié sur une colonne de gel de silice (granulométrie 0,06-0,200 mm, diamètre 3 cm, hauteur 30 cm), sous une pression de 0,1 bar d'argon en éluant avec un mélange cyclohexane et acétate d'éthyle (50/50 en volumes) et en recueillant des fractions de 50 cm³. Les fractions 15 à 30 sont réunies puis concentrées à sec sous pression réduite (2,7 kPa). On obtient 11,0 g du mélange des 2 diastéréoisomères formes B 1-{(R*)-(4-chlorophényl)[4-(hydroxyméthyl)phényl]méthyl}-3-[(R)-(3,5-difluorophényl)(méthylsulfonyl) méthyl]azétidin-3-ol, et 1-{(R*)-(4-chlorophényl)[4-(hydroxy-méthyl)phényl]méthyl}-3-[(S)-(3,5-difluorophényl)(méthylsulfonyl)méthyl]azétidin-3-ol, sous la forme d'une meringue blanche.

Le mélange des 2 diastéréoisomères formes B 3-acétoxy-1-{(R*)-(4-chlorophényl)[4-(méthoxycarbonyl)phényl]méthyl}-3-[(R)-(3,5-difluorophényl) (méthylsulfonyl)méthyl]azétidine, et 3-acétoxy-1-{(R*)-(4-chlorophényl)[4-(méthoxycarbonyl)phényl]méthyl}-3-[(R)-(3,5-difluorophényl)(méthylsulfonyl) méthyl]azétidine, peut être préparé en opérant comme il est décrit dans l'exemple 3, à partir de 11,2 g de (3,5-difluorobenzyl)méthylsulfone, 350 cm³ de tétrahydrofuranne, 34 cm³ d'une solution 1,6N de n-butyllithium dans l'hexane, de 11,2 g de 1-{(R*)-(4-chlorophényl)[4-(méthoxycarbonyl) phényl]méthyl} azétidin-3-one, isomère forme B, et 5,5 cm³ de chlorure d'acétyle. Le produit brut est chromatographié sur une colonne de gel de silice (granulométrie 0,06-0,200 mm, diamètre 4 cm, hauteur 40 cm), en éluant avec un mélange cyclohexane et acétate d'éthyle (70/30 en volumes) et en recueillant des fractions de 100 cm³. Les fractions 10 à 30 sont réunies puis concentrées à sec sous pression réduite (2,7 kPa). On obtient 21 g d'une meringue crème encore impure que l'on chromatographie sur une colonne de gel de silice (granulométrie 0,06-0,200 mm, diamètre 4 cm, hauteur 40 cm), en éluant avec du dichlorométhane et en recueillant des fractions de 100 cm³. Les fractions 11 à 30 sont réunies puis concentrées à sec sous pression réduite (2,7 kPa). On obtient 20,0 g du mélange des 2 diastéréoisomères formes B 3-acétoxy-1-{(R*)-(4-chlorophényl)[4-(méthoxycarbonyl)phényl]méthyl}-3-[(R)-(3,5-difluorophényl)(méthylsutfonyl) méthyl)]azétidine, et 3-acétoxy-{(R*)-(4-chlorophényl)[4-(méthoxycarbonyl) phényl]méthyl}-3-[(S)-(3,5-difluorophényl) (méthylsulfonyl)méthyl]azétidine, sous la forme d'une meringue blanche.

La 1-{(R*)-(4-chlorophényl)[4-(méthoxycarbonyl)phényl]méthyl}azétidin-3-one, isomère forme B, peut être préparée en opérant comme il est décrit dans l'exemple 3, à partir de 8,7 cm³ de chlorure d'oxalyle, de 350 cm³ de dichlorométhane, de 14,2 cm³ de diméthylsulfoxyde, de 29,0 g de 1-{(R*)-(4-chlorophényl)[4-(méthoxycarbonyl)phényl]méthyl}azétidin-3-ol, isomère forme B, et 43 cm³ de triéthylamine. Le produit brut est chromatographié sur une colonne de gel de silice (granulométrie 0,06-0,200 mm, diamètre 4 cm, hauteur 40 cm), en éluant avec du dichlorométhane et en recueillant des fractions de 250 cm³. Les fractions 7 à 25 sont réunies puis concentrées à sec sous pression réduite (2,7 kPa). On obtient 15,5 g de 1-{(R*)-(4-chlorophényl)[4-(méthoxycarbonyl)phényl]méthyl}azétidin-3-one, isomère forme B, sous la forme d'une huile orange.

Le 1-{(R*)-(4-chlorophényl)[4-(méthoxycarbonyl)phényl]méthyl}azétidin-3-ol, isomère forme B, peut être préparé comme il est décrit dans l'exemple 3, à partir de 25,5g de (-)-4-[1-(R*)-amino-1-(4-chlorophényl)méthyl]benzoate de méthyle, de 250 cm³ d'éthanol, 7,9 g d'hydrogénocarbonate de sodium, et 7,7 cm³ d'épibromhydrine. On obtient 29 g de 1-{(R*)-(4-chlorophényl)[4-(méthoxycarbonyl)phényl]méthyl}azétidin-3-ol, isomère forme B, sous la forme d'une huile jaune.

Le (-)-4-[(R*)-amino-(4-chlorophényl)méthyl]benzoate de méthyle, peut être préparé en effectuant deux recristallisations successives des cristaux blancs (3,4 g) nommés « cristaux A» de l'exemple 3, dans 68 cm³ d'éthanol à 5% d'eau au reflux. Les cristaux obtenus sont filtrés, essorés puis séchés sous pression réduite (2,7 kPa). On obtient 2,2 g de D-(-)-tartrate de (-)-4-[(R*)-amino-(4-chlorophényl)méthyl]benzoate de méthyle, sous la forme de cristaux blancs que l'on dissout dans 50 cm³ d'acétate d'éthyle. La solution obtenue est additionnée de 50 cm³ d'hydroxyde de sodium 1N, agitée, puis décantée. La phase organique est lavée avec 50 cm³ d'eau, puis séchée sur sulfate de magnésium, et concentrée à sec sous pression réduite (2,7 kPa). On obtient 1,9 g de (-)-4-[(R*)-amino-(4-chlorophényl)méthyl]benzoate de méthyle, sous la forme d'un solide blanc. [a]20°C, 365 nm =-58,1° +/- 1 (c = 0,5%)

### Exemple 5

La 1-[bis-(thièn-2-yl)-méthyl]-3-[(RS)-(3,5-difluorophényl)méthylsulfonylméthyl]azétidine peut être préparée en opérant comme il est décrit dans l'exemple 3, à partir de 0,10 g de 1-[bis-(thièn-2-yl)-méthyl]-3-[(3,5-difluorophényl)méthylsulfonyl-méthylène]azétidine, de 2 cm³ de méthanol, de 2 cm³ de dichlorométhane, et de 25 mg d'hydroborure de sodium, en agitant pendant 3 heures à 20°C. Le produit brut est chromatographié sur une colonne de gel de silice (granulométrie 0,063-0,200 mm, hauteur 7 cm, diamètre 1 cm), en éluant sous une pression de 0,1 bar d'argon avec du dichlorométhane et en recueillant des fractions de 4 cm³. Les fractions 2 à 5 sont réunies et concentrées à sec sous pression réduite (2,7 kPa). On obtient 83 mg de 1-[bis-(thièn-2-yl)-méthyl]-3-[(RS)-(3,5-difluorophényl)méthyl sulfonyl-méthyl]azétidine, sous la forme d'un solide blanc [Spectre de R.M.N ¹H (400 MHz, CDCl₃, δ en ppm) : de 2,60 à 2,70 (mt : 1H); 2,66 (s : 3H); 3,31 (mt : 2H); 3,40 (mt : 1H); 3,73 (t large, J = 7,5 Hz : 1H); 4,27 (d, J = 11 Hz : 1H); 4,92 (s : 1H); 6,83 (tt, J = 9 et 2,5 Hz : 1H); de 6,85 à 7,00 (mt : 6H); 7,21 (mt : 2H)].

La 1-[bis-(thièn-2-yl)-méthyl]-3-[(3,5-difluorophényl)méthylsulfonyl-méthylène]azétidine peut être préparée en opérant selon le mode opératoire décrit dans l'exemple 6, à partir de 2,2 g de 1-[bis-(thièn-2-yl)-méthyl]-3-[(3,5-difluorophényl)méthylsulfonyl-méthyl-(RS)]azétidin-3-ol, 0,64 cm³ de chlorure de méthylsulfonyle, 2,3 g de 4-diméthylaminopyridine et 75 cm³ de dichlorométhane, on obtient, après purification par chromatographie et cristallisation dans de l'oxyde de diisopropyle, 1,3 g de 1-[bis-(thièn-2-yl)-méthyl]-3-[(3,5-difluorophényl)méthylsulfonyl-méthylène]azétidine, sous la forme de cristaux blancs fondant à 165°C.

Le 1-[bis-(thièn-2-yl)-méthyl]-3-[(3,5-difluorophényl)méthylsulfonyl-méthyl-(RS)]azétidin-3-ol peut-être préparé en opérant de la façon suivante : A une solution refroidie à -60°C de 1,3 g de (3,5-difluorobenzyl)méthylsulfone dans 20 cm³ de tétrahydrofuranne, on ajoute sous argon en 10 minutes 4 cm³ de n-butyl lithium à 1,6 N dans l'hexane. Après 45 minutes d'agitation à -70°C, on coule en 10 minutes une solution de 1,5 g de 1-[bis-(thiè-2-yl)-méthyl]azétidin-3-one dans 20 cm³ de tétrahydrofuranne. Après 3 heures d'agitation à -70°C, le mélange réactionnel est laissé revenir à température ordinaire, puis est additionné de 10 cm³ d'une solution aqueuse saturée de chlorure d'ammonium. Le mélange est décanté, la phase organique est séchée sur sulfate de magnésium, filtrée puis concentrée à sec sous pression réduite (2,7 kPa). Le résidu est repris avec 20 cm³ d'un mélange de cyclohexane et d'acétate d'éthyle (60/40 en volumes), la suspension obtenue est filtrée, le solide est essoré, puis séché à l'air. On obtient, 2,2 g de 1-[bis-(thièn-2-yl)-méthyl]-3-[(3,5-difluorophényl)méthylsulfonyl-méthyl-(RS)]azétidin-3-ol, sous la forme de cristaux blancs fondant à 145°C.

La 1-[bis-(thièn-2-yl)-méthyl]azétidin-3-one peut-être préparée en opérant comme il est décrit dans l'exemple 1 (méthode 2) à partir de 4 g de 1-[bis-(thièn-2-yl)-méthyl]azétidin-3-ol, 2,6 cm³ de diméthylsulfoxyde, 7,7 cm³ de triéthylamine, 7,7 cm³ de chlorure d'oxalyle, et 100 cm³ de dichlorométhane. Le résidu obtenu est purifié par chromatographie sur colonne de gel de silice (granulométrie 0,04-0,06mm, diamètre 3 cm, hauteur 30 cm) avec comme éluant un mélange cyclohexane et acétate d'éthyle (1/1 en volumes). Les fractions obtenues sont évaporées à sec sous pression réduite (2,7 Kpa). On obtient 3,2 g de 1-[bis-(thièn-2-yl)-méthyl]azétidin-3-one, sous la forme de cristaux crèmes fondant à 70°C.

Le 1-[bis-(thlèn-2-yl)-méthyl]azétidin-3-ol peut-être préparé en opérant comme il est décrit dans l'exemple 3, à partir de 6 g de 1-[bis-(thièn-2-yl)-méthyl]amine, 2,5 cm³ d'épibromhydrine, 2,6g de bicarbonate de sodium et 50 cm³ d'éthanol. On obtient 4 g de 1-[bis-(thièn-2-yl)-méthyl]azétidin-3-ol, sous la forme de cristaux beiges fondant à 115°C.

La 1-[bis-(thièn-2-yl)-méthyl]amine peut être préparée de la façon suivante : à une suspension refroidie sous argon à 10°C de bromure de thièn-2-yl-magnésien (préparée à partir de 1,29 g de magnésium et 3,22 cm³ 2-bromothiophène dans 75 cm³ d'oxyde de diéthyle), on coule goutte à goutte une solution de 5 cm³ de thiophen-2-yl-carbonitrile dans 50 cm³ d'oxyde de diéthyle. Après 1 heure et 30 minutes de reflux, le milieu réactionnel est refroidi à 5°C puis on coule goutte à goutte 20 cm³ de méthanol, filtre la suspension, lave le solide avec du méthanol. Le filtrat obtenu une solution marron . Sous argon, on ajoute à cette solution 2,45 g de borohydrure de sodium, en plusieurs fois. Le mélange est agité à température ambiante pendant 16 heures, puis est dilué par de l'éthyle acétate et additionné d'eau lentement. la phase organique est extraite, lavée avec de l'eau, séchée sur sulfate de magnésium et évaporée à sec sous pression réduite (2,7 kpa) à 55°C. On obtient une huile marron qui est chromatographiée sur colonne de gel de silice(granulométrie 0,2-0,063 mm, diamètre 8 cm, hauteur 25 cm) et éluée par un mélange cyclohexane et acétate d'éthyle (90/10 puis 85/15 en volumes). Les fractions 21 à 30 sont réunies et évaporées à sec sous pression réduite (2,7 kpa) . On obtient 11g de 1-[bis-(thièn-2-yl)-méthyl]amine, sous la forme d'un solide cristallisé.

### Exemple 6

La 1-[bis-(p-tolyl)méthyl]-3-[(RS)-méthylsulfonyl-phényl-méthyl]azétidine peut être préparée en opérant comme il est décrit dans l'exemple 3, à partir de 0,10 g de 1-[bis-(p-tolyl)méthyl]-3-(méthylsulfonyl-phényl-méthylène) azétidine, de 2 cm³ de méthanol, de 2 cm³ de dichlorométhane, et de 25 mg d'hydroborure de sodium. Le produit brut est chromatographié sur une colonne de gel de silice (granulométrie 0,063-0,200 mm, hauteur 7 cm, diamètre 1 cm), en éluant sous une pression de 0,1 bar d'argon avec du dichlorométhane et en recueillant des fractions de 4 cm³. Les fractions 5 à 10 sont réunies et concentrées à sec sous pression réduite (2,7 kPa). On obtient 35 mg de 1-[bis-(p-tolyl)méthyl]-3-[(RS)-méthylsulfonyl-phényl-méthyl] azétidine, sous la forme d'un solide blanc [Spectre de R.M.N ¹H (300 MHz, CDCl₃, δ en ppm) : 2,24 (s : 3H); 2,27 (s : 3H); 2,53 (t, J = 7,5 Hz : 1H); 2,58 (s : 3H); 3,19 (mt : 2H); 3,49 (mt : 1 H); 3,69 (t large, J = 7,5 Hz : 1H); 4,22 (s : 1H); 4,28 (d, J = 11,5 Hz : 1H); de 6,95 à 7,45 (mts : 13H)].

La 1-[bis-(p-tolyl)méthyl]-3-(méthylsulfonyl-phényl-méthylène)azétidine peut être préparée en opérant de la façon suivante : A une solution de 0,48 g de 1-[bis-(p-tolyl)méthyl]-3-[méthylsulfonyl-phényl-méthyl-(RS)]azétidin-3-ol dans 25 cm³ de dichlorométhane anhydre, on ajoute 0,125 cm³ de chlorure de méthylsulfonyle, puis par petites fractions 0,465 g de 4-diméthylaminopyridine. Après 20 heures d'agitation à 20°C, le mélange réactionnel est lavé par 2 fois 80 cm³ d'eau, par 80 cm³ de saumure, séché sur sulfate de magnésium, puis concentré à sec sous pression réduite (2,7 kPa). Le résidu est chromatographié sur une colonne de gel de silice (granulométrie 0,040-0,063 mm, hauteur 17 cm, diamètre 3,2 cm), en éluant sous une pression de 0,5 bar d'argon avec un mélange de cyclohexane et d'acétate d'éthyle (80/20 en volumes) et en recueillant des fractions de 40 cm³. Les fractions 5 à 8 sont réunies et concentrées à sec sous pression réduite (2,7 kPa). Le résidu est agité avec de l'oxyde de diisopropyle, le solide est filtré, essoré puis séché sous pression réduite (2,7 kPa). On obtient 0,25 g de 1-[bis-(p-tolyl)méthyl]-3-(méthylsulfonyl-phényl-méthylène) azétidine, sous la forme d'un solide blanc [Spectre RMN dans DMSO-d6, T=300K, δ en ppm (250 Mhz) : 2,23 (6H, s, 2 Ph-CH₃), 2,98 (3H, s, SCH₃), 3,76 (2H, s, NCH₂), 4,20 (2H, s, NCH₂), 5,55 (1H, s, NCH), 7,10 (4H, d, J=7Hz, 4 CH arom.), 7,32 (4H, d, J=7Hz, 4 CH arom.), 7,43 (5H, s, Phényle)].

Le 1-[bis-(p-tolyl)méthyl]-3-[(méthylsulfonyl)-phényl-méthyl-(RS)]azétidin-3-ol peut-être préparé en opérant de la façon suivante : A une solution de 0,59 g de bromo(bis-p-tolyl)méthane, dans 20 cm³ d'acétonitrile, on ajoute 0,6 g de chlorhydrate de 3-[(méthylsulfonyl)(phényl)méthyl-(RS)]azétidin-3-ol puis 0,3 g de carbonate de potassium. Après 1 heure et 15 minutes de chauffage à la température du reflux, le mélange réactionnel est refroidi à 20°C et filtré. Le filtrat est concentré à sec sous pression réduite (2,7 kPa) et le résidu est chromatographié sur colonne de gel de silice (granulométrie 0,04-0,06mm, diamètre 4 cm, hauteur 16 cm) en éluant avec un mélange de cyclohexane et d'acétate d'éthyle (70/3O en volumes) et en recueillant des fractions de 50 cm³. Les fractions 8 à 13 sont concentrées à sec sous pression réduite (2,7 Kpa). On obtient 0,48 g de 1-[bis-(p-tolyl)méthyl]-3-[(méthylsulfonyl) (phényl)méthyl-(RS)]azétidin-3-ol sous la forme d'un solide blanc.

Le bromo(bis-p-tolyl)méthane peut-être préparé selon le mode opératoire décrit par BACHMANN W.E., J.Am.Chem.Soc., 2135, (1933).

Le chlorhydrate de 3-[(méthylsulfonyl)phényl-méthyl-(RS)]azétidin-3-ol peut-être préparé en opérant de la façon suivante : A une solution de 2,62 g de 3-[(méthylsulfonyl)(phényl)méthyl-(RS)]-1-(vinyloxycarbonyl)azétidin-3-ol dans 12,6 cm³ de dioxane, on ajoute 12,6 cm³ d'une solution 6,2N d'acide chlorhydrique dans le dioxane. Après 20 heures d'agitation à 20°C, le mélange est concentré à sec à 50°C sous pression réduite (2,7 kPa). Le résidu est repris par 25 cm³ d'éthanol, porté au reflux pendant 1 heure puis laissé revenir à 20°C et filtré. Le solide est rincé avec de l'oxyde de diéthyle, puis essoré et séché sous pression réduite (2,7 kPa). On obtient 1,89 g de chlorhydrate de 3-[(méthylsulfonyl)phényl-méthyl-(RS)]azétidin-3-ol, sous la forme de cristaux blancs.

Le 3-[(méthylsulfonyl)(phényl)méthyl-(RS)]-1-(vinyloxycarbonyl)azétidin-3-ol peut être préparé en opérant de la façon suivante : A un mélange refroidi à +5°C de 3,92 g de 1-benzhydryl-3-[(méthylsulfonyl)(phényl)méthyl-(RS)]azétidin-3-ol dans 500 cm³ de dichlorométhane anhydre, on coule goutte à goutte une solution de 0,99 cm³ de chloroformiate de vinyle dans 4 cm³ de dichlorométhane anhydre. Après 48 heures d'agitation à 20°C, le mélange réactionnel est concentré à sec sous pression réduite (2,7 kPa). Le résidu est chromatographié sur colonne de gel de silice (granulométrie 0,04-0,06mm, diamètre 5,6 cm, hauteur 15,5 cm) en éluant avec un mélange de cyclohexane et d'acétate d'éthyle (70/30 en volumes) et en recueillant des fractions de 50 cm³. Les fraction 17 à 36 sont concentrées à sec sous pression réduite (2,7 Kpa). On obtient 0,9 g de 3-[(méthylsulfonyl)(phényl)méthyl-(RS)]-1-(vinyloxycarbonyl)azétidin-3-ol, sous la forme d'un solide blanc.

Le 1-benzhydryl-3-[(méthylsulfonyl)(phényl)méthyl-(RS)]azétidin-3-ol peut être préparé en opérant comme il est décrit dans l'exemple 1 (méthode 1), à partir de 47 cm³ de diisopropylamine, de 205,6 cm³ de solution de n-butyllithium 1,6M dans l'hexane, de 2,2 litres de tétrahydrofuranne, de 50 g de benzyl-méthylsulfone et de 69,6 g de 1-benzhydryl-azétidin-3-one. On obtient 94,3 g de 1-benzhydryl-3-[(méthylsulfonyl)(phényl)méthyl-(RS)]azétidin-3-ol, sous la forme de cristaux blancs.

### Exemple 7

La 1-[bis-(3-fluorophényl)méthyl]-3-(RS)-[(3,5-difluorophényl)méthylsulfonylméthyl]azétidine peut être préparée en opérant comme il est décrit dans l'exemple 3, à partir de 0,10 g de 1-[bis-(3-fluorophényl)méthyl]-3-[(3,5-difluorophényl)méthylsulfonyl-méthylène]azétidine, de 2 cm³ de méthanol, de 2 cm³ de dichlorométhane, et de 20 mg d'hydroborure de sodium, en agitant pendant 48 heures à 20°C. Le produit brut est chromatographié sur une colonne de gel de silice (granulométrie 0,063-0,200 mm, hauteur 7 cm, diamètre 1 cm), en éluant sous une pression de 0,1 bar d'argon avec du dichlorométhane et en recueillant des fractions de 4 cm³. Les fractions 3 à 7 sont réunies et concentrées à sec sous pression réduite (2,7 kPa). On obtient 95 mg de 1-[bis-(3-fluorophényl)méthyl]-3-(RS)-[(3,5-difluorophényl) méthylsulfonyl-méthyl]azétidine, sous la forme de cristaux blancs [Spectre de R.M.N ¹H (300 MHz, CDCl₃, δ en ppm) : 2,57 (t, J = 7,5 Hz : 1H); 2,66 (s : 3H); de 3,15 à 3,30 (mt : 2H); de 3,30 à 3,50 (mt : 1H); 3,66 (t large, J = 7,5 Hz : 1H); 4,27 (d, J = 11,5 Hz : 1H); 4,28 (s : 1H); de 6,75 à 7,35 (mt : 11H)].

La 1-[bis-(3-fluorophényl)méthyl]-3-[(3,5-difluorophényl)méthylsulfonylméthylène]azétidine peut être préparée en opérant comme il est décrit dans l'exemple 6, à partir de 1,15 g de 1-[bis(3-fluorophényl)méthyl]-3-[(3,5-difluorophényl)(méthylsuffonyl)méthyl-(RS)]azétidin-3-ol, 30 cm³ de dichlorométhane, de 0,264 cm³ de chlorure de méthylsulfonyle, et de 0,98 g de 4-diméthylaminopyridine. On obtient après chromatographie sur colonne de gel de silice (granulométrie 0,06-0,200 mm, diamètre 2,8 cm, hauteur 25 cm), sous une pression de 1 bar d'argon avec un mélange d'acétate d'éthyle et cyclohexane (15/85 en volumes) comme éluant et en recueillant des fractions de 60 cm³, 0,55 g de 1-[bis(3-fluorophényl)méthyl]-3-[(3,5-difluorophényl)(méthylsulfonyl)méthylène] azétidine sous la forme d'un solide blanc fondant à 178°C.

Le 1-[bis(3-fluorophényl)méthyl]-3-[(3,5-difluorophényl)(méthylsulfonyl) méthyl-(RS)]azétidin-3-ol peut être préparé en opérant de la façon suivante : A un mélange refroidi à -60°C de diisopropylamine et de 10 cm³ de tétrahydrofuranne, on coule en 10 minutes 3,65 cm³ d'une solution 1,6M de n-butyllithium dans l'hexane, agite pendant 10 minutes à -30°C puis refroidit à - 70°C. On ajoute ensuite en 20 minutes une solution de 1,2 g de (3,5-difluorobenzyl)méthylsulfone dans 30 cm³ de tétrahydrofuranne. Après 30 minutes d'agitation à -70°C, le mélange est additionné en 30 minutes d'une solution de 1,5 g de 1-[bis(3-fluorophényl)méthyl]azétidin-3-one dans 10 cm³ de tétrahydrofuranne. Après 2 heures d'agitation à -70°C, le mélange réactionnel est ramené à température ordinaire, puis additionné de 20 cm³ d'une solution aqueuse saturée de chlorure d'ammonium et de 100 cm³ de dichlorométhane. Le mélange est décanté, la phase organique est lavée avec de l'eau, puis séchée sur sulfate de magnésium, filtrée, et concentrée à sec sous pression réduite (2,7 kPa). Le résidu est chromatographié sur une colonne de gel de silice (granulométrie 0,06-0,200 mm, diamètre 3,2 cm, hauteur 30 cm), en éluant sous une pression de 1 bar d'argon avec un mélange d'acétate d'éthyle et cyclohexane (20/80 en volumes) et en recueillant des fractions de 60 cm³. Les fractions 9 à 20 sont réunies et concentrées à sec sous pression réduite (2,7 kPa). On obtient 1,95 g de 1-[bis(3-fluorophényl)méthyl]-3-[(3,5-difluorophényl)(méthylsulfonyl)méthyl(RS)]azétidin-3-ol, sous la forme d'un solide blanc, fondant à 170°C (décomposition).

La 1-[bis(3-fluorophényl)méthyl]azétidin-3-one peut être préparée en opérant comme il est décrit dans l'exemple 1 (méthode 2), à partir de 0,7 cm³ de chlorure d'oxalyle, 16 cm³ de dichlorométhane, 1,12 cm³ de diméthylsulfoxyde, 2 g de 1-[bis(3-fluorophényl)méthyl]azétidin-3-ol, et de 3,7 cm³ de triéthylamine. On obtient 1,55 g de 1-[bis(3-fluorophényl)méthyl]azétidin-3-one, sous la forme d'une huile qui cristallise à 20°C.

Le 1-[bis(3-fluorophényl)méthyl]azétidin-3-ol peut être préparé en opérant comme il est décrit par KATRITSKY A. R. dans J. Heterocycl. Chem, 31, 271 (1994), à partir de 4,9 g de [bis(3-fluorophényl)méthyl]amine et 1,78 cm³ d'épichlorhydrine.

La [bis(3-fluorophényl)méthyl]amine peut être préparée de la façon suivante : à une suspension de 1,27 g d'hydrure de lithium et d'aluminium dans 80 cm³ de tétrahydrofuranne, on coule sous atmosphère d'argon en 30 minutes une solution de 5,17 g de 3,3'-difluorobenzophénone oxime dans 30 cm³ de tétrahydrofuranne. Après 5 heures d'agitation au reflux, on ajoute successivement 1,3 cm³ d'eau, 1,3 cm³ de soude 4N, 2,6 cm³ d'eau puis 50 cm³ d'acétate d'éthyle. Après séchage sur sulfate de magnésium et concentration à sec sous pression réduite (2,7 kPa), on obtient 4,9 g de [bis(3-fluorophényl)méthyl]amine, sous la forme d'une huile jaune.

La 3-3'-difluorobenzophénone oxime peut être préparée selon le mode opératoire suivant : dans une solution de 5,0 g de 3,3'-difluorobenzophénone dans 10 cm³ d'éthanol, on coule goutte à goutte une solution de 1,6 g de chlorhydrate d'hydroxylamine dans 8 cm³ d'eau, puis ajoute par petites fractions 1,2 g de soude en pastilles. Le mélange réactionnel, porté au reflux pendant 10 minutes est refroidi à 20°C puis acidifié par 7,5 cm³ d'acide chlorhydrique 4N. Le précipité huileux obtenu une fois trituré devient un solide blanc que l'on filtre, lave par de l'eau puis sèche à 35°C sous pression réduite (2,7 kPa). On obtient 5,17 g de 3,3'-difluorobenzophénone oxime, sous la forme d'un solide blanc.

### Exemple 8

La 1-[bis-(4-chlorophényl)méthyl]-3-(RS)-{[3-azétidin-1-yl-phényl]méthylsulfonyl-méthyl}azétidine peut être préparée en opérant comme il est décrit dans l'exemple 3, à partir de 0,10 g de 1-[bis-(4-chlorophényl)méthyl]-3-[(azétidin-1-yl-phényl)-méthylsulfonyl-méthylène]azétidine, de 2 cm³ de méthanol, de 2 cm³ de dichlorométhane, et de 30 mg d'hydroborure de sodium, en agitant pendant 24 heures à 20°C. Le produit brut est chromatographié sur une colonne de gel de silice (granulométrie 0,063-0,200 mm, hauteur 7 cm, diamètre 1 cm), en éluant sous une pression de 0,1 bar d'argon avec du dichlorométhane et en recueillant des fractions de 4 cm³. Les fractions 5 à 10 sont réunies et concentrées à sec sous pression réduite (2,7 kPa). On obtient 20 mg de 1-[bis-(4-chlorophényl)méthyl]-3-(RS)-{[3-azétidin-1-yl-phényl]méthylsulfonyl-méthyl}azétidine, sous la forme d'une laque blanc cassé [Spectre de R.M.N ¹H (300 MHz, CDCl₃, δ en ppm) : 2,39 (mt : 2H); de 2,50 à 2,65 (mt : 1H); 2,60 (s : 3H); 3,20 (mt : 2H); 3,47 (mt : 1H); 3,66 (t large, J = 7 Hz : 1H); 3,89 (t large, J = 7,5 Hz : 4H); 4,20 (d, J = 11 Hz : 1H); 4,26 (s : 1H); de 6,35 à 6,50 (mt : 2H); 6,67 (d large, J = 7,5 Hz : 1H); de 7,10 à 7,40 (mt : 9H)].

La 1-[bis-(4-chlorophényl)méthyl]-3-[(azétidin-1-yl-phényl)-méthylsulfonylméthylène]azétidine peut être préparée en opérant en opérant comme il est décrit dans l'exemple 6, à partir de 0,83 g de 1-[bis-(4-chlorophényl)méthyl]-3-[(azétidin-1-yl-phényl)-méthylsulfonyl-méthyl-(RS)]azétidine-3-ol, de 20 cm³ de dichlorométhane, de 0,18 cm³ de chlorure de méthylsulfonyle, et de 0,75 g de 4-diméthylaminopyridine. On obtient 0,40 g de 1-[bis-(4-chlorophényl) méthyl]-3-[(azétidin-1-yl-phényl)-méthylsulfonyl-méthylène]azétidine, sous la forme d'une meringue blanche.

Le 1-[bis-(4-chlorophényl)méthyl]-3-[(azétidin-1-yl-phényl)-méthylsulfonylméthyl-(RS)]azétidine-3-ol peut être préparé en opérant comme il est décrit dans l'exemple 5, à partir de 1,55 g de 1-(3-méthylsulfonylméthylphényl)azétidine, de 5,2 cm³ de solution 1,6M de n-butyllithium dans l'hexane, de 30 cm³ de tétrahydrofuranne et de 2,11 g de 1-[bis-(4-chlorophényl)méthyl]azétidin-3-one. On obtient 0,83 g de 1-[bis-(4-chlorophényl)méthyl]-3-[(azétidin-1-yl-phényl)-méthylsulfonyl-méthyl-(RS)]azétidine-3-ol, sous la forme d'un solide ocre, fondant à 172°C.

La 1-(3-méthylsulfonylméthyl-phényl)azétidine peut être préparée en opérant de la façon suivante : A un mélange refroidi à 0°C de 10 cm³ d'eau, de 5 cm³ d'acide acétique, 1,5 cm³ d'acide sulfurique 36N, et de 6,15 g d'oxone, on ajoute une solution de 1,9 g de 1-(3-méthylsulfanylméthyl-phényl)azétidine dans 10 cm³ d'éthanol. Après 20 heures d'agitation à 20°C, le mélange réactionnel est additionné de 100 cm³ d'eau, 100 cm³ d'acétate d'éthyle, puis est neutralisé en agitant avec de l'hydrogénocarbonate de sodium. Le mélange obtenu est décanté, la phase organique est séchée sur sulfate de magnésium, puis filtrée et concentrée à sec sous pression réduite (2,7 kPa). On obtient 1,55 g de 1-[3-(méthylsulfonylméthyl)-phényl]azétidine, sous la forme d'une gomme brun clair.

La 1-[3-(méthylsulfonylméthyl)-phényl]azétidine peut être préparée en opérant de la façon suivante : A une solution de 4,6 g de 1-iodo-3-(méthylsulfanylméthyl)-benzène, dans 60 cm³ de tétrahydrofuranne, on ajoute sous argon 2,57 g de tertbutylate de potassium, 0,64 g de chlorure de 1,1'-bis-(diphénylphosphino)ferrocènyl palladium, 1,49 g de 1,1'-bis-(diphénylphosphino)ferrocène, 0,12 g d'iodure de cuivre, et 2,0 g d'azétidine.

Après 3 heures de chauffage à la température du reflux, le mélange réactionnel est refroidi à température ordinaire, et filtré sur célite, en lavant celle ci ensuite avec 150 cm³ d'acétate d'éthyle. Le filtrat et le lavage réunis sont acidifiés avec 120 cm³ d'acide chlorhydrique 1 N, puis décantés. La phase aqueuse est additionnée de 60 cm³ d'acétate d'éthyle, puis est alcalinisée avec de l'hydrogénocarbonate de sodium et le mélange est décanté. La phase organique est séchée sur sulfate de magnésium, filtrée, et concentrée à sec sous pression réduite (2,7 kPa). Le résidu est est chromatographié sur une colonne de gel de silice (granulométrie 0,06-0,200 mm, diamètre 3,5 cm, hauteur 50 cm), en éluant sous une pression de 0,5 bar d'argon avec un mélange d'acétate d'éthyle et cyclohexane (20/80 en volumes) et en recueillant des fractions de 100 cm³. Les fractions 1 à 3 sont réunies et concentrées à sec sous pression réduite (2,7 kPa). On obtient 1,9 g de 1-[3-(méthylsulfanylméthyl)-phényl]azétidine, sous la forme d'une huile.

Le chlorure de 1,1'-bis-(diphénylphosphino)ferrocènyl palladium peut être préparé en opérant selon Hayashi T. et coll., dans J. Am. Chem. Soc, 106, 158(1984).

Le 1-iodo-3-(méthylsulfanylméthyl)-benzène peut être préparé en opérant de la façon suivante : A une solution de 25 g de bromure de 3-iodobenzyle dans 80 cm³ de N,N-diméthylformamide, on ajoute 6,4 g de méthylthiolate de sodium. Après 20 heures d'agitation à 20°C, le mélange réactionnel est additionné de 250 cm³ d'eau, de 200 cm³ d'acétate d'éthyle et est agité puis décanté. La phase organique est lavée par 4 fois 200 cm³ d'eau, séchée sur sulfate de magnésium, filtrée, puis concentrée à sec à 50°C sous pression réduite (2,7 kPa). On obtient 22 g de 1-iodo-3-(méthylsulfanylméthyl)-benzène, sous la forme d'une gomme.

### Exemple 9

Le mélange des 2 diastéréoisomères formes A 1-(R*)-{4-[(4-chlorophényl)-{3-[(3,5-difluorophényl)-méthylsulfonyl-méthyl-(R)]-3-imidazolyl-azétidin-1-yl}méthyl]benzyl}-1H-imidazole et 1-(R*)-{4-[(4-chlorophényl)-{3-[(3,5-difluorophényl)-méthylsulfonyl-méthyl-(S)}-3-imidazolyl-azétidin-1-yl}méthyl] benzyl]-1H-imidazole, peut être préparé en opérant de la façon suivante : A une solution de 50 mg de 1-{(R*)-[4-(chlorométhyl)phényl]-(4-chlorophényl) méthyl}-3-[(3,5-difluorophényl)méthylsulfonyl-méthylène]azétidine, isomère forme A, dans 1 cm³ de dichlorométhane, on ajoute 13,6 mg d'imidazole. Après 20 heures d'agitation à 20°C, le mélange réactionnel est directement chromatographié sur une colonne de gel de silice (granulométrie 0,063-0,200 mm, hauteur 7 cm, diamètre 1 cm), en éluant sous une pression de 0,1 bar d'argon avec un mélange de dichlorométhane et de méthanol (98/2 en volumes) et en recueillant des fractions de 4 cm³. Les fractions 4 à 9 sont réunies et concentrées à sec sous pression réduite (2,7 kPa). On obtint 20 mg du mélange des 2 diastéréoisomères formes A 1-(R*)-{4-[(4-chlorophény)-{3-[(3,5-difluorophényl)-méthylsulfonyl-méthyl-(R)]-3-imidazolylazétidin-1-yl}méthyl]benzyl}-1H-imidazole, et 1-(R*)-{4-[(4-chlorophényl)-{3-[(3,5-difluorophényl)-méthylsulfonyl-méthyl-(S)]-3-imidazolyl-azétidin-1-yl}méthyl]benzyl}-1H-imidazole sous la forme d'une laque blanche [Spectre de R.M.N ¹H (300 MHz, CDCl₃, δ en ppm) : on observe un mélange de diastéréoisomères, * 2,64 (s : 3H); 3,42 (d, J = 8 Hz : 1H); 3,50 (d, J = 8 Hz : 1H); 3,75 (mt : 1H); 4,31 (d large, J = 8 Hz : 1H); 4,40 (s : 1H); 4,54 et 4,55 (2s : 2H en totalité); 4,72 (s : 1H); 6,84 (mt : 2H); 6,87 (s : 1H); 6,95 (mt : 1H); 7,11 (s : 1H); de 7,20 à 7,40 (mt : 12H)].

### Exemple 10

Le (1-benzhydryl-azétidin-3-yl)-phényl-méthanone O-méthyl oxime, mélange des 2 isomères Z et E, peut être préparé en opérant de la façon suivante : A une suspension de 0,80 g de 1-benzhydryl 3-benzoyl azétidine dans 30 cm³ d'éthanol, on ajoute 0,286 g de chlorhydrate de O-méthylhydroxylamine, et 0,32 g d'acétate de sodium. Après 24 heures d'agitation à la température du reflux, le mélange réactionnel est laissé refroidir à température ordinaire, et filtré. Le filtrat est concentré à sec à 50°C sous pression réduite (2,7 kPa). Le résidu est repris par 100 cm³ de dichlorométhane, et la solution est additionnée de 20 cm³ d'eau et d'acide chlorhydrique 1 N en agitant jusqu'à pH acide. La phase organique est décantée, séchée sur sulfate de magnésium, puis filtrée et concentrée à sec sous pression réduite (2,7 kPa). Le résidu est chromatographié sur une colonne de gel de silice (granulométrie 0,063-0,200 mm, hauteur 36 cm, diamètre 3,8 cm), en éluant sous une pression de 0,5 bar d'argon avec un mélange de cyclohexane et d'acétate d'éthyle (95/5, 92/8 puis 80/20 en volumes) et en recueillant des fractions de 30 cm³. Les fractions 8 à 14 sont réunies et concentrées à sec sous pression réduite (2,7 kPa). On obtient 0,20 g de (1-benzhydryl-azétidin-3-yl)-phényl-méthanone O-méthyl oxime, mélange des 2 isomères Z et E, sous la forme d'un solide blanc [Spectre de R.M.N. ¹H (250 MHz, (CD₃)₂SO d6, d en ppm). On observe un mélange d' isomère 65/35, * 2,68-3,02 et de 3,25 à 3,90 (mts : 5H en totalité); 3,76 et 3,80 (2s : 3H en totalité); 4,26 et 4,38 (2s : 1H en totalité); de 7,10 à 7,50 (mt : 15H)].

La 1-benzhydryl 3-benzoyl azétidine peut être préparée en opérant de la façon suivante: A une solution refroidie à 0°C de 11,5 g d'acide (1-benzhydryl-azétidin-3-yl)carboxylique-N-méthoxy-N-méthylamide dans 350 cm³ de tétrahydrofuranne, on coule goutte à goutte sous argon 112 cm³ d'une solution 1 M de bromure de phénylmagnésien dans le tétrahydrofuranne, puis laisse revenir le mélange à température ordinaire. Après 20 heures d'agitation à 20°C, le mélange réactionnel est additionné de 400 cm³ d'une solution aqueuse saturée de chlorure d'ammonium, puis de 250 cm³ d'acétate d'éthyle. Après agitation, le mélange est décanté, la phase aqueuse réextraite avec 250 cm³ d'acétate d'éthyle, et les deux phases organiques réunies sont lavées par 2 fois 250 cm³ d'eau, séchées sur sulfate de magnésium, puis filtrées et concentrées à sec à 50°C sous pression réduite (2,7 kPa). Le résidu est agité avec 50 cm³ d'oxyde de diisopropyle, la suspension est filtrée, le solide essoré, puis séché sous pression réduite (2,7 kPa). On obtient 9,76 g de 1-benzhydryl 3-benzoyl azétidine, sous la forme d'un solide crème.

L'acide (1-benzhydryl-azétidin-3-yl)carboxylique-N-méthoxy-N-méthylamide peut être préparé en opérant de la façon suivante : A une suspension de 4,0 g de chlorhydrate de N,O-diméthylhydroxylamine dans 250 cm³ de dichlorométhane, on ajoute en agitant 13,35 g d'acide (1-benzhydryl-azétidin-3-yl)carboxylique, et 1,0 g d'hydrate d'hydroxybenzotriazole. A ce mélange mis sous argon et refroidi à +5°C, on ajoute 6,92 g de chlorhydrate de 1-(3-diméthylaminopropyl) 3-éthylcarbodiimide et 8,8 cm³ de N,N-diisopropyléthylamine. Après 3 heures d'agitation à +5°C puis 20 heures à 20°C, le mélange réactionnel est additionné de 200 cm³ d'eau, puis décanté.

La phase organique est lavée par 300 cm³ d'eau, séchée sur sulfate de magnésium, filtrée, puis concentrée à sec sous pression réduite (2,7 kPa). Le résidu est agité avec 100 cm³ d'oxyde de diisopropyle, la suspension filtrée, et le solide est essoré, puis séché sous pression réduite (2,7 kPa). On obtient 10,76 g d'acide (1-benzhydryl-azétidin-3-yl)carboxylique-N-méthoxy-N-méthyl amide, sous la forme d'un solide crème.

L'acide (1-benzhydryl-azétidin-3-yl)carboxylique peut être préparé en opérant de la façon suivante : A une suspension refroidie à +5°C de 14 g de (1-benzhydryl-azétidin-3-yl)carbonitrile dans 140 cm³ de 2-éthoxyéthanol on coule goutte à goutte une solution de 11 g d'hydroxyde de potassium dans 9 cm³ d'eau, puis chauffe le mélange à 95°C. Après 16 heures d'agitation à cette température, le mélange réactionnel est versé lentement en agitant sur de la glace, et laissé à 0°C pendant 68 heures puis concentré à sec à sec à 50°C sous pression réduite (2,7 kPa). Le résidu est repris par 400 cm³ d'eau, la solution est acidifiée jusqu'à pH 4 avec de l'acide chlorhydrique 6N, puis additionnée de 400 cm³ d'acétate d'éthyle. La suspension résultante est filtrée, le solide essoré, puis séché à 50°C sous pression réduite (2,7 kPa). On obtient 13,55 g d'acide (1-benzhydryl-azétidin-3-yl)carboxylique, sous la forme d'un solide crème.

Le (1-benzhydryl-azétidin-3-yl)carbonitrile peut être préparé en opérant de la façon suivante : A une solution de 40 g de méthylsulfonate de 1-benzhydrylazétidin-3-yle dans 350 cm³ de N,N-diméthylformamide, on ajoute goutte à goutte une solution de 18,54 g de cyanure de sodium dans 25 cm³ d'eau puis chauffe le mélange à 65°C. Après 24 heures d'agitation à 65°C, le mélange réactionnel, laissé revenir à température ordinaire, est versé ensuite en agitant dans un mélange de 550 cm³ d'eau et de 300 g de glace. La suspension obtenue est filtrée, le solide est lavé par 3 fois 110 cm³ d'eau, puis dissous dans 350 cm³ de dichlorométhane. La solution est séchée sur sulfate de magnésium, filtrée, et concentrée à sec sous pression réduite (2,7 kPa). Le résidu est agité avec 200 cm³ d'oxyde de diisopropyle, la suspension filtrée, le solide essoré puis séché sous pression réduite (2,7 kPa). On obtient 28,4 g de (1-benzhydryl-azétidin-3-yl)carbonitrile, sous la forme d'un solide rose crème.

Le méthylsulfonate de 1-benzhydryl-azétidin-3-yle peut être préparé en opérant à partir de 100 g de 1-benzhydryl-azéfidin-3-ol, de 800 cm³ de dichlorométhane, de 31 cm³ de chlorure de méthylsulfonyle et de 35 cm³ de pyridine. Le produit brut obtenu est chromatographié sur une colonne de gel de silice (granulométrie 0,063-0,200 mm, hauteur 50 cm, diamètre 11 cm), en éluant sous une pression de 0,5 bar d'argon avec un mélange de cyclohexane et d'acétate d'éthyle (80/20, 75/25, puis 70/30 et 60/40 en volumes) et en recueillant des fractions de 1 litre. Les fractions 12 à 31 sont réunies et concentrées à sec sous pression réduite (2,7 Kpa). On obtient 66 g de méthylsulfonate de 1-benzhydryl-azétidin-3-yle, sous la forme d'un solide blanc jaune.

Le 1-benzhydryl-azétidin-3-ol peut être préparé en opérant comme il est décrit par Alan R. KATRITZKY et coll, dans J. Heterocyclic Chem.; 31, 271 (1994).

### Exemple 11

La 1-[3-({1-[bis-(4-chlorophényl)méthyl]azétidin-3-yl}méthylsulfonylméthyl)phényl]pyrrolidine-(RS) peut être préparée en opérant comme il est décrit dans l'exemple 3, à partir de 0,10 g de 1-[3-({1-[bis-(4-chlorophényl)méthyl]azétidin-3-yl}méthylsulfonyl-méthytène)phényl] pyrrolidine, de 1,5 cm³ de méthanol anhydre, de 1,5 cm³ de dichlorométhane anhydre, et de 30 mg d'hydroborure de sodium, en agitant pendant 3 heures à 20°C, puis 8 heures à 50°C. Le produit brut est chromatographié sur une colonne de gel de silice (granulométrie 0,040-0,063 mm, hauteur 8 cm, diamètre 1 cm), en éluant sous une pression de 0,8 bar d'argon avec un mélange de cyclohexane et d'acétate d'éthyle (80/20 en volumes) et en recueillant des fractions de 5 cm³. Les fractions 22 à 28 sont réunies et concentrées à sec sous pression réduite (2,7 kPa), le résidu est agité avec 5 cm³ de pentane, le solide est filtré, essoré et séché. On obtient 18 mg de 1-[3-({1-[bis-(4-chlorophényl)méthyl]azétidin-3-yl}méthylsulfonylméthyl)phényl]pyrrolidine-(RS), sous la forme d'une poudre blanche [Spectre de R.M.N ¹H (300 MHz, CDCl₃, δ en ppm) : 2,01 (mt : 4H); 2,59 (mt : 1H); 2,61 (s : 3H); de 3,10 à 3,25 (mt : 2H); 3,27 (mt : 4H); de 3,40 à 3,55 (mt : 1H); 3,66 (mt : 1H); 4,20 (d, J = 12 Hz : 1H); 4,25 (s : 1H); de 6,45 à 6,65 (mt : 3H); de 7,10 à 7,35 (mt : 9H)].

La 1-[3-({1-[bis-(4-chlorophényl)méthyl]azétidin-3-yl}méthylsulfonylméthylène)phényl]pyrrolidine peut être préparée en opérant comme il est décrit dans l'exemple 6, à partir de 0,6 g de 1-[bis(4-chlorophényl)méthyl]-3-[(méthylsulfonyl)(3-pyrrolidinylphényl)méthyl-(RS)]azétidin-3-ol, de 0,1 cm³ de chlorure de méthylsulfonyle et 0,5 g de 4-diméthylaminopyridine, le résidu obtenu est purifié par chromatographie sur colonne de gel de silice (granulométrie 0,04-0,06 mm, diamètre 2 cm, hauteur 30 cm) sous une pression de 0,5 bar d'azote avec un mélange dichlorométhane et éthanol comme éluant (98,5/1,5 en volumes) et en recueillant des fractions de 10 cm³. La fraction 4 est concentrée à sec sous pression réduite (2,7 kPa). On obtient après recristallisation dans 5 cm³ d'oxyde de diéthyle 0,5 g de 1-[3-({1-[bis-(4-chlorophényl)méthyl]azétidin-3-yl}méthylsulfonylméthylène)phényl]pyrrolidine, sous la forme d'un solide fondant à 133°C.

Le 1-[bis(4-chlorophényl)méthyl]-3-[(méthylsulfonyl)(3-pyrrolidinylphényl) méthyl-(RS)]azétidin-3-ol peut être obtenu en opérant selon le mode opératoire de l'exemple 5, à partir de 0,5 g de 1-[3-(méthylsulfonylméthyl)phényl]pyrrolidine, de 0,6 g de 1-[bis(4-chlorophényl)méthyl]azétidin-3-one de 15 cm³ de tétrahydrofuranne et de 1,4 cm³ d'une solution 1,6 N de n butyllithium dans l'hexane. On obtient 0,6 g de 1-[bis(4-chlorophényl)méthyl]-3-[(méthylsulfonyl)(3-pyrrolidinylphényl)méthyl-(RS)]azétidin-3-ol sous forme d'un solide crème.

La 1-[3-(méthylsulfonylméthyl)phényl]pyrrolidine peut être préparée en opérant comme il est décrit dans l'exemple 8, à partir de 6 cm³ d'eau, de 6 cm³ d'acide acétique à 100%, de 3,5 cm³ d'acide sulfurique 36N, de 3,11 g d'oxone, de 0,96 g de 1-[3-(méthylsulfanylméthyl)phényl]pyrrolidine et de 6 cm³ d'éthanol. On obtient 0,478 g de 1-[3-(méthylsulfonylméthyl)phényl]pyrrolidine, sous la forme d'une gomme brun clair.

La 1-[3-(méthylsulfanylméthyl)phényl]pyrrolidine peut être préparée en opérant comme il est décrit dans l'exemple 8, à partir de 4,0 g de 1-iodo-3-(méthylsulfanylméthyl)-benzène, de 120 cm³ de tétrahydrofuranne, de 2,2 g de tertbutylate de sodium, de 0,556 g de chlorure de 1,1'-bis-(diphénylphosphino)ferrocènyl palladium, de 1,26 g de 1,1'-bis-(diphénylphosphino)ferrocène, et de 2,6 g de pyrrolidine. On obtient 1,9 g de 1-[3-(méthylsulfanylméthyl)phényl]pyrrolidine, sous la forme d'une huile.

### Exemple 12

L'ester tert-butyl de l'acide N-(RS)-[3-({1-[bis-(4-chlorophényl)méthyl]azétidin-3-yl}méthylsulfonyl-méthyl)phényl]-N-méthyl-carbamique peut être préparé en opérant comme il est décrit dans l'exemple 3, à partir de 0,10 g de l'ester tert-butyl de l'acide N-[3-({1-[bis-(4-chlorophényl)méthyl]azétidin-3-yl}méthylsulfonyl-méthylène)phényl]-N-méthyl-carbamique, de 1,5 cm³ de méthanol anhydre, de 1,5 cm³ de dichlorométhane anhydre, et de 30 mg d'hydroborure de sodium, en agitant pendant 3 heures à 20°C. Le produit brut est agité avec 10 cm³ d'oxyde de diisopropyle, le solide est filtré, essoré, puis séché sous pression réduite (2,7 kPa). On obtient 94 mg d'ester tert-butyl de l'acide N-(RS)-[3-({1-[bis-(4-chlorophényl)méthyl]azétidin-3-yl}méthylsulfonylméthyl)phényl]-N-méthyl-carbamique, sous la forme d'une poudre blanche [Spectre de R.M.N ¹H (300 MHz, (CD₃)₂SO d6, δ en ppm) : 1,36 (s : 9H); 2,46 (t, J = 7,5 Hz : 1H); 2,75 (s : 3H); de 3,00 à 3,55 (mt : 4H); 3,17 (s : 3H); 4,45 (s : 1H); 4,78 (d, J = 11 Hz : 1H); de 7,20 à 7,50 (mt : 12H)].

L'ester tert-butyl de l'acide N-[3-({1-[bis-(4-chlorophényl)méthyl]azétidin-3-yl}méthylsulfonyl-méthylène)phényl]-N-méthyl-carbamique peut être préparé en opérant comme il est décrit dans l'exemple 6, à partir de 5,6g de 1-[bis(4-chlorophényl)méthyl]-3-{[3-(N-tertbutyloxycarbonyl-N-méthylamino)phényl] méthylsulfonyl-méthyl-(RS)}azétidin-3-ol, de 100 cm³ de dichlorométhane, de 1,59 g de chlorure de méthylsulfonyle et de 4,5 g de 4-diméthylaminopyridine. Le produit brut obtenu est purifié par chromatographie sur une colonne de gel de silice (granulométrie 0,04-0,06 mm, diamètre 4 cm et poids en silice 250 g), en éluant sous une pression de 0,5 bar d'azote avec un mélange d'acétate d'éthyle et de cyclohexane (30/70 en volumes) et en recueillant des fractions de 100 cm³: Les fractions 12 à 18 sont réunies, concentrées à sec sous pression réduite (2,7 kpa). On obtient 3,2g de l'ester tert-butyl de l'acide N-[3-({1-[bis-(4-chlorophényl)méthyl]azétidin-3-yl}méthylsulfonylméthylène)phényl]-N-méthyl-carbamique sous la forme d'une meringue blanche.

Le 1-[bis(4-chlorophényl)méthyl]-3-{[3-(N-tertbutyloxycarbonyl-N-méthylamino)phényl]méthylsulfonyl-méthyl-(RS)}azétidin-3-ol peut-être préparé en opérant comme il est décrit dans l'exemple 5, à partir de 3,8 g d'ester tert-butyl de l'acide N-[3-(méthylsulfonylméthyl)-phényl]-N-méthyl-carbamique, de 50 cm³de tétrahydrofuranne, de 9,5 cm³ d'une solution de n-butyl lithium 1,6N dans l'hexane, et de 3,82 g de 1-[bis(4-chlorophényl)méthyl]azétidin-3-one. Le produit brut est purifié par chromatographie sur une colonne de gel de silice (granulométrie 0,04-0,06 mm, diamètre 4 cm, poids en silice 250 g), en éluant sous une pression de 0,5 bar d'azote avec du dichlorométhane puis avec un mélange dichlorométhane et éthanol (99/1 en volumes) et en recueillant des fractions de 500 cm³. Les fractions 10 à 16 sont réunies, concentrées à sec sous pression réduite (2,7 kPa). On obtient 5,6 g de 1-[bis(4-chlorophényl)méthyl]-3-{[3-(N-tertbutyloxycarbonyl-N-méthylamino) phényl]méthylsulfonyl-méthyl-(RS)}azétidin-3-ol, sous la forme d'une meringue.

L'ester tert-butyl de l'acide N-[3-(méthylsulfonylméthyl)-phényl]-N-méthylcarbamique peut être préparé en opérant de la façon suivante : A une solution de 3 g de N-[3-(méthylsulfonylméthyl)-phényl]-N-méthylamine dans 80 cm³ de dichlorométhane, on ajoute 3,7 g de di-tert-butyl di-carbonate. Après 20 heures d'agitation à 20°C, le mélange réactionnel est additionné de 100 cm³ d'eau, puis décanté. La phase organique est séchée sur sulfate de magnésium, filtrée, puis concentrée à sec sous pression réduite (2,7 kPa). Le résidu est chromatographié sur une colonne de gel de silice (granulométrie 0,04-0,06 mm, diamètre 4 cm et poids en silice 300g), en éluant sous une pression de 0,5 bar d'azote avec un mélange d'acétate d'éthyle et de cyclohexane (45/55 en volumes) et en recueillant des fractions de 100 cm³. Les fractions 11 à 16 sont réunies, et concentrées à sec sous pression réduite (2,7 kpa). On obtient 3,8 g d'ester tert-butyl de l'acide N-[3-(méthylsulfonylméthyl)-phényl]-N-méthyl-carbamique, sous la forme d'une gomme qui cristallise.

La N-[3-(méthylsulfonylméthyl)-phényl]-N-méthylamine peut être obtenue en opérant de la façon suivante : On chauffe à 50°C pendant 3 heures, sous argon, un mélange de 9,65 cm³ d'acide formique et de 19,63 cm³ d'anhydride acétique, puis laisse revenir la solution obtenue à température ordinaire. On coule 40 cm³ de tétrahydrofuranne et refroidit le milieu à -20°C. Après 2 heures d'agitation à -20°C on coule une solution de 14,8 g de 3-(méthylsulfonylméthyl)-phénylamine en maintenant la température. Après 2 heures d'agitation à -20°C, puis 48 heures à 20°C, le mélange est filtré, le solide est essoré, puis lavé avec 3 fois 50 cm³ d'oxyde de diisopropyle et séché sous pression réduite (2,7 kPa). On obtient un solide A. Le filtrat est concentré à demi volume, la suspension résultante est filtrée, le solide est essoré, lavé avec de l'oxyde de diisopropyle, et séché. On obtient un solide B. Les deux solides A et B sont réunis, et repris par 375 cm³ de tétrahydrofuranne et le mélange, refroidi à 0°C est additionné en 20 minutes de 80 cm³ d'une solution 2M de complexe borane diméthylsulfure dans le térahydrofuranne, puis porté à la température du reflux pendant 3 heures. Le mélange réactionnel, refroidi à +5°C, est additionné en 20 minutes de 60 cm³ de méthanol, agité pendant une heure à température ambiante, puis additionné d'acide chlorhydrique gazeux jusqu'à un pH de 1. Le mélange est porté au reflux pendant une heure, puis additionné de 300 cm³ d'eau et d'une solution d'hydroxyde de sodium 3N, jusqu'à pH 8. Le mélange résultant est extrait par 500 cm³ d'acétate d'éthyle, la phase organique est lavée successivement par une solution aqueuse saturée d'hydrogénocarbonate de sodium, et par de la saumure, puis est séchée sur sulfate de magnésium et concentrée à sec sous pression réduite(2,7 kPa). Le résidu est repris par 100 cm³ d'acide sulfurique 4N, la solution obtenue est lavée par 100 cm³ d'acétate d'éthyle, puis alcalinisée jusqu'à pH 8 avec de l'hydroxyde de sodium 3N et avec une solution aqueuse saturée de carbonate de sodium, puis est extraite avec 2 fois 75 cm³ d'acétate d'éthyle. Les extraits réunis sont séchés sur sulfate de magnésium et concentrés à sec sous pression réduite (2,7 kPa). On obtient 8,98 g de N-[3-(méthylsulfonylméthyl)-phényl]-N-méthylamine sous la forme d'un solide rose.

La 3-(méthylsulfonylméthyl)-phénylamine peut être préparée en opérant de la façon suivante : On chauffe au reflux une suspension de 23,7 g de 1-(méthylsulfonylméthyl)-3-nitrobenzène dans 150 cm³ de méthanol et 65 cm³ d'acide chlorhydrique à 36%, puis ajoute avec précaution en 10 minutes, par petites fractions, 18,5 g de fer. Après 4 heures de reflux puis 20 heures d'agitation à température ordinaire, le mélange réactionnel est additionné de 5 g de fer, puis chauffé à nouveau au reflux pendant une heure puis 20 heures à température ordinaire. Le mélange est ensuite alcalinisé jusqu'à pH 9 avec de l'ammoniaque et avec de l'hydrogénocarbonate de sodium, puis extrait avec 3 fois 250 cm³ d'acétate d'éthyle, séché sur sulfate de magnésium, et concentré à sec sous pression réduite (2,7 kPa). On obtient 14,9 g de 3-(méthylsulfonylméthyl)-phénylamine, sous la forme d'une poudre beige.

Le 1-(méthylsulfonylméthyl)-3-nitrobenzène peut être préparé en chauffant au reflux 23,8 g de chlorure de 3-nitrobenzyle, 20 g de méthylsulfinate de sodium et de 250 cm³ d'éthanol absolu. On obtient 23,74 g de 1-(méthylsulfonylméthyl)-3-nitrobenzène, sous la forme d'une poudre blanche.

### Exemple 13

La N-[3-({1-[bis-(4-chlorophényl)méthyl]azétidin-3-yl}méthylsulfonylméthyl)phényl]-N-méthyl-amine-(RS) peut être préparée en opérant comme il est décrit dans l'exemple 3, à partir de 0,10 g de N-[3-({1-[bis-(4-chlorophényl)méthyl]azétidin-3-yl}méthylsulfonyl-méthylène)phényl]-N-méthylamine, de 1,5 cm³ de méthanol anhydre, de 1,5 cm³ de dichlorométhane anhydre, et de 45 mg d'hydroborure de sodium, en agitant pendant 20 heures à 20°C puis pendant 5 heures à 50°C. Le produit brut est chromatographié sur une colonne de gel de silice (granulométrie 0,040-0,063 mm, hauteur 10 cm, diamètre 1 cm), en éluant sous une pression de 0,8 bar d'argon avec un mélange de cyclohexane et d'acétate d'éthyle (80/20, puis 60/40 en volumes) et en recueillant des fractions de 5 cm³. Les fractions 20 à 26 sont réunies et concentrées à sec sous pression réduite (2,7 kPa), le résidu est agité avec 5 cm³ de pentane, le solide est filtré, essoré et séché. On obtient 20 mg de N-[3-({1-[bis-(4-chlorophényl)méthyl]azétidin-3-yl}méthylsulfonyl-méthyl)phényl]-N-méthyl-amine-(RS), sous la forme d'une poudre blanche [Spectre de R.M.N ¹H (300 MHz, CDCl₃, δ en ppm) : de 2,50 à 2,65 (mt : 1H); 2,60 (s : 3H); 2,82 (d, J = 5 Hz : 3H); 3,18 (mt : 2H); de 3,35 à 3,50 (mt : 1H); 3,64 (t large, J = 7,5 Hz : 1H); 3,80 (mt : 1H); 4,18 (d, J = 11,5 Hz : 1H); 4,24 (s : 1H); de 6,50 à 6,70 (mt : 3H); de 7,10 à 7,35 (mt : 9H].

La N-[3-({1-[bis-(4-chlorophényl)méthyl]azétidin-3-yl}méthylsulfonylméthylène)phényl]-N-méthyl-amine peut être préparée en opérant de la façon suivante : On agite pendant 20 heures 2,7 g de 1-[bis(4-chlorophényl)méthyl]-3-{[3-(N-tertbutyloxycarbonyl-N-méthylamino)phényl] méthylsulfonylméthylènelazétidine dans 30 cm³ de dioxane et 30 cm³ d'une solution de dioxane chlorhydrique 4,7N . Le milieu réactionnel est évaporé à sec sous pression réduite (2,7 kpa), repris par 50 cm³ d'eau et 50 cm³ d'acétate d'éthyle, agité et neutralisé avec précaution par une solution aqueuse saturée de bicarbonate de sodium. La phase organique est séparée, séchée sur sulfate de magnésium, traitée au noir animal puis concentrée sous pression réduite (2,7 kpa) jusqu'à un volume d'environ 25 cm³, ensuite filtrée, concentrée à sec sous pression réduite. On obtient 1,3 g de N-[3-({1-[bis-(4-chlorophényl)méthyl]azétidin-3-yl}méthylsulfonyl-méthylène)phényl]-N-méthylamine, sous la forme de cristaux blancs fondant à 228°C.

### Exemple 14

La 1-[bis-(4-chlorophényl)méthyl]-3-[(3,5-bis-trifluorométhylphényl)méthylsulfonyl-méthyl]azétidine-(RS) peut être préparée en opérant comme il est décrit dans l'exemple 3, à partir de 0,10 g de 1-[bis-(4-chlorophény)méthyl]-3-[(3,5-bis-trifluorométhylphényl)méthylsulfonyl-méthylène]azétidine, de 1,5 cm³ de méthanol anhydre, de 1,5 cm³ de dichlorométhane anhydre, et de 15 mg d'hydroborure de sodium, en agitant pendant 3 heures à 20°C. Le produit brut est agité avec 5 cm³ de pentane, le solide est filtré, essoré et séché. On obtient 82 mg de 1-[bis-(4-chlorophényl)méthyl]-3-[(3,5-bis-trifluorométhyl phényl)méthylsulfonyl-méthyl]azétidine-(RS), sous la forme d'une poudre blanche [Spectre de R.M.N ¹H (400 MHz, (CD₃)₂SO d6, δ en ppm) : 2,84 (s : 3H); 3,08 (mt : 2H); de 3,25 à 3,40 (mt : 1H); de 3,45 à 3,65 (mt : 2H); 4,45 (s : 1H); 5,13 (d, J = 10,5 Hz : 1H); de 7,25 à 7,50 (mt : 8H); 8,11 (s large : 2H); 8,15 (s large : 1H)].

La 1-[bis-(4-chlorophényl)méthyl]-3-[(3,5-bis-trifluorométhylphényl)méthylsulfonyl-méthylène]azétidine peut être préparée en opérant de la façon suivante : A une solution de 3,16 g de 3-acétoxy-1-[bis-(4-chlorophényl)méthyl]-3-{[3,5-bis(trifluorométhyl)phényl]méthylsulfonyl-méthyl-(RS)}azétidine dans 40 cm³ de dioxane, on ajoute par fractions 0,96 g de soude broyée. Après 1 heure d'agitation à température ordinaire, le mélange réactionnel est additionné de 200 cm³ d'acétate d'éthyle, 200 cm³ d'eau, puis est décanté. La phase organique est lavée par 2 fois 80 cm³ d'eau, puis par 80 cm³ de saumure, séchée sur sulfate de magnésium, puis filtrée et concentrée à sec sous pression réduite (2,7 kPa). Le résidu est chromatographié sur une colonne de gel de silice (granulométrie 0,040-0,063 mm, hauteur 14,5 cm, diamètre 4,8 cm), en éluant sous une pression de 0,5 bar d'argon avec un mélange de cyclohexane et d'acétate d'éthyle (85/15 en volumes) et en recueillant des fractions de 40 cm³. Les fractions 8 à 15 sont réunies et concentrées à sec sous pression réduite (2,7 kPa) on obtient 1,49 g de 1-[bis(4-chlorophényl)méthyl]-3-{[3,5-bis(trifluorométhyl) phényl]méthylsulfonyl-méthylènelazétidine sous la forme d'une meringue blanche.

La 3-acétoxy-1-[bis(4-chlorophényl)méthyl]-3-{[3,5-bis(trifluorométhyl) phényljméthylsulfonyl-méthyl-(RS)}azétidine peut être préparée en opérant de la façon suivante : A une solution refroidie à -78°C de 2,0 g de [3,5-bis(trifluorométhyl)benzyl]méthylsulfone dans 35 cm³ de tétrahydrofuranne, on coule goutte à goutte sous argon 4,1 cm³ d'une solution 1,6N de n-butyllithium dans l'hexane. Après une heure d'agitation à -70°C, on ajoute goutte à goutte une solution de 2,0 g de 1-[bis(4-chlorophényl)méthyllazétidin-3-one dans 35 cm³ de tétrahydrofuranne. Après 2 heures d'agitation à -78°C, on coule une solution de 0,7 cm³ de chlorure d'acétyle dans 5 cm³ d'oxyde de diéthyle anhydre puis laisse le mélange revenir à température ordinaire. Après 2 heures et 30 minutes d'agitation, le mélange réactionnel est additionné de 100 cm³ d'eau, et décanté. La phase organique est lavée par 100 cm³ d'eau, puis par 100 cm³ de saumure puis est séchée sur sulfate de magnésium et concentrée à sec sous pression réduite (2,7 kPa). Le résidu est chromatographié sur une colonne de gel de silice (granulométrie 0,04-0,06 mm, diamètre 5,6 cm, hauteur 16 cm), en éluant sous une pression de 0,5 bar d'argon avec un mélange d'acétate d'éthyle et de cyclohexane (10/90 puis 40/60 en volumes) et en recueillant des fractions de 100 cm³. Les fractions 37 à 52 sont réunies et concentrées à sec sous pression réduite (2,7 kPa). On obtient 3,56 g de 3-acétoxy-1-[bis(4-chlorophényl)méthyl]-3-{[3,5-bis(trifluorométhyl)phényl]méthylsulfonyl-méthyl-(RS)}azétidine sous forme d'une meringue blanche.

La [3,5-bis(trifluorométhyl)benzyl]méthylsulfone peut être préparée en chauffant au reflux 1,8 g de chlorure de 3,5-bis-(trifluorométhyl)benzyle, 50 cm³ d'éthanol absolu et 1,22 g de méthylsulfinate de sodium. On obtient 1,86 g de [3,5-bis(trifluorométhyl)benzyl]méthylsulfone sous forme d'un solide blanc.

### Exemple 15

La N-[4-((4-chlorophényl)-{3-[(3,5-difluorophényl)méthylsulfonyl-méthyl-(RS)]azétidin-1-yl}méthyl-(RS))-benzyl]-N,N-diméthyl-amine, mélange de deux diastéréoisomères peut être préparée en opérant comme il est décrit dans l'exemple 3, à partir de 0,10 g de N-[4-((4-chtorophényl)-{3-[(3,5-difluorophényl)méthylsulfonyl-méthylène]azétidin-1-yl}-méthyl-(RS))-benzyl]-N,N-diméthyl-amine, de 1,5 cm³ de méthanol anhydre, de 1,5 cm³ de dichlorométhane anhydre, et de 45 mg d'hydroborure de sodium, en agitant pendant 16 heures à 20°C puis pendant 16 heures à 50°C. Le produit brut est chromatographié sur une colonne de gel de silice (granulométrie 0,040-0,063 mm, hauteur 22 cm, diamètre 1 cm), en éluant sous une pression de 0,8 bar d'argon avec un mélange d'acétate d'éthyle et de méthanol (97/3 en volumes) et en recueillant des fractions de 20 cm³. Les fractions 17 à 25 sont réunies et concentrées à sec sous pression réduite (2,7 kPa), le résidu est agité avec 5 cm³ de pentane, le solide est filtré, essoré et séché. On obtient 6 mg de N-[4-((4-chlorophényl)-{3-[(3,5-difluorophényl)méthyisulfonyle-méthyl-(RS)]azétidin-1-yl}méthyl-(RS))-benzyl]-N,N-diméthyl-amine, mélange de deux diastéréoisomères, sous la forme d'une poudre blanche [Spectre de R.M.N. ¹H (300 MHz, CDCl₃, δ en ppm) : 2,20 (s : 6H); 2,53 (t, J = 7 Hz : 1H); 2,65 (s : 3H); de 3,10 à 3,25 (mt : 2H); de 3,30 à 3,45 (mt : 1H); 3,35 (s large : 2H); 3,63 (t large, J = 7 Hz : 1H); 4,24 (s : 1H); 4,25 (d, J = 11 Hz : 1H); 6,82 (tt, J = 9 et 2 Hz : 1H); 6,94 (mt : 2H); de 7,15 à 7,35 (mt : 8H)].

La N-[4-((4-chlorophényl)-{3-[(3,5-difluorophényl)méthylsulfonylméthylène]azétidin-1-yl}méthyl-(RS))-benzyl]-N,N-diméthyl-amine peut être préparée en opérant de la façon suivante : A une solution de 0,93 cm³ d'une solution de diméthylamine 2M dans le méthanol dans 30 cm³ de 1,2-dichloroéthane anhydre, on ajoute sous argon 1,0 g de (RS)-4-((4-chlorophényl)-{3-[(3,5-difluorophényl)-méthylsulfonyl-méthylène]azétidin-1-yl}méthyl)-benzaldéhyde. Après 30 minutes d'agitation à température ordinaire, on ajoute par petites fractions 0,9 g de triacétoxyborohydrure de sodium. Après 48 heures d'agitation, le mélange réactionnel est additionné de 2,65 cm³ d'hydroxyde de sodium 1N, de 100 cm³ d'eau et de 100 cm³ de dichlorométhane, puis est décanté. La phase organique est lavée avec 2 fois 80 cm³ d'eau, et avec 80 cm³ de saumure, puis est séchée sur sulfate de magnésium, et concentrée à sec sous pression réduite (2,7 kPa). Le résidu est chromatographié sur une colonne de gel de silice (granulométrie 0,04-0,06 mm, diamètre 4 cm, hauteur 17,5 cm), en éludant sous une pression de 0,5 bar d'argon avec un mélange d'acétate d'éthyle et cyclohexane (30/70 en volumes) et en recueillant des fractions de 40 cm³. Les fractions 48 à 53 sont réunies et concentrées à sec sous pression réduite (2,7 kPa). On obtient 0,46 g de N-[4-((4-chlorophényl)-{3-[(3,5-difluorophényl)méthylsulfonylméthylène]azétidin-1-yl}méthyl-(RS))-benzyl]-N,N-diméthyl-amine, sous la forme d'un solide blanc.

Le (RS)-4-((4-chlorophényl)-{3-[(3,5-difluorophényl)-méthylsulfonylméthylène]azétidin-1-yl}méthyl)-benzaldéhyde peut être préparé en opérant de la façon suivante : A une solution de 18,9 g de 1-[(4-chlorophényl)-(4-[1,3]dioxolan-2-yl-phényl)méthyl]-(RS)-3-[(3,5-difluorophényl)méthylsulfony]-méthylène]azétidine dans 80 cm³ de tétrahydrofuranne, on ajoute 75,6 cm³ d'acide chlorhydrique 5N. Après 3 heures à température ambiante, le mélange est repris par du dichlorométhane et l'eau distillée puis amené à pH 14 par ajout de soude 30% et décanté. La phase organique est lavée 2 fois avec 100 cm³ d'eau puis 100 cm³ d'une solution aqueuse saturée de chlorure de sodium, séchée sur sulfate de magnésium, filtrée et concentrée à sec sous pression réduite (2,7 kPa). On obtient 16 g de (RS)-4-((4-chlorophényl)-{3-[(3,5-difluorophényl)méthylsulfonyl-méthylène]azétidin-1-yl}méthyl)-benzaldéhyde, sous forme d'une meringue blanche.

La 1-[(4-chlorophényl)-(4-[1,3]dioxolan-2-yl-phényl)méthyl]-(RS)-3-[(3,5-difluorophényl)méthylsulfonyl-méthylène]azétidine peut être préparée selon la méthode suivante : à une solution de 34,45 g du mélange des deux diastéréoisomères acétate de 1-[(4-chlorophényl)-(4-[1,3]dioxolan-2-ylphényl)méthyl-(RS)]-3-[(3,5-difluorophényl)-méthylsulfonyl-méthyl-(RS)]azétidin-3-yle dans 400 cm³ de tétrahydrofuranne sous argon à 0°C, on ajoute goutte à goutte 13,0 cm³ de 1,8-diazabicyclo[5-4-0]undec-7-éne et après traitement habituel le produit est chromatographié sur colonne de gel de silice (granulométrie 0,04-0,06 mm, diamètre 10,2 cm, hauteur 23 cm), en éluant sous une pression de 0,5 bar d'argon avec un mélange d'acétate d'éthyle et de cyclohexane (20/80 en volumes) et en recueillant des fractions de 250 cm³, on obtient 16,6 g de 1-[(4-chlorophényl)-(4-[1,3]dioxolan-2-ylphényl)méthyl]-(RS)-3-[(3,5-difluorophényl)méthylsulfonylméthylène]azétidine, sous la forme d'un solide blanc.

Le mélange des deux diastéréoisomères acétate de 1-[(4-chlorophényl)-(4-[1,3]dioxoian-2-yl-phényl)méthyl-(RS)]-3-[(3,5-difluorophényl)-méthylsulfonylméthyl-(RS)]azétidin-3-yle peut être obtenu de la manière suivante : en opérant selon l'exemple 1 (méthode 2), à partir de 11,6 g de (3,5-difluorobenzyl)méthylsulfone, 35,1 cm³ d'une solution 1,6N de n butyllithium dans l'hexane, 19,3 g de 1-{(4-chlorophényl)[4-([1,3]-dioxolan-2-yl)phényl]méthyl-(RS)}azétidin-3-one et 8,8 cm³ de chlorure d'acétyle dans 500 cm³ de tétrahydrofuranne, on obtient 37,8 g du mélange des deux diastéréoisomères acétate de 1-[(4-chlorophényl)-(4-[1,3]dioxolan-2-yl-phényl)méthyl-(RS)]-3-[(3,5-difluorophényl)-méthylsulfonyl-méthyl-(RS)]azétidin-3-yle, sous la forme d'une meringue blanche.

La 1-{(4-chlorophényl)[4-([1,3]-dioxolan-2-yl)phényl]méthyl-(RS)}azétidin-3-one peut être préparée de la manière suivante : à une solution de 28,32 g de 1-{(4-chlorophényl)[4-([1,3]-dioxolan-2-yl)phényl]méthyl-(RS)}azétidin-3-ol dans 200 cm³ de diméthylsulfoxyde, on ajoute à température ambiante 46 cm³ de triéthylamine puis on additionne goutte à goutte une solution de 34 g du complexe trioxyde de soufre-pyridine dans 100 cm³ diméthylsulfoxyde. Après 0,25 heure à température ambiante, le mélange réactionnel est versé sur de la glace, extrait avec de l'acétate d'éthyle, lavé par 3 fois 400 cm³ d'eau puis par 400 cm³ d'une solution saturée de chlorure de sodium, séché sur sulfate de magnésium, filtré et concentré à sec sous pression réduite (2,7 kPa). Le résidu obtenu est chromatographié sur colonne de gel de silice (granulométrie 0,04-0,06 mm, diamètre 9,2 cm, hauteur 21 cm), sous une pression de 0,5 bar d'argon avec un mélange d'acétate d'éthyle et cyclohexane (20/80 en volumes) comme éluant et en recueillant des fractions de 250 cm³. Les fractions 9 à 18 sont réunies puis concentrées à sec sous pression réduite (2,7 kPa). On obtient 20,4 g de 1-{(4-chlorophényl)[4-([1,3]-dioxolan-2-yl)phényl]méthyl-(RS)}azétidin-3-one sous la forme d'une huile jaune.

Le 1-{(4-chlorophényl)[4-([1,3]-dioxolan-2-yl)phényl]méthyl-(RS)}azétidin-3-ol peut être préparé comme il est décrit dans l'exemple 3, en partant de 35,0 g de {(4-chlorophényl)[4-(1,3-dioxolan-2-yl)phényl]méthyl}amine, 8,3 g d'épibromhydrine, 5,1 g d'hydrogénocarbonate de sodium et de 400 cm³ d'éthanol. On isole 30,3 g de 1-{(4-chlorophényl)[4-(1,3-dioxolan-2-yl)phényl]méthyl-(RS)}azétidin-3-ol.

Le chlorhydrate de {(4-chlorophényl)[4-([1,3]-dioxolan-2-yl)phényl]méthyl-(RS)}amine peut être préparé selon la méthode décrite par GRISAR M. et coil., J. Med. Chem., 885 (1973) à partir de 67,2 g de 4-([1,3]-dioxolan-2-yl)benzonitrile, 88,2 g de 1-bromo-4-chlorobenzène, 11 g de magnésium et 600 cm³ d'éther éthylique. On obtient 42,3 g de {(4-chlorophényl)[4-([1,3]-dioxolan-2-yl)phényl]méthyl-(RS)}amine sous forme d'une huile jaune.

### Exemple 16

La 1-[(4-chlorophényl)-(thièn-2-yl)-méthyl-(RS)]-3-[(3,5-difluorophényl)méthylsulfonyl-méthyl-(RS)]azétidine, mélange de deux diastéréoisomères, peut être préparée en opérant comme il est décrit dans l'exemple 3, à partir de 0,10 g de 1-[(4-chlorophényl)-(thièn-2-yl)-méthyl-(RS)]-3-[(3,5-difluorophényl) méthylsulfonyl-méthylène]azétidine, de 1,5 cm³ de méthanol anhydre, de 1,5 cm³ de dichlorométhane anhydre, et de 45 mg d'hydroborure de sodium, en agitant pendant 16 heures à 20°C puis pendant 16 heures à 50°C. Le produit brut est chromatographié sur une colonne de gel de silice (granulométrie 0,040-0,063 mm, hauteur 25 cm, diamètre 1 cm), en éluant sous une pression de 1 bar d'argon avec un mélange de cyclohexane et d'acétate d'éthyle (90/10 en volumes) et en recueillant des fractions de 20 cm³. Les fractions 37 à 42 sont réunies et concentrées à sec sous pression réduite (2,7 kPa), le résidu est agité avec 5 cm³ de pentane, le solide est filtré, essoré et séché. On obtient 8 mg de 1-[(4-chlorophényl)-(thièn-2-yl)-méthyl-(RS)]-3-[(3,5-difluorophényl)méthylsulfonyl-méthyl-(RS)]azétidine, mélange de deux diastéréoisomères, sous la forme d'une poudre blanche [Spectre de R.M.N ¹H (300 MHz, CDCl₃, δ en ppm). On observe un mélange de diastéréoisomères, de 2,50 à 2,70 (mt : 1H); 2,66 et 2,68 (2s : 3H en totalité); de 3,15 à 3,80 (mt : 4H); de 4,20 à 4,30 (mt : 1H); 4,31 et 4,57 (2s : 1H en totalité); de 6,80 à 7,00 et de 7,10 à 7,40 (mts : 10H en totalité)].

Le 1-[(4-chlorophényl)-(thièn-2-yl)-méthyl-(RS)]-3-[(3,5-difluorophényl)méthylsulfonyl-méthylène]azétidine peut être préparé en opérant comme il est décrit dans l'exemple 6, à partir de 0,52 g d'un mélange des deux diastéréoisomères 1-[(4-chlorophényl)-(thièn-2-yl)-méthyl-(RS)]-3-[(3,5-difluorophényl)méthylsulfonyl-méthyl-(RS)]azétidin-3-ol, de 0,14 cm³ de chlorure de méthylsulfonyle et de 0,49 g de 4-diméthylaminopyridine. On obtient après chromatographie sur une colonne de gel de silice (granulométrie 0,06-0,200 mm, diamètre 2,4 cm, hauteur 20 cm), en éluant sous une pression de 0,5 bar d'argon avec un mélange d'acétate d'éthyle et cyclohexane (20/80 en volumes) et en recueillant des fractions de 30 cm³, 0,32 g de (RS)-1-[(4-chlorophényl-(thièn-2-yl)-méthyl]-3-[(3,5-difluorophényl)-méthylsulfonylméthylène]azétidine, sous la forme d'un solide blanc, fondant à 176°C.

Le mélange des deux diastéréoisomères 1-[(4-chlorophényl)(thièn-2-yl)méthyl-(RS)]-3-[(3,5-difluorophényl)-méthylsulfonyl-méthyl-(RS)]azétidin-3-ol peut être préparé en opérant comme il est décrit dans l'exemple O, à partir de 1,60 cm³ de n-butyllithium 1,6N en solution dans l'hexane, de 0,83 g de (3,5-difluorobenzyl)méthylsulfone et de 1,06 g de 1-[(4-Chlorophényl)(thièn-2-yl)méthyl-(RS)]azétidin-3-one. On obtient après purification sur une colonne de gel de silice (granulométrie 0,06-0,200 mm, diamètre 2,8 cm, hauteur 30 cm), en éluant sous une pression de 0,5 bar d'argon avec un mélange d'acétate d'éthyle et cyclohexane (25/75 en volumes) et en recueillant des fractions de 40 cm³, 0,55 g du mélange de diastéréoisomères 1-[(4-chlorophényl)(thièn-2-yl)méthyl-(RS)]-3-[(3,5-difluorophényl)-méthylsulfonylméthyl-(RS)]azétidin-3-ol, sous la forme d'un solide blanc cassé.

La 1-[(4-chlorophényl)(thièn-2-yl)méthyl-(RS)]azétidin-3-one peut être préparée en opérant comme il est décrit dans l'exemple 1 (méthode 2), à partir de 1,83 cm³ de chlorure d'oxalyle, de 20 cm³ de dichlorométhane, de 3,04 cm³ de diméthylsulfoxyde, de 5,2 g de 1-[(4-chlorophényl)(thièn-2-yl)méthyl-(RS)]azétidin-3-ol, de 80 cm³ de dichlorométhane, et de 9,12 cm³ de triéthylamine. On obtient 3,3 g de 1-[(4-chlorophényl)(thièn-2-yl)méthyl-(RS)]zétidin-3-one, sous la forme d'une huile jaune qui cristallise à température ordinaire.

Le 1-[(4-chlorophényl)(thièn-2-yl)méthyl-(RS)]azétidin-3-ol peut être préparé en opérant de la façon suivante : à une solution de 11,0 g de [(4-chlorophényl)(thièn-2-yl)méthyl-(RS)]amine dans 80 cm³ d'éthanol on ajoute 4,12 g de bicarbonate de sodium. Le mélange chauffé à 65°C est additionné de 4,03 cm³ d'épibromhydrine. Après 20 heures d'agitation à 65°C, le mélange refroidi est filtré et le filtrat concentré à sec sous pression réduite (2,7 Kpa). Le résidu est chromatographié sur une colonne de gel de silice (granulométrie 0,06-0,200 mm, diamètre 3,6 cm, hauteur 32 cm), en éluant sous une pression de 0,5 bar d'argon avec un mélange d'acétate d'éthyle et cyclohexane (25/75 en volumes) et en recueillant des fractions de 60 cm³ On obtient 6,3 g de 1-[(4-chlorophényl)(thièn-2-yl)méthyl-(RS)]azétidin-3-ol, sous la forme d'une huile jaune pâle.

La [(4-chlorophényl)(thièn-2-yl)méthyl-(RS)]amine peut être préparée de la façon suivante : à une suspension refroidie à 10°C de bromure de 4-chlorophénylmagnésien (préparée à partir de 19,15 g de 4-bromochlorobenzène et 2,43 g de magnésium) dans 120 cm³ d'éther éthylique anhydre, on coule lentement une solution de 10,92 g de 2-thiophènecarbonitrile dans 80 cm³ d'oxyde de diéthyle. Après une heure de reflux, le mélange est refroidi à 10°C, additionné lentement de 40 cm³ de méthanol et ensuite filtré sur supercel. On ajoute sous argon et par petites fractions en 15 minutes 4,54 g de borohydrure de sodium puis agite le milieu réactionnel pendant 20 heures à 20°C. Le mélange obtenu est dilué avec de l'acétate d'éthyle, puis lavé avec de l'eau. La phase organique est séchée sur sulfate de magnésium, concentrée à sec à 50°C sous pression réduite (2,7 kPa). Le résidu est chromatographié sur une colonne de gel de silice (granulométrie 0,06-0,200 mm, diamètre 5 cm, hauteur 42 cm), en éluant sous une pression de 0,5 bar d'argon avec un mélange d'acétate d'éthyle et cyclohexane (4/6 en volumes) et en recueillant des fractions de 100 cm³. Les fractions 6 à 12 concentrées à sec correspondent à 13 g d'imine sous la forme d'une huile jaune que l'on reprend dans 100 cm³ de méthanol. La solution obtenue est additionnée de 2,4 g de borohydrure de sodium, et agitée pendant une heure à 5°C. Le mélange obtenu est dilué avec de l'acétate d'éthyle, puis lavé avec de l'eau. La phase organique est séchée sur sulfate de magnésium, concentrée à sec à 50°C sous pression réduite (2,7 Kpa). Le résidu est chromatographié sur une colonne de gel de silice (granulométrie 0,06-0,200 mm, diamètre 3,2 cm, hauteur 40 cm), en éluant sous une pression de 0,5 bar d'argon avec un mélange d'acétate d'éthyle et cyclohexane (4/6 en volumes) et en recueillant des fractions de 60 cm³. On obtient 11,0 g de [(4-chlorophényl)(thièn-2-yl)méthyl-(RS)]amine, sous la forme d'une huile jaune.

### Exemple 17

Le [3-({1-[bis-(4-chlorophényl)méthyl]azétidin-3-yl}méthylsulfonylméthyl)phényl]méthanol-(RS) peut être préparé en opérant comme il est décrit dans l'exemple 3, à partir de 0,050 g de, [3-({1-[bis-(4-chlorophényl)méthyl]azétidin-3-yl}méthylsulfonyl-méthylène)phényl]méthanol, de 1,0 cm³ de méthanol anhydre, de 1,0 cm³ de dichlorométhane anhydre, et de 20 mg d'hydroborure de sodium, en agitant pendant 3 heures à 20°C. Le produit brut est chromatographié sur une colonne de gel de silice (granulométrie 0,040-0,063 mm, hauteur 20 cm, diamètre 1 cm), en éluant sous une pression de 0,8 bar d'argon avec un mélange de cyclohexane et d'acétate d'éthyle (90/10 en volumes) et en recueillant des fractions de 10 cm³. Les fractions 30 à 38 sont réunies et concentrées à sec sous pression réduite (2,7 kPa), le résidu est agité avec 5 cm³ de pentane, le solide est filtré, essoré et séché. On obtient 13 mg de [3-({1-[bis-(4-chlorophényl)méthyl]azétidin-3-yl}méthylsulfonyl-méthyl)phényl]méthanol-(RS), sous la forme d'une poudre blanche [Spectre de R.M.N ¹H (300 MHz, CDCl₃, δ en ppm) : 1,75 (t, J = 6 Hz : 1H); 2,52 (t, J = 7,5 Hz : 1H); 2,59 (s : 3H); 3,17 (t large, J = 7,5 Hz : 2H); 3,48 (mt : 1H); 3,65 (mt : 1H); 4,23 (s : 1H); 4,28 (d, J = 11,5 Hz : 1H); 4,70 (d, J = 6 Hz : 2H); de 7,15 à 7,40 (mt : 12H)].

Le [3-({1-[bis-(4-chlorophényl)méthyl]azétidin-3-yl}méthylsulfonyl-méthylène)phényl]méthanol peut être préparé en opérant de la façon suivante : A une solution refroidie à +5°C de 5,1 g de 1-[bis(4-chlorophényl)méthyl]-3-{[3-(tert-butyldiméthylsilyloxyméthyl)phényl]méthylsulfonyl-méthylène}azétidine dans 51 cm³ de tétrahydrofuranne, on coule 17 cm³ d'une solution 1M de fluorure de tétrabutylammmonium dans le tétrahydrofuranne. Après 20 minutes d'agitation à froid puis 3 heures à 20°C, le mélange réactionnel est coulé dans un mélange de 200 cm³ d'eau et de 100 cm³ d'acétate d'éthyle, puis décanté. La phase organique est lavée avec de l'eau, séchée sur sulfate de magnésium, puis filtrée et concentrée à sec sous pression réduite (2,7 kPa). Le résidu obtenu est purifié par chromatographie sur colonne de gel de silice (granulométrie 0,04-0,06 mm, diamètre 2 cm, hauteur 30 cm) en éluant sous une pression de 0,5 bar d'azote avec un mélange dichlorométhane et éthanol (97/3 en volumes) et en recueillant des fractions de 100 cm³. Les fractions 10 à 14 sont réunies, concentrées à sec sous pression réduite (2,7 kPa). Le solide jaune obtenu est repris par 2 cm³ de dichlorométhane et 10 cm³ d'acétate d'éthyle puis filtré sur verre fritté et lavé par 2 cm³ d'acétate d'éthyle. On obtient 1,6 g de [3-({1-[bis-(4-chlorophényl)méthyl]azétidin-3-yl}méthylsulfonyl-méthylène) phényl]méthanol, sous la forme d'un solide blanc fondant à 214°C.

Le 1-[bis(4-chlorophényl)méthyl]-3-{[3-(tert-butyldiméthylsilyloxyméthyl)phényl]méthylsulfonyl-méthylène}azétidine peut être préparé en opérant selon le mode opératoire de l'exemple 1, à partir de 10,8 g de 1-[bis(4-chlorophényl)méthyl]-3-{[3-(tert-butyldiméthylsilyloxyméthyl)phényl]méthylsulfonyméthyl-(RS)}azétidin-3-ol, de 2 cm³ de chlorure de méthylsulfonyle et 8,5 g de 4-diméthylaminopyridine, le résidu obtenu est purifié par chromatographie sur colonne de gel de silice (granulométrie 0,04-0,06 mm, diamètre 4 cm, hauteur 40 cm) sous une pression de 0,5 bar d'azote avec du dichlorométhane comme éluant et en recueillant des fractions de 100 cm³. Les fractions 12 à 29 sont réunies, concentrées à sec sous pression réduite (2,7 kPa). On obtient 5,2 g de 1-[bis(4-chlorophényl)méthyl]-3-{[3-(tert-butyldiméthylsilyloxyméthyl)phényl]méthylsulfonyl-méthylène}azétidine sous la forme d'une gomme.

Le 1-[bis(4-chlorophényl)méthyl]-3-{[3-(tert-butyldiméthylsilyloxyméthyl) phényl] méthylsulfonyl-méthyl-(RS)}azétidin-3-ol peut être préparé en opérant selon le mode opératoire de l'exemple 5, à partir de 5,8 g de tert-butyl-(3-méthylsulfonylméthyl-benzyloxy)diméthyl-silane et de 5,6 g de 1-[bis(4-chlorophényl)méthyl]azétidin-3-one, on obtient 10,8 g de 1-[bis(4-chlorophényl)méthyl]-3-{[3-(tert-butyldiméthylsilyloxyméthyl)phényl]méthylsulfonyl-méthyl-(RS)}azétidin-3-ol sous forme d'une gomme.

La tert-butyl-(3-méthylsulfonylméthyl-benzyloxy)diméthyl-silane peut être préparée en opérant de la façon suivante : A une solution de 5,73 g de (3-méthylsulfonylméthyl-phényl)méthanol dans 50 cm³ de N,N-diméthylformamide, on ajoute 4,87 g d'imidazole puis 10,3 cm³ de tert-butylchlorodiméthylsilane. Après 20 heures d'agitation à température ordinaire, le mélange réactionnel est concentré à sec sous pression réduite (2,7 kPa). Le résidu est chromatographié sur une colonne de gel de silice (granulométrie 0,06-0,200 mm, diamètre 3,5 cm, poids de silice 100 g), en éluant sous une pression de 0,5 bar d'azote avec du dichlorométhane, et en recueillant des fractions de 100 cm³. Les fractions 2 à 7 sont réunies, concentrées à sec sous pression réduite (2,7 kPa). On obtient 5,8 g d'une huile qui cristallise à température ordinaire. PF = 75°C.

Le (3-méthylsulfonylméthyl-phényl)méthanol peut être préparé en opérant de la façon suivante : on agite 18 heures à une température proche de 20°C un mélange de 26 g d'acide 3-(méthylsulfonylméthyl)benzoïque et 4,6 g d'hydrure de lithium et d'aluminium dans 600 cm³ de tétrahydrofuranne. La solution est refroidie à 0°C puis on ajoute successivement 15 cm³ d'acétate d'éthyle, 5 cm³ d'eau, 5 cm³ d'une solution aqueuse à 15% de soude et enfin 30 cm³ d'eau. Le mélange est filtré sur célite, le filtrat repris par 600 cm³ d'acétate d'éthyle. La phase organique est reprise par 500 cm³ d'eau puis 200 cm³ d'une solution aqueuse saturée par du chlorure de sodium, décantée, séchée sur du sulfate de magnésium anhydre, filtrée et concentrée à sec sous pression réduite (2,7 kPa). On obtient 10,4 g de (3-méthylsulfonylméthyl-phényl)méthanol, sous la forme d'une gomme.

L'acide 3-(méthylsulfonylméthyl)benzoïque peut être préparé de la manière suivante : en opérant selon le mode opératoire de l'exemple 14 à partir de 23,3 g d'acide 3-chlorométhylbenzoïque et 23,3 g de méthanesulfinate de sodium, on obtient 26 g d'acide 3-(méthylsulfonylméthyl)benzoïque sous forme d'un solide blanc fondant à 210°C.

### Exemple 18

La 1-[bis-(4-chlorophényl)méthyl]-3-(phénylsulfonylméthyl)-azétidine peut être préparée en opérant de la façon suivante : A une solution de 0,15 g de 1-[bis-(4-chlorophényl)méthyl)]-3-(phénylsulfonylméthylène)-azétidine dans 3 cm³ d'éthanol anhydre et 3,5 cm³ de dichlorométhane anhydre, on ajoute sous argon 13 mg de borohydrure de sodium. Après 1 heure et 45 minutes d'agitation, on ajoute à nouveau 14 mg de borohydrure de sodium puis laisse agiter pendant 20 heures à 20°C. Le mélange réactionnel est ensuite chauffé à 50°C, additionné de 9,5 mg d'hydroborure de sodium et laissé agité pendant 2 heures et 30 minutes à 50°C puis refroidi à température ordinaire. On ajoute alors au mélange 0,5 cm³ d'eau, 10 cm³ de dichlorométhane puis 50 mg de sulfate de magnésium, et filtre puis évapore à sec sous pression réduite (2,7 kPa). Le résidu est chromatographié sur une colonne de gel de silice (granulométrie 0,063-0,200 mm, hauteur 15 cm, diamètre 1 cm), en éluant sous une pression de 0,5 bar d'argon avec un mélange de cyclohexane et d'acétate d'éthyle (80/20 en volumes) et en recueillant des fractions de 5 cm³. Les fractions 12 à 19 sont réunies et concentrées à sec sous pression réduite (2,7 kPa). On obtient 29 mg de 1-[bis-(4-chlorophényl)méthyl)]-3-(phénylsulfonylméthyl)-azétidine, sous la forme d'un solide blanc [Spectre de R.M.N ¹H (300 MHz, CDCl₃, δ en ppm) : de 2,75 à 8,90 (mt : 3H); 3,32 (mt : 2H); 3,37 (d, J = 7 Hz : 2H); 4,22 (s : 1H); de 7,20 à 7,30 (mt : 8H); 7,57 (t large, J = 7,5 Hz : 2H); 7,67 (tt, J = 7,5 et 1,5 Hz : 1H); 7,88 (d large, J = 7,5 Hz : 2H)].

### Exemple 19

La 1-[bis-(4-chlorophényl)méthyl)]-3-(phénylsulfonylméthylène)-azétidine peut être préparée en opérant de la façon suivante : A une solution refroidie à - 70°C, sous argon, de 4,34 g de (phénylsulfonylméthyl)triméthylsilane dans 40 cm³ de diméthyléther, on coule en 5 minutes 12 cm³ d'une solution 1,6M de n-butyllithium dans l'hexane. Après 30 minutes d'agitation du mélange à-60°C, on coule en 10 minutes une solution de 1-[bis-(4-chlorophényl)méthyl]azétidin-3-one dans 30 cm³ de diméthyléther [produit sous la forme de base préparée en traitant 7,35 g du bromhydrate de 1-[bis-(4-chlorophényl)méthyl]azétidin-3-one dissous dans 30 cm³ d'eau, par 25 cm³ d'hydroxyde de sodium 1 N et extraction de la base obtenue par 30 cm³ d'oxyde de diéthyle, puis séchage et concentration à sec sous pression réduite (2,7 kPa)]. Après 45 minutes d'agitation à -70°C, puis 2 heures à 20°C, le mélange réactionnel est additionné de 12 cm³. d'une solution aqueuse saturée de chlorure d'ammonium, de 20 cm³ d'eau, puis extrait par 2 fois 40 cm³ d'acétate d'éthyle. Les phases organiques réunies sont par 40 cm³ d'eau, séchées sur sulfate de magnésium puis concentrées à sec sous pression réduite (2,7 kPa). L'huile obtenue est chromatographiée sur une colonne de gel de silice (granulométrie 0,063-0,200 mm, hauteur 40 cm, diamètre 5 cm), en éluant sous une pression de 0,5 bar d'argon avec un mélange de cyclohexane et d'acétate d'éthyle (90/10 puis 85/15 en volumes) et en recueillant des fractions de 100 cm³. Les fractions 10 à 16 sont réunies et concentrées à sec sous pression réduite (2,7 kPa). L'huile obtenue est triturée dans 10 cm³ d'oxyde de diéthyle, la suspension filtrée, et le solide séché. On obtient 1,17 g de 1-[bis-(4-chlorophényl)méthyl)]-3-(phénylsulfonylméthytène)-azétidine, sous la forme d'un solide blanc [Spectre de R.M.N ¹H (300 Hz, CDCl₃, d en ppm) : 3,88 (mt : 2H); 4,29 (mt : 2H); 4,50 (s : 1H); 6,17 (mt : 1H); de 7,20 à 7,40 (mt : 8H); 7,56 (t large, J = 7,5 Hz : 2H); 7,64 (tt, J = 7,5 et 1,5 Hz : 1H); 7,87 (d large, J = 7,5 Hz : 2H)].

Le (phénylsulfonylméthyl)triméthylsilane peut être préparé en opérant de la façon suivante : A une solution refroidie à -70°C de 3 g de méthyl phényl sulfone dans 40 cm³ de tétrahydrofuranne anhydre, on coule en agitant sous argon en 20 minutes 13 cm³ d'une solution 1,6M de n-butyllithium dans l'hexane. Après 30 minutes d'agitation à-70°C, on ajoute au mélange 2,66 cm³ de triméthylchlorosilane, et arrête le chauffage. Après 4 heures d'agitation à température ordinaire le mélange réactionnel est additionné de 30 cm³ d'eau et extraite par 30 cm³ d'acétate d'éthyle. La phase organique est lavée par 30 cm³ d'eau, séchée sur sulfate de magnésium, filtrée puis concentrée à sec sous pression réduite (2, 7 kPa). On obtient 4,34 g de (phénylsulfonylméthyl)triméthylsilane, sous la forme d'un liquide jaune.

### Exemple 20

La 2-{1-[bis-(4-chlorophényl)méthyl]-azétidin-3-ylméthylsulfonyl}pyridine peut être préparée en opérant de la façon suivante : A une solution de 0,25 g de 2-{1-[bis-(4-chlorophényl)méthyl)]-azétidin-3-ylidèneméthylsulfonyl}pyridine dans 20 cm³ d'un mélange 50/50 de dichlorométhane et d'éthanol, on ajoute 0,125 g d'hydroborure de sodium. Après 1 heure d'agitation à 50°C, le mélange réactionnel est refroidi à 20°C, additionné de 20 cm³ de dichlorométhane, de 1 cm³ d'eau et de 0,1 g de sulfate de magnésium. Le mélange est filtré, et le filtrat est concentré à 50°C sous pression réduite (2,7 kPa). Le résidu est chromatographié sur une colonne de gel de silice (granulométrie 0,040 -0,063 mm, hauteur 15 cm, diamètre 1 cm), en éluant sous une pression de 0,5 bar d'argon avec un mélange de cyclohexane et d'acétate d'éthyle (40/60 en volumes) et en recueillant des fractions de 10 cm³. Les fractions 5 à 10 sont réunies et concentrées à sec sous pression ; réduite (2,7 kPa). On obtient 0,18 g de 2-{1-[bis-(4-chlorophényl)méthyl]-azétidin-3-ylméthylsulfonyl}pyridine, sous la forme d'une poudre blanche [Spectre de R.M.N ¹H (300 MHz, CDCl₃, δ en ppm) : de 2,80 à 3,00 (mt : 3H); 3,34 (mt : 2H); 3,70 (d, J = 7 Hz : 2H); 4,25 (s : 1H); de 7,20 à 7,40 (mt : 8H); 7,57 (ddd, J = 8-5 et 1 Hz : 1H); 7,97 (t dédoublé, J = 8 et 1,5 Hz : 1H); 8,07 (d large, J = 8 Hz : 1H); 8,75 (d large, J = 5 Hz : 1H)].

La 2-{1-[bis-(4-chlorophényl)méthyl)]-azétidin-3-ylidèneméthylsuifonyl} pyridine peut être préparée en opérant de la façon suivante : A une solution de 0,9 g de 1-[bis-(4-chlorophényl)méthyl)]-3-(-pyrid-2-yl-sulfonylméthyl) azétidin-3-ol dans 50 cm³ de dichlorométhane, on ajoute 0,25 cm³ de chlorure de méthylsulfonyle, agite 15 minutes et ajoute 0,9 g de 4-diméthylaminopyridine. Après 3 heures d'agitation à 20°C, on ajoute au mélange 30 cm³ d'eau, et 30 cm³ de dichlorométhane, puis la phase organique est décantée, séchée sur sulfate de magnésium, filtrée et concentrée à sec sous pression réduite (2,7 kPa). Le résidu est chromatographié sur une colonne de gel de silice (granulométrie 0,04-0,063 mm, hauteur 25 cm, diamètre 2 cm), en éluant sous une pression de 0,5 bar d'argon avec un mélange de cyclohexane et d'acétate d'éthyle (40/60 en volumes) et en recueillant des fractions de 20 cm³. Les fractions 4 à 8 sont réunies et concentrées à sec sous pression réduite (2,7 kPa). On obtient 0,50 g de 2-{-[bis-(4-chlorophényl)méthyl)]azétidin-3-ylidèneméthylsulfonyl}pyridine, sous la forme d'une poudre jaune.

Le 1-[bis-(4-chlorophényl)méthyl)]-3-(-pyrid-2-yl-sulfonylméthyl) azétidin-3-ol peut être préparé en opérant de la façon suivante : A une solution refroidie à -78°C, sous argon de 2,92 g de 1-[bis-(4-chlorophényl)méthyl)]azétidin-3-one et de 3 g de 2-méthylsulfonyl-pyridine dans 50 cm³ de tétrahydrofuranne, on ajoute 2,13 g de tertiobutylate de potassium. Après 3 heures d'agitation à -78°C, on laisse le mélange réactionnel revenir à 0°C, puis on ajoute 50 cm³ d'oxyde de diéthyle, 10 cm³ d'eau et 10 cm³ de solution aqueuse saturée de chlorure d'ammonium. La phase organique est décantée, séchée sur sulfate de magnésium, filtrée, et concentrée à sec sous pression réduite (2,7 kPa). Le résidu est chromatographié sur une colonne de gel de silice (granulométrie 0,063-0,200 mm, hauteur 30 cm, diamètre 3 cm), en éluant sous une pression de 0,5 bar d'argon d'abord avec du dichlorométhane, puis avec un mélange de dichlorométhane et de méthanol (97/3 en volumes) et en recueillant des fractions de 20 cm³. Les fractions 8 à 15 sont réunies et concentrées à sec sous pression réduite (2,7 kPa). On obtient une huile brune encore impure que l'on chromatographie sur colonne de gel de silice (granulométrie 0,04-0,063 mm, hauteur 20 cm, diamètre 2 cm), en éluant sous une pression de 0,5 bar d'argon d'abord avec du dichlorométhane, puis avec un mélange de dichlorométhane et de méthanol (97/3 en volumes) et en recueillant des fractions de 20 cm³. Les fractions 5 à 25 sont réunies et concentrées à sec et le résidu obtenu est à nouveau chromatographié sur une même colonne, et dans les mêmes conditions mais en éluant avec du dichlorométhane. Les fractions 12 à 20 sont réunies et concentrées à sec sous pression réduite (2,7 kPa). On obtient 0,3 g de 1-[bis-(4-chlorophényl)méthyl)]-3-(-pyrid-2-yl-sulfonylméthyl) azétidin-3-ol, sous la forme d'une meringue blanche.

La 2-méthylsulfonyl-pyridine peut être préparée en opérant de la façon suivante : A une solution de 20 g de tungstate de sodium dihydrate, dans 10 cm³ d'eau, on ajoute en agitant sous argon 0,25 cm³ d'acide acétique à 100%, puis 7,0 g de 2-méthylsulfanyl-pyridine. On chauffe ce mélange à 65°C, coule lentement en 15 minutes 10 cm³ d'eau oxygénée à 30%, agite ensuite à 85°C pendant 30 minutes puis refroidit le mélange à +10°C. On ajoute au milieu 1,0 cm³ d'ammoniaque à 32%, 5,0 cm³ d'une solution aqueuse à 37,5% d'hydrogénosulfite de sodium, puis 10 cm³ d'eau et 50 cm³ de dichlorométhane. Le mélange est décanté, la phase organique séchée sur sulfate de magnésium, filtrée et concentrée à sec sous pression réduite (2,7 kPa). L'huile incolore obtenue est délitée avec 50 cm³ d'éther de pétrole et la gomme insoluble est filtrée et reprise par 30 cm³ de dichlorométhane. La solution obtenue est concentrée à sec à 50°C sous pression réduite (2,7 kPa). On obtient 3,5 g de 2-méthylsulfonyl-pyridine, sous la forme d'une huile incolore.

La 2-méthylsulfanyl-pyridine peut être préparée en opérant de la façon suivante: A une solution de 11,0 g de 2-mercaptopyridine dans 105 cm³ d'hydroxyde de sodium 1 N, on ajoute lentement 6,2 cm³ d'iodure de méthyle. Le mélange réactionnel dont la température s'est élevée à 30°C, est refroidi à température ordinaire. Après 2 heures d'agitation, le mélange est extrait par 100 cm³ de dichlorométhane, la phase organique est séchée sur sulfate de magnésium, et concentrée à sec à 50°C sous pression réduite (2,7 kPa). L'huile obtenue, est purifiée par distillation sous pression réduite. On obtient 9,0 g de 2-méthylsulfanyl-pyridine, sous la forme d'un liquide incolore, pE = 84°C/45 mm Hg.

### Exemple 21

La 3-{1-[bis-(4-chlorophényl)méthyl]-azétidin-3-ylméthylsulfonyl}pyridine peut être préparée en opérant comme il est décrit dans l'exemple 20, à partir de 0,15 g de 3-{1-[bis-(4-chlorophényl)méthyl)]-azétidin-3-ylidèneméthylsuifonyl}pyridine, de 20 cm³ d'un mélange 50/50 de dichlorométhane et d'éthanol, et de 80 mg d'hydroborure de sodium. On obtient 0,11 g de 3-{1-[bis-(4-chlorophényl)méthyl]-azétidin-3-ylméthylsulfonyl}pyridine, sous la forme d'une poudre blanche [Spectre de R.M.N ¹H (300 MHz, CDCl₃, δ en ppm) : de 2,75 à 2,95 (mt : 3H); 3,35 (mt : 2H); 3,43 (d, J = 6,5 Hz : 2H); 4,25 (s : 1H); de 7,20 à 7,40 (mt : 8H); 7,54 (ddd, J = 8-5 et 1 Hz : 1H); 8,18 (ddd, J = 8-2,5 et 1,5 Hz : 1H); 8,90 (dd, J = 5 et 1,5 Hz : 1H); 9,11 (dd, J = 2,5 et 1 Hz : 1 H)].

La 3-{1-[bis-(4-chlorophényl)méthyl)]-azétidin-3-ylidèneméthylsulfonyl} pyridine peut être préparée en opérant comme il est décrit dans l'exemple 20, à partir de 0,8 g de 1-[bis-(4-chlorophényl)méthyl)]-3-(-pyrid-3-yl-sulfonylméthyl) azétidin-3-ol, de 50 cm³ de dichlorométhane, de 0,22 cm³ de chlorure de méthylsulfonyle, et de 0,8 g de 4-diméthylaminopyridine. On obtient 0,50 g de 3-{1-[bis-(4-chlorophényl)méthyl)]-azétidin-3-ylidèneméthylsulfonyl}pyridine, sous la forme d'une poudre crème.

Le 1-[bis-(4-chlorophényl)méthyl)]-3-(-pyrid-3-yl-sulfonylméthyl) azétidin-3-ol, peut être préparé en opérant comme il est décrit dans l'exemple 20, à partir de 3,3 g de 1-[bis-(4-chlorophényl)méthyl)]azétidin-3-one, de 50 cm³ de tétrahydrofuranne, de 3,5 g de 3-méthylsulfonyl-pyridine, et de 2,4 g de tertiobutylate de potassium. On obtient 1,4 g de 1-[bis-(4-chlorophényl)méthyl)]-3-(-pyrid-3-yl-sulfonylméthyl) azétidin-3-ol, sous la forme d'une poudre blanche.

La 3-méthylsulfonyl-pyridine peut être préparée en opérant comme il est décrit dans l'exemple 20, à partir de 33 g de tungstate de sodium, de 10 cm³ d'eau, de 0,25 cm³ d'acide acétique à 100%, de 9,5 g de 3-méthylsulfanyl-pyridine, de 15 cm³ d'eau oxygénée à 30% puis de 2 cm³ d'ammoniaque à 32% et de 2 cm³ de solution aqueuse à 37,5% d'hydrogénosulfite de sodium. L'huile brute obtenue est cristallisée avec 20 cm³ d'oxyde de diisopropyle, les cristaux sont filtrés, essorés, et séchés sous pression réduite (2,7 kPa). On obtient 4,5 g de 3-méthylsulfonyl-pyridine, sous la forme de cristaux blancs. PF = 58°C.

La 3-méthylsulfanyl-pyridine peut être préparée en opérant de la façon suivante : A un mélange chauffé à 80°C, sous argon, de 9,4 g de 3-aminopyridine et de 100 cm³ de diméthyldisulfure, on ajoute 20 cm³ de nitrite d'isoamyle. Après 2 heures d'agitation à 90°C, le mélange réactionnel est refroidi à 20°C, puis purifié par distillation fractionnée sous pression réduite.

On obtient 8,4 g de 3-méthylsulfanyl-pyridine, sous la forme d'un liquide jaune pâle, PE = 90°C/30 mm de mercure.

### Exemple 22

La 4-{1-[bis-(4-chlorophényl)méthyl]-azétidin-3-ylméthylsulfonyl}pyridine peut être préparée en opérant comme il est décrit dans l'exemple 20, à partir de 0,15 g de 4-{1-[bis-(4-chlorophényl)méthyl)]-azétidin-3-ylidèneméthylsulfonyl} pyridine, 20 cm³ d'un mélange 50/50 de dichlorométhane et d'éthanol, et de 80 mg d'hydroborure de sodium. On obtient 0,13 g de 4-{1-[bis-(4-chlorophényl)méthyl]-azétidin-3-ylméthylsulfonyl}pyridine, sous la forme d'une poudre blanche [Spectre de R.M.N ¹H (300 MHz, CDCl₃, δ en ppm) : de 2,75 à 2,90 (mt : 1 H); 2,88 (t, J = 7 Hz : 2H); 3,36 (t, J = 7 Hz : 2H); 3,42 (d, J = 7 Hz : 2H); 4,25 (s : 1H); de 7,20 à 7,35 (mt : 8H); 7,75 (d large, J = 6 Hz : 2H); 8,93 (d large, J = 6 Hz : 2H)].

La 4-{1-[bis-(4-chlorophényl)méthyl)]-azétidin-3-ylidèneméthylsulfonyl} pyridine peut être préparée en opérant comme il est décrit dans l'exemple 20, à partir de 0,8 g de 1-[bis-(4-chlorophényl)méthyl)]-3-(-pyrid-4-yl-sulfonylméthyl) azétidin-3-ol, de 50 cm³ de dichlorométhane, de 0,22 cm³ de chlorure de méthylsulfonyle, et de 0,8 g de 4-diméthylaminopyridine. Le produit brut est purifié par chromatographie sur une colonne de gel de silice (granulométrie 0,04-0,063 mm, hauteur 20 cm, diamètre 2 cm), en éluant sous une pression de 0,5 bar d'argon avec un mélange de cyclohexane et d'acétate d'éthyle (40/60 en volumes) et en recueillant des fractions de 20 cm³. Les fractions 5 à 10 sont réunies et concentrées à sec sous pression réduite (2,7 kPa). On obtient 0,42 g de 4-{1-[bis-(4-chlorophényl)méthyl)]-azétidin-3-ylidèneméthylsulfonyl}pyridine, sous la forme d'une poudre cristalline blanche.

Le 1-[bis-(4-chlorophényl)méthyl)]-3-(-pyrid-4-yl-sulfonylméthyl) azétidin-3-ol, peut être préparé en opérant comme il est décrit dans l'exemple 20, à partir de 2,5 g de 1-[bis-(4-chlorophényl)méthyl)]azétidin-3-one, de 50 cm³ de tétrahydrofuranne, de 2,6 g de 4-méthylsulfonyl-pyridine, et de 1,8 g de tertiobutylate de potassium. Le produit brut obtenu est purifié par chromatographie sur une colonne de gel de silice (granulométrie 0,04-0,063 mm, hauteur 30 cm, diamètre 3 cm), en éluant sous une pression de 0,5 bar d'argon avec du dichlorométhane puis avec un mélange de dichlorométhane et de méthanol (98/2 en volumes) et en recueillant des fractions de 20 cm³. Les fractions 8 à 27 sont réunies et concentrées à sec sous pression réduite (2,7 kPa). On obtient une poudre crème que l'on recristallise dans 5 cm³ d'acétonitrile. Les cristaux sont filtrés, essorés, et séchés sous pression réduite (2,7 kPa). On obtient 1,4 g de 1-[bis-(4-chlorophényl)méthyl)]-3-(-pyrid-4-yl-sulfonylméthyl)azétidin-3-ol, sous la forme de cristaux blancs PF = 130°C

La 4-méthylsulfonyl-pyridine peut être préparée en opérant comme il est décrit dans l'exemple 20, à partir de 14 g de tungstate de sodium, de 4 cm³ d'eau, de 0,05 cm³ d'acide acétique à 100%, de 3,3 g de 4-méthylsulfanyl-pyridine, de 6,5 cm³ d'eau oxygénée à 30% puis de 0,25 cm³ d'ammoniaque à 32% et de 1 cm³ de solution aqueuse à 37,5% d'hydrogénosulfite de sodium. L'huile brute obtenue est cristallisée avec 10 cm³ d'oxyde de diisopropyle, les cristaux sont filtrés, essorés, et séchés sous pression réduite (2,7 kPa). On obtient 2,6 g de 4-méthylsulfonyl-pyridine, sous la forme de cristaux blancs.

La 4-méthylsulfanyl-pyridine peut être préparée en opérant comme il est décrit dans l'exemple 20, à partir de 11,0 g de 4-mercaptopyridine, de 105 cm³ d'hydroxyde de sodium 1N, et de 6,2 cm³ d'iodure de méthyle. L'huile brute obtenue, est purifiée par distillation sous pression réduite. On obtient 4,0 g de 4-méthylsulfanyl-pyridine, sous la forme d'une pâte blanche, pEb = 120°C/45 mm de mercure.

### Exemple 23

La 1-[bis-(4-chlorophényl)méthyl]-3-[(3,5-difluorophényl)sulfonylméthyl] azétidine peut être préparée en opérant de la façon suivante : A une solution de 0,50 g de 1-[bis-(4-chlorophényl)méthyl]-3-[(3,5-difluorophényl) sulfonylméthylène]azétidine dissous dans 25 cm³ de méthanol anhydre et 25 cm³ de dichlorométhane anhydre, on ajoute sous argon 78 mg de borohydrure de sodium. Après 24 heures d'agitation, on ajoute 80 cm³ d'eau et 50 cm³ de dichlorométhane, décante, lave avec 80 cm³ d'eau puis 80 cm³ d'une solution aqueuse saturée de chlorure de sodium. La phase organique est séchée avec du sulfate de magnésium, filtrée puis évaporée à sec sous pression réduite (2,7 kPa). Le résidu est chromatographié sur une colonne de gel de silice (granulométrie 0,02-0,04 mm, hauteur 20 cm, diamètre 14 cm), en éluant sous une pression de 0,7 bar d'argon avec un mélange de cyclohexane et d'acétate d'éthyle (90/10 en volumes) et en recueillant des fractions de 5 cm³. Les fractions 60 à 82 sont réunies et concentrées à sec sous pression réduite (2,7 kPa). On obtient 0,29 g de 1-[bis-(4-chlorophényl)méthyl]-3-[(3,5-difluorophényl)sulfonylméthyl]azétidine sous la forme d'un solide blanc [Spectre de R.M.N. ¹H (300 MHz, CDCl₃, δ en ppm) : de 2,75 à 2,95 (mt : 1H); 2,88 (t, J = 7 Hz : 2H); 3,36 (t, J = 7 Hz : 2H); 3,41 (d, J = 7 Hz : 2H); 4,26 (s : 1H); 7,13 (tt, J = 9 et 2,5 Hz : 1H); de 7,20 à 7,35 (mt : 8H); 7,44 (mt : 2H)].

### Exemple 24

La 1-[bis-(4-chlorophényl)méthyl]-3-[(3,5-difluorophényl)sulfonylméthylène] azétidine peut être préparée en opérant de la façon suivante : à 18,8 g de 1-[bis-(4-chlorophényl)méthyl]-3-[(3,5-difluorophényl)sulfonylméthyl]azétidin-3-ol dissous dans 800 cm³ de dichlorométhane à température ambiante, on ajoute 4,3 cm³ de chlorure de méthylsulfonyle puis, par petites portions, 16 g de 4-diméthylamino pyridine. Après 22 heures, le mélange réactionnel est lavé avec 3 fois 700 cm³ d'eau puis 700 cm³ d'une solution aqueuse saturée de chlorure de sodium. La phase organique est séchée avec du sulfate de magnésium, filtrée puis évaporée à sec sous pression réduite (2,7 kPa). Le résidu (25 g) est chromatographié sur une colonne de gel de silice (granulométrie 0,02-0,04 mm, hauteur 36 cm, diamètre 8,5 cm), en éluant sous une pression de 0,7 bar d'argon avec un mélange de cyclohexane et d'acétate d'éthyle (90/10 en volumes) et en recueillant des fractions de 250 cm³. Les fractions 2 à 148 sont réunies et concentrées à sec sous pression réduite (2,7 kPa). On obtient 2,79 g de 1-[bis-(4-chlorophényl)méthyl]-3-[(3,5-difluorophényl)sulfonylméthylène]azétidine sous la forme d'un solide blanc [Spectre de R.M.N. ¹H (300 MHz, CDCl₃, δ en ppm) : 3,91 (mt : 2H); 4,28 (mt : 2H); 4,51 (s : 1H); 6,15 (mt : 1H); 7,08 (tt, J = 9 et 2,5 Hz : 1H); de 7,25 à 7,40 (mt : 8H); 7,40 (mt : 2H)].

Le 1-[bis-(4-chlorophényl)méthyl]-3-[(3,5-difluorophényl)sulfonylméthyl] azétidin-3-ol peut être préparé en opérant de la façon suivante : à une solution de 13,2 g de (3,5-difluorophényl)méthylsulfone dans 800 cm³ de tétrahydrofuranne, on additionne goutte à goutte 42,9 cm³ de butyllithium 1,6M dans l'hexane. Après 0,5 heure à -70°C et 0,5 heure à -30°C, on additionne goutte à goutte à -70°C, 14 g de 1-[bis(4-chlorophényl)méthyl]azétidin-3-one dissous dans 150 cm³ de tétrahydrofuranne. Après 3 heures à -70°C, le mélange réactionnel est versé sur une solution saturée de chlorure d'ammonium et extrait avec de l'acétate d'éthyle. La phase organique est lavée 2 fois avec 400 cm³ d'eau puis 400 cm³ d'une solution aqueuse saturée de chlorure de sodium, séchée avec du sulfate de magnésium, filtrée puis évaporée à sec sous pression réduite (2,7 kPa). Le résidu (25,14 g) est chromatographié sur une colonne de gel de silice (granulométrie 0,06-0,04 mm, hauteur 31 cm, diamètre 7,5 cm), en éluant sous une pression de 0,5 bar d'argon avec un mélange de cyclohexane et d'acétate d'éthyle (85/15 en volumes) et en recueillant des fractions de 200 cm³. Les fractions 13 à 16 sont réunies et concentrées à sec sous pression réduite (2,7 kPa). Après cristallisation dans l'éther éthylique, filtration et séchage, on obtient 4,5 g de 1-[bis-(4-chlorophényl)méthyl]-3-[(3,5-difluorophényl)sulfonylméthyl]azétidin-3-ol sous la forme d'un solide blanc.

La (3,5-difluorophényl)méthylsulfone peut être préparée en opérant de la façon suivante : à une solution de 13,3 g de (3,5-difluorophényl)méthylsulfure dissous dans 450 cm³ de méthanol, on ajoute 225 cm³ d'eau et, par petites quantités à 5°C, 56,3 g d'oxone^{R}. Après 20 heures à température ambiante, le mélange réactionnel est dilué avec du dichlorométhane et de l'eau et décanté. La phase organique est lavée 2 fois avec 700 cm³ d'eau puis 700 cm³ d'une solution aqueuse saturée de chlorure de sodium, séchée avec du sulfate de magnésium, filtrée puis évaporée à sec sous pression réduite (2,7 kPa). On obtient 13,2 g de (3,5-difluorophényl)méthylsulfone sous la forme d'un solide blanc.

Le (3,5-difluorophényl)méthylsulfure peut être préparé en opérant de la façon suivante : à 11,8 cm³ de 1-bromo-3,5-difluorobenzène dilué dans 200 cm³ d'éther éthylique, on ajoute goutte à goutte à -70°C goutte 64 cm³ de n-butyllithium 1,6M dans l'hexane. Après 0,5 heure à -70°C, on additionne goutte à goutte à -70°C, 14,2 g de méthylthiosulfonate de S-méthyle dissous dans 60 cm³ de tétrahydrofuranne. Après 3 heures à -70°C puis 18 heures à température ambiante, le mélange réactionnel est versé sur une solution saturée de chlorure d'ammonium et extrait avec de l'acétate d'éthyle. La phase organique est lavée 2 fois avec 300 cm³ d'eau puis 300 cm³ d'une solution aqueuse saturée de chlorure de sodium, séchée avec du sulfate de magnésium, filtrée puis évaporée à sec sous pression réduite (2,7 kPa). On obtient 13,3 g de (3,5-difluorophényl)méthylsulfure sous la forme d'une huile jaune.

### Exemple 25

A une solution de 0,40 g de 1-[bis-(4-chlorophényl)-méthyl]-3-(phénylsulfanyl)-azétidine dans 20 cm³ de dichlorométhane, on ajoute à température ambiante 0,25 g d'acide métachloroperbenzoique. Après 3 heures d'agitation à température ambiante, le mélange réactionnel est lavé par 30 cm³ d'une solution saturée d'hydrogénocarbonate de sodium, séché sur sulfate de magnésium, filtré et concentré à sec sous pression réduite (2,7 kPa). Après chromatographie sur une colonne de gel de silice (granulométrie 0,06-0,200 mm, hauteur 25 cm, diamètre 2 cm), en éluant sous une pression de 0,8 bar d'argon avec un mélange acétate d'éthyle/cyclohexane 20/80 en volume et en recueillant des fractions de 60 cm³, les fractions 9 à 16 sont réunies et concentrées à sec sous pression réduite (2,7 kPa), reprises dans l'heptane pour isoler 100 mg de 1-[bis-(4-chlorophényl)-méthyl]-3-[(RS)-phénylsulfinyl]-azétidine sous la forme d'un solide blanc [Spectre de R.M.N. ¹H (300 MHz, CDCl₃, δ en ppm) : 3,01 (t large, J = 7,5 Hz : 1H); 3,32 (t large, J = 7,5 Hz : 1H); 3,45 (t large, J = 7,5 Hz : 2H); 3,59 (mt : 1H); 4,45 (s large : 1H); de 7,15 à 7,65 (mts : 13H)].

### Exemple 26

A une solution de 0,80 g de 1-[bis-(4-chlorophényl)-méthyl]-3-(phénylsulfanyl)-azétidine dans 3,4 cm³ d'eau, 3,4 cm³ d'acide acétique, 3,4 cm³ d'éthanol et 1,7 cm³ d'acide sulfurique, on ajoute en plusieurs fois 1,2 g d'oxone^{R}. Après 20 heures d'agitation à température ambiante, le mélange réactionnel est dilué avec 100 cm³ de dichlorométhane, lavé par 3 fois 100 cm³ d'eau, séché sur sulfate de magnésium, filtré et concentré à sec sous pression réduite (2,7 kPa). Après chromatographie sur une colonne de gel de silice (granulométrie 0,06-0,200 mm, hauteur 40 cm, diamètre 2 cm), en éluant sous une pression de 0,8 bar d'argon avec un mélange acétate d'éthyle/cyclohexane 20/80 en volume et en recueillant des fractions de 60 cm³, les fractions 9 à 15 sont réunies et concentrées à sec sous pression réduite (2,7 kPa), reprises dans l'heptane, le solide filtré et séché pour isoler 0,23 g de 1-[bis-(4-chlorophényl)-méthyl]-3-(phénylsulfonyl)-azétidine sous la forme d'un solide blanc [Spectre de R.M.N. ¹H (300 MHz, CDCl₃, δ en ppm) : de 3,35 à 3,50 (mt : 4H); 3,96 (mt : 1H); 4,44 (s : 1H); de 7,20 à 7,35 (mt : 8H); 7,57 (t large, J = 7,5 Hz : 2H); 7,68 (tt, J = 7,5 et 1,5 Hz : 1H); 7,88 (d large, J = 7,5 Hz : 2H)].

### Exemple 27

A une solution de 0,6 g de 5-({1-[bis(4-chlorophényl)méthyl]azétidin-3-ylidène}-méthylsulfonyl-méthyl)-thièn-2-yl-carboxylate de méthyle dans 70 cm³ de méthanol refroidie vers 0°C on ajoute 43,5 mg de borohydrure de sodium. Le milieu réactionnel est agité 15 minutes à cette température, puis 5 heures à 20°C avant d'être à nouveau refroidi vers 0°C et additionné de 8,7 mg de borohydrure de sodium. Après 18 heures à température ambiante, le mélange réactionnel est concentré à sec sous pression réduite (2,7 kPa). Le résidu obtenu est additionné de 100 cm³ de dichlorométhane et 20 cm³ d'eau distillée. Le mélange est décanté, la phase organique lavée avec deux fois 20 cm³ d'eau distillée, séchée sur sulfate de magnésium, filtrée et concentrée à sec sous pression réduite (2,7 kPa). Le résidu obtenu est purifié par chromatographie-flash sur gel de silice [éluant : cyclohexane/acétate d'éthyle (70 /30 en volumes)]. On obtient 0,18 g de (RS)-5-({1-[bis(4-chlorophényl)méthyl]azétidin-3-yl}-méthylsulfonyl-méthyl)-thièn-2-yl-carboxylate de méthyle sous forme d'une poudre blanche [Spectre de R.M.N ¹H (400 MHz, (CD₃)₂SO d6, δ en ppm) : 2,60 (t, J = 7,5 Hz : 1H); 2,86 (s : 3H); 3,14 (mt : 2H); de 3,20 à 3,35 (mt : 1H); 3,45 (t large, J = 7,5 Hz : 1H); 3,82 (s : 3H); 4,47 (s : 1H); 5,27 (d, J = 11 Hz : 1H); 7,28 (d, J = 4 Hz : 1H); de 7,30 à 7,50 (mt : 8H); 7,72 (d, J = 4 Hz : 1H)].

Le 5-({1-[bis(4-chlorophényl)méthyl]azétidin-3-ylidène}-méthylsulfonylméthyl)-thièn-2-yl-carboxylate de méthyle peut être obtenu de la manière suivante : à une solution de 6,12 g de 1-[bis(4-chlorophényl)méthyl]azétidin-3-one dans 200 cm³ de tétrahydrofuranne on ajoute, à température ambiante sous atmosphère d'argon, 5,15 g de 5-(méthylsulfonylméthyl)thièn-2-yl-carboxylate de méthyle, puis la suspension obtenue est refroidie à -70°C. On ajoute successivement 2,47 g de *tert*-butylate de potassium, puis après 1 heure 30 à cette température une solution de 1,7 cm³ de chlorure de méthylsulfonyle dans 8 cm³ d'éther éthylique en 2 minutes. Le milieu réactionnel est maintenu 1 heure à -70°C, puis on laisse remonter la température vers 20°C avant de couler 80 cm³ d'eau distillée. Le tétrahydrofuranne est chassé sous pression réduite et le résidu aqueux obtenu est extrait par 500 cm³ de dichlorométhane. Le mélange est décanté, la phase organique est lavée avec 3 fois 80 cm³ d'eau distillée, séchée sur sulfate de magnésium, filtrée et concentrée à sec sous pression réduite (2,7 kPa). Le résidu obtenu est purifié par chromatographie-flash sur gel de silice [éluant : cyclohexane/acétate d'éthyle (70 /30 en volumes)]. On obtient 1,6 g de 5-({1-[bis(4-chlorophényi)méthyl]azétidin-3-ylidène}-méthylsulfonylméthyl)-thièn-2-yl-carboxylate de méthyle sous la forme d'une meringue couleur crème.

Le 5-(méthylsulfonylméthyl)thièn-2-yl-carboxylate de méthyle peut être obtenu de la manière suivante : A la solution de 73 g de 5-(bromométhyl)thièn-2-yl-carboxylate de méthyle dans 150 cm³ d'éthanol on ajoute 31,7 g de méthylsulfinate de sodium et la suspension obtenue est chauffée au reflux pendant 7 heures. Le milieu réactionnel est ensuite concentré à sec sous pression réduite (2,7 kPa). Le résidu est extrait par quatre fois 500 cm³ d'acétate d'éthyle, les phases organiques réunies sont lavées successivement avec 250 cm³ d'eau distillée et 250 cm³ d'une solution saturée de chlorure de sodium, séchées sur du sulfate de magnésium, filtrées et concentrées d'une manière incomplète sous pression réduite. Le solide apparu est isolé par filtration, rincé par trois fois 25 cm³ d'acétate d'éthyle glacé et fournit 21,4 g de 5-(méthylsulfonylméthyl)thièn-2-yl-carboxylate de méthyle sous forme d'une poudre de couleur crème.

Le 5-(bromométhyl)thièn-2-yl-carboxylate de méthyle peut être préparé selon la méthode décrite par Wityak J. et coll., Bioorg. Med. Chem. Lett. (1995), 5(18), 2097-100.

### Exemple 28

La (RS)-1-[bis-(4-chlorophényl)-méthyl]-3-[(3,5-difluorophényl)-méthylsulfonylméthyl]-azétidin-3-yl-cyclopropyl-amine peut être préparée de la manière suivante : A une solution de 3 g de 1-[bis(4-ehlorophényl)-méthyl)]-3-[(3,5-difluorophényl)-(méthylsulfonyl)-méthylène]-azétidine dans 30 cm³ de dichlorométhane, à une température voisine de 24°C, sous atmosphère inerte d'argon, on ajoute 2,52 cm³ de cyclopropylamine. Après 39 heures à une température voisine de 24°C, le milieu réactionnel est concentré sous pression réduite (3 mbar) à une température voisine de 40°C. On obtient ainsi 3,26 g d'une meringue jaune-pâle que l'on reprend avec 30 cm³ de dichlorométhane et 2,52 cm³ de cyclopropylamine. La solution obtenue est agitée à une température voisine de 21°C, sous atmosphère inerte d'argon, pendant 87 heures, puis concentrée sous 3 mbar à une température voisine de 40°C. On obtient ainsi 3,64 g de (RS)-1-[bis-(4-chlorophényl)-méthyl]-3-[(3,5-difluorophényl)-méthylsulfonyl-méthyl]-azétidin-3-yl-cyclopropyl-amine sous forme d'une meringue jaune-pâte [Spectre de R.M.N ¹H (300 MHz, CDCl₃, δ en ppm) : 0,29 (mt : 1H); de 0,40 à 0,75 (mt : 3H); 2,50 (mt : 1H); 2,73 (s : 3H); 2,90 (mf : 1H); de 3,45 à 3,70 (mt : 3H); 4,36 (s large : 1H); de 4,60 à 4,80 (mf étalé : 1H); 6,87 (tt, J = 9 et 2,5 Hz : 1H); de 7,20 à 7,40 (mt : 10H)].

### Exemple 29

La (RS)-{1-[bis-(4-chlorophényl)-méthyl]-3-[(3,5-difluorophényl)-méthylsulfonyl-méthyl]-azétidin-3-yl}-(2-pyrrolidin-1-yl-éthyl)-amine peut être préparée de la manière suivante : A une solution de 50 mg de 1-[bis(4-chlorophényl)-méthyl)]-3-[(3,5-difluorophényl)-(méthylsulfonyl)-méthylène]-azétidine dans 0,5 cm³ de dichlorométhane, à une température voisine de 21 °C, sous atmosphère inerte d'argon, on ajoute 0,076 cm³ de 1-(2-aminoéthyl)pyrrolidine. La solution obtenue est agitée à une température voisine de 21 °C, sous atmosphère d'argon, pendant 22 heures, concentrée sous flux d'air à une température voisine de 42°C, puis le résidu brut obtenu est séché sous pression réduite (environ 3 mbar) à une température voisine de 40°C. On obtient la (RS)-{1-[bis-(4-chlorophényl)-méthyl]-3-[(3,5-difluorophényl)-méthylsulfonyl-méthyl]-azétidin-3-yl}-(2-pyrrolidin-1-yl-éthyl)-amine sous forme d'une meringue ocre [Spectre de R.M.N. ¹H (300 MHz, CDCl₃, δ en ppm) : de 1,75 à 1,95 (mt : 4H); de 2,55 à 2,85 (mt : 6H); 2,79 (s : 3H); 2,91 (t, J = 6,5 Hz : 2H); 3,06 (d, J = 8,5 Hz : 1H); 3,17 (d, J = 8,5 Hz : 1H); 3,32 (d, J = 8,5 Hz : 1H); 3,41 (d, J = 8,5 Hz : 1H); 4,31 (s : 1H); 4,56 (s : 1H); 6,88 (tt, J = 8,5 et 2,5 Hz : 1H); 7,22 (s : 4H); 7,25 (s : 4H); 7,34 (mt : 2H)].

### Exemple 30

La (RS)-{1-[bis-(4-chlorophényl)-méthyl]-3-[(3,5-difluorophényl)-méthane-sulfonyl-méthyl]-azétidin-3-yl}-méthyl-amine peut être préparée selon l'exemple 29 à partir de 50 mg de 1-[bis(4-chlorophényl)-méthyl)]-3-[(3,5-difluorophényl)-(méthylsulfonyl)-méthylène]-azétidine, 0,5 cm³ de dichlorométhane, et 0,3 cm³ d'une solution de méthylamine dans le tétrahydrofuranne (solution 2M). On obtient la (RS)-{1-[bis-(4-chlorophényl)-méthyl]-3-[(3,5-difluorophényl)-méthylsulfonyl-méthyl]-azétidin-3-yl}-méthylamine sous forme d'une gomme jaune [Spectre de R.M.N. ¹H (400 MHz, CDCl₃, δ en ppm) : de 2,00 à 2,20 (mf étalé : 1H); 2,62 (s : 3H); 2,76 (s : 3H); 3,04 (d, J = 9 Hz : 1H); 3,18 (d, J = 9 Hz : 1H); 3,37 (AB, J = 9 Hz : 2H); 4,31 (s : 1H); 4,55 (s : 1H); 6,89 (tt, J = 9 et 2,5 Hz : 1H); 7,22 (s : 4H); 7,24 (s : 4H); 7,32 (mt : 2H)].

### Exemple 31

La (PS)-{1-[bis-(4-chlorophényl)-méthyl]-3-[(3,5-difluorophényl)-méthylsulfonyl-méthyl]-azétidin-3-yl}-isobutyl-amine peut être préparée selon l'exemple 29 à partir de 50 mg de 1-[bis(4-chlorophényl)-méthyl)]-3-[(3,5-difluorophényl)-(méthylsulfonyl)-méthylène]-azétidine, 0,5 cm³ de dichlorométhane, et 0,0596 cm³ d'isobutylamine. On obtient la (RS)-{1-[bis-(4-chlorophényl)-méthyl]-3-[(3,5-difluorophényl)-méthylsulfonyl-méthyl]-azétidin-3-yl}-isobutyl-amine sous forme d'une meringue blanche [Spectre de R.M.N ¹H (300 MHz, CDCl₃, δ en ppm) : 1,01 (2 d, J = 7 Hz : 6H); de 1,70 à 2,15 (mf étalé : 1H); 1,76 (mt : 1H); 2,51 (dd, J = 10,5 et 7 Hz : 1H); 2,76 (s : 3H); 2,80 (dd, J = 10,5 et 6 Hz : 1H); 3,01 (d, J = 8,5 Hz : 1H); 3,14 (d, J = 8,5 Hz : 1H); 3,32 (d, J = 8,5 Hz : 1H); 3,44 (d, J = 8,5 Hz : 1H); 4,31 (s : 1H); 4,58 (s : 1H); 6,88 (tt, J = 8,5 et 2,5 Hz : 1H); de 7,15 à 7,30 (mt : 8H); 7,35 (mt : 2H)].

### Exemple 32

La (RS)-{1-[bis-(4-chlorophényl)-méthyl]-3-[(3,5-difluorophényl)-méthylsulfonyl-méthyl]-azétidin-3-yl}-éthyl-amine peut être préparée selon l'exemple 29 à partir de 50 mg de 1-[bis(4-chlorophényl)-méthyl)]-3-[(3,5-difluorophényl)-(méthylsulfonyl)-méthylène]-azétidine, 0,5 cm³ de dichlorométhane, et 0,3 cm³ d'une solution d'éthylamine dans le tétrahydrofuranne (solution 2M). On obtient la (RS)-{1-[bis-(4-chlorophényl)-méthyl]-3-[(3,5-difluorophényl)-méthylsulfonyl-méthyl]-azétidin-3-yl}-éthyl-amine sous forme d'une gomme jaune [Spectre de R.M.N. ¹H (300 MHz, CDCl₃, δ en ppm) : 1,22 (t, J = 7 Hz : 3H); de 2,70 à 2,85 (mt : 2H); 2,77 (s : 3H); de 2,95 à 3,10 (mf : 1H); 3,05 (d, J = 8,5 Hz : 1H); 3,17 (d, J = 8,5 Hz : 1H); 3,33 (d, J = 8,5 Hz : 1H); 3,40 (d, J = 8,5 Hz : 1H); 4,30 (s : 1H); 4,54 (s : 1H); 6,89 (tt, J = 9 et 2,5 Hz : 1H); de 7,15 à 7,30 (mt : 8H); 7,34 (mt : 2H].

### Exemple 33

La (RS)-N-{1-[bis-(4-chlorophényl)-méthyl]-3-[(3,5-difluorophényl)-méthylsuifonyl-méthyl]-azétidin-3-yl}-N',N'-diméthyl-ethane-1,2-diamine peut être préparée selon l'exemple 29 à partir de 50 mg de 1-[bis(4-chlorophényl)-méthyl)]-3-[(3,5-difluorophényl)-(méthylsulfonyl)-méthylène]-azétidine, 0,5 cm³ de dichlorométhane, et 0,0659 cm³ de N,N-diméthyléthylènediamine. On obtient la (RS)-N-{1-[bis-(4-chlorophényl)-méthyl]-3-[(3,5-difluorophényl)-méthylsulfonyl-méthyl]-azétidin-3-yl}-N',N'-diméthyl-ethane-1,2-diamine sous forme d'une meringue blanche [Spectre de R.M.N ¹H (300 MHz, CDCl₃, δ en ppm) : 2,32 (s : 6H); 2,53 (t, J = 6 Hz : 2H); 2,79 (s : 3H); 2,94 (t, J = 6 Hz : 2H); 3,06 (d, J = 8,5 Hz : 1H); 3,16 (d, J = 8,5 Hz : 1H); 3,30 (d, J = 8,5 Hz : 1H); 3,41 (d, J = 8,5 Hz : 1H); 4,30 (s : 1H); 4,55 (s : 1H); 6,88 (tt, J = 9 et 2,5 Hz : 1H); 7,21 (s : 4H); 7,24 (s : 4H); 7,34 (mt : 2H)].

### Exemple 34

La (RS)-1-[bis-(4-chlorophényl)-méthyl]-3-[(3,5-difluorophényl)-méthane-sulfonyl-méthyl]-3-méthyl-azétidine peut être préparée de la manière suivante : A une suspension de 400 mg de magnésium en tournures dans 2,5 cm³ de diéthyléther anhydre, est ajouté, sous atmosphère d'argon, à une température voisine de 24°C, quelques gouttes d'iodure de méthyle pur puis 1 cm³ d'iodure de méthyle en solution dans 22,5 cm³ de diéthyléther. La suspension obtenue est agitée 30 minutes à une température voisine de 24°C, puis refroidie à une température voisine de 0°C par un mélange glace + eau. On ajoute, à une température voisine de 0°C, 1,65 g de complexe CuBr.Me₂S, puis le mélange réactionnel est agité 15 minutes à une température voisine de 0°C. A la suspension jaune obtenue est ajoutée, à une température voisine de 0°C, une solution de 0,5 g de 1-[bis(4-chlorophényl)-méthyl)]-3-[(3,5-difluorophényl)-(méthylsulfonyl)-méthylène]-azétidine dans un mélange de 1 cm³ de tétrahydrofuranne et 1 cm³ de diéthyléther. La suspension obtenue est agitée 4 heures à une température voisine de 0°C, puis à une température voisine de 25°C pendant 16 heures. La suspension noire obtenue est diluée avec 100 cm³ d'acétate d'éthyle et 15 cm³ d'une solution aqueuse saturée en chlorure d'ammonium. Le mélange réactionnel est filtré sur verre fritté garni de Célite, le résidu solide est rincé avec de l'acétate d'éthyle puis de l'eau. Après décantation du filtrat, la phase organique est séparée, lavée avec de 10 cm³ d'eau, 10 cm³ d'une solution aqueuse saturée en chlorure de sodium, séchée sur sulfate de magnésium, filtré sur verre fritté, et concentrée sous pression réduite (20 mbar) à une température voisine de 43°C. On obtient ainsi 550 mg d'une meringue orangée qui est purifiée par chromatographie préparative sur couche mince de silice [14 plaques préparatives Merck Kieselgel 60F254; 20x20 cm; épaisseur 0,5 mm; dépôt en solution dans le dichlorométhane], en éluant par un mélange méthanol-dichlorométhane (0,5-99,5 en volumes). Après élution de la zone correspondant au produit recherché par un mélange méthanol-dichlorométhane (15-85 en volumes), filtration sur verre fritté, puis évaporation des solvants sous pression réduite à une température voisine de 40°C, on obtient 290 mg d'une meringue blanche qui est dissoute dans 15 cm³ de dichlorométhane anhydre et mise en réaction avec 500 mg de résine thiophénol (fournisseur Argonaut, 1,45 mMol/g) et 1 g de résine éthylène-diamine (0.8mMol/g) pendant 38 heures à une température voisine de 20°C. La suspension est filtrée sur verre fritté, les résines sont rincées avec du dichlorométhane, et le filtrat est concentré sous pression réduite (5 mbar) à une température voisine de 43°C. On obtient 272,8 mg d'une meringue blanche qui est dissoute dans 2 cm³ de dichlorométhane et mise en réaction avec 1 cm³ d'éthylènediamine pendant 72 heures à une température voisine de 24°C. Le résidu brut obtenu est repris avec 50 cm³ d'acétate d'éthyle et 10 cm³ d'eau. Après décantation, la phase organique est lavée avec 10 cm³ d'un solution aqueuse d'acide chlorhydrique 1N, 10 cm³ d'une solution aqueuse saturée en hydrogénocarbonate de sodium, 10 cm³ d'eau, 10 cm³ d'une solution aqueuse saturée en chlorure de sodium, séchée sur sulfate de magnésium, filtrée sur verre fritté, et concentrée sous pression réduite (8 mbar) à une température voisine de 42°C. On obtient 263,4 mg d'une meringue jaune-pâle qui est purifiée par chromatographie préparative sur couche mince de silice [7 plaques préparatives Merck Kieselgel 60F254; 20x20 cm; épaisseur 0,5 mm; dépôt en solution dans le dichlorométhane], en éluant par un mélange méthanol-dichlorométhane (0,5-99,5 en volumes). Après élution de la zone correspondant au produit recherché par un mélange méthanol-dichlorométhane (15-85 en volumes), filtration sur verre fritté, puis évaporation des solvants sous pression réduite à une température voisine de 40°C, on obtient 191,4 mg de (RS)-1-[bis-(4-chlorophényl)-méthyl]-3-[(3,5-difluorophényl)-méthylsulfonyl-méthyl]-3-méthyl-azétidine sous forme d'une meringue blanche [Spectre de R.M.N ¹H (300 MHz, CDCl₃, δ en ppm) : 1,75 (s : 3H); 2,67 (s : 3H); 2,74 (d large, J = 7,5 Hz : 1H); 2,93 (d, J = 7,5 Hz : 1H); 3,21 (d large, J = 7,5 Hz : 1H); 3,46 (d, J = 7,5 Hz : 1H); 4,33 (s large : 2H); 6,87 (tt, J = 9 et 2,5 Hz : 1H); 7,12 (mt : 2H); de 7,15 à 7,35 (mt : 8H].

### Exemple 35

La (RS)-1-(2-{1-[bis-(4-chlorophényl)-méthyl]-3-[(3,5-difluorophényl)-méthylsulfonyl-méthyl]-azétidin-3-ylsulfanyl}-éthyl)-4-méthyl-pipérazine peut être préparée de la manière suivante : A une solution de 99 mg de 1-[bis(4-chlorophényl)-méthyl)]-3-[(3,5-difluorophényl)-(méthylsulfonyl)-méthylène]-azétidine dans 2 cm³ de dichlorométhane, à une température voisine de 20°C, on ajoute 128 mg de 1-(éthanethiol-2-yl)-4-méthyl-pipérazine. Après une nuit d'agitation à une température voisine de 20°C, on ajoute 706 mg de résine de Merrifield (1,7 mMol/g). Après une nuit d'agitation à une température voisine de 20°C, la suspension est filtrée, et la résine est rincée avec 2 fois 1 cm³ de dichlorométhane. Le filtrat est concentré sous pression réduite. On obtient ainsi 125 mg d'une huile blanche que l'on purifie par chromatographie sur silice (13 cm³ de silice 0,06-0,2 mm), en éluant avec un mélange méthanol-dichlorométhane (0-100 puis 5-95 en volumes). Les fractions ne contenant que le produit cherché sont réunies et concentrées à sec sous pression réduite. On obtient ainsi 62 mg de (RS)-1-(2-{1-[bis-(4-chlorophényl)-méthyl]-3-[(3,5-difluorophényl)-méthylsulfonyl-méthyl]-azétidin-3-yisulfanyl}-éthyl)-4-méthyl-pipérazine sous forme de cristaux blancs [Spectre de R.M.N ¹H (300 MHz, (CD₃)₂SO d6, δ en ppm) : 2,15 (s : 3H); 2,30 et 2,41 (2mfs : 8H); 2,55 (mt : 2H); 2,85 (s : 3H); 3,02 (mt : 2H); 3,09 (d, J = 8,5 Hz : 1H); 3,38 (d, J = 8,5 Hz : 1H); 3,42 (d, J = 8,5 Hz : 1H); 3,79 (d, J = 8,5 Hz : 1H); 4,68 (s : 1H); 5,37 (s : 1H); de 7,30 à 7,50 (mt : 11H)].

### Exemple 36

La (RS)-(2-{1-[bis-(4-chlorophényl)-méthyl]-3-[(3,5-difluorophényl)-méthylsulfonyl-méthyl]-azétidin-3-ylsulfanyl}-éthyl)-diméthyl-amine peut être préparée en opérant comme dans l'exemple 35, à partir de 99 mg de 1-[bis(4-chlorophényl)-méthyl)]-3-[(3,5-difluorophényl)-(méthylsulfonyl)-méthylène]-azétidine , 2 cm³ de dichlorométhane, 84 mg 2-(diméthylamino)-éthanethiol, et 706 mg de résine de Merrifield (1,7 mMol/g). On obtient ainsi 36 mg (RS)-(2-{1-[bis-(4-chlorophényl)-méthyl]-3-[(3,5-difluorophényl)-méthylsulfonylméthyl]-azétidin-3-ylsulfanyl}-éthyl)-diméthyl-amine sous forme de poudre blanc cassé.

### Exemple 37

La (RS)-{1-[[4-(chlorométhyl)phényl]-(4-chlorophényl)méthyl]-3-[(3,5-difluorophényl)-méthylsulfonyl-méthyl]-azétidin-3-yl}-éthyl-amine peut être préparée de la manière suivante : Une solution de 40 mg de (RS)-{1-[[4-(chlorométhyl)phényl]-(4-chlorophényl)méthyl]-3-[(3,5-difluorophényl)-méthylsulfonyl-méthylène]-azétidine dans 0,1125 cm³ d'éthylamine (solution 2M dans le tétahydrofuranne), contenant un grain d'iodure de sodium, est agitée à une température voisine de 20°C pendant 2 heures, puis diluée avec 20 cm³ d'acétate d'éthyle et 5 cm³ d'une solution aqueuse saturée en hydrogénocarbonate de sodium. La phase organique séparée est lavée avec 5 cm³ d'eau, 5 cm³ d'une solution aqueuse saturée en chlorure de sodium, séchée sur sulfate de magnésium, filtrée sur verre fritté, et concentrée sous pression réduite (15 mbar) à une température voisine de 40°C. L'huile jaune obtenue est purifiée par chromatographie préparative sur couche mince de silice [2 plaques préparatives Merck Kieselgel 60F254; 20x20 cm; épaisseur 0,5 mm; dépôt en solution dans le dichlorométhane], en éluant par un mélange méthanol-dichlorométhane (3-97 en volumes). Après élution de la zone correspondant au produit recherché par un mélange méthanol-dichlorométhane (15-85 en volumes), filtration sur verre fritté, puis évaporation des solvants sous pression réduite à une température voisine de 40°C, on obtient 17 mg de (RS)-{1-[[4-(chlorométhyl)phényl]-(4-chlorophényl)méthyl]-3-[(3,5-difluorophényl)-méthylsulfonyl-méthyl]-azétidin-3-yl}-éthyl-amine sous forme d'un solide blanc [Spectre de R.M.N. ¹H (400 MHz, (CD₃)₂SO d6, δ en ppm) : 1,11 (t, J = 7 Hz : 3H); de 2,10 à 3,55 (mt : 8H); 2,95 (s : 3H); 4,44 (s : 1H); 5,09 (s : 1H); de 7,10 à 7,55 (mt : 11H)].

La (RS)-{1-[[4-(chlorométhyl)phényl]-(4-chlorophényl)méthyl]-3-[(3,5-difluorophényl)-méthylsulfonyl-méthylène]-azétidine peut être préparée de la manière suivante : A une solution de 590 mg de (RS)-[4-((4-Chlorophényl)-{3-[(3,5-difluorophényl)-méthylsulfonyl-méthylene]-azétidin-1-yl}-méthyl)-phényl]-méthanol dans 5 cm³ de dichlorométhane anhydre, on ajoute, à une température voisine de de 21°C, 0,525 cm³ de N,N-diisopropyl-éthylamine puis 0,19 cm³ de chlorure de méthane sulfonyle. Après 1 heure à une température voisine de 21°C, on ajoute 2 cm³ d'un mélange méthanol/dichlorométhane (2,5/97,5 en volumes), puis après 5 minutes, le mélange réactionnel est concentré sous pression réduite (20mbar) à une température voisine de 40°C. La meringue jaune obtenue est purifiée par chromatographie sur silice (50 g de silice 0,06-0,2 mm contenus dans une colonne de diamètre 3 cm), en éluant avec un mélange méthanol/dichlorométhane (0/100 puis 1/99 en volumes) en recueillant des fractions de 10 cm³. Les fractions ne contenant que le produit cherché sont réunies et concentrées à sec sous pression réduite. On obtient ainsi 421,2 mg de (RS)-{1-[[4-(chlorométhyl)phényl]-(4-chlorophényl)méthyl]-3-[(3,5-difluorophényl)-méthylsulfonyl-méthylènel-azétidine sous forme d'une meringue jaune.

Le (RS)-[4-((4-Chlorophényl)-{3-[(3,5-difluorophényl)-méthylsulfonyl-méthylene]-azétidin-1-yl}-méthyl)-phényl]-méthanol peut être préparé de la manière suivante : A une solution de 420 mg de (RS)-4-((4-chlorophényl)-{3-[(3,5-difluorophényl)-méthylsulfonyl-méthylène]azétidin-1-yl}méthyl)-benzaldéhyde dans 7 cm³ de méthanol, refroidie à une température voisine de 0°C (glace + eau) sont ajoutés par portions 49 mg de tétrahydroborure de sodium. Après 2 heures à une température voisine de 0°C, le milieu réactionnel est concentré sous pression réduite (15 mbar) à une température voisine de 35°C. Le résidu obtenu est purifié par chromatographie sur silice (40 g de silice 0,06-0,2 mm contenus dans une colonne de diamètre 3 cm), en éluant avec un mélange méthanol/dichlorométhane (1/99 puis 2,5/97,5 en volumes) en recueillant des fractions de 10 cm³. Les fractions ne contenant que le produit cherché sont réunies et concentrées à sec sous pression réduite. On obtient ainsi 418 mg de RS)-[4-((4-Chlorophényl)-{3-[(3,5-difluorophényl)-méthylsulfonyl-méthylene]-azétidin-1-yl}-méthyl)-phényl]-méthanol sous forme d'une meringue blanche.

### Exemple 38

La (RS)-{1-[bis-(4-chlorophényl)-méthyl]-3-[(3,5-bis-trifluorométhyl-phényl)-méthylsulfonyl-méthyl]-azétidin-3-yl}-isobutyl-amine peut être préparée selon l'exemple 29 à partir de 50 mg de 1-[bis(4-chlorophényl)-méthyl)]-3-[(3,5-bis-trifluorométhyl-phényl)-(méthylsulfonyl)-méthylène]-azétidine, 0,5 cm³ de dichlorométhane, et 0,05 cm³ d'isobutylamine. On obtient 57 mg de la (RS)-{1-[bis-(4-chlorophényl)-méthyl]-3-[(3,5-bis-trifluorométhyl-phényl)-méthylsulfonyl-méthyl]-azétidin-3-yl}-isobutyl-amine sous forme d'une meringue jaune-pâle [Spectre de R.M.N ¹H (300 MHz, CDCl₃, δ en ppm) : 1,01 (d, J = 7,5 Hz : 6H); 1,76 (mt : 1H); 2,47 (dd, J = 10,5 et 7,5 Hz : 1H); de 2,75 à 2,85 (mt : 1H); 2,79 (s : 3H); 2,82 (dd, J = 10,5 et 5,5 Hz : 1H); 3,00 (d, J = 9 Hz : 1H); 3,10 (d, J = 9 Hz : 1H); 3,31 (d, J = 9 Hz : 1H); 3,40 (d, J = 9 Hz : 1H); 4,30 (s : 1H); 4,74 (s : 1H); 7,13 (d, J = 8,5 Hz : 2H); de 7,15 à 7,25 (mt : 6H); 7,96 (s large : 1H); 8,31 (s large : 2H)].

### Exemple 39

La (RS)-1-[bis-(4-chlorophényl)-méthyl]-3-cyano-3-[(3,5-difluorophényl)-méthylsulfonyl-méthyl]-azétidine peut être préparée de la manière suivante : A une solution de 99 mg de 1-[bis(4-chlorophényl)-méthyl)]-3-[(3,5-difluorophényl)-(méthylsulfonyl)-méthylène]-azétidine dans 2,5 cm³ de diméthylsulfoxyde, à une température voisine de 20°C, on ajoute 17 mg de cyanure de potassium. La solution jaune, puis marron, obtenue est chauffée 15 minutes à une température voisine de 40°C, puis refroidie à une température voisine de 20°C. Le milieu réactionnel est concentré sous pression réduite, puis repris avec 10 cm³ de dichlorométhane, lavé avec 3 fois 5 cm³ d'eau. La phase organique obtenue est séchée sur sulfate de magnésium, filtré et concentrée sous pression réduite. On obtient ainsi 100 mg d'une pâte jaune que l'on purifie par chromatographie sur silice (10 cm³ de silice 0,06-0,2 mm contenus dans une colonne de diamètre 1 cm), en éluant avec du dichlorométhane. Les fractions ne contenant que le produit cherché sont réunies et concentrées à sec sous pression réduite. On obtient ainsi 60 mg de (RS)-1-[bis-(4-chlorophényl)-méthyl]-3-cyano-3-[(3,5-difluorophényl)-méthylsulfonyl-méthyl]-azétidine sous forme d'une pâte jaune [Spectre de R.M.N ¹H (300 MHz, (CD₃)₂SO d6, δ en ppm) : 2,94 (s : 3H); 3,07 (d, J = 7,5 Hz : 1H); de 3,20 à 3,40 (mt : 1H); 3,61 (d large, J = 7,5 Hz : 1H); 3,68 (d large, J = 7,5 Hz : 1H); 4,64 (s : 1H); 5,51 (s : 1H); de 7,25 à 7,50 (mt : 11H)].

### Exemple 40

La (RS)-{1-[bis-(4-chlorophényl)-méthyl]-3-[(3,5-difluorophényl)-méthylsulfonyl-méthyl]-azétidin-3-ylméthyl}-(1-cyclopropyl-éthyl)-amine peut être préparée de la manière suivante : A une solution de 53 mg de (RS)-C-{1-[bis-(4-chlorophényl)-méthyl]-3-[(3,5-difluorophényl)-méthylsulfonyl-méthyl]-azétidin-3-yl}-méthylamine dans 2 cm³ de 1,2-dichloroéthane, à une température voisine de 20°C, sont ajoutés successivement 0,03 cm³ de cyclopropylméthylcétone, 0,006 cm³ d'acide acétique puis 32 mg de triacétoxyborohydrure de sodium. La solution obtenue est agitée à une température voisine de 20°C pendant 18 heures, puis on ajoute 2 cm³ d'une solution aqueuse saturée en hydrogénocarbonate de sodium. Après décantation, la phase organique est concentrée sous pression réduite. On obtient ainsi 60 mg d'une huile visqueuse jaune qui est triturée par de l'oxyde d'isopropyle, de l'éther de pétrole. Après séchage sous 0,1 mbar, on obtient 55 mg d'un résidu qui est purifié par chromatographie sur silice (4 cm³ de silice 0,06-0,2 mm contenus dans une colonne de diamètre 1,2 cm), en éluant avec un mélange méthanol/dichlorométhane (0/100 puis 5/95 en volumes).

Les fractions ne contenant que le produit cherché sont réunies, concentrées à sec sous pression réduite, et repurifiées par chromatographie sur silice (4 cm³ de silice 0,04-0,063 mm contenus dans une colonne de diamètre 1,2 cm), en éluant avec un mélange méthanol/dichlorométhane (0/100 puis 1/99 en volumes). Les fractions ne contenant que le produit cherché sont réunies et concentrées à sec. On obtient ainsi 20 mg de (RS)-{1-[bis-(4-chlorophényl)-méthyl]-3-[(3,5-difluorophényl)-méthylsulfonyl-méthyl]-azétidin-3-ylméthyl}-(1-cyclopropyl-éthyl)-amine [Spectre de R.M.N ¹H (300 MHz, CDCl₃, δ en ppm) : On observe un mélange de diastéréoisomère, de 0,00 à 0,30 et de 0,40 à 0,80 (mts : 5H); 1,09 et 1,17 (2d, J = 6,5 Hz : 3H en totalité); 1,87 (mt : 1H); de 2,55 à 2,75-de 2,75 à 2,95 et de 3,25 à 3,55 (mts : 4H); 2,68 (s : 3H); 3,12 (d, J = 8,5 Hz : 1H); de 3,80 à 3,90 (mt : 1H); 4,42 et 4,43 (2s : 1H en totalité); 4,79 et 4,84 (2s : 1H en totalité); 6,89 (tt, J = 9 et 2,5 Hz : 1H); 7,16 (mt : 2H); de 7,15 à 7,35 (mt : 8H]).

La (RS)-C-{1-[bis-(4-chlorophényl)-méthyl]-3-[(3,5-difluorophényl)-méthylsulfonyl-méthyl]-azétidin-3-yl}-méthylamine peut être préparée de la manière suivante : A une solution de 250 mg de (RS)-1-[bis-(4-chlorophényl)-méthyl]-3-cyano-3-[(3,5-difluorophényl)-méthylsulfonyl-méthyl]-azétidine dans 10 cm³ de tétrahydrofuranne anhydre, refroidie à une température voisine de 0°C, est ajouté goutte à goutte 1,27 cm³ d'une solution d'hydrure de diisobutylaluminium 1,5 M dans le tétrahydrofuranne. Après 30 minutes à une température voisine de 0°C, puis 4 heures à une température voisine de 20°C, la solution est de nouveau refroidie à une température de 0°C. On ajoute successivement 6,35 cm³ d'eau, puis 1,06 cm³ d'acide chlorhydrique aqueux (12N). Après décantation, la phase aqueuse est extraite avec 3 fois 10 cm³ d'acétate d'éthyle. Les phases organiques sont rassemblées, séchées sur sulfate de magnésium, filtrées et concentrées sous pression réduite. On obtient ainsi 0,42 g d'une huile jaune foncé que l'on purifie par chromatographie sur silice (40 cm³ de silice 0,063-0,2 mm contenus dans une colonne de diamètre 2,7 cm), en éluant avec un mélange méthanol/dichlorométhane (0/100 puis 5/95 en volumes). Les fractions ne contenant que le produit cherché sont réunies et concentrées à sec sous pression réduite. On obtient ainsi 110 mg de (RS)-C-{1-[bis-(4-chlorophényl)-méthyl]-3-[(3,5-difluorophényl)-méthylsulfonyl-méthyl]-azétidin-3-yl}-méthylamine.

### Exemple 41

Le (RS)-N-{1-[bis-(4-chlorophényl)-méthyl]-3-[(3,5-difluorophényl)-méthylsulfonyl-méthyl]-azétidin-3-ylméthyl}-isobutyramide peut être préparé de la manière suivante : A une solution de 53 mg de (RS)-C-{1-[bis-(4-chlorophényl)-méthyl]-3-[(3,5-difluorophényl)-méthylsulfonyl-méthyl]-azétidin-3-yl}-méthylamine dans 2 cm³ de dichlorométhane anhydre, à une température voisine de 20°C, sont ajoutés successivement 0,0187 cm³ d'acide isobutyrique, 0,032 cm³ de 1,3-diisopropylcarbodiimide et 2,5 mg de 4-diméthylaminopyridine. Après 72 heures d'agitation, à une température voisine de 20°C, on ajoute 2 cm³ d'eau, on décante, puis la phase organique est séchée sur sulfate de magnésium, filtrée et concentrée à sec sous pression réduite. Le résidu brut obtenu est purifié par chromatographie préparative sur couche mince de silice [1 plaque préparative Merck Kieselgel 60F254; 20x20 cm; épaisseur 1 mm], en éluant par un mélange acétate d'éthyle-dichlorométhane (5-95 en volumes). Après élution de la zone correspondant au produit recherché, filtration sur verre fritté, puis évaporation des solvants sous pression réduite à une température voisine de 40°C, on obtient 16 mg de (RS)-N-{1-[bis-(4-chlorophényl)-méthyl]-3-[(3,5-difluorophényl)-méthylsulfonyl-méthyl]-azétidin-3-ylméthyl}-isobutyramide sous forme d'une poudre jaune pâle [Spectre de R.M.N ¹H (300 MHz, CDCl₃, δ en ppm) : 1,22 (d, J = 7 Hz : 6H); 2,46 (mt : 1H); 2,69 (s : 3H); 2,99 (d, J = 8,5 Hz : 1H); 3,23 (AB, J = 8,5 Hz : 2H); 3,40 (d, J = 8,5 Hz : 1H); 3,57 (dd, J = 14 et 4,5 Hz : 1H); 4,09 (dd, J = 14 et 7,5 Hz : 1H); 4,34 (s : 1H); 4,35 (s : 1H); 6,71 (dd, J = 7,5 et 4,5 Hz : 1H); 6,95 (tt, J = 9 et 2,5 Hz : 1H); de 7,10 à 7,35 (mt : 10H)].

### Exemple 42

Le (RS)-N-{1-[bis-(4-chlorophényl)-méthyl]-3-[(3,5-difluorophényl)-méthylsulfonyl-méthyl]-azétidin-3-ylméthyl}-cyclopropanecarboxamide peut être préparée d'une manière semblable à l'exemple 39 à partir de 53 mg de (RS)-C-{1-[bis-(4-chlorophényl)-méthyl]-3-[(3,5-difluorophényl)-méthane-sulfonyl-méthyl]-azétidin-3-yl}-méthylamine, 2 cm³ de dichlorométhane anhydre, 0,0167 cm³ d'acide cyclopropanecaboxylique, 0,032 cm³ de 1,3-diisopropylcarbodiimide et 2,5 mg de 4-diméthylaminopyridine. On obtient 28 mg de (RS)-N-{1-[bis-(4-chlorophényl)-méthyl]-3-[(3,5-difluorophényl)-méthylsulfonyl-méthyl]-azétidin-3-ylméthyl}-cyclopropanecarboxamide sous forme d'une poudre beige [Spectre de R.M.N. ¹H (300 MHz, COCl₃, δ en ppm) : 0,81 (mt : 2H); 1,01 (mt : 2H); de 1,35 à 1,55 (mt : 1H); 2,70 (s : 3H); 3,02 (d, J = 8,5 Hz : 1H); 3,21 (AB limite, J = 8 Hz : 2H); 3,45 (d, J = 8,5 Hz : 1H); 3,62 (dd, J = 14 et 4,5 Hz : 1H); 4,10 (dd, J = 14 et 7,5 Hz : 1H); 4,31 (s : 1H); 4,36 (s : 1H); 6,75 (dd, J = 7,5 et 4,5 Hz : 1H); 6,95 (tt, J = 9 et 2 Hz : 1H); de 7,15 à 7,35 (mt : 10H)].

### Exemple 43

La (RS)-{1-[bis-(4-chlorophényl)-méthyl]-3-[(3,5-difluorophényl)-méthylsulfonyl-méthyl]-azétidin-3-ylméthyl}-diéthyl-amine peut être préparée d'une manière semblable à l'exemple 40 à partir de 53 mg de (RS)-C-{1-[bis-(4-chlorophényl)-méthyl]-3-[(3,5-difluorophényl)-méthylsulfonyl-méthyl]-azétidin-3-yl}-méthylamine, 2 cm³ de 1,2-dichloroéthane, 0,017 cm³ d'acétaldéhyde, 0,006 cm³ d'acide acétique et 32 mg de triacétoxyborohydrure de sodium. On obtient ainsi 12 mg de (RS)-{1-[bis-(4-chlorophényl)-méthyl]-3-[(3,5-difluorophényl)-méthylsulfonyl-méthyl]-azétidin-3-ylméthyl}-diéthyl-amine sous forme d'une poudre blanc cassé [Spectre de R.M.N. ¹H (300 MHz, CDCl₃, δ en ppm) : 1,00 (t, J = 7 Hz : 6H); 2,49 (q, J = 7 Hz : 4H); 2,54 (d, J = 13,5 Hz : 1H); 2,69 (s : 3H); 2,76 (d large, J = 7,5 Hz : 1H); 3,07 (d large, J = 7,5 Hz : 1H); 3,15 (d, J = 13,5 Hz : 1H); 3,24 (d large, J = 7,5 Hz : 1H); 4,06 (d large, J = 7,5 Hz : 1H); 4,35 (s : 1H); 5,02 (s : 1H); 6,91 (mt : 1H); de 7,15 à 7,40 (mt : 10H)].

### Exemple 44

Le (RS)-N-{1-[bis-(4-chlorophényl)-méthyl]-3-[(3,5-difluorophényl)-méthylsulfonyl-méthyl]-azétidin-3-ylméthyl}-méthanesulfonamide peut être préparé de la manière suivante : A une solution de 53 mg de (RS)-C-{1-[bis-(4-chlorophényl)-méthyl]-3-[(3,5-difluorophényl)-méthylsulfonyl-méthyl]-azétidin-3-yl}-méthylamine dans 2 cm³ d'acétate d'éthyle, à une température voisine de 20°C, on ajoute 150 mg de résine IRA-68 anhydre, puis 0,012 cm³ de chlorure de méthylsulfonyle. Après une nuit d'agitation à une température voisine de 20°C, on ajoute 0,001 cm³ d'eau puis 150 mg de résine IRA-68 anhydre. Après 1 heure d'agitation à une température voisine de 20°C, le mélange réactionnel est filtré, et le filtrat est concentré sous pression réduite. On obtient ainsi 81 mg d'une pâte jaune qui est purifiée par chromatographie préparative sur couche mince de silice [1 plaque préparative Merck Kieselgel 60F254; 20x20 cm; épaisseur 1 mm], en éluant par un mélange acétate d'éthyle-dichlorométhane (5-95 en volumes). Après élution de la zone correspondant au produit recherché, filtration sur verre fritté, puis évaporation des solvants sous pression réduite à une température voisine de 40°C, on obtient 25 mg de (RS)-N-{1-[bis-(4-chlorophényl)-méthyl]-3-[(3,5-difluorophényl)-méthylsulfonyl-méthyl]-azétidin-3-ylméthyl}-méthanesulfonamide sous forme d'une poudre jaune pâle [Spectre de R.M.N. ¹H (300 MHz, CDCl₃, δ en ppm) : 2,70 (s : 3H); de 2,95 à 3,10 (mt : 2H); 3,05 (s : 3H); 3,22 (d large, J = 8 Hz : 1H); 3,52 (d, J = 8 Hz : 1H); 3,74 (dd, J = 13,5 et 8 Hz : 1H); 3,90 (dd, J = 13,5 et 5,5 Hz : 1H); 4,23 (s : 1H); 4,46 (s : 1H); 5,54 (mt : 1H); 7,00 (tt, J = 9 et 2 Hz : 1H); de 7,05 à 7,35 (mt : 10H)].

### Exemple 45

La (RS)-1-{1-[bis-(4-chlorophényl)-méthyl]-3-[(3,5-difluorophényl)-méthylsulfonyl-méthyl]-azétidin-3-ylméthyl}-3-isopropyl-urée peut être préparée de la manière suivante : A une solution de 53 mg de (RS)-C-{1-[bis-(4-chlorophényl)-méthyl]-3-[(3,5-difluorophényl)-méthylsulfonyl-méthyl]-azétidin-3-yl}-méthylamine dans 2 cm³ de dichlorométhane, à une température voisine de 20°C, on ajoute 0,0197 cm³ d'isocyanate d'isopropyle. Après une nuit d'agitation à une température voisine de 20°C, on ajoute 0,05 cm³ d'eau, puis après 15 minutes d'agitation à une température voisine de 20°C, le mélange réactionnel est séché sur sulfate de magnésium, filtré, et concentré sous pression réduite. Le résidu obtenu (75 mg) est purifié par chromatographie préparative sur couche mince de silice [1 plaque préparative Merck Kieselgel 60F254; 20x20 cm; épaisseur 1 mm], en éluant par un mélange acétate d'éthyle-dichlorométhane (5-95 en volumes). Après élution de la zone correspondant au produit recherché, filtration sur verre fritté, puis évaporation des solvants sous pression réduite à une température voisine de 40°C, on obtient 16 mg de (RS)-1-{1-[bis-(4-chlorophényl)-méthyl]-3-[(3,5-difluorophényl)-méthylsulfonyl-méthyl]-azétidin-3-ylméthyl}-3-isopropyl-urée sous forme d'une poudre jaune pâle [Spectre de R.M.N. ¹H (300 MHz, CDCl₃, δ en ppm) : 1,17 (d, J = 7 Hz : 6H); 2,68 (s : 3H); 3,00 (d large, J = 8,5 Hz : 1H); 3,11 (d, J = 8,5 Hz : 1H); 3,17 (d, J = 8,5 Hz : 1H); 3,46 (d large, J = 8,5 Hz : 1H); 3,64 (dd, J = 14 et 5 Hz : 1H); 3,86 (mt : 1H); 3,96 (dd, J = 14 et 7,5 Hz : 1H); 4,15 (d, J = 8 Hz : 1H); 4,29 (s : 1H); 4,43 (s : 1H); 5,11 (mt : 1H); 6,94 (tt, J = 9 et 2 Hz : 1H); de 7,10 à 7,30 (mt : 10H)].

### Exemple 46

L'ester d'isobutyl de l'acide (RS)-{1-[bis-(4-chlorophényl)-méthyl]-3-[(3,5-difluorophényl)-méthylsulfonyl-méthyl]-azétidin-3-ylméthyl}-carbamique peut être préparé de la manière suivante : A une solution de 53 mg de (RS)-C-{1-[bis-(4-chlorophényl)-méthyl]-3-[(3,5-difluorophényl)-méthylsulfonyl-méthyl]-azétidin-3-yl}-méthylamine dans 2 cm³ de pyridine, à une température voisine de 20°C, on ajoute 0,016 cm³ de chlorofomiate d'isobutyle. Après une nuit d'agitation à une température voisine de 20°C, le mélange réactionnel est concentré sous pression réduite. On obtient ainsi 68 mg d'une pâte jaune qui est purifiée par chromatographie préparative sur couche mince de silice [1 plaque préparative Merck Kieselgel 60F254; 20x20 cm; épaisseur 1 mm], en éluant par un mélange acétate d'éthyle-dichlorométhane (5-95 en volumes). Après élution de la zone correspondant au produit recherché, filtration sur verre fritté, puis évaporation des solvants sous pression réduite à une température voisine de 40°C, on obtient 14 mg de l'ester d'isobutyl de l'acide (RS)-{1-[bis-(4-chlorophényl)-méthyl]-3-[(3,5-difluorophényl)-méthylsulfonylméthyl]-azétidin-3-ylméthyl}-carbamique sous forme d'une poudre blanc cassé [Spectre de R.M.N. ¹H (300 MHz, CDCl₃, δ en ppm) : 0,96 (d, J = 7 Hz : 6H); 1,95 (mt : 1H); 2,68 (s : 3H); 3,04 (d, J = 8,5 Hz : 1H); 3,19 (s : 2H); 3,51 (d, J = 8 Hz : 1H); 3,75 (dd, J = 14 et 5 Hz : 1H); de 3,80 à 4,00 (mt : 3H); 4,30 (s : 1H); 4,34 (s : 1H); 5,63 (mf : 1H); 6,95 (tt, J = 9 et 2 Hz : 1H); de 7,10 à 7,30 (mt : 10H)].

### Exemple 47

La (RS)-{1-[bis-(4-chlorophényl)-méthyl]-3-[(3,5-difluorophényl)-méthylsulfonyl-méthyl]-azétidin-3-ylméthyl}-diméthyl-amine peut être préparée de la manière suivante : A une solution de 52 mg de (RS)-C-{1-[bis-(4-chlorophényl)-méthyl]-3-[(3,5-difluorophényl)-méthylsulfonyl-méthyl]-azétidin-3-yl}-méthylamine dans 2 cm³ d'acétonitrile est ajouté, à une température voisine de 20°C, 138 mg de carbonate de potassium puis 0,0075 cm³ d'iodure de méthyle. Après agitation pendant une nuit à une température voisine de 20°C, le milieu réactionnel est filtré sur verre fritté, le solide est rincé avec du dichlorométhane, et le filtrat est concentré sous pression réduite. Le résidu brut obtenu (90 mg) est purifié par chromatographie préparative sur couche mince de silice [1 plaque préparative Merck Kieselgel 60F254; 20x20 cm; épaisseur 1 mm], en éluant par un mélange acétate d'éthyle-dichlorométhane (5-95 en volumes). Après élution de la zone correspondant au produit recherché, filtration sur verre fritté, puis évaporation des solvants sous pression réduite à une température voisine de 40°C, on obtient 11 mg de (RS)-{1-[bis-(4-chlorophényl)-méthyl]-3-[(3,5-difluorophényl)-méthylsulfonyl-méthyl]-azétidin-3-ylméthyl}-diméthyl-amine [Spectre de R.M.N. ¹H (300 MHz, CDCl₃, δ en ppm) : 2,18 (s : 6H); 2,30 (d. J = 13 Hz : 1H); de 4,65 à 4,78 (mt : 1H); 2,70 (s : 3H); 2,98 (d, J = 13 Hz : 1H); 3,09 (d, J = 8 Hz : 1H); 3,32 (d, J = 7,5 Hz : 1H); 4,11 (d, J = 8 Hz : 1H); 4,35 (s : 1H); 4,94 (s : 1H); 6,92 (mt : 1H); de 7,10 à 7,40 (mt : 10H)].

### Exemple 48

La (RS)-1-[bis-(4-chlorophényl)méthyl]-3-[(4-méthoxyphényl)méthylsulfonylméthyl]azétidine peut être préparée de la manière suivante : A une solution de 400 mg de 1-[bis-(4-chlorophényl)méthyl]-3-[(4-méthoxyphényl)méthylsulfonyl-méthylène]azétidine dans 4,5 cm³ d'éthanol, sous atmosphère d'argon, à une température voisine de 20°C, on ajoute 25,5 mg de tétrahydroborure de sodium. Après 16 heures d'agitation à une température voisine de 20°C, on ajoute 26 mg de tétrahydroborure de sodium. Le milieu réactionnel est agité à une température voisine de 20°C pendant 4,5 heures, puis à une température voisine de 50°C pendant 3 heures. Après refroidissement jusqu'à une température voisine de 20°C, le milieu réactionnel est concentré sous pression réduite. Le dépôt blanc obtenu est repris avec 2 cm³ d'eau et 2cm³ de dichlorométhane. Après décantation, la phase organique est concentrée sous pression réduite, et la meringue jaune obtenue est purifiée par chromatographie préparative sur couche mince de silice [2 plaques préparatives Merck Kieselgel 60F254; 20x20 cm; épaisseur 0,5 mm], en éluant par un mélange méthanol-dichlorométhane (1-99 en volumes). Après élution de la zone correspondant au produit recherché par un mélange méthanol-dichlorométhane (10-90 en volumes), filtration sur verre fritté, puis évaporation des solvants sous pression réduite à une température voisine de 40°C, on obtient 14 mg de (RS)-1-[bis-(4-chlorophényl)méthyl]-3-[(4-méthoxyphényl)méthylsulfonyl-méthyt]azétidine sous forme d'une meringue blanche [Spectre de R.M.N ¹H (300 MHz, CDCl₃, δ en ppm) : 2,56 (mt : 1H); 2,58 (s : 3H); 3,20 (mt : 2H); de 3,35 à 3,55 (mt : 1H); 3,66 (t large, J = 7,5 Hz : 1H); 3,81 (s : 3H); 4,21 (d, J = 4,5 Hz : 1H); 4,26 (s large : 1H); 6,90 (d, J = 8,5 Hz : 2H); de 7,15 à 7,40 (mt : 10H)].

La 1-[bis-(4-chlorophényl)méthyl]-3-[(4-méthoxyphényl)méthylsulfonyl-méthylène]azétidine peut être préparée en opérant comme il est décrit dans l'exemple 1 à partir de 1 g de (RS)-1-[bis-(4-chlorophényl)-méthyl]-3-[méthylsulfonyl-(4-methoxy-phényl)-méthyl]-azétidin-3-ol, 20 cm³ de dichlorométhane, 0,229 cm³ de chlorure de méthylsulfonyle et 722 mg de 4-diméthylamino-pyridine. Après purification par chromatographie sur silice à pression atmosphérique (100 g de silice, granulométrie 0,063-0,2 mm contenus dans une colonne de diamètre 3 cm), en éluant avec du dichlorométhane), les fractions ne contenant que le produit cherché sont réunies et concentrées à sec sous pression réduite. On obtient ainsi 650 mg de 1-[bis-(4-chlorophényl)méthyl]-3-[(4-méthoxyphényl)méthylsulfonyl-méthylène]azétidine sous forme d'une gomme jaune.

Le (RS)-1-[bis-(4-chlorophényl)- méthyl]-3-[méthylsulfonyl-(4-methoxy-phényl)-méthyl]-azétidin-3-ol peut être préparé en opérant comme il est décrit dans l'exemple 1 à partir de 19,6 cm³ de n-butyllithium 1,6N en solution dans l'hexane, 5,7 g de 4-méthoxybenzyl méthyl sulfone et 8,71 g de 1-[bis-(4-chlorophényl)-méthyl]-azétidin-3-one dans 450 cm³ de tétrahydrofuranne. On obtient ainsi 8,3 g de (RS)-1-[bis-(4-chlorophényl)-méthyl]-3-[méthylsulfonyl-(4-methoxy-phényl)-méthyl]-azétidin-3-ol sous forme d'un solide beige.

La 4-méthoxybenzyl-méthyl-sulfone peut être préparée en opérant comme décrit dans l'exemple 27, à partir de 13,6 cm³ de chlorure de 4-méthoxybenzyle, 30 mg d'iodure de sodium, 14,4 g de méthylsulfinate de sodium dans 125 cm³ d'éthanol. On obtient ainsi 5,75 g de 4-méthoxybenzyl-méthyl-sulfone sous forme d'une poudre blanche.

### Exemple 49

La (RS)-2-{1-[bis-(4-chlorophényl)méthyl]-azétidin-3-yl}-2-(3,5-difluorophényl)-N-acétylmorpholine peut être préparé de la manière suivante : A 37 mg de chlorhydrate de l'acide (RS)-{1-[bis-(4-chlorophényl) méthyl]-azétidin-3-yl}-(3,5-difluorophényl)-acétique est ajouté successivement, à une température voisine de 20°C, 252 mg d'EDCI supporté (2,3 équivalents, le réactif EDCI supporté est commercial, et peut être également préparé selon la référence suivante : M. Desai, L. Stramiello, Tetrahedron Letters, 34, 48, 7685-7688 (1993)), 2 cm³ de dichlorométhane anhydre, 0,006 cm³ de morpholine, puis 0,010 cm³ de triéthylamine. Après 12 heures d'agitation, à une température voisine de 20°C, le mélange réactionnel est filtré sur verre fritté. Le filtrat est lavé avec 2 cm³ d'eau, séché sur sulfate de magnésium, filtré, et concentré à sec sous pression réduite (2,7 kPa) à une température voisine de 20°C. On obtient ainsi 15 mg de (RS)-2-{1-[bis-(4-chlorophényl)méthyl]-azétidin-3-yl}-2-(3,5-difluorophényl)-N-acétylmorpholine sous forme d'une meringue beige [Spectre de R.M.N. ¹H (300 MHz, CDCl₃, δ en ppm) : de 2,60 à 3,80 (mt : 13H); 3,93 (d, J = 10 Hz : 1H); 4,27 (s : 1H); de 6,65 à 6,85 (mt : 3H); de 7,20 à 7,40 (mt : 8H)].

### Exemple 50

Le (RS)-2-{1-[bis-(4-chlorophényl)méthyl]-azétidin-3-yl}-2-(3,5-difluorophényl)-N-cyclohéxylacétamide peut être préparé de la manière suivante : A une solution de 250 mg de chlorhydrate de l'acide (RS)-{1-[bis-(4-chlorophényl) méthyl]-azétidin-3-yl}-(3,5-difluorophényl)-acétique dans 10cm³ de dichlorométhane anhydre, à une température voisine de 20°C, sont ajoutés successivement, 0,070 cm³ de cyclohexylamine, 144 mg de chlorhydrate de 1-(3-diméthylaminopropyl)-3-éthylcarbodiimide, 0,141 cm³ de triéthylamine, puis 4 mg d'hydrate d'hydroxybenzotriazole. La solution obtenue est agitée à une température voisine de 20°C pendant environ 12 heures. Le milieu réactionnel est déposé sur une cartouche Varian (25cm³) garnie avec 12 g de silice fine (0,040-0,063 mm), conditionnée puis éluée avec un mélange dichlorométhane-éther de pétrole (80-20 en volumes) à l'aide d'une pompe Duramat en recueillant des fractions de 1,5 cm³. Les fractions 11 à 17 sont réunies et concentrées à sec sous pression réduite (2,7 kPa) à 40°C. On obtient ainsi 169 mg de (RS)-2-{1-[bis-(4-chlorophényl)méthyl]-azétidin-3-yl}-2-(3,5-difluorophényl)-N-cyclohexylacétamide sous forme d'une meringue blanche [Spectre de R.M.N.¹H (300 MHz, CDCl₃, δ en ppm) : de 0,90 à 1,45 (mt : 6H); de 1,50 à 1,90 (mt : 4H); 2,66 (mt : 1H); 2,90 (mt : 1H); de 3,00 à 3,15 (mt : 2H); 3,42 (t large, J = 7,5 Hz : 1H); 3,52 (d, J = 10,5 Hz : 1H); de 3,60 à 3,80 (mt : 1H); 4,27 (s : 1H); 5,25 (d large, J = 8 Hz : 1H); 6,90 (tt, J = 9 et 2,5 Hz : 1H); 6,82 (mt : 2H); de 7,20 à 7,35 (mt : 8H].

### Exemple 51

La (RS)-2-{1-[bis-(4-chlorophényl)méthyl]-azétidin-3-yl}-2-(3,5-difluorophényl)-N-acétylpipéridine peut être préparé de la manière suivante : A une solution de 50 mg de chlorhydrate de l'acide (RS)-{1-[bis-(4-chlorophényl)méthyl]-azétidin-3-yl}-(3,5-difluorophényl)-acétique dans 2 cm³ de dichlorométhane anhydre, à une température voisine de 20°C, sont ajoutés successivement 0,012 cm³ de pipéridine, 29 mg de chlorhydrate de 1-(3-diméthylaminopropyl)-3-éthylcarbodiimide, 0,028 cm³ de triéthylamine et 1,5 mg d'hydrate d'hydroxybenzotriazole. La solution obtenue est agitée à une température voisine de 20°C pendant environ 12 heures. Le milieu réactionnel est déposé sur une cartouche Varian (6 cm³) garnie avec 3 g de silice fine (0,040-0,063 mm) en éluant au dichlorométhane à l'aide d'une pompe Duramat. Les fractions comprises entre 8 et 15 cm³ sont réunies et concentrées à sec sous pression réduite (2,7 kPa) à 40°C. On obtient ainsi 14 mg (RS)-2-{1-[bis-(4-chlorophényl)méthyl]-azétidin-3-yl}-2-(3,5-difluorophényl)-N-acétylpipéridine sous forme d'une poudre blanche cristalline [Spectre de R.M.N.¹H (300 MHz, CDCl₃, δ en ppm) : de 0,95 à 1,15 et de 1,30 à 1,50 (2mts : 6H en totalité); 2,74 (mt : 2H); de 3,00 à 3,15 (mt : 2H); de 3,30 à 3,45 (mt : 4H); de 3,55 à 3,70 (mt : 1H); 3,95 (d, J = 10 Hz : 1H); 4,26 (s : 1H); 6,68 (tt, J = 9 et 2,5 Hz : 1H); 6,80 (mt : 2H); de 7,20 à 7,35 (mt : 8H)].

### Exemple 52

La (RS)-2-{1-[bis-(4-chlorophényl)méthyl]-azétidin-3-yl}-2-(3,5-difluorophényl)-N-acétylpyrrolidine peut être préparé de la manière suivante : A une solution de 50 mg de chlorhydrate de l'acide (RS)-{1-[bis-(4-chlorophényl)méthyl]-azétidin-3-yl}-(3,5-difluorophényl)-acétique dans 2 cm³ de dichlorométhane anhydre, à une température voisine de 20°C, sont ajoutés successivement 0,010 cm³ de pyrrolidine, 0.023 cm³ de diisopropylcarbodiimide, 0,028 cm³ de triéthylamine et 1,5 mg d'hydrate d'hydroxybenzotriazole. La solution obtenue est agitée à une température voisine de 20°C pendant environ 12 heures, Le milieu réactionnel est déposé sur une cartouche Varian (6 cm³) garnie avec 3 g de silice fine (0,040-0,063 mm) en éluant au dichlorométhane à l'aide d'une pompe Duramat. Les fractions comprises entre 11 et 19 cm³ sont réunies et concentrées à sec sous pression réduite (2,7 kPa) à 40°C. On obtient ainsi 29 mg de (RS)-2-{1-[bis-(4-chlorophényl)méthyl]-azéfidin-3-yl}-2-(3,5-difluorophényl)-N-acétylpyrrolidine sous forme d'une meringue blanche [Spectre de R.M.N.¹H (300 MHz, CDCl₃, δ en ppm) : de 1,75 à 2,00 (mt : 4H); 2,74 (mt : 2H); de 3,00 à 3,30 (mt : 3H); de 3,35 à 3,60 (mt : 4H); 3,80 (d, J = 10,5 Hz : 1H); 4,25 (s : 1 H); 6,68 (tt, J = 9 et 2,5 Hz : 1 H); 6,84 (mt : 2H); de 7,20 à 7,40 (mt : 8H)].

### Exemple 53

Le (RS)-2-{1-[bis-(4-chlorophényl)méthyl]-azétidin-3-yl}-2-(3,5-difluorophényl)-N-cyclopropylacétamide peut être préparé de la manière suivante : A une solution de 50 mg de chlorhydrate de l'acide (RS)-{1-[bis-(4-chlorophényl)méthyl]-azétidin-3-yl}-(3,5-difluorophényl)-acétique dans 2cm³ de dichlorométhane anhydre, à une température voisine de 20°C, sont ajoutés successivement 0,009 cm³ de cyclopropylamine, 29 mg de chlorhydrate de 1-(3-diméthylaminopropyl)-3-éthylcarbodiimide, 0,028 cm³ de triéthylamine et 1,5 mg d'hydrate d'hydroxybenzotriazole. La solution obtenue est agitée à une température voisine de 20°C pendant environ 12 heures. Le milieu réactionnel est déposé sur une cartouche Varian (6 cm³) garnie avec 3 g de silice fine (0,040-0,063 mm), conditionnée puis éluée au dichlorométhane. à l'aide d'une pompe Duramat en recueillant des fractions de 2 cm³. Les fractions 2 à 6 sont réunies et concentrées à sec sous pression réduite (2,7 kPa) à 40°C. On obtient ainsi 29 mg de (RS)-2-{1-[bis-(4-chlorophényl)méthyl]-azétidin-3-yl}-2-(3,5-difluorophényl)-N-cyclopropyl acétamide sous forme d'une meringue blanche [Spectre de R.M.N ¹H (300 MHz, CDCl₃, δ en ppm) : 0,40 (mt : 2H); 0,74 (mt : 2H); 2,64 (mt : 2H); 2,89 (dd, J = 7,5 et 5 Hz : 1H); 3,08 (mt : 2H); 3,42 (t large, J = 7,5 Hz : 1 H); 3,51 (d, J = 10,5 Hz : 1 H); 4,25 (s : 1 H); 5,50 (mf : 1 H); 6,70 (tt, J = 9 et 2,5 Hz : 1 H); 6,81 (mt : 2H); de 7,15 à 7,35 (mt : 8H)].

### Exemple 54

Le (RS)-2-{1-[bis-(4-chlorophényl)méthyl]-azétidin-3-yl}-2-(3,5-difluoro phényl)-N-cyclohéxyl-N-méthylacétamide peut être préparé de la manière suivante : A une solution de 50 mg de chlorhydrate de l'acide (RS)-{1-[bis-(4-chlorophényl)méthyl]-azétidin-3-yl}-(3,5-difluorophényl)-acétique dans 2 cm³ de dichlorométhane anhydre, à une température voisine de 20°C, sont ajoutés successivement 0,016 cm³ de N-méthylcyclohexylamine, 29 mg de chlorhydrate de 1-(3-diméthytaminopropyl)-3-éthylcarbodiimide, 0,028 cm³ de triéthylamine et 1,5 mg d'hydrate d'hydroxybenzotriazole. La solution obtenue est agitée à une température voisine de 20°C pendant environ 12 heures. Le milieu réactionnel est déposé sur une cartouche Varian (6 cm³) garnie avec 3 g de silice fine (0,040-0,063 mm), conditionnée puis éluée au dichlorométhane à l'aide d'une pompe Duramat en recueillant des fractions de 2 cm³. Les fractions 4 à 6 sont réunies et concentrées à sec sous pression réduite (2,7 kPa) à 40°C. On obtient ainsi 17 mg de (RS)-2-{1-[bis-(4-chlorophényl)méthyl]-azétidin-3-yl}-2-(3,5-difluorophényl)-N-cyclohéxyl-N-méthylacétamide sous forme d'une meringue blanche [Spectre de R.M.N ¹H (250 MHz, (CD₃)₂SO d6, à une température de 373K, δ en ppm) : de 0,98 à 1,85 (mt : 10H); de 2,60 à 3,05 (mt : 8H); 3,26 (t large, J = 7,5 Hz : 1 H); 4,25 (d large, J = 9 Hz : 1H); 4,45 (s : 1H); 7,00 (mt : 3H); de 7,25 à 7,45 (mt : 8H)].

### Exemple 55

Le (RS)-2-{1-[bis-(4-chlorophényl)méthyl]-azétidin-3-yl}-2-(3,5-difluorophényl)-N-(tétrahydrofuran-2-ylméthyl)-acétamide peut être préparé de la manière suivante : A une solution de 50 mg de chlorhydrate de l'acide (RS)-{1-[bis-(4-chlorophényl)méthyl]-azétidin-3-yl}-(3,5-difluorophényl)-acétique dans 2cm³ de dichlorométhane anhydre, à une température voisine de 20°C, sont ajoutés successivement 0,013 cm³ de tétrahydrofurfurylamine, 29 mg de chlorhydrate de 1-(3-diméthylaminopropyl)-3-éthylcarbodiimide, 0,028 cm³ de triéthylamine et 1,5 mg d'hydrate d'hydroxybenzotriazole. La solution obtenue est agitée à une température voisine de 20°C pendant environ 12 heures. Le milieu réactionnel est déposé sur une cartouche Varian (6 cm³) garnie avec 3 g de silice fine (0,040-0,063 mm), conditionnée puis éluée au dichlorométhane à l'aide d'une pompe Duramat en recueillant des fractions de 2 cm³. Les fractions 3 à 8 sont réunies et concentrées à sec sous pression réduite (2,7 kPa) à 40°C. On obtient ainsi 27 mg de 2(RS)-2-{1-[bis-(4-chlorophényl)méthyl]-azétidin-3-yl}-2-(3,5-difluorophényl)-N-(tétrahydrofuran-2-ylméthyl)-acétamide sous forme d'une solide blanc [Spectre de R.M.N ¹H (300 MHz, CDCl₃, δ en ppm) : de 1,65 à 1,95 (mt : 4H); 2,64 (mt : 1H); 2,89 (dd, J = 7,5 et 5,5 Hz : 1 H); de 3,00 à 3,20 (mt : 3H); de 3,30 à 3,60 (mt : 2H); 3,58 (d, J = 10,5 Hz : 1 H); de 3,65 à 3,95 (mt : 3H); 4,25 (s : 1 H); 5,81 (mt : 1 H); 6,68 (tt, J = 9 et 2,5 Hz : 1 H); 6,82 (mt : 2H); de 7,15 à 7,35 (mt : 8H)].

### Exemple 56

Le (RS)-2-{1-[bis-(4-chlorophényl)méthyl]-azétidin-3-yl}-2-(3,5-difluorophényl)-N-(2-morpholin-4-yl-éthyl)-acétamide peut être préparé de la manière suivante : A une solution de 50 mg de chlorhydrate de l'acide (RS)-{1-[bis-(4-chlorophényl)méthyl]-azétidin-3-yl}-(3,5-difluorophényl)-acétique dans 2 cm³ de dichlorométhane anhydre, à une température voisine de 20°C, sont ajoutés successivement 0,016 cm³ d'aminoéthylmorpholine, 29 mg de chlorhydrate de 1-(3-diméthylaminopropyl)-3-éthylcarbodiimide, 0,028 cm³ de triéthylamine et 1,5 mg d'hydrate d'hydroxybenzotriazole. La solution obtenue est agitée à une température voisine de 20°C pendant environ 12 heures. Le milieu réactionnel est déposé sur une cartouche Varian (6 cm³) garnie avec 3 g de silice fine (0,040-0,063 mm), conditionnée au dichlorométhane à l'aide d'une pompe Duramat, éluée successivement avec un mélange dichlorométhane-acétate d'éthyle (70-30 en volumes) en recueillant des fractions de 2 cm³ pour les fractions 1 à 12, puis avec un mélange dichlorométhane-méthanol (98-2 en volumes) en recueillant des fractions de 2 cm³ pour les fractions 12 à 27. Les fractions 13 à 27 sont réunies et concentrées à sec sous pression réduite (2,7 kPa) à 40°C. On obtient ainsi 34 mg de (RS)-2-{1-[bis-(4-chlorophényl)méthyl]-azétidin-3-yl}-2-(3,5-difluorophényl)-N-(2-morpholin-4-yl-éthyl)-acétamide sous forme d'une meringue blanche [Spectre de R.M.N ¹H (300 MHz, CDCl₃, δ en ppm) : 2,33 (t large, J = 5 Hz : 4H); 2,38 (t, J = 7 Hz : 2H); 2,65 (mt : 1 H); de 2,85 à 3,20 (mt : 3H); 3,25 (q large, J = 7 Hz : 2H); 3,43 (mt : 1H); 3,57 (t, J = 5 Hz : 4H); 3,61 (d, J = 10,5 Hz : 1 H); 4,26 (s : 1 H); 5,98 (mf : 1 H); 6,70 (tt, J = 9 et 2,5 Hz : 1 H); 6,83 (mt : 2H); de 7,15 à 7,35 (mt : 8H)].

### Exemple 57

Le (RS)-2-{1-[bis-(4-chlorophényl)méthyl]-azétidin-3-yl}-2-(3,5-difluorophényl)-N-(1-éthylpyrrolidin-2-ylméthyl)- acétamide peut être préparé de la manière suivante : A une solution de 50 mg de chlorhydrate de l'acide (RS)-{1-[bis-(4-chlorophényl)méthyl]-azétidin-3-yl}-(3,5-difluorophényi)-acétique dans 2 cm³ de dichlorométhane anhydre, à une température voisine de 20°C, sont ajoutés successivement 0,018 cm³ d'aminométhyléthylpyrrolidine, 29 mg de chlorhydrate de 1-(3-diméthylaminopropyl)-3-éthylcarbodiimide, 0,028 cm³ de triéthylamine et 1,5 mg d'hydrate d'hydroxybenzotriazole. La solution obtenue est agitée à une température voisine de 20°C pendant environ 12 heures. Le milieu réactionnel est déposé sur une cartouche Varian (de 12 mm de diamètre) garnie avec 4 cm³ de silice (0,060-0,200 mm) conditionnée au dichlorométhane à l'aide d'un appareil à vide, élution au dichlorométhane entre 0 et 6 cm³ puis avec un mélange dichlorométhane-méthanol (95-5 en volumes). Les fractions contenant le produit cherché sont réunies et concentrées à sec sous pression réduite (2,7 kPa) à 40°C pendant 2 heures. La pâte jaune ainsi obtenue est reprise par 2 cm³ d'acétate d'éthyle, puis par 2 cm³ d'eau distillée. Après agitation, le mélange est congelé, la phase organique est concentrée à sec sous pression réduite (2,7 kPa) à 40°C. Le résidu obtenu est repris par 2 cm³ d'oxyde de di-isopropyle puis est concentré à sec sous pression réduite (2,7 kPa) à 40°C. On obtient ainsi 38 mg de (RS)-2-{1-[bis-(4-chlorophényl)méthyl]-azétidin-3-yl}-2-(3,5-difluorophényl)-N-(1-éthylpyrrolidin-2-ylméthyl)-acétamide sous forme d'une meringue blanche [Spectre de R.M.N ¹H (300 MHz, CDCl₃ avec ajout de quelques gouttes de CD₃COOD d4, δ en ppm) : 0,96 et 1,17 (2t, J = 7,5 Hz : 3H en totalité); de 1,60 à 2,00 (mt: 4H); de 2,50 à 2,95-de 3,10 à 3,85 (mts : 11 H); de 3,95 à 4,10 (mt : 1H); 4,10 et 4,14 (2d, J = 10,5 Hz : 1H en totalité); 5,18 et 5,25 (2s larges : 1H en totalité); 6,66 (mt : 1H); de 6,80 à 7,00 (mt : 2H); de 7,15 à 7,45 (mt : 8H)].

### Exemple 58

Le (RS)-2-{1-[bis-(4-chlorophényl)méthyl]-azétidin-3-yl}-2-(3,5-difluorophényl)-N- isobutylacétamide peut être préparé de la manière suivante : A une solution de 50 mg de chlorhydrate de l'acide (RS)-{1-[bis-(4-chlorophényl)méthyl]-azétidin-3-yl}-(3,5-difluorophényl)-acétique dans 2 cm³ de dichlorométhane anhydre, à une température voisine de 20°C, sont ajoutés successivement 0,012 cm³ d'isobuthylamine, 29 mg de chlorhydrate de 1-(3-diméthylaminopropyl)-3-éthylcarbodiimide, 0,028 cm³ de triéthylamine et 1,5 mg d'hydrate d'hydroxybenzotriazole. La solution obtenue est agitée à une température voisine de 20°C pendant environ 12 heures. Le milieu réactionnel est déposé sur une cartouche Varian (de 12 mm de diamètre) garnie avec 5 cm³ de silice (0,060-0,200 mm) conditionnée au dichlorométhane à l'aide d'un appareil à vide, élution au dichlorométhane entre 0 et 6 cm³ puis avec un mélange dichlorométhane-acétate d'éthyle (95-5 en volumes). Les fractions ne contenant que le produit cherché sont réunies et concentrées à sec sous pression réduite (2,7 kPa) à 40°C. On obtient ainsi 40 mg de (RS)-2-{1-[bis-(4-chlorophényl)méthyl]-azétidin-3-yl}-2-(3,5-difluorophényl)-N- isobutylacétamide sous forme d'une meringue blanche [Spectre de R.M.N ¹H (300 MHz, (CD₃)₂SO d6, δ en ppm) : 0,75 (d, J = 7,5 Hz : 6H); 1,62 (mt : 1H); 2,61 (mt : 1H); de 2,70 à 2,95 (mt : 3H); 3,03 (mt : 2H); 3,22 (t large, J = 7 Hz : 1 H); 3,83 (d, J = 10,5 Hz : 1 H); 4,45 (s : 1 H); 7,00 (mt : 2H); 7,08 (tt, J = 9 et 2,5 Hz : 1H); de 7,25 à 7,45 (mt : 8H); 8,11 (t, J=6 Hz : 1H)].

### Exemple 59

Le (RS)-2-{1-[bis-(4-chlorophényl)méthyl]-azétidin-3-yl}-2-(3,5-difluorophényl)-N,N-diméthylacétamide peut être préparé de la manière suivante : A une solution de 50 mg de chlorhydrate de l'acide (RS)-{1-[bis-(4-chlorophényl)méthyl]-azétidin-3-yl}-(3,5-difluorophényl)-acétique dans 2 cm³ de dichlorométhane anhydre, à une température voisine de 20°C, sont ajoutés successivement 0,060 cm³ de diméthylamine en solution 2M dans le tétrahydrofuranne, 29 mg de chlorhydrate de 1-(3-diméthylaminopropyl)-3-éthylcarbodiimide, 0,028 cm³ de triéthylamine et 1,5 mg d'hydrate d'hydroxybenzotriazole. La solution obtenue est agitée à une température voisine de 20°C pendant environ 12 heures. Le milieu réactionnel est déposé sur une cartouche Varian (de 12 mm de diamètre) garnie avec 4,4 cm³ de silice (0,060-0,200 mm) conditionnée au dichlorométhane à l'aide d'un appareil à vide, élution au dichlorométhane entre 0 et 6 cm³ puis avec un mélange dichlorométhane-acétate d'éthyle (95-5 en volumes). Les fractions ne contenant que le produit cherché sont réunies et concentrées à sec sous pression réduite (2,7 kPa) à 40°C. On obtient ainsi 13 mg de (RS)-2-{1-[bis-(4-chlorophényl)méthyl]-azétidin-3-yl}-2-(3,5-difluorophényl)-N,N-diméthylacétamide sous forme d'une poudre blanche [Spectre de R.M.N ¹H (300 MHz, CDCl₃, δ en ppm) : de 2,70 à 2,80 (mt : 2H); 2,92 (s : 3H); 2,95 (s : 3H); de 3,00 à 3,15 (mt : 2H); 3,38 (t large, J = 7,5 Hz : 1H); 3,96 (d, J = 10 Hz : 1 H); 4,26 (s : 1 H); 6,69 (tt, J = 9 et 2,5 Hz : 1 H); 6,81 (mt : 2H); de 7,20 à 7,35 (mt : 8H)].

### Exemple 60

Le (RS)-2-{1-[bis-(-4-chlorophényl)méthyl]-azétidin-3-yl}-2-(3,5-difluorophényl) N-benzylacétamide peut être préparé de la manière suivante : A une solution de 50 mg de chlorhydrate de l'acide (RS)-{1-[bis-(4-chlorophényl)méthyl]-azétidin-3-yl}-(3,5-difluorophényl)-acétique dans 2 cm³ de dichlorométhane anhydre, à une température voisine de 20°C, sont ajoutés successivement 0,013 cm³ de benzylamine, 29 mg de chlorhydrate de 1-(3-diméthylaminopropyl)-3-éthylcarbodiimide, 0,028 cm³ de triéthylamine et 1,5 mg d'hydrate d'hydroxybenzotriazole. La solution obtenue est agitée à une température voisine de 20°C pendant environ 12 heures. Le milieu réactionnel est déposé sur une cartouche Varian (de 12 mm de diamètre) garnie avec 4,4 cm³ de silice (0,060-0,200 mm) conditionnée au dichlorométhane à l'aide d'un appareil à vide, élution au dichlorométhane entre 0 et 6 cm³ puis avec un mélange dichlorométhane-acétate d'éthyle (95-5 en volumes). Les fractions ne contenant que le produit cherché sont réunies et concentrées à sec sous pression réduite (2,7 kPa) à 40°C. On obtient ainsi 37 mg de (RS)-2-{1-[bis-(4-chlorophényl)méthyl]-azétidin-3-yl}-2-(3,5-difluorophényl)-N-benzylacétamide sous forme de cristaux blancs [Spectre de R.M.N ¹H (300 MHz, CDCl₃, δ en ppm) : 2,67 (mt : 1 H); 2,93 (mt : 1 H); 3,11 (mt : 2H); 3,43 (t large, J = 7,5 Hz : 1 H); 3,62 (d, J = 10,5 Hz : 1 H); 4,26 (s : 1H); 4,38 (mt: 2H); 5,71 (mt: 1H); 6,71 (t large, J = 9 Hz : 1H); 6,83 (mt: 2H); de 7,10 à 7,40 (mt : 13H)].

### Exemple 61

Le (RS)-2-{1-[bis-(4-chlorophényl)méthyl]-azétidin-3-yl}-2-(3,5-difluorophényl)-N-cyclohéxylméthylacétamide peut être préparé de la manière suivante : A une solution de 50 mg de chlorhydrate de l'acide{1-[bis-(4-chlorophényl)méthyl]-azétidin-3-yl}-(3,5-difluorophényl)-acétique dans 2cm³ de dichlorométhane anhydre, à une température voisine de 20°C, sont ajoutés successivement 0,016 cm³ d'aminométhylcyclohéxane, 29 mg de chlorhydrate de 1-(3-diméthylaminopropyl)-3-éthylcarbodiimide, 0,028 cm³ de triéthylamine et 1,5 mg d'hydrate d'hydroxybenzotriazole. La solution obtenue est agitée à une température voisine de 20°C pendant environ 12 heures. Le milieu réactionnel est déposé sur une cartouche Varian (6 cm³) garnie avec 3 g de silice fine (0,040-0,063 mm), conditionnée puis éluée au dichlorométhane à l'aide d'une pompe Duramat en recueillant des fractions de 2 cm³. Les fractions 2 à 3 sont réunies et concentrées à sec sous pression réduite (2,7 kPa) à 40°C. On obtient ainsi 49 mg de (RS)-2-{1-[bis-(4-chlorophényl)méthyl]-azétidin-3-yl}-2-(3,5-difluorophényl)-N-cyclohéxylméthyl-acétamide sous forme d'une meringue blanche [Spectre de R.M.N ¹H (300 MHz, CDCl₃, δ en ppm) : de 0,75 à 1,75 (mt : 11H); 2,65 (mt : 1H); de 2,85 à 3,15 (mt : 5H); 3,42 (t large, J = 7,5 Hz : 1 H); 3,57 (d, J = 10,5 Hz : 1 H); 4,27 (s : 1 H); 5,40 (mt : 1 H); 6,71 (t large, J = 9 Hz : 1 H); 6,83 (mt : 2H); de 7,15 à 7,40 (mt : 8H)].

### Exemple 62

Le (RS)-2-{1-[bis-(4-chlorophényl)méthyl]-azétidin-3-yl}-2-(3,5-difluorophényl)-N-[3-(2-oxo-pyrrolidin-1-yl)-propyl]-acétamide peut être préparé de la manière suivante : A une solution de 50 mg de chlorhydrate de l'acide (RS)-{1-[bis-(4-chlorophényl)méthyl]-azétidin-3-yl}-(3,5-difluorophényl)-acétique dans 2cm³ de dichlorométhane anhydre, à une température voisine de 20°C, sont ajoutés successivement 0,017 cm³ d'aminopropylpyrrolidinone, 29 mg de chlorhydrate de 1-(3-diméthylaminopropyl)-3-éthylcarbodümide, 0,028 cm³ de triéthylamine et 1,5 mg d'hydrate d'hydroxybenzotriazole. La solution obtenue est agitée à une température voisine de 20°C pendant environ 12 heures. Le milieu réactionnel est déposé sur une cartouche Varian (6 cm³) garnie avec 3 g de silice fine (0,040-0,063 mm), conditionnée puis éluée au dichlorométhane à l'aide d'une pompe Duramat en recueillant des fractions de 2 cm³. Les fractions 4 à 12 sont réunies et concentrées à sec sous pression réduite (2,7 kPa) à 40°C. On obtient ainsi 35 mg de (RS)-2-{1-[bis-(4-chlorophényl)méthyl]-azétidin-3-yl}-2-(3,5-difluorophényl)-N-[3-(2-oxo-pyrrolidin-1-yl)-propyl]-acétamide sous forme d'une meringue blanche [Spectre de R.M.N ¹H (300 MHz, CDCl₃, δ en ppm) : de 1,50 à 1,65 (mt : 2H); 2,04 (mt : 2H); 2,43 (t, J = 8 Hz : 2H); 2,64 (mt : 1 H); 2,88 (dd, J = 7,5 et 5,5 Hz : 1H); de 3,00 à 3,30 (mt : 6H); de 3,30 à 3,45 (mt : 3H); 3,64 (d, J = 10,5 Hz : 1 H); 4,27 (s : 1 H); 6,67 (tt, J = 9 et 2,5 Hz : 1 H); 6,90 (mt : 2H); 7,15 (t, J = 6 Hz : 1H); de 7,20 à 7,35 (mt : 8H)].

### Exemple 63

Le (RS)-2-{1-[bis-(4-chlorophényl)méthyl]-azétidin-3-yl}-2-(3,5-difluorophényl)-N-acétyl(4-méthylpipérazine) peut être préparé de la manière suivante : A une solution de 50 mg de chlorhydrate de l'acide (RS)-{1-[bis-(4-chlorophényl)méthyl]-azétidin-3-yl}-(3,5-difluorophényl)-acétique dans 2 cm³ de dichlorométhane anhydre, à une température voisine de 20°C, sont ajoutés successivement 0,013 cm³ de N-méthylpipérazine, 29 mg de chlorhydrate de 1-(3-diméthylaminopropyl)-3-éthylcarbodiimide, 0,028 cm³ de triéthylamine et 1,5 mg d'hydrate d'hydroxybenzotriazole. La solution obtenue est agitée à une température voisine de 20°C pendant environ 12 heures. Le milieu réactionnel est déposé sur une cartouche Varian (6 cm³) garnie avec 3 g de silice fine (0,040-0,063 mm), conditionnée au dichlorométhane à l'aide d'une pompe Duramat, éluée avec un mélange dichlorométhane-éthanol (98-02 en volumes) en recueillant des fractions de 2 cm³. Les fractions 10 à 24 sont réunies et concentrées à sec sous pression réduite (2,7 kPa) à 40°C. On obtient ainsi 39 mg de (RS)-2-{1-[bis-(4-chlorophényl)méthyl]-azétidin-3-yl}-2-(3,5-difluorophényl)-N-acétyl(4-méthylpipérazine)sous forme d'une meringue blanche [Spectre de R.M.N ¹H (300 MHz, CDCl₃, δ en ppm) : 1,83 (mt : 1H); de 2,10 à 2,45 (mt : 3H); 2,21 (s : 3H); 2,74 (mt : 2H); de 2,95 à 3,15 (mt : 2H); de 3,30 à 3,55 (mt : 4H); 3,73 (mt : 1 H); 3,93 (d, J = 10 Hz : 1 H); 4,25 (s : 1H); 6,69 (tt, J = 9 et 2,5 Hz : 1 H); 6,78 (mt : 2H); de 7,15 à 7,35 (mt : 8H)].

### Exemple 64

Le (RS)-2-{1-[bis-(4-chlorophényl)méthyl]-azétidin-3-yl}-2-(3,5-difluorophényl) -N-(2,2-diméthyl-propyl)-acétamide peut être préparé de la manière suivante : A une solution de 50 mg de chlorhydrate de l'acide (RS)-{1-[bis-(4-chlorophényl)méthyl]-azétidin-3-yl}-(3,5-difluorophényl)-acétique dans 2 cm³ de dichlorométhane anhydre, à une température voisine de 20°C, sont ajoutés successivement 0,014 cm³ de néopentylamine, 29 mg de chlorhydrate de 1-(3-diméthylaminopropyl)-3-éthylcarbodiimide, 0,028 cm³ de triéthylamine et 1,5 mg d'hydrate d'hydroxybenzotriazole. La solution obtenue est agitée à une température voisine de 20°C pendant environ 12 heures. Le milieu réactionnel est déposé sur une cartouche Varian (6 cm³) garnie avec 3 g de silice fine (0,040-0,063 mm), conditionnée au dichlorométhane à l'aide d'une pompe Duramat, éluée avec un mélange dichlorométhane-éther de pétrole (80-20 en volumes) en recueillant des fractions de 1,5 cm³. Les fractions 4 à 8 sont réunies et concentrées à sec sous pression réduite (2,7 kPa) à 40°C. On obtient ainsi 40 mg de (RS)-2-{1-[bis-(4-chlorophényl)méthyl]-azétidin-3-yl}-2-(3,5-difluorophényl)-N-(2,2-diméthylpropyl)-acétamide sous forme d'une meringue blanche [Spectre de R.M.N ¹H (300 MHz, CDCl₃, δ en ppm) : 0,81 (s : 9H); 2,66 (mt : 1H); de 2,90 à 3,20 (mt : 5H); 3,44 (t large, J = 7,5 Hz : 1 H); 3,61 (d, J = 10,5 Hz : 1 H); 4,28 (s : 1 H); 5,40 (mt : 1H); 6,72 (tt, J = 9 et 2,5 Hz : 1 H); 6,85 (mt : 2H); de 7,20 à 7,35 (mt : 8H)].

### Exemple 65

Le (RS)-2-{1-[bis-(4-chlorophényl)méthyl]-azétidin-3-yl}-2-(3,5-difluorophényl)-N-(2-pyrrolidin-1-yl-éthyl)-acétamide peut être préparé de la manière suivante : A une solution de 50 mg de chlorhydrate de l'acide (RS)-{1-[bis-(4-chlorophényl)méthyl]-azétidin-3-yl}-(3,5-difluorophényl)-acétique dans 2 cm³ de dichlorométhane, anhydre, à une température voisine de 20°C, sont ajoutés successivement 0,015 cm³ d'aminoéthylpyrrolidine, 29 mg de chlorhydrate de 1-(3-diméthylaminopropyl)-3-éthylcarbodiimide, 0,028 cm³ de triéthylamine et 1,5 mg d'hydrate d'hydroxybenzotriazole. La solution obtenue est agitée à une température voisine de 20°C pendant environ 12 heures. Le milieu réactionnel est déposé sur une cartouche Varian (6 cm³) garnie avec 3 g de silice fine (0,040-0,063 mm), conditionnée au dichlorométhane à l'aide d'une pompe Duramat, éluée avec un mélange dichlorométhane-éthanol (98-04 en volumes) en recueillant des fractions de 1.5 cm³. Les fractions 13 à 20 sont réunies et concentrées à sec sous pression réduite (2,7 kPa) à 40°C. On obtient ainsi 33 mg de (RS)-2-{1-[bis-(4-chlorophényl)méthyl]-azétidin-3-yl}-2-(3,5-difluorophényl)-N-(2-pyrrolidin-1-yl-éthyl)-acétamide sous forme d'une meringue blanche [Spectre de R.M.N ¹H (300 MHz, CDCl₃, δ en ppm) : 1,78 (mt : 4H); de 2,50 à 2,70 (mt : 3H); 2,54 (mt : 4H); 2,91 (dd, J = 7,5 et 5 Hz : 1 H); 3,10 (mt : 2H); de 3,20 à 3,45 (mt : 3H); 3,64 (d, J = 10,5 Hz : 1 H); 4,27 (s : 1 H); 6,67 (tt, J = 9 et 2,5 Hz : 1H); 6,86 (mt : 2H); de 7,15 à 7,35 (mt : 8H)].

### Exemple 66

Le (RS)-2-{1-[bis-(4-chlorophényl)méthyl]-azétidin-3-yl}-2-(3,5-difluorophényl)-N-cyclopropylméthyl acétamide peut être préparé de la manière suivante : A une solution de 50 mg de chlorhydrate de l'acide (RS)-{1-[bis-(4-chlorophényl)méthyl]-azétidin-3-yl}-(3,5-difluorophényl)-acétique dans 2 cm³ de dichlorométhane anhydre, à une température voisine de 20°C, sont ajoutés successivement 0,011 cm³ de cyclopropaneméthylamine, 29 mg de chlorhydrate de 1-(3-diméthylaminopropyl)-3-éthylcarbodiimide, 0,028 cm³ de triéthylamine et 1,5 mg d'hydrate d'hydroxybenzotriazole. La solution obtenue est agitée à une température voisine de 20°C pendant environ 12 heures. Le milieu réactionnel est déposé sur une cartouche Varian (6 cm³) garnie avec 3 g de silice fine (0,040-0,063 mm), conditionnée puis éluée avec un mélange dichlorométhane-éther de pétrole (80-20 en volumes) à l'aide d'une pompe Duramat en recueillant des fractions de 1.5 cm³. Les fractions 3 à 8 sont réunies et concentrées à sec sous pression réduite (2,7 kPa) à 40°C. On obtient ainsi 37 mg de (RS)-2-{1-[bis-(4-chlorophényl)méthyl]-azétidin-3-yl}-2-(3,5-difluorophényl)-N-cyclopropylméthyl acétamide sous forme d'une meringue blanche [Spectre de R.M.N ¹H (300 MHz, CDCl₃, δ en ppm) : 0,13 (mt : 2H); 0,45 (mt : 2H); 0,86 (mt : 1H); 2,65 (mt : 1 H); 2,91 (dd, J = 7,5 et 5 Hz : 1H); de 3,00 à 3,15 (mt : 4H); 3,41 (t large, J = 7,5 Hz : 1 H); 3,57 (d, J = 10,5 Hz : 1 H); 4,25 (s : 1 H); 5,50 (mt : 1H); 6,70 (tt, J = 9 et 2,5 Hz : 1 H); 6,84 (mt : 2H); de 7,15 à 7,35 (mt : 8H)].

### Exemple 67

Le (RS)-2-{1-[bis-(4-chlorophényl)méthyl]-azétidin-3-yl}-2-(3,5-difluorophényl) -N-propylacétamide peut être préparé de la manière suivante : A une solution de 50 mg de chlorhydrate de l'acide (RS)-{1-[bis-(4-chlorophényl)méthyl]-azétidin-3-yl}-(3,5-difluorophényl)-acétique dans 2 cm³ de dichlorométhane anhydre, à une température voisine de 20°C, sont ajoutés successivement 0,015 cm³ de propylamine, 29 mg de chlorhydrate de 1-(3-diméthylaminopropyl)-3-éthylcarbodiimide, 0,028 cm³ de triéthylamine et 1,5 mg d'hydrate d'hydroxybenzotriazole. La solution obtenue est agitée à une température voisine de 20°C pendant environ 12 heures. Le milieu réactionnel est déposé sur une cartouche Varian (6cm³) garnie avec 3 g de silice fine (0,040-0,063 mm) conditionnée et éluée au dichlorométhane à l'aide d'une pompe Duramat. Les fractions ne contenant que le produit cherché sont réunies et concentrées à sec sous pression réduite (2,7 kPa) à 40°C. On obtient ainsi 21 mg de (RS)-2-{1-[bis-(4-chlorophényl)méthyl]-azétidin-3-yl}-2-(3,5-difluorophényl)-N-propylacétamide sous forme de solide blanc [Spectre de R.M.N ¹H (300 MHz, CDCl₃, δ en ppm) : 0,84 (t, J = 7,5 Hz : 3H); 1,45 (mt : 2H); 2,65 (mt : 1H); 2,92 (dd, J = 7,5 et 5,5 Hz : 1H); de 3,00 à 3,20 (mt : 2H); 3,15 (q, J = 7 Hz : 2H); 4,43 (t large, J = 7,5 Hz : 1H); 3,56 (d, J = 10,5 Hz : 1 H); 4,26 (s : 1 H); 5,39 (mt : 1 H); 6,70 (tt, J = 9 et 2,5 Hz : 1 H); 6,83 (mt : 2H); de 7,15 à 7,35 (mt : 8H].

### Exemple 68

Le (RS)-2-{1-[bis-(4-chlorophényl)méthyl]-azétidin-3-yl}-2-(3,5-difluorophényl)-N-méthylacétamide peut être préparé de la manière suivante : A une solution de 50 mg de chlorhydrate de l'acide (RS)-{1-[bis-(4-chlorophényl)méthyl]-azétidin-3-yl}-(3,5-difluorophényl)-acétique dans 2 cm³ de dichlorométhane anhydre, à une température voisine de 20°C, sont ajoutés successivement 0,050 cm³ d'une solution de méthylamine 2M dans le tétrahydrofuranne, 29 mg de chlorhydrate de 1-(3-diméthylaminopropyl)-3-éthylcarbodiimide, 0,028 cm³ de triéthylamine et 1,5 mg d'hydrate d'hydroxybenzotriazole. La solution obtenue est agitée à une température voisine de 20°C pendant environ 12 heures. Le milieu réactionnel est déposé sur une cartouche Varian (6cm³) garnie avec 3 g de silice fine (0,040-0,063 mm) conditionnée et éluée au dichlorométhane à l'aide d'une pompe Duramat. Les fractions ne contenant que le produit cherché sont réunies et concentrées à sec sous pression réduite (2,7 kPa) à 40°C. On obtient ainsi 19 mg de (RS)-2-{1-[bis-(4-chlorophényl)méthyl]-azétidin-3-yl}-2-(3,5-difluorophényl)-N-méthylacétamide sous forme de solide blanc [Spectre de R.M.N ¹H (300 MHz, CDCl₃, δ en ppm) : 2,66 (mt : 1 H); 2,76 (d, J = 5 Hz : 3H); 2,93 (mt : 1 H); 3,10 (mt : 2H); 3,44 (t large, J = 7,5 Hz : 1H); 3,59 (d, J = 10,5 Hz : 1 H); 4,28 (s : 1 H); 5,41 (mf : 1 H); 6,71 (tt, J = 9 et 2,5 Hz : 1 H); 6,83 (mt : 2H); de 7,20 à 7,35 (mt : 8H)].

### Exemple 69

Le (RS)-2-{1-[bis-(4-chlorophényl)méthyl]-azétidin-3-yl}-2-(3,5-difluorophényl)-N-isopropylacétamide peut être préparé de la manière suivante : A une solution de 50 mg de chlorhydrate de l'acide (RS)-{1-[bis-(4-chlorophényl)méthyl]-azétidin-3-yl}-(3,5-difluorophényl)-acétique dans 2 cm³ de dichlorométhane anhydre, à une température voisine de 20°C, sont ajoutés successivement 0,011 cm³ d'isopropylamine, 29 mg de chlorhydrate de 1-(3-diméthylaminopropyl)-3-éthylcarbodiimide, 0,028 cm³ de triéthylamine et 1,5 mg d'hydrate d'hydroxybenzotriazole. La solution obtenue est agitée à une température voisine de 20°C pendant environ 12 heures. Le milieu réactionnel est déposé sur une cartouche Varian (6 cm³) garnie avec 3 g de silice fine (0,040-0,063 mm), conditionnée puis éluée au dichlorométhane à l'aide d'une pompe Duramat en recueillant des fractions de 1,5 cm³. Les fractions 6 à 14 sont réunies et concentrées à sec sous pression réduite (2,7 kPa) à 40°C. On obtient ainsi 21 mg de (RS)-2-{1-[bis-(4-chlorophényl)méthyl]-azétidin-3-yl}-2-(3,5-difluorophényl)-N-isopropyl-acétamide sous forme d'une meringue blanche [Spectre de R.M.N ¹H (300 MHz, CDCl₃, δ en ppm) : 1,05 (d, J = 7 Hz : 3H); 1,10 (d, J = 7 Hz : 3H); 2,65 (mt : 1 H); 2,90 (dd, J = 7,5 et 5,5 Hz : 1H); de 3,00 à 3,15 (mt : 2H); 3,42 (t large, J = 7,5 Hz : 1H); 3,51 (d, J = 10,5 Hz : 1H); 4,00 (mt : 1 H); 4,26 (s : 1 H); 5,19 (d large, J = 7,5 Hz : 1 H); 6,70 (tt, J = 9 et 2,5 Hz : 1H); 6,82 (mt : 2H); de 7,20 à 7,40 (mt : 8H)].

### Exemple 70

Le (RS)-2-{1-[bis-(4-chlorophényl)méthyl]-azétidin-3-yl}-2-(3,5-difluorophényl)-N-piperidin-1-yl-acétamide peut être préparé de la manière suivante : A une solution de 50 mg de chlorhydrate de l'acide (RS)-{1-[bis-(4-chlorophényl)méthyl]-azétidin-3-yl}-(3,5-difluorophényl)-acétique dans 2 cm³ de dichlorométhane anhydre, à une température voisine de 20°C, sont ajoutés successivement 0,013 cm³ d'aminopipéridine, 29 mg de chlorhydrate de 1-(3-diméthylaminopropyl)-3-éthylcarbodiimide, 0,028 cm³ de triéthylamine et 1,5 mg d'hydrate d'hydroxybenzotriazole. La solution obtenue est agitée à une température voisine de 20°C pendant environ 12 heures. Le milieu réactionnel est déposé sur une cartouche Varian (6 cm³) garnie avec 3 g de silice fine (0,040-0,063 mm), conditionnée au dichlorométhane à l'aide d'une pompe Duramat, éluée avec un mélange dichlorométhane-éthanol (98-02 en volumes) en recueillant des fractions de 2 cm³. Les fractions 7 à 12 sont réunies et concentrées à sec sous pression réduite (2,7 kPa) à 40°C. On obtient ainsi 33 mg de (RS)-2-{1-[bis-(4-chlorophényl)méthyl]-azétidin-3-yl}-2-(3,5-difluorophényl)-N-piperidin-1-yl-acétamide sous forme d'un solide blanc [Spectre de R.M.N ¹H (300 MHz, CDCl₃, δ en ppm) : de 0,95 à 1,85-2,05 et 2,29 (mts : 10H); de 2,60 à 2,80 (mt : 2H); de 3,00 à 3,20 (mt : 2H); 3,39 (t large, J = 7,5 Hz : 1 H); 4,26 (s : 1 H); 4,32 (d, J = 10,5 Hz : 1 H); 6,00 (s : 1 H); 6,65 (tt, J = 9 et 2,5 Hz : 1 H); 6,85 (mt : 2H); de 7,15 à 7,35 (mt : 8H)].

### Exemple 71

Le (RS)-2-{1-[bis-(4-chlorophényl)méthyl]-azétidin-3-yl}-2-(3,5-difluorophényl) -N-cycloheptylacétamide peut être préparé de la manière suivante : A une solution de 50 mg de chlorhydrate de l'acide (RS)-{1-[bis-(4-chlorophényl)méthyl]-azétidin-3-yl}-(3,5-difluorophényl)-acétique dans 2 cm³ de dichlorométhane anhydre, à une température voisine de 20°C, sont ajoutés successivement 0,015 cm³ cyclohéptylamine, 29 mg de chlorhydrate de 1-(3-diméthylaminopropyl)-3-éthylcarbodiimide, 0,028 cm³ de triéthylamine et 1,5 mg d'hydrate d'hydroxybenzotriazole. La solution obtenue est agitée à une température voisine de 20°C pendant environ 12 heures. Le milieu réactionnel est déposé sur une cartouche Varian (6 cm³) garnie avec 3 g de silice fine (0,040-0,063 mm), conditionnée puis éluée au dichlorométhane à l'aide d'une pompe Duramat en recueillant des fractions de 2 cm³. Les fractions 3 à 6 sont réunies et concentrées à sec sous pression réduite (2,7 kPa) à 40°C. On obtient ainsi 24 mg de (RS)-2-{1-[bis-(4-chlorophényl)méthyl]-azétidin-3-yl}-2-(3,5-difluorophényl)-N-cycloheptylacétamide sous forme de meringue blanche [Spectre de R.M.N ¹H (300 MHz, CDCl₃, δ en ppm) : de 1,20 à 1,95 (mt : 12H); 2,65 (mt : 1H); 2,90 (dd, J =7.5 et 5,5 Hz : 1H); de 3,00 à 3,15 (mt : 2H); 3,42 (t large, J = 7,5 Hz : 1 H); 3,52 (d, J = 10,5 Hz : 1 H); 3,86 (mt : 1H); 4,26 (s : 1 H); 5,31 (d large, J = 7,5 Hz : 1 H); 6,70 (tt, J = 9 et 2,5 Hz : 1 H); 6,82 (mt : 2H); de 7,20 à 7,35 (mt : 8H)].

### Exemple 72

Le (RS)-2-{1-[bis-(4-chlorophényl)méthyl]-azétidin-3-yl}-2-(3,5-difluorophényl)-acétamide peut être préparé de la manière suivante : A une solution de 98 mg de chlorhydrate de l'acide (RS)-{1-[bis-(4-chlorophényl)méthyl]-azétidin-3-yl}-(3,5-difluorophényl)-acétique dans 4 cm³ de dichloroéthane anhydre, à une température voisine de 20°C, sont ajoutés successivement 60 mg de chlorhydrate de 1-(3-diméthylaminopropyl)-3-éthylcarbodiimide, et 3 mg d'hydrate d'hydroxybenzotriazol on fait buller de l'ammoniac pendant 2 heures avec agitation à une température voisine de 20°C. Le milieu réactionnel est lavé à l'eau puis est déposé sur une cartouche Varian (6 cm³) garnie avec 3g de silice fine (0,040-0,063 mm) conditionnée et éluée avec un mélange dichlorométhane-acétate d'éthyle (9-1 en volumes) à l'aide d'une pompe Duramat. Les fractions comprises entre 36 et 80 ml sont réunies et concentrées à sec sous pression réduite (2,7 kPa) à 40°C. On obtient ainsi 32 mg de (RS)-2-{1-[bis-(4-chlorophényl)méthyl]-azétidin-3-yl}-2-(3,5-difluorophényl)-acétamide sous forme de poudre amorphe [Spectre de R.M.N ¹H (300 MHz, CDCl₃, δ en ppm) : 2,66 (mt : 1H); 2,95 (dd, J = 7 et 5 Hz : 1H); de 2,95 à 3,15 (mt : 2H); 3,45 (t large, J = 7 Hz : 1H); 3,67 (d, J = 10,5 Hz : 1 H); 4,27 (s : 1 H); de 5,20 à 5,40 (mf : 2H); 6,72 (tt, J = 9 et 2,5 Hz : 1 H); 6,84 (mt : 2H); de 7,20 à 7,35 (mt : 8H)].

### Exemple 73

Le (RS)-2-{1-[bis-(4-chlorophényl)méthyl]-azétidin-3-yl}-2-(3,5-difluorophényl)-N-acétylamino acétate de méthyle peut être préparé de la manière suivante : A une solution de 200 mg de chlorhydrate de l'acide (RS)-{1-[bis-(4-chlorophényl)méthyl]-azétidin-3-yl}-(3,5-difluorophényl)-acétique dans 8 cm³ de dichloroéthane, à une température voisine de 20°C, sont ajoutés successivement 100 mg de chlorhydrate d'ester méthylique de la glycine, 115 mg de chlorhydrate de 1-(3-diméthylaminopropyl)-3-éthylcarbodiimide, et 6 mg d'hydrate d'hydroxybenzotriazole. La solution obtenue est agitée à une température voisine de 20°C pendant environ 12 heures. Le milieu réactionnel est lavé à l'eau, séché, filtré, puis concentré à sec sous pression réduite (2,7 kPa) à 40°C. Le résidu ainsi obtenu est repris par 1 cm³ de dichlorométhane, puis déposé sur une cartouche IST FlashPack de référence SIL-020-005 conditionnée et éluée avec un mélange dichlorométhane-acétate d'éthyle (95-05 en volumes) à l'aide d'une pompe Duramat. Les fractions comprises entre 18 et 42 ml sont réunies et concentrées à sec sous pression réduite (2,7 kPa) à 40°C. On obtient ainsi 68 mg (RS)-2-{1-[bis-(4-chlorophényl)méthyl]-azétidin-3-yl}-2-(3,5-difluorophényl)-N-acétylamino acétate de méthyle sous forme de cristaux cotonneux blancs [Spectre de R.M.N ¹H (300 MHz, CDCl₃, δ en ppm) : 2,67 (met : 1H); 2,93 (dd large, J = 7,5 et 5 Hz : 1 H); de 3,00 à 3,15 (mt : 2H); 3,41 (t large, J = 7,5 Hz : 1 H); 3,68 (d, J = 10,5 Hz : 1 H); 3,74 (s : 3H); 3,93 (dd, J = 18 et 5 Hz : 1 H); 4,03 (dd, J = 18 et 5 Hz : 1 H); 4,27 (s : 1 H); 5,96 (mt : 1 H); 6,71 (tt, J = 9 et 2 Hz : 1H); 6,85 (mt : 2H); de 7,20 à 7,35 (mt : 8H)].

### Exemple 74

Le (RS)-2-{1-[bis-(4-chlorophényl)méthyl]-axétidin-3-yl}-2-(3,5-difluorophényl)-N-(3-diméthylamino-propyl)-acétamide peut être préparé de la manière suivante : A une solution de 50 mg de chlorhydrate de l'acide (RS)-{1-[bis-(4-chlorophényl)méthyl]-azétidin-3-yl}-(3,5-difluorophényl)-acétique dans 2 cm³ de dichlorométhane anhydre, à une température voisine de 20°C, sont ajoutés successivement 0,015 cm³ de N,N-diméthylpropane-1,3-diamine, 29 mg de chlorhydrate de 1-(3-diméthylaminopropyl)-3-éthylcarbodiimide, 0,028 cm³ de triéthylamine et 1,5 mg d'hydrate d'hydroxybenzotriazole. La solution obtenue est agitée à une température voisine de 20°C pendant environ 12 heures. Le milieu réactionnel est déposé sur une cartouche Varian (de 12 mm de diamètre) garnie avec 4 cm³ de silice (0,060-0,200 mm) conditionnée au dichlorométhane à l'aide d'un appareil à vide, élution au dichlorométhane entre 0 et 6 cm³ puis avec un mélange dichlorométhane-méthanol (95-5 en volumes). Les fractions ne contenant que le produit cherché sont réunies et concentrées à sec sous pression réduite (2,7 kPa) à 40°C. On obtient ainsi 33 mg (RS)-2-{1-[bis-(4-chlorophényl)méthyl]-azétidin-3-yl}-2-(3,5-difluorophényl)-N-(3-diméthylamino-propyl)-acétamide sous forme de cristaux blancs [Spectre de R.M.N ¹H (300 MHz, CDCl₃ avec ajout de quelques gouttes de CD₃COOD d4, δ en ppm) : 1,91 (mt : 2H); 2,71 (s : 6H); 2,95 (mt : 2H); de 3,15 à 3,40 (mt : 2H); de 3,40 à 3,60 (mt : 1H); de 3,60 à 3,80 (mt : 2H); 4,00 (mt : 2H); 4,28 (d, J = 10,5 Hz : 1H); 5,22 (s : 1H); 6,68 (tt, J = 9 et 2,5 Hz : 1 H); 6,90 (mt : 2H); 7,33 (mt : 4H); 7,46 (d, J = 8 Hz : 4H)].

### Exemple 75

Le (RS)-2-{1-[bis-(4-chlorophényl)méthyl]-azétidin-3-yl}-2-(3,5-difluorophényl)-N-(2-hydroxy-éthyl)-acétamide peut être préparé de la manière suivante : A une solution de 50mg de chlorhydrate de l'acide (RS)-{1-[bis-(4-chlorophényl)méthyl]-azétidin-3-yl}-(3,5-difluorophényl)-acétique dans 2 cm³ de dichlorométhane anhydre, à une température voisine de 20°C, sont ajoutés successivement 0,024 cm³ d'éthanolamine, 29 mg de chlorhydrate de 1-(3-diméthylaminopropyl)-3-éthylcarbodiimide, 0,028cm³ de triéthylamine et 1,5 mg d'hydrate d'hydroxybenzotriazole. La solution obtenue est agitée à une température voisine de 20°C. Le milieu réactionnel est lavé avec 2 cm³ d'une solution saturée de bicarbonate de sodium, séché sur sulfate de magnésium, puis est déposée sur une cartouche IST FlashPack de référence SIL-016-002 conditionnée au dichlorométhane et éluée avec un mélange dichlorométhane-acétate d'éthyle (95-05 en volumes) à l'aide d'une pompe Duramat. Les fractions comprises entre 25 et 60 cm³ sont réunies et concentrées à sec sous pression réduite (2,7 kPa) à 40°C. Le résidu obtenu est à nouveau chromatographié sur une cartouche IST FlashPack de référence SIL-016-002 conditionnée au dichlorométhane et éluée avec un mélange dichlorométhane-acétate d'éthyle (95-05 en volumes) à l'aide d'une pompe Duramat. Les fractions comprises entre 25 et 35 cm³ sont réunies et concentrées à sec sous pression réduite (2,7 kPa) à 40°C. On obtient ainsi 14 mg de (RS)-2-{1-[bis-(4-chlorophényl)méthyl]-azétidin-3-yl}-2-(3,5-difluorophényl)-N-(2-hydroxy-éthyl)-acétamide sous forme de solide blanc [Spectre de R.M.N. ¹H (300 MHz, CDCl₃, δ en ppm) : 2,65 (mt : 1H); 2,91 (mt : 1H); de 3,00 à 3,15 (mt : 2H); de 3,30 à 3,50 (mt : 3H); 3,60 (d, J = 10,5 Hz : 1H); 3,66 (t, J = 5,5 Hz : 2H); 4,26 (s : 1 H); 5,88 (mt : 1 H); 6,71 (tt, J = 9 et 2 Hz : 1 H); 6,84 (mt : 2H); de 7,20 à 7,35 (mt : 8H)].

### Exemple 76

La (RS)-1-[{1-[bis-(4-chlorophényl)méthyl]-azétidin-3-yl}-(3,5-difluorophényl)-méthyl]-3-propylurée peut être préparé de la manière suivante : A une solution de 50 mg de chlorhydrate de l'acide (RS)-{1-[bis-(4-chlorophényl)méthyl]-azétidin-3-yl}-(3,5-difluorophényl)-acétique dans 3 cm³ de toluène anhydre, sous atmosphère inerte d'argon, à une température voisine de 20°C, sont ajoutés successivement, 0,056 cm³ de triéthylamine et 0,064 cm³ de diphénylphosphonoazide. La solution obtenue est agitée à une température voisine de 50°C pendant environ 1 heure. On ajoute 0,016 cm³ de propylamine, l'agitation est maintenue à une température voisine de 20°C pendant environ 12 heures. Le milieu réactionnel est concentré à sec sous pression réduite (2,7 kPa) à une température voisine de 40°C. Le résidu est repris par 1cm³ de dichlorométhane puis déposé sur une cartouche Varian (6 cm³) garnie avec 3 g de silice fine (0,040-0,063 mm) conditionnée et éluée avec un mélange dichlorométhane-acétate d'éthyle (95-5 en volumes) à l'aide d'une pompe Duramat. Les fractions comprises entre 12 et 16 cm³ sont réunies et concentrées à sec sous pression réduite (2,7 kPa) à 40°C. On obtient ainsi 13 mg de (RS)-1-[{1-[bis-(4-chlorophényl)méthyl]-azétidin-3-yl}-(3,5-difluorophényl)-méthyl]-3-propylurée sous forme d'un solide beige [Spectre de R.M.N ¹H (400 MHz, CDCl₃, δ en ppm) : 0,92 (t, J = 7,5 Hz : 3H); 1,53 (mt : 2H); de 2,55 à 2,75 (mt : 1 H); de 2,80 à 3,25 (mt : 6H); 4,26 (t, J = 5,5 Hz : 1 H); 4,29 (s : 1 H); 4,92 (t, J = 7 Hz : 1 H); 5,31 (d, J = 5,5 Hz : 1 H); 6,66 (tt, J = 9 et 2,5 Hz : 1 H); 6,79 (mt : 2H); de 7,15 à 7,40 (mt : 8H)].

### Exemple 77

Le chlorhydrate de l'acide (RS)-{1-[bis-(4-chlorophényl)méthyl]-azétidin-3-yl}-(3,5-difluorophényl)-acétique peut être préparé de la manière suivante : A une solution de 0,44 g d'ester éthylique de l'acide (RS)-{1-[bis-(4-chlorophényl)méthyl]-azétidin-3-yl}-(3,5-difluorophényl)-acétique dans 7 cm³ de dioxane, est rajouté 3 cm³ d'acide chlorhydrique 6 N. La solution obtenue est agitée au reflux pendant environ 2 heures, puis laissé à une température voisine de 20°C pendant environ 12 heures. Le précipité formé est filtré sur verre fritté N° 3, lavé par 10 cm³ d'oxyde de di-isopropyle, puis séché sous pression réduite (0,27 kPa) à une température voisine de 40°C. On obtient ainsi 0,185 g de chlorhydrate de l'acide (RS)-{1-[bis-(4-chlorophényl)méthyl]-azétidin-3-yl}-(3,5-difluorophényl)-acétique sous forme de poudre blanche [Spectre de R.M.N ¹H (400 MHz, (CD₃)₂SO d6, à une température de 363K, δ en ppm) : 2,87 (dd, J = 14 et 4 Hz : 1 H); 2,95 (mt : 1 H); 3,18 (mt : 1H); 3,96 (d, J = 10,5 Hz : 1H); 4,18 (t, J = 9 Hz : 1H); 4,72 (t, J = 9 Hz : 1H); 5,26 (mf : 1 H); 7,00 (mt : 2H); 7,06 (tt, J = 9,5 et 2,5 Hz : 1 H); de 7,30 à 7,60 (mt : 8H)].

### Exemple 78

L'ester éthylique de l'acide (RS){1-[bis-(4-chlorophényl)méthyl]-azétidin-3-yl}-(3,5-difluorophényl)-acétique peut être préparé de la manière suivante : A une suspension de 0,78 g d'ester éthylique de l'acide (RS)-{1-[bis-(4-chlorophényl)méthyl]-azétidin-3-ylidene}-(3,5-difluorophényl)-acétique dans 20 cm³ d'éthanol, on rajoute 121 mg de borohydrure de sodium, à une température voisine de 0°C. La suspension obtenue est agitée à une température voisine de 20°C pendant environ 12 heures. Le milieu réactionnel est versé sur 200 cm³ d'eau distillée puis extrait par 3 fois 40 cm³ d'acétate d'éthyle. La phase organique est successivement lavée par 3 fois 40 cm³ d'eau distillée puis par 40 cm³ d'une solution saturée en chlorure de sodium. Après décantation, la phase organique est séchée sur sulfate de magnésium, filtrée, et concentrée à sec sous pression réduite (2,7 kPa) à une température voisine de 40°C. Le résidu obtenu est chromatographié sur colonne garnie de 75 cm³ de silice fine (0,040-0,063 mm) sous une pression de 0,7 bar avec un mélange dichlorométhane-acétate d'éthyle (le pourcentage d'acétate d'éthyle variant de 0 à 10 %) en recueillant des fractions de 15 cm³ . Les fractions 4 à 11 sont réunies et concentrées à sec sous pression réduite (2,7 kPa) à 40°C. On obtient ainsi 0,46 g d'ester éthylique de l'acide (RS)-{1-[bis-(4-chlorophényl)méthyl]-azétidin-3-yl}-(3,5-difluorophényl)-acétique sous forme de laque jaune [Spectre de R.M.N. ¹H (300 MHz, CDCl₃, δ en ppm) : 1,19 (t, J = 7 Hz : 3H); 2,62 (t large, J = 6 Hz : 1 H); 2,87 (dd, J = 7,5 et 6 Hz : 1H); de 2,95 à 3,15 (mt : 2H); 3,39 (t large, J = 7,5 Hz : 1H); 3,78 (d, J = 10,5 Hz : 1H); 4,10 (mt : 2H); 4,25 (s : 1H); 6,69 (tt,

J = 9 et 2,5 Hz : 1H); 6,80 (mt : 2H); de 7,20 à 7,35 (mt : 8H)].

Les fractions 16 à 26 de la chromatographie précédente sont réunies et concentrées à sec sous pression réduite (2,7 kPa) à 40°C. On obtient ainsi 0,24 g de (RS)-2-{1-[bis-(4-chlorophényl)méthyl]-azétidin-3-yl}-2-(3,5-difluorophényl)-éthanol sous forme de meringue jaune [Spectre de R.M.N.¹H (300 MHz, CDCl₃, δ en ppm) : 1,98 (mf : 1H); 2,69 (mt : 1H); de 2,70 à 2,85 (mt : 1 H); de 2,90 à 3,10 (mt : 2H); 3,18 (mt : 1 H); 3,44 (mt : 1H); de 3,65 à 3,85 (mt : 2H); 4,28 (s : 1H); de 6,60 à 6,80 (mt : 3H); de 7,20 à 7,35 (mt : 8H)].

L'ester éthylique de l'acide {1-[bis-(4-chlorophényl)méthyl]-azétidin-3-ylidène}-(3,5-difluorophényl)-acétique peut être préparé de la manière suivante : A une solution de 9,1 g d'ester éthylique de l'acide {1-[bis-(4-chlorophényl)méthyl]-3-hydroxy-azétidin-3-yl}-(3,5-difluorophényl)-acétique dans 200 cm³ de dichlorométhane, on rajoute 6.6 g de 4-diméthylaminopyridine, 2,1 cm³ de chlorure de méthylsulfonyle. La solution obtenue est agitée à une température voisine de 20°C pendant environ 12 heures. Le mélange réactionnel est lavé 3 fois avec 250 cm³ d'eau distillée, puis séché avec du sulfate de magnésium, filtré, et concentré à sec sous pression réduite (2,7 kPa) à une température voisine de 40°C. Le résidu obtenu est repris à chaud par 50 cm³ d'oxyde d'isopropyle puis abandonné à une température voisine de 20°C pendant environ 12 heures. La suspension blanche obtenue est filtré sur verre fritté, lavée par 20cm³ d'éther de pétrole puis séchée sous vide sous pression réduite (0,27 kPa) à une température voisine de 40°C pendant 2 heures. On obtient ainsi 7,9 g d'ester éthylique de l'acide {1-[bis-(4-chlorophényl)méthyl]-azétidin-3-ylidene}-(3,5-difluorophényl)-acétique sous forme de poudre crème [Spectre de R.M.N. ¹H (300 MHz; CDCl₃, δ en ppm) : 1,25 (t, J = 7 Hz : 3H); 3,85 (mt : 2H); 4,12 (AB, J = 7,5 Hz : 2H); 4,23 (mt : 2H); 4,51 (s : 1H); de 6,65 à 6,80 (mt : 3H); de 7,20 à 7,40 (mt : 8H)].

L'ester éthylique de l'acide {1-[bis-(4-chlorophényl)méthyl]-3-hydroxy-azétidin-3-yl}-(3,5-difluorophényl)-acétique peut être préparé de la manière suivante : A une solution de 7,73 cm³ de diisopropylamine dans 125 cm³ de tétrahydrofuranne anhydre, sous atmosphère inerte d'azote, à une température voisine de -70°C, sont ajoutés au goutte à goutte 34,54 cm³ d'une solution de butyllithium 1,6 M dans l'hexane en 15 minutes, l'agitation est maintenue à cette température pendant 45 minutes, est ajouté en 15 minutes une solution de 11,01 g de 3,5-difluorophénylacétate d'éthyle dans 85 cm³ de tétrahydrofuranne anhydre, l'agitation est poursuivie pendant 1 heure à -78°C, est ajouté 16,84 g 1-[bis-(4-chlorophényl)méthyl]-azétidin-3-one dans 90 cm³ de tétrahydrofuranne anhydre, l'agitation est poursuivie 1 heure à -70°C, est ajouté à 0°C sous vive agitation 300 cm³ d'une solution saturée de chlorure d'ammonium en 30 minutes., le mélange réactionnel est décanté après 12 heures, la phase organique est lavée 3 fois avec une solution saturée de bicarbonate de sodium, séchée sur sulfate de magnésium, filtrée puis concentré à sec sous pression réduite (2,7 kPa). Le résidu obtenu est chromatographié sur colonne de diamètre 70 mn garnie de 2000 cm³ de silice fine (0,040-0,063mm) sous une pression de 0,7 bars avec un mélange dichlorométhane-acétate d'éthyle (99-01 en volumes). Les fractions ne contenant que le produit cherché sont réunies et concentrées à sec sous pression réduite (2,7 kPa) à 40°C pendant 2 heures. On obtient ainsi 9,1 g d'ester éthylique de l'acide {1-[bis-(4-chlorophényl)méthyl]-3-hydroxy-azétidin-3-yl}-(3,5-difluorophényl)-acétique sous forme de solide crème [Spectre de R.M.N. ¹H (300 MHz, CDCl₃, δ en ppm) : 1,25 (t, J = 7 Hz : 3H); 2,87 (d, J = 8 Hz : 1 H); 3,07 (d, J = 8 Hz : 1 H); 3,13 (d large, J = 8 Hz : 1 H); 3,28 (d large, J = 8 Hz : 1 H); 4,12 (s : 2H); 4,21 (mt : 2H); 4,36 (s : 1 H); 6,78 (tt, J = 9 et 2,5 Hz : 1 H); 6,98 (mt : 2H); de 7,20 à 7,40 (mt : 8H)].

Le 3,5-difluorophénylacétate d'éthyle peut être préparé de la manière suivante : A une solution de 12 cm³ d'éthanol dans 300 cm³ de dichlorométhane anhydre, à une température voisine de 20°C, sont ajoutés successivement 20,4cm³ de triéthylamine, puis 27,6 g du chlorure de l'acide 3,5-difluorophénylacétique en solution dans 60 cm³ de dichlorométhane. La solution obtenue est agitée à une température voisine de 20°C pendant environ 12 heures. Le mélange réactionnel est lavé successivement par 2 fois 150 cm³ d'une solution d'acide chlorhydrique décinormale, puis par 2 fois 150 cm³ d'une solution saturée de bicarbonate de sodium. La phase organique est séchée avec du sulfate de magnésium, filtré, et concentré à sec sous pression réduite (2,7 kPa) à une température voisine de 40°C. On obtient ainsi 29 g de 3,5-difluorophénylacétate d'éthyle sous forme d'huile jaune.

Le chlorure de l'acide 3,5-difluorophénylacétique peut être préparé de la manière suivante : A une solution de 25 g d'acide 3,5-difluorophénylacétique dans 350 cm³ de 1,2-dichloroéthane, à une température voisine de 20°C, sont ajoutés successivement 19,3 cm³ de chlorure d'oxalyle, puis quelques gouttes de diméthylformamide, après 3 heures d'agitation à une température voisine de 20°C sont à nouveau successivement ajoutés 30 cm³ de chlorure d'oxalyle, puis quelques gouttes de diméthylformamide. Le milieu réactionnel est concentré à sec sous pression réduite (2,7 kPa) à une température voisine de 40°C. On obtient ainsi 27,6 g de chlorure de l'acide 3,5-difluorophénylacétique sous forme d'huile jaune.

### Exemple 79

La (RS)-1-[bis-(4-chlorophényl)-méthyl]-3-[1-(3,5-difluorophényl)-1-méthylsulfonyl-éthyl]-azétidine est obtenu de la façon suivante : A une solution de 0,5 g de (RS)-1-[bis(4-chlorophényl)méthyl)]-3-[(3,5-difluorophényl) (méthylsulfonyl) méthyl]-azétidine dans 10 cm³ de tétrahydrofuranne refroidie à -78°C et maintenue sous atmosphère inerte, on ajoute 0,5 cm³ une solution 2M de diisopropylamidure de lithium. On laisse la température remonter à -20°C puis on ajoute 0,06 cm³ de iodométhane. On laisse la température remonter à 0°C sur une période de 2 heures puis on ajoute 20 cm³ d'une solution aqueuse saturée de chlorure d'ammonium. Le milieu réactionnel est décanté et les phases aqueuses sont extraites par deux fois 20 cm³ d'acétate d'éthyle. Les extraits organiques sont réunis, séchés sur sulfate de magnésium et évaporés à sec à 40°C sous 2,7 kPa, fournissant 550 mg de résidu de couleur crème. Le résidu est chromatographié sur colonne de silice (Dynamax, référence 83-121-C taille 21.4 mm x 250 mm, précolonne 21.4 mm x 50 mm référence R00083121G, silice 8µ porosité 60 Angstroem; Rainin Instrument Co. Inc., Mac Road, Woburn, MA 01801, USA) en éluant avec un mélange heptane : isopropanol (99 : 1 en volumes) à 15 cm³ par minute (détection 254nm, fractions de 10 cm³). Les fractions contenant le composé de Rf=32/77 (cyclohexane : acétate d'éthyle 70: 30, 254nm, Plaques de Silice référence 1.05719, Merck KGaA, 64271 Darmstatd, Allemagne) sont réunies et évaporées à 40°C sous 2,7 kPa, fournissant 80 mg de 1-[bis-(4-chlorophényl)-méthyl]-3-[1-(3,5-difluorophényl)-1-méthylsulfonyl-éthyl]-azétidine sous forme d'une poudre blanche amorphe [Spectre de R.M.N ¹H (300 MHz, CDCl₃, δ en ppm) : 2,05 (s : 3H); 2,54 (s : 3H); 2,63 (t, J = 7,5 Hz : 1 H); 3,17 (t large, J = 8 Hz : 1 H); 3,32 (mt : 1 H); 3,44 (t large, J = 8 Hz : 1 H); 3,71 (mt : 1 H); 4,27 (s : 1 H); 6,83 (tt, J = 9 et 2,5 Hz : 1H); 7,15 (mt : 2H); de 7,20 à 7,40 (mt : 8H)].

### Exemple 80

La (RS)-1-[bis-(4-fluoro-phényl)-méthyl]-3-[(3,5-difluorophényl)-méthylsulfonyl-méthyl]-azétidine peut être préparée de la manière suivante : A une suspension de 0,191 cm³ de bis-(4-fluoro-phényl)-chlorométhane, 300 mg de chlorhydrate de (RS)-3-[(3,5-difluorophényi)-méthylsulfonyl-méthyl]-azétidine et 167 mg de carbonate de potassium dans 5 cm³ d'acétonitrile, à une température voisine de 20°C, sont ajoutés quelques grains d'iodure de potassium. Après 48 heures à une température voisine de 20°C, le milieu réactionnel est filtré sur verre fritté, le solide est rincé avec de l'acétonitrile, et le filtrat est purifié par chromatographie préparative sur couche mince de silice [3 plaques préparatives Merck Kieselgel 60F254; 20x20 cm; épaisseur 1 mm], en éluant par un mélange méthanol-dichlorométhane (1-99 en volumes). Après élution de la zone correspondant au produit recherché par un mélange méthanol-dichlorométhane (10-90 en volumes), filtration sur verre fritté, puis évaporation des solvants sous pression réduite à une température voisine de 40°C, on obtient 39 mg de (RS)-1-[bis-(4-fluoro-phényl)-méthyl]-3-[(3,5-difluorophényl)-méthylsulfonyl-méthyl]-azétidine sous forme d'une meringue jaune [Spectre de R.M.N ¹H (300 MHz, CDCl₃, δ en ppm) : 2,54 (t large, J = 7 Hz : 1H); 2,66 (s : 3H); 3,18 (mt : 2H); de 3,20 à 3,45 (mt : 1 H); 3,63 (t large, J = 7 Hz : 1 H); 4,27 (s : 1 H); 4,28 (d, J = 11 Hz : 1 H); 6,83 (tt, J = 9 et 2 Hz : 1 H); de 6,90 à 7,05 (mt : 6H); de 7,25 à 7,40 (mt : 4H)].

Le chlorhydrate de (RS)-3-[(3,5-difluorophényl)-méthylsuifonyt-méthyl]-azétidine peut être préparé peut être préparé de la manière suivante : Une suspension de 8,5 g de 1-benzhydryl-3-[(3,5-difluorophényl)(méthylsulfonyl méthylène]-azétidine, et 1,3 g d'hydroxyde de palladium (20% en poids de palladium), dans 600 cm³ de méthanol, 20 cm³ d'acide chlorhydrique 1N et 4 cm³ d'acide acétique, est agitée à une température voisine de 20°C sous atmosphère d'hydrogène (1,5 bars) jusqu'à absorption totale d'un volume de 2,1 litres d'hydrogène. Le milieu réactionnel est alors filtré sur verre fritté garni de noir. Le filtrat est concentré à sec sous pression réduite, puis le résidu obtenu est repris avec de l'éthanol. Le produit blanc cristallisé est filtré et séché. On obtient ainsi 5,4 g de chlorhydrate de (RS)-3-[(3,5-difluorophényl)-méthylsulfonyl-méthyl]-azétidine sous forme de cristaux blancs.

La 1-benzhydryl-3-[(3,5-difluorophényl)(méthylsulfonyl)méthylène]-azétidine peut être préparée peut être préparée de la manière suivante : Un mélange de 18,8 g de 3-acétoxy-1-benzhydryl-3-[(3,5-difluorophényl)(méthylsulfonyl) méthyl]-azétidine et 3,9 g de monohydrate d'hydroxyde de lithium dans 120 cm³ d'acétonitrile est porté à une température voisine de 70°C pendant 3 heures. Après refroidissement jusqu'à une température voisine de 2°C, on ajoute successivement 120 cm³ d'éther de ter-butyl et de méthyl, 80 cm³ d'eau distillée puis lentement 5 cm³ d'acide acétique. Après décantation, la phase organique est lavée avec 80 cm³ d'une solution aqueuse saturée en hydrogénocarbonate de sodium; 80 cm³ d'eau distillée, 80 cm³ d'une solution aqueuse saturée en chlorure de sodium, séchée sur sulfate de magnésium, filtrée, et concentrée à sec sous pression réduite. Le résidu obtenu est repris avec de l'éthanol. Après une nuit à une température voisine de 20°C, le mélange obtenu est filtré sur verre fritté, les cristaux blancs obtenus sont rincés avec de l'éthanol, de l'oxyde de diisopropyle et séchés sous pression réduite à une température voisine de 45°C. On obtient ainsi 14,6 g de 1-benzhydryl-3-[(3,5-difluorophényl)(méthylsulfonyl)méthylène]-azétidine sous forme de cristaux blancs.

La 3-acétoxy-1-benzhydryl-3-[(3,5-difluorophényl)(méthylsulfonyl)méthyl]-azétidine peut être préparée peut être préparée de la manière suivante : A une suspension de 12,37 g de 3,5-difluorobenzyl-méthyl-sulfone dans 200 cm³ de tétrahydrofuranne, sous atmosphère inerte d'azote, à une température voisine de -30°C, on ajoute goutte à goutte en 25 minutes environ 47,1 cm³ de n-butyllithium 1,6N en solution dans l'hexane. La solution jaune trouble est agitée à une température voisine de -30°C pendant 2 heures, puis on ajoute goutte à goutte une solution de 11,87 g de 1-benzhydryl azétidin-3-one dans 75 cm³ de dichlorométhane. Le mélange réactionnel est agité 1,5 heures à une température voisine de -30°C, puis on ajoute 6,07 cm³ de chlorure d'acétyle, et on laisse revenir la température du milieu à une température voisine de -10°C en environ 30 minutes. On ajoute 200 cm³ d'eau et 100 cm³ de dichlorométhane. Après agitation vigoureuse pendant 30 minutes et décantation, la phase organique est lavée avec 3 fois 150 cm³ d'une solution aqueuse saturée en hydrogénocarbonate de sodium, 150 cm³ d'une solution aqueuse saturée en chlorure de sodium, séchée sur sulfate de magnésium, filtrée, et concentrée à sec sous pression réduite. Le résidu cristallin obtenu est repris par 50 cm³ d'éthanol bouillant. La suspension blanche obtenue est laissée reposer une nuit à une température voisine de 20°C, puis le solide obtenu est essoré sur verre fritté, rincé par de l'éther de diisopropyle, et séché sous pression réduite à une température voisine de 50°C. On obtient ainsi 19,5 g de 3-acétoxy-1-benzhydryl-3-[(3,5-difluorophényl)(méthylsulfonyl)méthyl]-azétidine sous forme de cristaux blancs.

La 1-benzhydryl azétidin-3-one peut être préparée selon le mode opératoire décrit par KATRITZKY A.R. et coll. dans J. Heterocycl. Chem., 271 (1994).

La 3,5-difluorobenzyl-méthyl-sulfone peut être préparée peut être préparée de la manière suivante : Un mélange de 66,69 cm³ de bromure de 3,5-difluorobenzyle, 71,97 g du sel de sodium de l'acide méthanesulfinique et 150 mg d'iodure de sodium dans 625 cm³ d'éthanol est porté au reflux, sous atmosphère d'argon, pendant environ 16 heures. Après refroidissement jusqu'à une température voisine de 20°C, le milieu réactionnel est dilué avec 3 litres d'acétate d'éthyle, lavé avec 500 cm³ d'eau, 500 cm³ d'une solution aqueuse saturée en chlorure de sodium, séché sur sulfate de magnésium, filtré et évaporé sous pression réduite (50 mbar) à une température voisine de 40°C. Le résidu obtenu est repris avec 300 cm³ d'éther éthylique, et le solide est filtré sur verre fritté, rincé avec 200 cm³ d'éther éthylique, séché sous pression réduite à une température voisine de 20°C. On obtient ainsi 86,9 g de 3,5-difluorobenzyl-méthyl-sulfone sous forme d'une poudre blanche.

### Exemple 81

La (RS)-{1-[(3-pyridyl)-(4-chlorophényl)méthyl]-3-[(3,5-difluorophényl)-méthylsulfonyl-méthyl]-azétidine peut être préparée de la manière suivante : Un mélange de 47 mg de (3-pyridyl)-(4-chlorophényl)-bromométhane, 50 mg de chlorhydrate de (RS)-3-[(3,5-difluorophényl)-méthylsulfonyl-méthyl]-azétidine et 58 mg de carbonate de potassium dans 2 cm³ d'acétonitrile, est agité environ 3 heures à une température voisine de 20°C, 2 heures au reflux du solvant, environ 16 heures à une température voisine de 20°C, puis 1,5 heures au reflux du solvant. On ajoute alors quelques grains d'iodure de potassium, et le mélange réactionnel est maintenu environ 2 heures au reflux du solvant. Après refroidissement jusqu'à une température voisine de 20°C, le milieu réactionnel est purifié par chromatographie préparative sur couche mince de silice [2 plaques préparatives Merck Kieselgel 60F254; 20x20 cm; épaisseur 0,5 mm], en éluant par un mélange méthanol-dichlorométhane (2,5-97,5 en volumes). Après élution de la zone correspondant au produit recherché par un mélange méthanol-dichlorométhane (10-90 en volumes), filtration sur verre fritté, puis évaporation des solvants sous pression réduite à une température voisine de 40°C, on obtient 11 mg de (RS)-{1-[(3-pyridyl)-(4-chlorophényl)méthyl]-3-[(3,5-difluorophényl)-méthylsulfonyl-méthyl]-azétidine sous forme d'une laque incolore [Spectre de R.M.N ¹H (300 MHz, CDCl₃, δ en ppm) : 2,59 (mt : 1H); 2,66 (s : 3H); 3,21 (mt : 2H); de 3,30 à 3,50 (mt : 1H); 3,67 (mt : 1H); 4,28 (d large, J = 11 Hz : 1H); 4,32 (s large : 1H); 6,84 (tt, J = 9 et 2 Hz : 1H); 6,95 (mt : 2H); 7,19 (dd large, J = 8 et 5 Hz : 1H); de 7,20 à 7,40 (mt : 4H); 7,64 (d large, J = 8 Hz : 1H); 8,45 (mt : 1H); 8,59 (s très large : 1H].

Le (3-pyridyl)-(4-chlorophényl)-bromométhane peut être préparé de la manière suivante : Un mélange de 150 mg de (3-pyridyl)-(4-chlorophényl)-méthanol dans 0,356 cm³ d'acide bromhydrique (à 33% dans l'acide acétique) et 0,101 cm³ de bromure d'acétyle est porté au reflux pendant 1 heure, puis laissé à une température voisine de 20°C pendant 2 heures, avant d'être concentré sous pression réduite, et coévaporé avec quelques cm³ de toluène. On obtient ainsi 234 mg de (3-pyridyl)-(4-chlorophényl)-bromométhane sous forme d'un solide beige gommeux.

Le (3-pyridyl)-(4-chlorophényl)-méthanol peut être préparé de la manière suivante : A une solution de 5,83 cm³ de bromure de 4-chlorophénylmagnésium (solution 1 M dans l'éther éthylique) dans 5 cm³ de tétrahydrofuranne, sous atmosphère inerte d'argon, est ajouté lentement 0,5 cm³ de 3-pyridine-carboxaldéhyde. Après environ 3 heures, on ajoute au milieu réactionnel 3 cm³ d'une solution aqueuse saturée en chlorure d'ammonium et 10 cm³ d'eau. Après agitation 5 minutes à une température voisine de 20°C, le milieu réactionnel est acidifié jusqu'à un pH d'environ 2 avec une solution aqueuse d'acide chlorhydrique 1N. La phase aqueuse est extraite avec 3 fois 15 cm³ de dichlorométhane. La phase aqueuse restante est traitée avec 10 cm³ d'une solution aqueuse de soude 1N et re-extraite avec 3 fois 15 cm³ d'acétate d'éthyle. Les phases organiques contenant l'acétate d'éthyle sont rassemblées, séchées sur sulfate de magnésium, filtrées, et concentrées sous pression réduite. On obtient ainsi 466 mg de (3-pyridyl)-(4-chlorophényl)-méthanol sous forme d'un solide jaune vif.

### Exemple 82

La (RS)-{1-[(4-pyridyl)-(4-chlorophényl)méthyl]-3-[(3,5-difluorophényl)-méthylsulfonyl-méthyl]-azétidine peut être préparée de la manière suivante : Un mélange de 160 mg de (4-pyridyl)-(4-chlorophényl)-bromométhane, 169 mg de chlorhydrate de (RS)-3-[(3,5-difluorophényl)-méthylsulfonylméthyl]-azétidine et 94 mg de carbonate de potassium dans 5 cm³ d'acétonitrile, est agité environ 17 heures à une température voisine de 20°C. On ajoute alors quelques grains d'iodure de sodium, et après 2 heures d'agitation à une température voisine de 20°C, le mélange réactionnel est maintenu environ 1,5 heures au reflux du solvant. Après refroidissement jusqu'à une température voisine de 20°C, le milieu réactionnel est purifié par chromatographie préparative sur couche mince de silice [4 plaques préparatives Merck Kieselgel 60F254; 20x20 cm; épaisseur 0,5 mm], en éluant par un mélange méthanol-dichlorométhane (2,5-97,5 en volumes). Après élution de la zone correspondant aux produits recherchés par un mélange méthanol-dichlorométhane (10-90 en volumes), filtration sur verre fritté, puis évaporation des solvants sous pression réduite à une température voisine de 40°C, on obtient un premier mélange de diastéréoisomères, soit 24 mg de (RS)-{1-[(4-pyridyl)-(4-chlorophényl)méthyl]-3-[(3,5-difluorophényl)-méthylsulfonyl-méthyl]-azétidine sous forme d'une laque jaune, et un second mélange de diastéréoisomères, soit 31 mg de (RS)-{1-[(4-pyridyl)-(4-chlorophényl)méthyl]-3-[(3,5-difluorophényl)-méthylsulfonyl-méthyl]-azétidine sous forme d'une meringue jaune. Le premier mélange de diastéréoisomères possède les caractéristiques sont les suivantes Spectre de R.M.N ¹H (300 MHz, CDCl₃, δ en ppm) : 2,62 (t, J = 7 Hz : 1H); 2,67 (s : 3H); 3,21 (t large, J = 7 Hz : 2H); 3,42 (mt : 1H); 3,70 (t large, J = 7 Hz : 1H); 4,28 (s : 1H); 4,28 (d, J = 11 Hz : 1H); 6,85 (tt, J = 9 et 2,5 Hz : 1H); 6,97 (mt : 2H); de 7,20 à 7,35 (mt : 6H); 8,52 (dd, J = 4,5 et 1,5 Hz : 2H).

Le second mélange de diastéréoisomères possède les caractéristiques sont les suivantes : Spectre de R.M.N ¹H (300 MHz, CDCl₃, δ en ppm) : 2,59 (t, J = 7 Hz : 1H); 2,67 (s : 3H); 3,26 (mt : 2H); de 3,35 à 3,50 (mt : 1H); 3,63 (t large, J = 7 Hz : 1H); 4,28 (s : 1H); 4,28 (d, J = 11 Hz : 1H); 6,85 (tt, J = 9 et 2 Hz : 1H); 6,97 (mt : 2H); de 7,20 à 7,40 (mt : 6H); 8,50 (dd, J = 4,5 et 1,5 Hz : 2H).

Le (4-pyridyl)-(4-chlorophényl)-bromométhane peut être préparé de la manière suivante : Une solution de 100 mg de (4-pyridyl)-(4-chlorophényl)-méthanol dans 0,24 cm³ d'acide bromhydrique (à 33% dans l'acide acétique) est portée au reflux pendant 1 heure, puis laissé revenir à une température voisine de 20°C. On ajoute alors 0,675 cm³ de bromure d'acétyle et le mélange réactionnel est porté au reflux pendant 1,5 heures, puis laissé revenir à une température voisine de 20°C, avant d'être concentré sous pression réduite. On obtient ainsi 163 mg de (4-pyridyl)-(4-chlorophényl)-bromométhane sous forme d'une meringue-gomme beige.

Le (4-pyridyl)-(4-chlorophényl)-méthanol peut être préparé de la manière suivante : A une solution de 2 g de 4-(4-chlorobenzoyle)-pyridine dans 160 cm³ d'éthanol, sont ajoutés, à une température voisine de 20°C, 348 mg de tétrahydroborure de sodium. Après 2 heures d'agitation à une température voisine de 20°C, on ajoute 90 mg de tétrahydroborure de sodium. Après environ 1,5 heures à la même température, on dilue le milieu réactionnel avec 200 cm³ de dichlorométhane et 200 cm³ d'eau. Le pH de la phase aqueuse est ajusté à environ une valeur de 5 par ajout d'environ 13 cm³ d'une solution aqueuse d'acide chlorhydrique 1N. Après décantation, la phase aqueuse est extraite avec 3 fois 100 cm³ de dichlorométhane. Les phases organiques sont rassemblées, séchées sur sulfate de magnésium, filtrées, et concentrées sous pression réduite. On obtient ainsi 2 g de (4-pyridyl)-(4-chlorophényl)-méthanol sous forme d'une poudre blanche.

### Exemple 83

La (RS)-{1-[(2-chloro-pyrid-5-yl)-(4-chlorophényl)méthyl]-3-[(3,5-difluorophényl)-méthylsulfonyl-méthyl]-azétidine peut être préparée de la manière suivante : Un mélange de 300 mg de (2-chloro-pyrid-5-yl)-(4-chlorophényl)-bromométhane, 225 mg de chlorhydrate de (RS)-3-[(3,5-difluorophényl)-méthylsulfonyl-méthyl]-azétidine, 125 mg d'iodure de potassium et 521 mg de carbonate de potassium dans 5 cm³ d'acétonitrile, est chauffé environ 2 heures au reflux du solvant. Après refroidissement jusqu'à une température voisine de 20°C, le milieu réactionnel est filtré sur verre fritté. Le résidu solide est rincé avec du dichlorométhane, et les filtrats sont évaporés sous pression réduite. On obtient ainsi 402 mg d'une meringue chocolat qui est purifiée par chromatographie préparative sur couche mince de silice [4 plaques préparatives Merck Kieselgel 60F254; 20x20 cm; épaisseur 0,5 mm], en éluant par un mélange méthanol-dichlorométhane (2,5-97,5 en volumes). Après élution de la zone correspondant au produit recherché par un mélange méthanol-dichlorométhane (10-90 en volumes), filtration sur verre fritté, puis évaporation des solvants sous pression réduite à une température voisine de 40°C, on obtient un premier mélange de diastéréoisomères, soit 14 mg de (RS)-{1-[(2-chloro-pyrid-5-yl)-(4-chlorophényl)méthyl]-3-[(3,5-difluorophényl)-méthylsulfonyl-méthyl]-azétidine sous forme d'une meringue marron, et un second mélange de diastéréoisomères, soit 10 mg de (RS)-{1-[(2-chloro-pyrid-5-yl)-(4-chlorophényl)méthyl]-3-[(3,5-difluorophényl)-méthylsulfonyl-méthyl]-azétidine sous forme d'une meringue beige.

Le premier mélange de diastéréoisomères possède les caractéristiques sont les suivantes : Spectre de R.M.N. ¹H (300 MHz, CDCl₃, δ en ppm) : 2,57 (t, J = 7,5 Hz : 1H); 2,65 (s : 3H); de 3,15 à 3,30 (mt : 2H); 3,40 (mt : 1H); 3,63 (t large, J = 7,5 Hz : 1H); 4,27 (d, J = 11 Hz : 1H); 4,31 (s : 1H); 6,84 (tt, J = 9 et 2 Hz : 1H); 6,95 (mt : 2H); de 7,20 à 7,35 (mt : 5H); 7,63 (dd, J = 8 et 2,5 Hz : 1H); 8,38 (d, J = 2,5 Hz : 1H).

Le second mélange de diastéréoisomères possède les caractéristiques sont les suivantes : Spectre de R.M.N. ¹H (300 MHz, CDCl₃, δ en ppm) : 2,57 (t, J = 7,5 Hz : 1H); 2,64 (s : 3H); 3,18 (t large, J = 7,5 Hz : 2H); 3,38 (mt : 1H); 3,63 (t large, J = 7,5 Hz : 1H); 4,24 (d, J = 11,5 Hz : 1H); 4,29 (s : 1H); 6,83 (tt, J = 9 et 2 Hz : 1H); 6,94 (mt : 2H); 7,20 (d, J = 8 Hz : 1H); de 7,20 à 7,35 (mt : 4H); 7,59 (dd, J = 8 et 2,5 Hz : 1H); 8,34 (d, J = 2,5 Hz : 1H).

Le (2-chloro-pyrid-5-yl)-(4-chlorophényl)-bromométhane peut être préparé de la manière suivante : A une solution de 100 mg de (2-chloro-pyrid-5-yl)-(4-chlorophényl)-méthanol dans 2 cm³ de tétrachlorure de carbone, sous atmosphère inerte d'argon, à une température voisine de 0°C, on ajoute 0,153 cm³ de bromure de thionyle. Après 3,5 heures à une température voisine de 0°C, le milieu réactionnel est concentré sous pression réduite, et coévaporé avec quelques cm³ de toluène. On obtient ainsi 1,3 g d'un liquide brun qui est repris avec du dichlorométhane, additionné d'eau et de dithionite de sodium. Après décantation, la phase organique est séchée sur sulfate de magnésium, filtrée, et concentrée à sec sous pression réduite. On obtient ainsi 0,33 g de (2-chloro-pyrid-5-yl)-(4-chlorophényl)-bromométhane sous forme d'une huile brune.

Le (2-chloro-pyrid-5-yl)-(4-chlorophényl)-méthanol peut être préparé en opérant du manière semblable à l'exemple 84 à partir de 22,5 cm³ de bromure de 4-chlorophénylmagnésium (solution 1 M dans l'éther éthylique) dans 30 cm³ de tétrahydrofuranne, sous atmosphère inerte d'argon, et 2,9 g de 2-chloro-pyridine-5-carboxaldéhyde dans 30 cm³ de tétrahydrofuranne. On obtient ainsi 3,42 g de (2-chloro-pyrid-5-yl)-(4-chlorophényl)-méthanol sous forme d'une poudre vert pâle.

La 2-chloro-pyridine-5-carboxaldéhyde peut être préparée selon la référence suivante : G. Pandey, T.D. Bagul, A.K. Sahoo, J. Org. Chem., 1998, 63, 760-768.

### Exemple 84

La (RS)-5-((4-chlorophényl)-{3-[(3,5-difluorophényl)-méthylsulfonyl-méthyl]-azétidin-1-yl}-méthyl)-pyrimidine peut être préparée de la manière suivante : Un mélange de 50 mg 5-[bromo-(4-chlorophényl)-méthyl]-pyrimidine, 52,6 mg de chlorhydrate de (RS)-3-[(3,5-difluorophényl)-méthylsulfonyl-méthyl]-azétidine, 44 mg d'iodure de potassium et 73 mg de carbonate de potassium dans 2 cm³ d'acétonitrile, est chauffé environ 5 heures au reflux du solvant. Après refroidissement jusqu'à une température voisine de 20°C, le milieu réactionnel est purifié par dépôt direct sur chromatographie préparative sur couche mince de silice [2 plaques préparatives Merck Kieselgel 60F254; 20x20 cm; épaisseur 0,5 mm], en éluant par un mélange méthanol-dichlorométhane (2,5-97,5 en volumes). Après élution de la zone correspondant au produit recherché par un mélange méthanol-dichlorométhane (10-90 en volumes), filtration sur verre fritté, puis évaporation des solvants sous pression réduite à une température voisine de 40°C, on obtient un premier mélange de diastéréoisomères, soit 8 mg de (RS)-5-((4-chlorophényl)-{3-[(3,5-difluorophényl)-méthylsulfonyl-méthyl]-azétidin-1-yl}-méthyl)-pyrimidine sous forme d'une meringue jaune, et un second mélange de diastéréoisomères, soit 6 mg de (RS)-5-((4-chlorophényl)-{3-[(3,5-difluorophényl)-méthylsulfonyl-méthyl]-azétidin-1-yl}-méthyl)-pyrimidine sous forme d'une meringue jaune.

Le premier mélange de diastéréoisomères possède les caractéristiques sont les suivantes : Spectre de R.M.N. ¹H (300 MHz, COCl₃, δ en ppm) : 2,60 (t large, J = 7 Hz : 1H); 2,66 (s : 3H); 3,24 (t large, J = 7 Hz : 2H); 3,41 (mt : 1H); 3,66 (t large, J = 7 Hz : 1H); 4,28 (d, J = 11,5 Hz : 1H); 4,33 (s large: 1H); 6,84 (t large, J = 9 Hz : 1H); 6,95 (mt : 2H); de 7,25 à 7,35 (mt : 4H); 8,71 (s large : 2H); 9,08 (s large : 1 H).

Le second mélange de diastéréoisomères possède les caractéristiques sont les suivantes : Spectre de R.M.N. ¹H (300 MHz, CDCl₃, δ en ppm) : 2,61 (t, J = 7 Hz : 1H); 2,66 (s : 3H); 3,24 (t large, J = 7 Hz : 2H); 3,43 (mt : 1 H); 3,65 (t large, J = 7 Hz : 1H); 4,28 (d, J = 11,5 Hz : 1H); 4,33 (s : 1H); 6,85 (tt, J = 9 et 2 Hz : 1H); 6,96 (mt : 2H); de 7,25 à 7,35 (mt : 4H); 8,69 (s : 2H); 9,06 (s : 1 H).

La 5-[bromo-(4-chlorophényl)-méthyl]-pyrimidine peut être préparé de la manière suivante : A une solution de 205 mg de (4-chlorophényl)-pyrimidin-5-yl-méthanol dans 1 cm³ de tétrachlorure de carbone et 1cm³ de dchlorométhane, sous atmosphère inerte d'argon, à une température voisine de 0°C, on ajoute 0,36 cm³ de bromure de thionyle. Après 2,5 heures à une température voisine de 0°C, le milieu réactionnel est ramené à une température voisine de 20°C, concentré sous pression réduite, et coévaporé avec quelques cm³ de toluène. Le liquide brun obtenu est repris avec 10 cm³ de dichlorométhane, lavé avec 5 cm³ d'une solution aqueuse saturée en dithionite de sodium, puis avec de l'eau. Après décantation, la phase organique est séchée sur sulfate de magnésium, filtrée, et concentrée à sec sous pression réduite. On obtient ainsi 227 mg d'un liquide visqueux beige qui est repris avec un minimum de dichlorométhane et purifié par chromatographie préparative sur couche mince de silice [2 plaques préparatives Merck Kieselgel 60F254; 20x20 cm; épaisseur 0,5 mm], en éluant par un mélange méthanol-dichlorométhane (2,5-97,5 en volumes).

Après élution de la zone correspondant au produit recherché par un mélange méthanol-dichlorométhane (10-90 en volumes), filtration sur verre fritté, puis évaporation des solvants sous pression réduite à une température voisine de 40°C, on obtient 51 mg de 5-[bromo-(4-chlorophényl)-méthyl]-pyrimidine sous forme d'une meringue jaune.

Le (4-chlorophényl)-pyrimidin-5-yl-méthanol peut être préparé en opérant du manière semblable à l'exemple 83 : à une solution de 636 mg de 5-bromopyrimidine, dans 10 cm³ de tétrahydrofuranne, sous atmosphère inerte d'argon, à une température voisine de -78°C, sont ajoutés goutte à goutte 2,5 cm³ de n.butyl lithium (solution 1,6 M dans l'hexane). Après 10 minutes à une température voisine de -78°C, on ajoute goutte à goutte une solution de 562 mg de 4-chlorobenzaldéhyde dans 1 cm³ de tétrahydrofuranne. Après 30 minutes d'agitation à une température voisine de -78°C, on laisse remonter lentement la température du milieu réactionnel jusqu'à une température voisine de 20°C, et on ajoute successivement 15 cm³ d'une solution aqueuse saturée en chlorure d'ammonium, 60 cm³ d'acétate d'éthyle et 10 cm³ d'eau. La phase aqueuse est extraite avec 15 cm³ d'acétate d'éthyle, les phases organiques sont rassemblées, séchées sur sulfate de magnésium, filtrées sur verre fritté, et concentrées sous pression réduite (20 mbar) à une température voisine de 44°C. L'essentiel de l'huile orange obtenue (1,09 g) est purifiée par chromatographie sur colonne de diamètre 30 mn garnie de 60 g de silice moyenne (0,063-0,200mm) à pression atmosphérique en éluant avec un gradient méthanol/dichlorométhane (0/100 à 7/93 en volumes). Les fractions ne contenant que le produit cherché sont réunies et concentrées à sec sous pression réduite. On obtient ainsi 293 mg de (4-chlorophényl)-pyrimidin-5-yl-méthanol sous forme d'une huile jaune.

### Exemple 85

L'ester phénolique de l'acide 4-({1-[bis-(4-chlorophényl)-méthyl]-azétidin-3-ylidene}-méthylsulfonyl-méthyl)-3,6-dihydro-2H-pyridine-1-carbothioïque peut être préparé de la façon suivante : A une solution de 0,23 g de l'ester phénolique de l'acide 4-({1-[bis-(4-chlorophényl)-méthyl]-3-hydroxy-azétidin-3-yl}-méthylsulfonyl-méthyl)-3,6-dihydro-2H-pyridine-1-carbothioïque dans 5 cm³ dichlorométhane on ajoute 0,140 g de 4-diméthylamino-pyridine puis 0,042 cm³ de chlorure de méthane sulfonyle. Le mélange réactionnel est agité 20 heures à 20°C, puis dilué par 10 cm³ d'eau. Après décantation, la phase organique est successivement lavée avec 100 cm³ d'eau et 100 cm³ d'une solution saturée de NaCl, séchée sur sulfate de magnésium et concentrée à sec à 40°C sous 2,7 kPa. L'huile obtenue est triturée pendant 45 minutes dans 50 cm³ d'éther diisopropylique. Le solide formé est filtré fournissant 120 mg de l'ester phénolique de l'acide 4-({1-[bis-(4-chlorophényl)-méthyl]-azétidin-3-ylidene}-méthylsulfonyl-méthyl)-3,6-dihydro-2H-pyridine-1-carbothioïque sous forme d'un solide beige fondant à 184°C [Spectre de R.M.N. ¹H (400 MHz, CDCl₃, δ en ppm) : 2,53 (mf : 2H) ; 2,95 (s : 3H) ; 3,90 (mf : 2H) ; 4,04 (t, J = 5,5 Hz : 1H) ; 4,24 (mt : 3H) ; 4,49 (mf : 2H) ; 4,60 (mt : 1H) ; 5,90 (mf : 1H) ; de 7,05 à 7,50 (mt : 13H)].

L'ester phénolique de l'acide 4-({1-[bis-(4-chlorophényl)-méthyl]-3-hydroxyazétidin-3-yl}-méthylsulfonyl-méthyl)-3,6-dihydro-2H-pyridine-1-carbothioïque est préparé de la façon suivante : A un mélange de 0,72 g de l'ester phénolique de l'acide 4-méthylsulfonylméthyl-3,6-dihydro-2H-pyridine-1-carbothioïque et de 0,708 g de 1-[bis(4-chlorophényl)méthyl]azétidin-3-one dans 15 cm³ de tétrahydrofuranne sec refroidi sous atmosphère inerte à -78°C on ajoute 0,52 g de tertiobutylate de potassium. Le mélange réactionnel est agité à -78°C pendant 4 heures puis on ajoute 0,354 g de 1-[bis(4-chlorophényl)méthyl]azétidin-3-one. Après deux heures à -78°C, on laisse revenir la température à 20°C. Le mélange réactionnel est dilué dans 100 cm³ d'eau puis le tétrahydrofuranne est évaporé sous 2,7 kPa à 40°C. La phase aqueuse est extraite par deux fois 100 cm³ d'acétate d'éthyle. Les extraits organiques sont combinés et séchés sur sulfate de magnésium, concentrés sous 2,7kPa à 40°C. Le résidu obtenu est chromatographié sur silice (200g de silice, Amicon, 20-45 µm de porosité 60Angstroem, colonne de 5 cm de diamètre) en éluant avec un mélange cyclohexane : acétate d'éthyle (6 : 4 en volume). Les fractions de Rf=11/64 (cyclohexane :acétate d'éthyle 6 : 4, plaque de silice, Merck référence 1.05719, Merck KGaA, 64271 Darmstatd, Allemagne) sont réunies et concentrées sous 2,7 kPa à 40°C pour conduire à 240 mg de l'ester phénolique de l'acide 4-({1-[bis-(4-chlorophényl)-méthyl]-3-hydroxy-azétidin-3-yl}-méthylsulfonyl-méthyl)-3,6-dihydro-2H-pyridine-1-carbothioïque.

L'ester phénolique de l'acide 4-méthylsulfonylméthyl-3,6-dihydro-2H-pyridine-1-carbothioïque peut être préparé de la façon suivante : A une solution de 1 g de 1-benzyl-4-méthylsulfonylméthyl-1,2,3,6-tétrahydro-pyridine dans 10 cm³ de dichlorométhane sous atmosphère d'argon, on ajoute 0,778 cm³ de thiochloroformiate de phényle. La solution prend instantanément une couleur ambrée très foncée. Le mélange réactionnel est agité pendant 4 heures à 21 °C puis dilué dans 100 cm³ de dichlorométhane. Le milieu organique est lavé par 2 fois 50 cm³ d'eau, séché sur sulfate de magnésium et évaporé à sec à 40°C sous 2,7 kPa . Le résidu obtenu est purifié par chromatographie sur cartouche de silice (référence SIL-020-005, FlashPack, Jones Chromatography Limited, New Road, Hengoed, Mid Glamorgan, CF82 8AU, Royaume Uni) en éluant avec un mélange de cyclohexane : d'acétate d'éthyle 6 : 4 (10 cm³/min, fractions de 5 cm³). Les fractions de Rf=12/74 (cyclohexane : acetate d'éthyle 1 :1, plaque de silice, Merck référence 1.05719, Merck KGaA, 64271 Darmstatd, Allemagne) sont réunies et concentrées sous 2,7 kPa à 40°C pour conduire à 700 mg de l'ester phénolique de l'acide 4-méthylsulfonylméthyl-3,6-dihydro-2H-pyridine-1-carbothioïque.

La 1-benzyl-4-méthylsulfonylméthyl-1,2,3,6-tétrahydro-pyridine peut être préparé de la façon suivante : A une solution de 17,6 g de bromure de 1-benzyl-4-méthylsulfonylméthyl-pyridinium dans 700 cm³ d'eau refroidie à 5°C, on ajoute goutte à goutte en une heure une solution de 5,14 g de borohydrure de sodium et de 25 g de carbonate de sodium dans 700 cm³ d'eau en ne dépassant pas une température de 5°C dans le milieu réactionnel. Le milieu réactionnel est agité pendant quatre heures à 0°C puis on laisse revenir à température ambiante pendant la nuit. Le solide jaune formé est isolé par filtration et séché sous 2,7 kPa, fournissant 9,6 g de 1-benzyl-4-méthylsulfonylméthyl-1,2,3,6-tétrahydro-pyridine ayant un Rf de 44/81 (dichlorométhane : méthanol, 95: 5 en volumes, plaque de silice, Merck référence 1.05719, Merck KGaA, 64271 Darmstatd, Allemagne).

Le bromure de 1-benzyl-4-méthylsulfonylméthyl-pyridinium peut être préparé de la façon suivante : A une solution de 10g de 4-méthylsulfonylméthyl-pyridine dans 200 cm³ l'acétonitrile on ajoute 14 cm³ de bromure de benzyle puis on chauffe à reflux pendant 3 heures puis on laisse revenir à température ambiante pendant la nuit. Le solide formé est filtré, séché sous vide à 2,7 kPa, fournissant 17,6 g de bromure de 1-benzyl-4-méthylsulfonyfméthyl-pyridinium.

La 4-méthylsulfonylméthyl-pyridine peut être préparé de la façon suivante : A une solution de 57,4 g de chlorhydrate de 4-chlorométhyl pyridine dans 700 cm³ d'éthanol on ajoute lentement 14g d'hydroxyde de sodium en pastilles puis 35,7g de méthanesulfinate de sodium. Après addition la température est de 28°C. On chauffe le mélange réactionnel au reflux pendant deux heures puis on laisse revenir à température ambiante pendant la nuit. Le milieu réactionnel est porté à 50°C puis filtré à chaud sur papier. Le filtrat est évaporé à sec à 40°C sous 2,7 kPa. Le résidu est recristallisé dans 300 cm³ d'isopropanol fournissant 29,6 g de 4-méthylsulfonylméthyl-pyridine.

### Exemple 86

La (RS)-1-[2-{1-[bis-(4-chloro-phényl)-méthyl]-azétidin-3-yl}-2-(3,5-difluorophényl)-éthyl]-3-propyl-urée peut être préparée en opérant de la façon suivante : A une solution de 90 mg de (RS)-2-{1-[bis-(4-chloro-phényl)-méthyl]-azétidin-3-yl}-2-(3,5-difluoro-phényl)-éthylamine dans 5 cm³ de tétrahydrofuranne, on ajoute 0,052 cm³ d'isocyanate de n.propyle. Après environ 72 heures d'agitation à une température voisine de 20°C, le mélange réactionnel est filtré, concentré à sec sous pression réduite, repris avec de l'éther diisopropylique. Le mélange obtenu est filtré et concentré à sec sous pression réduite. On obtient ainsi 80 mg d'un solide jaune pâle, que l'on dissout dans 5 cm³ de tétrahydrofuranne, et auquel on ajoute 80 mg de résine scavenger. Après environ 18 heures d'agitation à une température voisine de 20°C, le mélange réactionnel est filtré, puis concentré à sec sous pression réduite. On obtient ainsi 36 mg d'un solide pâteux qui est purifié par chromatographie sous pression sur cartouche de silice, en éluant avec un mélange de cyclohexane et d'acétate d'éthyle (50/50 en volumes). Les fractions 16 à 20 sont réunies et concentrées à sec sous pression réduite. On obtient 6 mg de (RS)-1-[2-{1-[bis-(4-chloro-phényl)-méthyl]-azétidin-3-yl}-2-(3,5-difluoro-phényl)-éthyl]-3-propyl-urée sous la forme d'une huile [Spectre de R.M.N. ¹H (300 MHz, CDCl₃, δ en ppm) : 0,88 (t, J = 7,5 Hz : 3H); de 1,35 à 1,60 (mt : 2H); de 2,25 à 2,55 et de 2,65 à 3,05 (2 série de mts : 6H en totalité); 3,04 (mt : 2H); 3,22 (mt : 1H); 3,38 (mt : 1H); 4,07 (mt : 1H); de 4,10 à 4,20 (mt : 1H); 4,17 (s : 1H); 6,62 (tt, J = 9 et 2,5 Hz : 1H); 6,82 (mt : 2H); de 7,20 à 7,45 (mt : 8H].

La (RS)-2-{1-[bis-(4-chloro-phényl)-méthyl]-azétidin-3-yl}-2-(3,5-difluorophényl)-éthylamine peut être préparée en opérant de la façon suivante : Dans un autoclave, refroidi par un bain d'acétone et de carboglace, sont introduits 1,2 g de l'ester éthylique de l'acide méthanesulfonique (RS)-2-{1-[bis-(4-chloro-phényl)-méthyl]-azétidin-3-yl}-2-(3,5-difluoro-phényl) en solution dans 10 cm³ de méthanol, puis une solution de 30 cm³ d'ammoniac dans 30 cm³ de méthanol. L'autoclave, fermé, est agité et porté à une température voisine de 60°C pendant 24 heures. Après refroidissement jusqu'à une température voisine de 20°C,on laisse l'ammoniac s'évaporer à l'air, à une température voisine de 20°C, puis la solution restante est concentrée à sec sous pression réduite. On obtient ainsi une gomme qui est triturée ave de l'éther éthylique, à une température voisine de 20°C pendant environ 16 heures. L'insoluble obtenu est filtré, séché au dessiccateur pendant 3 heures. On obtient ainsi 830 mg de la (RS)-2-{1-[bis-(4-chloro-phényl)-méthyl]-azétidin-3-yl}-2-(3,5-difluoro-phényl)-éthylamine sous forme d'un solide blanchâtre.

L'ester éthylique de l'acide méthanesulfonique (RS)-2-{1-[bis-(4-chlorophényl)-méthyl]-azétidin-3-yl}-2-(3,5-difluoro-phényl) peut être préparée en opérant de la façon suivante : A une solution de 1,9 g de (RS)-2-{1-[bis-(4-chlorophényl)méthyl]-azétidin-3-yl}-2-(3,5-difluorophényl)-éthanol dans 20 cm³ de dichlorométhane, à une température voisine de 20°C, est ajouté 0,34 cm³ de chlorure de méthane sulfonyle. Après refroidissement du mélange réactionnel jusqu'à une température voisine de 10°C, on ajoute 0,89 cm³ de triéthylamine. Après agitation de la solution pendant 20 heures à une température voisine de 20°C, on ajoute goutte à goutte 100 cm³ d'eau puis 150 cm³ de dichlorométhane. La phase organique décantée, et séparée, est lavée avec deux fois 50 cm³ d'eau, 50 cm³ d'une solution aqueuse satrurée en chlorure de sodium, 50 cm³ d'eau, puis séchée sur sulfate de magnésium, filtrée, et évaporée à sec sous pression réduite. La meringue jaune ainsi obtenue (2 g) est purifiée sur colonne de silice (granulométrie 0,020 - 0,045 mm), sous 0,4 bar de pression en éluant avec un mélange de cyclohexane et d'acétate d'éthyle (80/20 en volumes). Les fractions 49 à 111 sont réunies et concentrées à sec sous pression réduite. On obtient 1,2 g de l'ester éthylique de l'acide méthanesulfonique (RS)-2-{1-[bis-(4-chlorophényl)-méthyl]-azétidin-3-yl}-2-(3,5-difluoro-phényl) sous la forme d'une meringue blanche.

### Exemple 87

Le (RS)-N-[2-{1-[bis-(4-ch)oro-phényl)-méthyl]-azétidin-3-yl}-2-(3,5-difluorophényl)-éthyl]-cyclopropanecarboxamide peut être préparé en opérant de la façon suivante : A une solution de 90 mg de (RS)-2-{1-[bis-(4-chloro-phényl)-méthyl]-azétidin-3-yl}-2-(3,5-difluoro-phényl)-éthylamine dans 5 cm³ de tétrahydrofuranne, à une température voisine de 20°C, sont ajoutés successivement 0,018 cm³ de diisopropylcarbodiimide, 10 mg d'acide cyclopropanecarboxylique, 16 mg d'hydrate d'hydroxybenzotriazole, puis 0,4 g de morpholine supportée sur polystyrène. La suspension obtenue est agitée à une température voisine de 20°C pendant 20 heures. Le milieu réactionnel est filtré, et concentré à sec sous pression réduite. On obtient ainsi 80 mg d'un produit pâteux qui est purifié par passage sur une cartouche SPE (phase SCX, 1 g de phase). On obtient ainsi 76 mg d'un résidu qui est purifié par chromatographie sous pression sur une cartouche de silice, en éluant avec un mélange de cyclohexane et d'acétate d'éthyle (70/30 en volumes) Les fractions 9 à 19 sont réunies et concentrées à sec sous pression réduite. On obtient 12 mg de (RS)-N-[2-{1-[bis-(4-chloro-phényl)-méthyl]-azétidin-3-yl}-2-(3,5-difluoro-phényl)-éthyl]-cyclopropanecarboxamide sous la forme d'une huile incolore [Spectre de R.M.N. ¹H (300 MHz, CDCl₃, δ en ppm) : 0,70 (mt : 1H); de 0,80 à 1,00 (mt : 2H); de 1,15 à 1,35 (mt : 1H); de 2,35 à 2,55 et de 2,70 à 3,10 (2 séries de mts : 7H en totalité); 3,26 (mt : 1H); 3,47 (mt : 1H); 4,19 (s : 1H); 5,63 (mt : 1H); 6,62 (tt, J = 9 et 2,5 Hz : 1H); 6,81 (mt : 2H); de 7,20 à 7,45 (mt : 8H)].

### Exemple 88

Le (RS)-N-[2-{1-[bis-(4-chloro-phényl)-méthyl]-azétidin-3-yl}-2-(3,5-difluorophényl)-éthyl]-3-méthyl-butyramide peut être préparé en opérant de la façon suivante : A une solution de 45 mg de (RS)-2-{1-[bis-(4-chloro-phényl)-méthyl]-azétidin-3-yl}-2-(3,5-difluoro-phényl)-éthylamine dans 5 cm³ de tétrahydrofuranne, à une température voisine de 20°C, sont ajoutés 114 mg de HATU, 30,6 mg d'acide isovalérique, puis 0,2 g de morpholine supportée sur polystyrène. La suspension obtenue est agitée à une température voisine de 20°C pendant 20 heures. Le milieu réactionnel est filtré, et concentré à sec sous pression réduite. On obtient ainsi 46 mg d'une huile orangée qui est purifiée par chromatographie sous pression sur une cartouche de 5 g de silice, en éluant avec un mélange de cyclohexane et d'acétate d'éthyle (70/30 en volumes). Les fractions ne contenant que le produit recherché sont réunies et concentrées à sec sous pression réduite. On obtient 6 mg de (RS)-N-[2-{1-[bis-(4-chloro-phényl)-méthyl]-azétidin-3-yl}-2-(3,5-difluoro-phényl)-éthyl]-3-méthyl-butyramide sous la forme d'une huile incolore [Spectre de R.M.N. ¹H (400 MHz, CDCl₃, δ en ppm) : 0,88 (d, J = 6,5 Hz : 3H); 0,91 (d, J = 6,5 Hz : 3H); de 1,85 à 2,10 (mt : 3H); de 2,30 à 2,55 et de 2, 70 à 3,10 (2 séries de mts : 6H en totalité); 3,37 (mt : 2H); 4,19 (s : 1H); 5,45 (mt : 1H); 6,65 (tt, J = 9 et 2,5 Hz : 1H); 6,82 (mt : 2H); de 7,20 à 7,45 (mt : 8H)].

### Exemple 89

Le (RS)-N-[2-{1-[bis-(4-chloro-phényl)-méthyl]-azétidin-3-yl}-2-(3,5-difluorophényl)-éthyl]-isobutyramide peut être préparé en opérant d'une manière semblable à l'exemple 3 précédent : A partir de 45 mg de (RS)-2-{1-[bis-(4-chloro-phényl)-méthyl]-azétidin-3-yl}-2-(3,5-difluoro-phényl)-éthylamine, 5 cm³ de tétrahydrofuranne, 114 mg de HATU, 26 mg d'acide isobutyrique, et 0,2 g de morpholine supportée sur polystyrène, on obtient 10 mg de (RS)-N-[2-{1-[bis-(4-chloro-phényl)-méthyl]-azétidin-3-yl}-2-(3,5-difluoro-phényl)-éthyl]-isobutyramide sous la forme d'une huile opaque [Spectre de R.M.N. ¹H (400 MHz, (CD₃)₂SO d6, δ en ppm) : 0,87 (d, J = 7 Hz : 3H); 0,93 (d, J = 7 Hz : 3H); de 2,15 à 2,85 (mt : 7H); de 3,00 à 3,25 (mt : 2H); 4,40 (s : 1H); de 7,00 à 7,20 (mt : 3H); 7,38 (mt : 4H); 7,52 (mt : 4H); 7,77 (mt : 1H)].

### Exemple 90

La {1-[bis-(4-chloro-phényl)-méthyl]-azétidin-3-yl}-(3,4-difluoro-phényl)-méthanone peut être préparée en opérant de la façon suivante : A une solution de 128 mg de l'amide N-méthoxy-N-méthyl de l'acide 1-[bis-(4-chloro-phényl)-méthyl]-azétidine-3-carboxylique dans 3 cm³ de tétrahydrofuranne, refroidie dans un bain d'acétone et de carboglace, est ajouté 3 cm³ d'une solution de bromure de 3,4-difluoro-phénylmagnésium 0,5N dans le tétrahydrofuranne. Après 20 heures d'agitation à une température voisine de 0°C. on ajoute 10 cm³ d'eau, puis le milieu réactionnel est agité 1 heure à une température voisine de 20°C. La phase aqueuse décantée est extraite avec 20 cm³ d'acétate d'éthyle. Les phases organiques rassemblées sont lavées avec 2 fois 15 cm³ d'eau, séchées sur sulfate de magnésium, et concentrées à sec sous pression réduite. On obtient ainsi 123 mg d'un résidu qui est purifié par chromatographie sous pression sur une cartouche de 20 g de silice, en éluant avec du dichlorométhane (stabilisé sur amylène). On obtient ainsi 47 mg de {1-[bis-(4-chloro-phényl)-méthyl]-azétidin-3-yl}-(3,4-difluoro-phényl)-méthanone sous forme d'une poudre blanche [Spectre de R.M.N. ¹H (300 MHz, CDCl₃, δ en ppm) : 3,34 (t, J = 7,5 Hz : 2H); 3,56 (t, J = 8 Hz : 2H); 4,05 (mt : 1H); 4,35 (s : 1H); de 7,15 à 7,40 (mt : 9H); 7,58 (dmt, J = 9 Hz : 1H); 7,70 (ddd, J = 9/7,5 et 2,5 Hz : 1H].

L'amide N-méthoxy-N-méthyl de l'acide 1-[bis-(4-chloro-phényl)-méthyl]-azétidine-3-carboxylique peut être préparé en opérant de la façon suivante : A une suspension de 2,03 g de chlorhydrate de N,O-diméthylhydroxylamine dans 40 cm³ de dichlorométhane, refroidie à une température voisine de 0°C par un bain d'eau glacée, sont ajoutés 2,65 cm³ de 1-méthylpipéridine. La solution jaune obtenue (solution A) est conservée à une température voisine de 0°C. A une suspension de 7 g de l'acide 1-[bis-(4-chloro-phényl)-méthyl]-azétidine-3-carboxylique dans 300 cm³ de dichlorométhane et 40 cm³ de tétrahydrofuranne, refroidie à une température voisine de -8°C par un bain de glace et d'isopropanol, sont ajoutés successivement 2,65 cm³ de 1-méthylpipéridine, puis 1,6 cm³ de chloroformiate de méthyle. Après environ 5 minutes d'agitation à une température voisine de -8°C, on ajoute goutte à goutte la solution A précédemment préparée. Après 10 minutes d'agitation à une température voisine de -8°C, le bain refroidissant est enlevé, et le mélange réactionnel est agité à une température voisine de 20°C pendant environ 20 heures, puis lavé avec 3 fois 150 cm³ d'eau, et concentré à sec sous pression réduite. On obtient ainsi 8,19 g d'un résidu qui est purifié sous pression sur 500 g de silice Amicon (diamètre des particules : 20 - 45 ?m) en éluant avec un mélange acétate d'éthyle/dichlorométhane (8 - 92 en volumes). On obtient ainsi 6,6 g de l'amide N-méthoxy-N-méthyl de l'acide 1-[bis-(4-chloro-phényl)-méthyl]-azétidine-3-carboxylique sous forme d'une huile jaune pâle.

L'acide 1-[bis-(4-chloro-phényl)-méthyl]-azétidine-3-carboxylique peut être préparé d'une manière semblable à celle décrite par ANDERSON A.G. et LOK R. J.Org.Chem., 37, 3953-3955 (1972) à partir du 1-benzhydrylazétidin-3-ol, en utilisant comme matière première le 1-[bis-(4-chloro-phényl)-méthyl]-azétidin-3-ol.

### Exemple 91

La {1-[bis-(4-chloro-phényl)-méthyl]-azétidin-3-yl}-(3,5-difluoro-phényl)-méthanone peut être préparée en opérant comme pour la {1-[bis-(4-chlorophényl)-méthyl]-azétidin-3-yl}-(3,4-difluoro-phényl)-méthanone, à partir de 1,1 g de l'amide N-méthoxy-N-méthyl de l'acide 1-[bis-(4-chloro-phényl)-méthyl]-azétidine-3-carboxylique, 1,5 cm³ de 1-bromo-3,5-difluorobenzène, et 316 mg de magnésium en tournures. On obtient ainsi 880 mg de {1-[bis-(4-chloro-phényl)-méthyl]-azétidin-3-yl}-(3,5-difluoro-phényl)-méthanone sous forme d'une huile visqueuse jaune pâle [Spectre de R.M.N. ¹H (300 MHz, CDCl₃, δ en ppm) : 3,34 (t, J = 7,5 Hz : 2H); 3,55 (t, J = 8 Hz : 2H); 4,03 (mt : 1H); 4,44 (s : 1H); 7,01 (tt, J = 9 et 2,5 Hz : 1H); de 7,20 à 7,40 (mt : 10H)].

### Exemple 92

La {1-[bis-(4-chloro-phényl)-méthyl]-azétidin-3-yl}-cyclohexyl-méthanone peut être préparée en opérant comme pour la {1-[bis-(4-chloro-phényl)-méthyl]-azétidin-3-yl}-(3,4-difluoro-phényl)-méthanone, à partir de 284 mg de l'amide N-méthoxy-N-méthyl de l'acide 1-[bis-(4-chloro-phényl)-méthyl]-azétidine-3-carboxylique et 1,68 cm³ de chlorure de cyclohexylmagnésium 2N dans le THF. On obtient ainsi 116 mg {1-[bis-(4-chloro-phényl)-méthyl]-azétidin-3-yl}-cyclohexyl-méthanone sous forme d'une huile visqueuse jaune [Spectre de R.M.N. ¹H (400 MHz, CDCl₃, δ en ppm) : de 1,10 à 1,35 et de 1,55 à 1,85 (2 série de mt : 10H en totalité); 2,30 (mt : 1H); 3,14 (t, J = 8 Hz : 2H); 3,36 (t, J = 8 Hz : 2H); 3,56 (mt : 1H); 4,31 (s : 1H); de 7,20 à 7,35 (mt : 8H)].

### Exemple 93

La {1-[bis-(4-chloro-phényl)-méthyl]-azétidin-3-yl}-phényl-méthanone peut être préparée en opérant comme pour la {1-[bis-(4-chloro-phényl)-méthyl]-azétidin-3-yl}-(3,4-difluoro-phényl)-méthanone, à partir de 258 mg de l'amide N-méthoxy-N-méthyl de l'acide 1-[bis-(4-chloro-phényl)-méthyl]-azétidine-3-carboxylique et 1,02 cm³ de bromure de phénylmagnésium 3N dans le THF. On obtient ainsi 208 mg de {1-[bis-(4-chloro-phényl)-méthyl]-azétidin-3-yl}-phényl-méthanone sous forme d'une huile visqueuse jaune [Spectre de R.M.N. ¹H (300 MHz, CDCl₃, δ en ppm) : 3,35 (t, J = 8 Hz : 2H); 3,57 (t, J = 8 Hz : 2H); 4,13 (mt : 1 H); 4,35 (s : 1H); 7,25 (dmt, J = 8 Hz : 4H); 7,34 (dmt, J = 8 Hz : 4H); 7,45 (t large, J = 8 Hz : 2H); 7,56 (tt, J = 8 et 1,5 Hz : 1H); 7,84 (dmt, J = 8 Hz : 2H)].

### Exemple 94

Le (RS)-1-{1-[bis-(4-chloro-phényl)-méthyl]-azétidin-3-yl}-1-(3,5-difluorophényl)-ethanol peut être préparé en opérant de la façon suivante : A une solution de 100 mg de {1-[bis-(4-chloro-phényl)-méthyl]-azétidin-3-yl}-(3,5-difluoro-phényl)-méthanone dans 4 cm³ de tétrahydrofuranne, refroidie à une température inférieure à -40°C, est ajouté 0,167 cm³ d'une solution de bromure de méthylmagnésium 3N. Après 20 heures d'agitation à une température voisine de 0°C, on ajoute 5 cm³ d'eau, puis la phase aqueuse décantée est extraite avec 5 cm³ d'acétate d'éthyle. Les phases organiques rassemblées sont séchées sur sulfate de magnésium, et concentrées à sec sous pression réduite. On obtient ainsi 91 mg d'un résidu qui est purifié par chromatographie sous pression sur une cartouche de 10 g de silice, en éluant avec un mélange acétate d'éthyle/cyclohexane (1/9 en volumes). On obtient ainsi 74 mg de (RS)-1-{1-[bis-(4-chloro-phényl)-méthyl]-azétidin-3-yl}-1-(3,5-difluoro-phényl)-ethanol sous forme d'une huile incolore [Spectre de R.M.N. ¹H (300 MHz, CDCl₃, δ en ppm) : 1,46 (s : 3H); 2,71 (mt : 1H); 2,82 (mt : 1H); 2,98 (t, J = 7,5 Hz : 1H); 3,24 (t, J = 7,5 Hz : 1H); 3,34 (mt : 1H); 4,31 (s : 1H); 4,33 (mf : 1H); 6,67 (tt, J = 9 et 2,5 Hz : 1H); 6,98 (mt : 2H); de 7,25 à 7,35 (mt : 8H)].

### Exemple 95

La O-allyl-oxime de la {1-[bis-(4-chloro-phényl)-méthyl]-azétidin-3-yl}-(3,5-difluoro-phényl)-méthanone peut être préparée en opérant de la façon suivante : Une solution de 100 mg de {1-[bis-(4-chloro-phényl)-méthyl]-azétidin-3-yl}-(3,5-difluoro-phényl)-méthanone et 101 mg de chlorhydrate de O-allyl-hydroxylamine dans 5 cm³ de pyridine est agité, à une température voisine de 20°C pendant 20 heures. On ajoute alors 5 cm³ d'eau, et l'on extrait le mélange réactionnel avec 2 fois 5 cm³ d'acétate d'éthyle. Les phases organiques rassemblées sont séchées sur sulfate de magnésium, puis concentrées à sec sous pression réduite. On obtient ainsi 111 mg d'une huile jaune qui est purifiée par chromatographie sous pression sur une cartouche de 20 g de silice (diamètre des particules de 0,04 à 0,063 mm) en éluant avec un mélange acétate d'éthyle/cyclohexane (2/98 en volumes). On obtient ainsi 68 mg O-allyl-oxime de la {1-[bis-(4-chloro-phényl)-méthyl]-azétidin-3-yl}-(3,5-difluoro-phényl)-méthanone sous forme d'une huile visqueuse incolore [Spectre de R.M.N. ¹H (300 MHz, CDCl₃, δ en ppm). On observe un mélange des 2 isomères Z et E dans les proportions approximatives 65/35 ou inversement; 2,84 et 3,11 (2 t larges, respectivement J = 8 Hz et J = 7,5 Hz : 2H en totalité); 3,44 et 3,66 (2 t larges, respectivement J = 7,5 Hz et J = 8 Hz : 2H en totalité); 3,58 et 3,81 (2 mts : 1H totalité); 4,16 et 4,30 (2 s : 1H en totalité); 4,59 (mt : 2H); 5,22 (dmt, J = 11 Hz : 1H); 5,27 (dmt, J = 18 Hz : 1H); 5,96 (mt : 1H); 6,80 (tt, J = 9 et 2,5 Hz : 1H); 6,91 (mt : 2H); de 7,20 à 7,35 (mt : 8H].

### Exemple 96

La O-éthyl-oxime de la {1-[bis-(4-chloro-phényl)-méthyl]-azétidin-3-yl}-(3,5-difluoro-phényl)-méthanone peut être préparée en opérant comme décrit pour la préparation de la O-allyl-oxime de la {1-[bis-(4-chloro-phényl)-méthyl]-azétidin-3-yl}-(3,5-difluoro-phényl)-méthanone : A partir de 100 mg de {1-[bis-(4-chloro-phényl)-méthyl]-azétidin-3-yl}-(3,5-difluoro-phényl)-méthanone et 90 mg de chlorhydrate de O-éthyl-hydroxylamine, on obtient ainsi 83 mg O-éthyl-oxime de la {1-[bis-(4-chloro-phényl)-méthyl]-azétidin-3-yl}-(3,5-difluorophényl)-méthanone sous forme d'une huile visqueuse incolore [Spectre de R.M.N. ¹H (300 MHz, CDCl₃, δ en ppm). On observe un mélange des 2 isomères Z et E dans les proportions approximatives 65/35 ou inversement; 1,25 et 1,27 (2 t, J = 7 Hz: 3H en totalité); 2,82 et 3,12 (2 t larges, respectivement J = 8 Hz et J = 7,5 Hz : 2H en totalité); 3,45 et 3,66 (2 t larges, respectivement J = 7,5 Hz et J = 8 Hz : 2H en totalité); 3,58 et 3,78 (2 mts : 1H totalité); de 4,05 à 4,20 (mt : 2H); 4,16 et 4,30 (2 s : 1H en totalité); 6,80 (tt, J = 9 et 2,5 Hz : 1 H); 6,91 (mt : 2H); de 7,20 à 7,35 (mt : 8H)].

### Exemple 97

La (RS)-1-[{1-[bis-(4-chloro-phényl)-méthyl]-azétidin-3-yl}-(3,5-difluorophényl)-méthyl]-3-méthylurée peut être préparée de la manière suivante : A une solution de 300 mg du chlorhydrate de l'acide {1-[bis-(4-chloro-phényl)-méthyl]-azétidin-3-yl}-(3,5-difluoro-phényl)-acétique dans 15 cm³ de toluène anhydre, sous atmosphère inerte d'azote, à une température voisine de 20°C, sont ajoutés successivement, 0,336 cm³ de triéthylamine et 0,384 cm³ de diphénylphosphonoazide. La solution obtenue est agitée à une température voisine de 60°C pendant environ 90 minutes. On ajoute 2,6 cm³ d'une solution de méthylamine 2M dans le tétrahydrofuranne, l'agitation est maintenue à une température voisine de 20°C pendant environ 12 heures, le mélange réactionnel est concentré à sec sous pression réduite (2,7 kPa) à une température voisine de 40°C. Le résidu est repris par 1cm³ de méthanol puis déposé sur une cartouche BOND-ELUT SCX VARIAN 5g de référence 1225-6027 conditionnée au méthanol. La cartouche est lavée au méthanol puis éluée au méthanol amoniacal 2N. Les fractions ammoniacales sont réunies et concentrées à sec sous pression réduite (2,7 kPa) à une température voisine de 40°C. On obtient ainsi 250 mg d'une huile claire qui est reprise par 1 cm³ de dichlorométhane puis déposée sur une cartouche de 16 mn de diamètre, remplie de 5 g de silice de granulométrie 0,015-0,035 mm, conditionnée et éluée au dichlorométhane entre 0 et 40 cm³ puis éluée avec un mélange dichlorométhane-acétate d'éthyle (80-20 en volumes) à l'aide d'un système de pompage. Les fractions comprises entre 50 et 80 cm³ sont réunies et concentrées à sec sous pression réduite (2,7 kPa) à 40°C. On obtient ainsi 140 mg de (RS)-1-[{1-[bis-(4-chloro-phényl)-méthyl]-azétidin-3-yl}-(3,5-difluoro-phényl)-méthyl]-3-méthylurée sous forme d'une meringue [Spectre de R.M.N. ¹H (400 MHz, CDCl₃, δ en ppm) : 2,66 (mt : 1H); 2,82 (d, J = 5 Hz : 3H); 2,95 (mt : 1H); 3,03 (mt : 1H); 3,18 (mt : 2H); 4,24 (mt : 1 H); 4,30 (s : 1H); 4,92 (t, J = 7 Hz : 1H); 5,36 (d large, J = 7 Hz : 1H); 6,67 (tt, J = 9 et 2,5 Hz : 1H); 6,80 (mt : 2H); de 7,20 à 7,30 (mt : 8H)].

La préparation du chlorhydrate de l'acide {1-[bis-(4-chloro-phényl)-méthyl]-azétidin-3-yl}-(3,5-difluoro-phényl)-acétique a été décrite dans le brevet « dérivés carbonés », exemple 77.

### Exemple 98

La (RS)-1-[{1-[bis-(4-chloro-phényl)-méthyl]-azétidin-3-yl}-(3,5-difluorophényl)-méthyl]-3-isopropylurée peut être préparée de la manière suivante : A une solution de 17 µl d'isopropylamine dans 1 cm³ de toluène anhydre, sous atmosphère inerte d'azote, à une température voisine de 20°C, sont ajoutés 3 cm³, soit 0,1 mM d'une solution de (RS)-1-[bis-(4-chloro-phényl)-méthyl]-3-[(3,5-difluoro-phényl)-isocyanato-méthyl]-azétidine fraîchement préparée. La solution obtenue est agitée à une température voisine de 20°C pendant environ 12 heures. Le milieu réactionnel est concentré à sec sous pression réduite (2,7 kPa) à une température voisine de 40°C. Le résidu solide est repris par 1cm³ de méthanol puis déposé sur une cartouche BOND-ELUT SCX VARIAN 500mg de référence 1210-2040 conditionnée au méthanol. La cartouche est lavée au méthanol puis éluée au méthanol amoniacal 2N. Les fractions ammoniacales sont réunies et concentrées à sec sous pression réduite (2,7 kPa) à une température voisine de 40°C. Le résidu solide ainsi obtenu est repris par 1cm³ de dichlorométhane puis déposé sur une cartouche IST FlashPack de référence SIL 016-002 remplie de 2 g de silice (0,065-0,090 mm) conditionnée au dichlorométhane et éluée avec un mélange dichlorométhane-acétate d'éthyle (90-10 en volumes) à l'aide d'un système de pompage. Les fractions comprises entre 20 et 38 cm³ sont réunies et concentrées à sec sous pression réduite (2,7 kPa) à 40°C. On obtient ainsi 14 mg de (RS)-1-[{1-[bis-(4-chloro-phényl)-méthyl]-azétidin-3-yl}-(3,5-difluoro-phényl)-méthyl]-3-isopropylurée sous forme de meringue blanche [Spectre de R.M.N. ¹H (400 MHz, CDCl₃, δ en ppm) : 1,14 (d, J = 6,5 Hz : 3H); 1,15 (d, J = 6,5 Hz : 3H); 2,66 (mt : 1H); 2,92 (dd large, J = 8 et 5,5 Hz : 1H); 3,01 (dd large, J = 8 et 5,5 Hz : 1H); 3,16 (t, J = 8 Hz : 1H); 3,20 (t, J = 8 Hz : 1H); 3,85 (mt : 1H); 4,06 (d, J = 8 Hz : 1H); 4,29 (s : 1H); 4,91 (t, J = 7 Hz : 1H); 5,17 (d, J = 6,5 Hz : 1H); 6,66 (tt, J = 9 et 2 Hz : 1H); 6,78 (mt : 2H); de 7,20 à 7,35 (mt : 8H)].

La-(RS)-1-[bis-(4-chloro-phényl)-méthyl]-3-[(3,5-difluoro-phényl)-isocyanato-méthyl]-azétidine peut être préparée de la manière suivante : A une solution de 150 mg du chlorhydrate de l'acide {1-[bis-(4-chloro-phényl)-méthyl]-azétidin-3-yl}-(3,5-difluoro-phényl)-acétique dans 9 cm³ de toluène anhydre, sous atmosphère inerte d'azote, à une température voisine de 20°C, sont ajoutés successivement: 0,126 cm³ de triéthylamine et 0,195 cm³ de diphénylphosphonoazide. La solution obtenue est agitée à une température voisine de 50°C pendant environ 1 heure. On laisse refroidir et l'on obtient ainsi le (RS)-1-[bis-(4-chloro-phényl)-méthyl]-3-[(3,5-difluoro-phényl)-isocyanato-méthyl]-azétidine en solution dans le toluène que l'on utilisera ultérieurement sous cette forme.

### Exemple 99

La (RS)-1-[{1-[bis-(4-chloro-phényl)-méthyl]-azétidin-3-yl}-(3,5-difluorophényl)-méthyl]-3-isobutylurée peut être préparée de la manière suivante : A une solution de 20 µl d'isobutylamine dans 1 cm³ de toluène anhydre, sous atmosphère inerte d'azote, à une température voisine de 20°C, sont ajoutés 3 cm³, soit 0,1 mM d'une solution de (RS)-1-[bis-(4-chloro-phényl)-méthyl]-3-[(3,5-difluoro-phényl)-isocyanato-méthyl]-azétidine fraîchement préparée. La solution obtenue est agitée à une température voisine de 20°C pendant environ 12 heures. Le milieu réactionnel est concentré à sec sous pression réduite (2,7 kPa) à une température voisine de 40°C. Le résidu solide est repris par 1cm³ de méthanol puis déposé sur une cartouche BOND-ELUT SCX VARIAN 500mg de référence 1210-2040 conditionnée au méthanol. La cartouche est lavée au méthanol puis éluée au méthanol amoniacal 2N. Les fractions ammoniacales sont réunies et concentrées à sec sous pression réduite (2,7 kPa) à une température voisine de 40°C. Le résidu ainsi obtenu est repris par 1cm³ de dichlorométhane puis déposé sur une cartouche IST FlashPack de référence SIL 016-002 remplie de 2 g de silice (0,065-0,090 mm) conditionnée au dichlorométhane et éluée avec un mélange dichlorométhane-acétate d'éthyle (90-10 en volumes) à l'aide d'un système de pompage. Les fractions comprises entre 0 et 15 cm³ sont réunies et concentrées à sec sous pression réduite (2,7 kPa) à 40°C. On obtient ainsi 14 mg de(RS)-1-[{1-[bis-(4-chloro-phényl)-méthyl]-azétidin-3-yl}-(3,5-difluorophényl)-méthyl]-3-isobutylurée sous forme de meringue blanche [Spectre de R.M.N. ¹H (400 MHz, CDCl₃, δ en ppm) : 0,89 (d, J = 6,5 Hz : 3H); 0,90 (d, J = 6,5 Hz : 3H); 1,74 (mt : 1H); 2,66 (mt : 1H); de 2,90 à 3,25 (mt : 6H); 4,29 (s et mt : 2H en totalité); 4,90 (t, J = 7 Hz : 1H); 5,34 (d large, J = 6,5 Hz : 1H); 6,66 (tt, J = 9 et 2 Hz : 1 H); 6,80 (mt : 2H); de 7,20 à 7,35 (mt : 8H)].

### Exemple 100

Le N-méthyl-N-phényl-1-[bis-(4-chloro-phényl)-méthyl]-azétidine-3-carboxamide peut être préparé de la manière suivante : A une solution de 100 mg d'acide 1-[bis-(4-chloro-phényl)-méthyl]-azétidine-3-carboxylique dans 2 cm³ de dichlorométhane anhydre, à une température voisine de 20°C, sont ajoutés successivement : 0,039 cm³ de N-méthylaniline, 87 mg de chlorhydrate de 1-(3-diméthylaminopropyl)-3-éthylcarbodiimide, 0,063 cm³ de triéthylamine, puis 4 mg d'hydrate d'hydroxybenzotriazol. La solution obtenue est agitée à une température voisine de 20°C pendant environ 12 heures. Le milieu réactionnel est déposé sur une cartouche IST FlashPack de référence

SIL 016-005 remplie de 5 g de silice (0,065-0,090 mm) conditionnée au dichlorométhane et éluée avec un gradient de mélange dichlorométhane-acétate d'éthyle (le pourcentage d'acétate d'éthyl variant de 0 à 5 en volumes) à l'aide d'un système de pompage en recueillant des fractions de 1,5 cm³. Les fractions 3 à 15 sont réunies et concentrées à sec sous pression réduite (2,7 kPa) à 50°C. On obtient ainsi 95 mg de 1-[bis-(4-chloro-phényl)-méthyl]-azétidine-N-méthyl-N-phényl-3-carboxamide sous forme d'une meringue crème [Spectre de R.M.N. ¹H (400 MHz, CDCl₃, δ en ppm) : 3,08 (mt : 2H); 3,21 (mt : 3H); 3,27 (s : 3H); 4,35 (s : 1H); 7,06 (d, J = 7,5 Hz : 2H); de 7,15 à 7,45 (mt : 11H)].

### Exemple 101

Le 1-[bis-(4-chloro-phényl)-méthyl]-azétidine-N-benzyl-N-méthyl-3-carboxamide peut être préparé de la manière suivante : A une suspension de 150 mg d'acide 1-[bis-(4-chloro-phényl)-méthyl]-azétidine-3-carboxylique activé sur résine TFP (165 µM) dans 2 cm³ de dichlorométhane est ajouté 0,0213 cm³ de N-benzylméthylamine. La suspension est agitée à une température voisine de 20 °C pendant 22 heures, puis filtrée sur fritté. Le résidu solide est relavé avec 2 fois 1 cm³ de dichlorométhane. Les filtrats sont réunis et concentrés à sec sous pression réduite (2,7 kPa) à une température voisine de 40°C. On obtient ainsi 38 mg de 1-[bis-(4-chloro-phényl)-méthyl]-azétidine-N-benzyl-N-méthyl-3-carboxamide sous forme de gomme incolore [Spectre de R.M.N. ¹H (300 MHz, CDCl₃, δ en ppm) : 2,78 (s : 3H); de 3,25 à 3,55 (mt : 5H); 4,38 (mt : 2H); 4,57 (s : 1H); de 7,15 à 7,40 (mt : 13H].

L'acide 1-[bis-(4-chloro-phényl)-méthyl]-azétidine-3-carboxylique activé sur résine TFP peut être préparé de la manière suivante : A une suspension de 2,7 g de résine TFP (fonction phénol libre, 1,1 mmole/g, soit 2,975 mM) dans 40 cm³ de diméthylformamide anhydre sont ajoutés : 2 g d'acide 1-[bis-(4-chloro-phényl)-méthyl]-azétidine-3-carboxylique, 73 mg de 4-diméthylaminopyridine, 0,927 cm³ de 1,3-diisopropylcarbodiimide. Après 19 heures d'agitation à une température voisine de 20°C, la suspension est filtrée, la résine est lavée avec 40 cm³ de diméthylformamide, 40 cm³ de tétrahydrofuranne, 40 cm³ de dichlorométhane, puis séchée sous vide à poids constant. On obtient ainsi 3,6 g de l'acide 1-[bis-(4-chloro-phényl)-méthyl]-azétidine-3-carboxylique activé sur résine TFP.

L'acide 1-[bis-(4-chloro-phényl)-méthyl]-azétidine-3-carboxylique peut être préparé d'une manière semblable à celle décrite par ANDERSON A.G. et LOK R. J.Org.Chem., 37, 3953-3955 (1972) à partir du 1-benzhydrylazétidin-3-ol, en utilisant comme matière première le 1-[bis-(4-chloro-phényl)-méthyl]-azétidin-3-ol.

La résine TFP (fonction phénol libre) peut être préparée selon le mode opératoire décrit dans le brevet WO9967228.

### Exemple 102

La (RS)-[{1-[bis-(4-chloro-phényl)-méthyl]-azétidin-3-yl}-(3,5-difluoro-phényl)-méthyl]-méthylamine peut être préparée de la manière suivante : A une solution de 108 mg de {1-[bis-(4-chloro-phényl)-méthyl]-azétidin-3-yl}-(3,5-difluoro-phényl)-méthanone dans 2 cm³ de dichlo-1,2 éthane anhydre est ajouté, 0,099 cm³ de méthylamine, puis successivement 84 mg de triacétoxyborohydrure de sodium, 0,014 cm³ d'acide acétique. Après 12 heures d'agitation à une température voisine de 20°C sont rajoutés à nouveau successivement 0,992 cm³ de méthylamine, 85 mg de triacétoxyborohydrure de sodium, puis 0,143 cm³ d'acide acétique. La solution obtenue est agitée à une température voisine de 20°C pendant environ 24 heures puis lavée par 4 cm³ d'une solution saturée de bicarbonate de sodium. La phase organique est décantée puis séchée sur du sulfate de magnésium, filtrée puis concentrée sous pression réduite (2,7 kPa). Le résidu ainsi obtenu est purifié sur une cartouche IST FlashPack de référence SIL 016-002 remplie de 2 g de silice (0,065-0,090 mm) conditionnée au dichlorométhane et éluée avec un gradient de mélange dichlorométhane-méthanol (le pourcentage de méthanol variant de 0 à 6 en volumes) à l'aide d'un système de pompage en recueillant des fractions de 1cm³. Les fractions 8 à 18 sont réunies et concentrées à sec sous pression réduite (2,7 kPa) à 50°C. On obtient ainsi 66 mg de [{1-[bis-(4-chloro-phényl)-méthyl]-azétidin-3-yl}-(3,5-difluoro-phényl)-méthyl]-méthylamine sous forme de miel incolore [Spectre de R.M.N. ¹H (300 MHz, CDCl₃, δ en ppm) : 2,25 (s : 3H); 2,60 (mt : 1H); 2,68 (t, J = 7 Hz : 1H); 2,94 (t, J = 7 Hz : 1H); 3,02 (t large, J = 7 Hz : 1H); 3,34 (t large, J = 7 Hz : 1H); 3,58 (d, J = 9 Hz : 1H); 4,25 (s : 1H); 6,67 (tt, J = 9 et 2,5 Hz : 1H); 6,80 (mt : 2H); de 7,20 à 7,35 (mt : 8H)].

### Exemple 103

La (RS)-[{1-[bis-(4-chloro-phényl)-méthyl]-azétidin-3-yl}-(3,5-difluoro-phényl)-méthyl]-isobutylamine peut être préparée de la manière suivante : A une solution de 109 mg de {1-[bis-(4-chloro-phényl)-méthyl]-azétidin-3-yl}-(3,5-difluoro-phényl)-méthanone dans 2 cm³ de dichlo-1,2 éthane anhydre est ajouté, 0,028 cm³ d'isobuthylamine, puis successivement: 85 mg de triacétoxyborohydrure de sodium, 0,015 cm³ d'acide acétique. La solution obtenue est agitée à une température voisine de 20°C pendant environ 12 heures. Le milieu réactionnel est déposé sur une cartouche remplie de 5 g de silice, conditionnée au dichlorométhane et éluée avec un gradient de mélange dichlorométhane-acétate d'éthyle (le pourcentage d'acétate d'éthyle variant de 0 à 10 en volumes) à l'aide d'un système de pompage. Les fractions contenant le produit cherché sont réunies et concentrées à sec sous pression réduite (2,7 kPa) à 40°C. On obtient ainsi 8 mg de (RS)-[{1-[bis-(4-chloro-phényl)-méthyl]-azétidin-3-yl}-(3,5-difluoro-phényl)-méthyl]-isobutylamine [Spectre de R.M.N. ¹H (300 MHz, CDCl₃, δ en ppm) : 0,85 (d, J = 7 Hz : 3H); 0,87 (d, J = 7 Hz : 3H); 1,63 (mt : 1H); 2,15 (dd, J = 11 et 7,5 Hz : 1H); 2,25 (dd, J = 11 et 7 Hz : 1H); 2,57 (mt : 1H); 2,70 (t, J = 7 Hz : 1H); 2,92 (t, J = 7 Hz: 1H); 3,01 (t large, J = 7, 5 Hz: 1H); 3,33 (t large, J = 7,5 Hz : 1H); 3,66 (d, J = 9 Hz : 1H); 4,25 (s : 1H); 6,66 (tt, J = 9 et 2,5 Hz : 1H); 6,81 (mt : 2H); de 7,20 à 7,35 (mt : 8H)].

### Exemple 104

La (RS)-[{1-[bis-(4-chloro-phényl)-méthyl]-azétidin-3-yl}-(3,5-difluoro-phényl)-méthyl]-butylamine peut être préparée de la manière suivante : A une solution de 108 mg de {1-[bis-(4-chloro-phényl)-méthyl]-azétidin-3-yl}-(3,5-difluorophényl)-méthanone dans 2 cm³ de dichlo-1,2 éthane anhydre est ajouté, 0,0265 cm³ de n.butylamine, puis successivement 95 mg de triacétoxyborohydrure de sodium, 0,0143 cm³ d'acide acétique. Après environ 16 heures d'agitation à une température voisine de 20°C sont rajoutés à nouveau successivement 0,0265 cm³ de n.butylamine, 85 mg de triacétoxyborohydrure de sodium, puis 0,143 cm³ d'acide acétique. La solution obtenue est agitée à une température voisine de 20°C pendant environ 24 heures puis lavée par 4 cm³ d'une solution saturée de bicarbonate de sodium. La phase organique est décantée puis séchée sur du sulfate de magnésium, filtrée puis concentrée sous pression réduite (2,7 kPa). Le résidu ainsi obtenu est purifié sur une cartouche IST FlashPack de référence SIL 016-002 remplie de 2 g de silice (0,065-0,090 mm) conditionnée au dichlorométhane et éluée avec un gradient de mélange dichlorométhane-méthanol (le pourcentage de méthanol variant de 0 à 6 en volumes) à l'aide d'un système de pompage en recueillant des fractions de 1 cm³. Les fractions 25 à 30 sont réunies et concentrées à sec sous pression réduite (2,7 kPa) à 50°C. On obtient ainsi 37 mg de RS)-[{1-[bis-(4-chloro-phényl)-méthyl]-azétidin-3-yl}-(3,5-difluoro-phényl)-méthyl]-butylamine sous forme d'un miel incolore [Spectre de R.M.N. ¹H (300 MHz, CDCl₃, δ en ppm) : 0,85 (t, J = 7,5 Hz : 3H); de 1,20 à 1,50 (mt : 4H); 2,37 (t large, J = 7 Hz : 2H); 2,56 (mt : 1H); 2,67 (t, J = 7 Hz : 1H); 2,89 (t, J = 7 Hz : 1H); 2,99 (t large, J = 7 Hz : 1H); 3,32 (t large, J = 7 Hz : 1H); 3,67 (d, J = 9 Hz : 1H); 4,24 (s : 1H); 6,65 (tt, J = 9 et 2,5 Hz : 1H); 6,80 (mt : 2H); de 7,20 à 7,35 (mt : 8H].

### Exemple 105

Le (RS)-N-[{1-[bis-(4-chloro-phényl)-méthyl]-azétidin-3-yl}-(3,5-difluorophényl)-méthyl]-3-méthyl-butyramide peut être préparé de la manière suivante : A une solution de 0,017 cm³ d'acide isovalérique dans 2 cm³ de dichlorométhane anhydre, à une température voisine de 20°C, sont ajoutés successivement: 0,025 cm³ de N,N'-diisopropylcarbodiimide, 10 mg d'hydrate d'hydroxybenzotriazole, 30 mg de (RS)-C-{1-[bis-(4-chloro-phényl)-méthyl]-azétidin-3-yl}-C-(3,5-difluoro-phényl)-méthylamine. La solution obtenue est agitée à une température voisine de 20°C pendant environ 12 heures. Le milieu réactionnel est déposé sur une cartouche BOND-ELUT

SCX VARIAN 500mg de référence 1210-2040 conditionnée au méthanol. La cartouche est lavée au méthanol puis éluée au méthanol amoniacal 2N. Les fractions ammoniacales sont réunies et concentrées à sec sous pression réduite (2,7 kPa) à une température voisine de 40°C. On obtient ainsi 35 mg de (RS)-N-[{1-[bis-(4-chloro-phényl)-méthyl]-azétidin-3-yl}-(3,5-difluorophényl)-méthyl]-3-méthyl-butyramide sous forme de miel jaune [Spectre de R.M.N. ¹H (300 MHz, CDCl₃, δ en ppm) : 0,98 (d, J = 5 Hz : 6H); de 2,05 à 2,25 (mt : 3H); 2,71 (mt : 1 H); 2,90 (mt : 1H); 3,00 (mt : 1H); 3,20 (mt : 2H); 4,30 (s : 1H); 5,14 (t, J = 7,5 Hz : 1H); 6,48 (d large, J = 7,5 Hz : 1H); 6,67 (tt, J = 9 et 2,5 Hz : 1H); 6,75 (mt : 2H); de 7,20 à 7,40 (mt : 8H)].

La (RS)-C-{1-[bis-(4-chloro-phényl)-méthyl]-azétidin-3-yl}-C-(3,5-difluorophényl)-méthylamine peut être préparée de la manière suivante : A une solution de 216 mg de (RS)-{1-[bis-(4-chloro-phényl)-méthyl]-azétidin-3-yl}-(3,5-difluoro-phényl)-méthanone dans 10 cm³ de méthanol, puis successivement 385 mg d'acétate d'ammonium et 29 mg de cyanoborohydrure de sodium. La solution obtenue est agitée à une température voisine de 20°C pendant environ 12 heures, puis tiédie à 45°C pendant 6h. A cette solution est rajouté 29 mg de cyanoborohydrure de sodium. L'agitation est poursuivie à une température voisine de 20°C pendant 72 heures. Le milieu réactionnel est versé dans un mélange de 30 cm³ d'eau glacée avec 5cm³ d'une solution aqueuse d'hydroxyde de sodium 4N, puis extrait par deux fois 30 cm³ d'acétate d'éthyle, la phase organique est extraite par deux fois 30 cm³ d'acide chlorhydrique N, la phase aqueuse ainsi obtenue est alcalinisée par une solution aqueuse d'hydroxyde de sodium normale puis extraite par trois fois 20 cm³ d'acétate d'éthyle. Les phases organiques réunies sont séchées sur sulfate de magnésium, filtrées puis concentrées à sec sous pression réduite (2,7 kPa) à 40°C. On obtient ainsi 35 mg de (RS)-C-{1-[bis-(4-chloro-phényl)-méthyl]-azétidin-3-yl}-C-(3,5-difluoro-phényl)-méthylamine sous forme de miel jaune.

Les médicaments selon l'invention sont constitués par un composé de formule (I) ou un isomère ou un sel d'un tel composé, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops ets élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles pour le traitement et/ou la prévention des psychoses y compris la schizophrénie, des troubles anxieux, de la dépression, de l'épilepsie, de la neurodégénération, des désordres cérébelleux et spinocérébelleux, des désordres cognitifs, du trauma crânien, des attaques de panique, des neuropathies périphériques, des glaucomes, de la migraine, de la maladie de Parkinson, de la maladie d'Alzheimer, de la chorée de Huntington, du syndrome de Raynaud, des tremblements, du désordre compulso-obsessionnel, de la démence sénile, des désordres thymiques, du syndrome de Tourette, de la dyskinésie tardive, des désordres bipolaires, des cancers, des désordres du mouvement induit par les médicaments, des dystonies, des chocs endotoxémiques, des chocs hémorragiques, de l'hypotension, de l'insomnie, des maladies immunologiques, de la sclérose en plaques, des vomissements, de l'asthme, des troubles de l'appétit (boulimie, anorexie), de l'obésité, des troubles de la mémoire, des troubles du transit intestinal, dans le sevrage aux traitements chroniques et abus d'alcool ou de médicaments (opioides, barbituriques, cannabis, cocaïne, amphétamine, phencyclide, hallucinogènes, benzodiazépines par exemple), comme analgésiques ou potentialisateurs de l'activité analgésique des médicaments narcotiques et non narcotiques.

Les doses dépendent de l'effet recherché, de la durée du traitement et de la voie d'administration utilisée; elles sont généralement comprises entre 5 mg et 1000 mg par jour par voie orale pour un adulte avec des doses unitaires allant de 1 mg à 250 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :

| | |
|---|---|
| - Composé de formule (I) | 50 mg |
| - Cellulose | 18 mg |
| - Lactose | 55 mg |
| - Silice colloïdale | 1 mg |
| - Carboxyméthylamidon sodique | 10 mg |
| - Talc | 10 mg |
| - Stéarate de magnésium | 1 mg |

### EXEMPLE B

On prépare selon la technique habituelle des comprimés dosés à 50 mg de produit actif ayant la composition suivante :

| | |
|---|---|
| - Composé de formule (I) | 50 mg |
| - Lactose | 104 mg |
| - Cellulose | 40 mg |
| - Polyvidone | 10 mg |
| - Carboxyméthylamidon sodique | 22 mg |
| - Talc | 10 mg |
| - Stéarate de magnésium | 2 mg |
| - Silice colloïdale | 2 mg |
| - Mélange d'hydroxyméthylcellulose, glycérine, oxyde de | |
| titane (72-3,5-24,5) q.s.p. 1 comprimé pelliculé terminé à | 245 mg |

### EXEMPLE C

On prépare une solution injectable contenant 10 mg de produit actif ayant la composition suivante :

| | |
|---|---|
| - Composé de formule (I) | 10 mg |
| - Acide benzoïque | 80 mg |
| - Alcool benzylique | 0,06 ml |
| - Benzoate de sodium | 80 mg |
| - Ethanol à 95 % | 0,4 ml |
| - Hydroxyde de sodium | 24 mg |
| - Propylène glycol | 1,6 ml |
| - Eau q.s.p. | 4 ml |

## Revendications

1. Un composé de formule 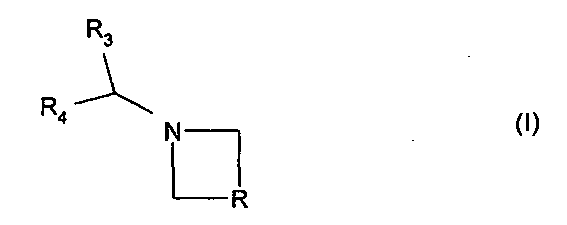 dans laquelle
R représente un radical CR₁R₂, C=C(R₅)SO₂R₆ ou C=C(R₇)SO₂alk,
soit R₁ représente un atome d'hydrogène et R₂ représente un radical -C(R₈)(R₉)(R₁₀), -C(R₈)(R₁₁)(R₁₂), -CO-NR₁₃R₁₄, -CH₂-CO-NR₁₃R₁₄, -CH₂-CO-R₆, -CO-R₆, -CO-cycloalkyle, -SO-R₆, -SO₂-R₆, -C(OH)(R₁₂)(R₆), -C(OH)(R₆)(alkyle), -C(=NOalk)R₆, -C(=NO-CH₂-CH=CH₂)R₆, -CH₂-CH(R₆)NR₃₁R₃₂, -CH₂-C(=NOalk)R₆, -CH(R₆)NR₃₁R₃₂, -CH(R₆)NHSO₂alk, -CH(R₆)NHCONHalk ou -CH(R₆)NHCOalk,
soit R₁ représente un radical alkyle, NH-R₁₅, cyano, -S-alk-NR₁₆R₁₇, -CH₂-NR₁₈R₁₉, ou -NR₂₀R₂₁ et R₂ représente un radical -C(R₈)(R₁₁)(R₁₂),
R₃ et R₄, identiques ou différents, représentent soit un radical alkyle ou cycloalkyle, soit un aromatique choisi parmi phényle, naphtyle ou indényle, ces aromatiques étant non substitués ou substitués par un ou plusieurs halogène, alkyle, alcoxy, formyle, hydroxy, trifluorométhyle, trifluorométhoxy, -CO-alk, cyano, -COOH, -COOalk, -CONR₂₂R₂₃, -CO-NH-NR₂₄R₂₅, alkylsulfanyle, alkylsulfinyle, alkylsulfonyle, alkylsulfanylalkyle, alkylsulfinylalkyle, alkylsulfonylalkyle, hydroxyalkyle ou -alk-NR₂₄R₂₅; soit un hétéroaromatique choisi parmi les cycles benzofuryle, benzothiazolyle, benzothiényle, benzoxazolyle, chromannyle, 2,3-dihydrobenzofuryle, 2,3-dihydrobenzothiényle, furyle, imidazolyle, isochromannyle, isoquinolyle, pyrrolyle, pyridyle, pyrimidinyle, quinolyle, 1,2,3,4-tétrahydroisoquinolyle, thiazolyle, thiényle, ces hétéroaromatiques pouvant être non substitués ou substitués par un ou plusieurs halogène, alkyle, alcoxy, hydroxy, trifluorométhyle, trifluorométhoxy, cyano, -COOH, -COOalk, -CO-NH-NR₂₄R₂₅, -CONR₂₂R₂₃, -alk-NR₂₄R₂₅, alkylsulfanyle, alkylsulfinyle, alkylsulfonyle, alkylsulfanylalkyle, alkylsulfinylalkyle, alkylsulfonylalkyle ou hydroxyalkyle,
R₅ représente un atome d'hydrogène ou un radical alkyle,
R₆ représente un radical Ar ou Het,
R₇ représente un radical cycloalkyle, hétérocycloalkyle ou hétérocyclényle éventuellement substitué par un radical -CSO-phényle,
R₈ représente un atome d'hydrogène ou un radical alkyle,
R₉ représente un radical -CO-NR₂₆R₂₇, -COOH, -COOalk, -CH₂OH, -NH-CO-NH-alk, -CH₂-NHR₂₈ ou -NHCOOalk,
R₁₀ représente un radical Ar ou Het,
R₁₁ représente un radical -SO₂-alk, -SO₂-Ar, -SO₂-Het,
R₁₂ représente un atome d'hydrogène ou un radical Ar ou Het,
R₁₃ représente un atome d'hydrogène ou un radical alkyle,
R₁₄ représente un radical Ar, Het, -alk-Ar ou -alk-Het,
R₁₅ représente un radical alkyle, cycloalkyle ou -alk-NR₂₉R₃₀,
R₁₆ et R₁₇, identiques ou différents, représentent un atome d'hydrogène ou un radical alkyle ou bien R₁₆ et R₁₇ forment ensemble avec l'atome d'azote auquel ils sont rattachés un hétérocycle mono ou bicyclique insaturé ou saturé, ayant 3 à 10 chaînons et contenant éventuellement un ou plusieurs autres hétéroatomes choisis parmi oxygène, soufre et azote et éventuellement substitué par un ou plusieurs radicaux alkyle,
R₁₈ représente un atome d'hydrogène ou un radical alkyle,
R₁₉ représente un atome d'hydrogène ou un radical alkyle, cycloalkyle, cycloalkylalkyle, cycloalkylcarbonyle, -SO₂alk, -CO-NHalk ou -COOalk,
ou bien R₁₈ et R₁₉ forment avec l'atome d'azote auquel ils sont rattachés un hétérocycle mono ou bicyclique insaturé ou saturé, ayant 3 à 10 chaînons et contenant éventuellement un ou plusieurs hétéroatomes choisis parmi oxygène, soufre et azote et éventuellement substitué par un ou plusieurs radicaux alkyle,
-NR₂₀R₂₁ représente un hétérocycle monocyclique saturé ou insaturé ayant 3 à 8 chaînons et contenant éventuellement un autre hétéroatome choisi parmi oxygène, azote et soufre,
R₂₂ et R₂₃, identiques ou différents, représentent un atome d'hydrogène ou un radical alkyle ou bien R₂₂ et R₂₃ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs radicaux alkyle,
R₂₄ et R₂₅, identiques ou différents, représentent un atome d'hydrogène ou un radical alkyle, -COOalk, cycloalkyle, alkylcycloalkyle, -alk-O-alk, hydroxyalkyle ou bien R₂₄ et R₂₅ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs radicaux alkyle, -COalk, -COOalk, -CO-NHalk, -CS-NHalk, oxo, hydroxyalkyle, -alk-O-alk, -CO-NH₂,
R₂₆ et R₂₇, identiques ou différents, représentent un atome d'hydrogène ou un radical alkyle, hydroxyalkyle, cycloalkyle, cycloalkylalkyle, -alk-COOalk, -alk-Ar, -alk-Het, Het, -alk-N(alk)₂, R₂₆ et R₂₇ peuvent également former avec l'atome d'azote auquel ils sont rattachés un hétérocycle mono ou bicyclique insaturé ou saturé, ayant 3 à 10 chaînons et contenant éventuellement un ou plusieurs autres hétéroatomes choisis parmi oxygène, soufre et azote et éventuellement substitué par un ou plusieurs radicaux alkyle, alcoxy, halogène,
R₂₈ représente un radical -CH₂-alk, benzyle, -SO₂alk, -CONHalk, -COalk, cycloalkylalkylcarbonyle, cycloalkylcarbonyle, -CO-(CH₂)ₙOH,
n est égal à 1, 2 ou 3,
R₂₉ et R₃₀, identiques ou différents, représentent un atome d'hydrogène ou un radical alkyle ou bien R₂₉ et R₃₀ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs radicaux alkyle,
R₃₁ et R₃₂, identiques ou différents, représentent un atome d'hydrogène ou un radical alkyle, Ar ou -alk-Ar ou bien R₃₁ et R₃₂ forment ensemble avec l'atome d'azote auquel ils sont rattachés un hétérocycle choisi parmi aziridinyle, azétidinyle, pyrrolidinyle et pipéridinyle,
alk représente un radical alkyle ou alkylène,
Ar représente un radical phényle ou naphtyle éventuellement substitué par un ou plusieurs substituants choisis parmi halogène, alkyle, alcoxy, -CO-alk, cyano, -COOH, -COOalk, -CONR₂₂R₂₃, -CO-NH-NR₂₄R₂₅, alkylsulfanyle, alkylsulfinyle, alkylsulfonyle, alkylsulfanylalkyle, alkylsulfinylalkyle, alkylsulfonylalkyle, hydroxyalkyle, -alk-NR₂₄R₂₅, -NR₂₄R₂₅, alkylthioalkyle, formyle, hydroxy, CF₃, OCF₃, Het, -O-alk-NH-cycloalkyle ou SO₂NH₂,
Het représente un hétérocycle mono ou bicyclique insaturé ou saturé, ayant 3 à 10 chaînons et contenant un ou plusieurs hétéroatomes choisis parmi oxygène, soufre et azote et éventuellement substitué par un ou plusieurs halogène, alkyle, alcoxy, alcoxycarbonyle, -CONR₂₂R₂₃, hydroxy, hydroxyalkyle, oxo ou SO₂NH₂,
les radicaux et portions alkyle et alkylène et les radicaux et portions alcoxy sont en chaîne droite ou ramifiée et contiennent 1 à 6 atomes de carbone, les radicaux cycloalkyle contiennent 3 à 10 atomes de carbone et les radicaux hétérocycloalkyle et hétérocyclényle contiennent 3 à 10 atomes de carbone, leurs isomères optiques et leurs sels avec un acide minéral ou organique.

2. Un composé selon la revendication 1 pour lequel Het est choisi parmi benzofuryle, benzothiazolyle, benzothiényle, benzoxazolyle, chromannyle, 2,3-dihydrobenzofuryle, 2,3-dihydrobenzothiényle, furyle, indolinyle, indolyle, isochromannyle, isoquinolyle, pipéridyle, pyrrolyle, pyridyle, pyrimidinyle, quinolyle, 1,2,3,4-tétrahydroisoquinolyle, 1,2,3,4-tétrahydroquinolyle, thiazolyle, thiényle.

3. Un composé de formule (I) selon la revendication 1 pour lequel lorsque R₁₆ et R₁₇ forment ensemble avec l'atome d'azote auquel ils sont rattachés un hétérocycle mono ou bicyclique, saturé ou insaturé ayant 3 à 10 chaînons celui-ci est un cycle azétidinyle, pyrrolidinyle, pipéridinyle, morpholinyle, thiamorpholinyle ou pipérazinyle.

4. Un composé de formule (I) selon la revendication 1 pour lequel lorsque R₁₈ et R₁₉ forment ensemble avec l'atome d'azote auquel ils sont rattachés un hétérocycle mono ou bicyclique, saturé ou insaturé ayant 3 à 10 chaînons celui-ci est un cycle azétidinyle, pyrrolidinyle, pipéridinyle, morpholinyle, thiamorpholinyle ou pipérazinyle.

5. Un composé de formule (I) selon la revendication 1 pour lequel l'hétérocycle formé par NR₂₀R₂₁ est un cycle azétidinyle, pyrrolidinyle, pipéridinyle, morpholinyle, thiamorpholinyle, pipérazinyle ou imidazolyle.

6. Un composé de formule (I) selon la revendication 1 pour lequel lorsque R₂₂ et R₂₃ forment ensemble avec l'atome d'azote auquel ils sont rattachés un hétérocycle mono ou bicyclique, saturé ou insaturé ayant 3 à 10 chaînons celui-ci est un cycle azétidinyle, pyrrolidinyle, pipéridinyle, morpholinyle, thiamorpholinyle ou pipérazinyle.

7. Un composé de formule (I) selon la revendication 1 pour lequel lorsque R₂₄ et R₂₅ forment ensemble avec l'atome d'azote auquel ils sont rattachés un hétérocycle mono ou bicyclique, saturé ou insaturé ayant 3 à 10 chaînons celui-ci est un cycle azétidinyle, pyrrolidinyle, pipéridinyle, morpholinyle, thiamorpholinyle ou pipérazinyle.

8. Un composé de formule (I) selon la revendication 1 pour lequel lorsque R₂₆ et R₂₇ forment ensemble avec l'atome d'azote auquel ils sont rattachés un hétérocycle mono ou bicyclique, saturé ou insaturé ayant 3 à 10 chaînons celui-ci est un cycle azétidinyle, pyrrolidinyle, pipéridinyle, morpholinyle, thiamorpholinyle ou pipérazinyle.

9. Un composé de formule (I) selon la revendication 1 pour lequel lorsque R₂₉ et R₃₀ forment ensemble avec l'atome d'azote auquel ils sont rattachés un hétérocycle mono ou bicyclique, saturé ou insaturé ayant 3 à 10 chaînons celui-ci est un cycle azétidinyle, pyrrolidinyle, pipéridinyle, morpholinyle, thiamorpholinyle ou pipérazinyle.

10. Un composé de formule (I) selon la revendication 1 pour lequel
R représente un radical CR₁R₂,
soit R₁ représente un atome d'hydrogène et R₂ représente un radical -C(R₈)(R₁₁)(R₁₂) ou C(R₈)(R₉)(R₁₀),
soit R1 représente un radical alkyle et R₂ représente un radical -C(R₈)(R₁₁)(R₁₂)
R₃ et R₄, identiques ou différents, représentent soit un phényle non substitué ou substitué par un ou plusieurs halogène, alkyle, alcoxy, trifluorométhyle, trifluorométhoxy, cyano, -CONR₂₂R₂₃, hydroxyalkyle, ou -alk-NR₂₄R₂₅; soit un hétéroaromatique choisi parmi les cycles pyridyle, pyrimidinyle, thiazolyle, thiényle, ces hétéroaromatiques pouvant être non substitués ou substitués par un ou plusieurs halogène, alkyle, alcoxy, hydroxy, trifluorométhyle, trifluorométhoxy, cyano, -CONR₂₂R₂₃, -alk-NR₂₄R₂₅ ou hydroxyalkyle,
R₈ représente un atome d'hydrogène,
R₉ représente un radical -CO-NR₂₆R₂₇, -COOalk, -CH₂OH, -NH-CO-NH-alk, -CH₂-NHR₂₈ ou -NHCOOalk,
R₁₀ représente un radical Ar ou Het,
R₁₁ représente un radical -SO₂-alk, -SO₂-Ar, -SO₂-Het,
R₁₂ représente représente un atome d'hydrogène ou un radical Ar ou Het,
R₂₂ et R₂₃, identiques ou différents, représentent un atome d'hydrogène ou un radical alkyle ou bien R₂₂ et R₂₃ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs radicaux alkyle,
R₂₄ et R₂₅, identiques ou différents représentent un atome d'hydrogène ou un radical alkyle, cycloalkyle, alkylcycloalkyle, hydroxyalkyle ou bien R₂₄ et R₂₅ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs radicaux alkyle, -COalk, -COOalk, -CO-NHalk, -CS-NHalk, oxo, -CO-NH₂,
Ar représente un radical phényle ou naphtyle éventuellement substitué par 1 ou 2 substituants choisis parmi halogène, alkyle, alcoxy, -CO-alk, cyano, -COOalk, -CONR₂₂R₂₃, alkylsulfonyle, hydroxyalkyle, -alk-NR₂₄R₂₅, -NR₂₄R₂₅, hydroxy, CF₃, OCF₃, -O-alk-NH-cycloalkyle ou SO₂NH₂,
Het représente un cycle benzofuryle, benzothiazolyle, benzothiényle, benzoxazolyle, furyle, isoquinolyle, pyrrolyle, pyridyle, quinolyle, 1,2,3,4-tétrahydroisoquinolyle, 1,2,3,4-tétrahydroquinolyle, thiazolyle ou thiényle
leurs isomères optiques et leurs sels avec un acide minéral ou organique.

11. Un composé choisi parmi les composés suivants :
(RS)-1-[bis(4-chlorophényl)méthyl)]-3-[(3,5-difluorophényl)(méthylsulfonyl) méthyl] -azétidine,
(R)-1-[bis(4-chlorophényl)méthyl)]-3-[(3,5-difluorophényl)(méthylsulfonyl) méthyl]-azétidine,
(S)-1-[bis(4-chlorophényl)méthyl)]-3-[(3,5-difluorophényl)(méthylsulfonyl) méthyl]-azétidine,
(RS)-1-[bis(4-chlorophényl)méthyl)]-3-[(pyrid-3-yl)(méthylsulfonyl)méthyl]-azétidine,
(R)-1-[bis(4-chlorophényl)méthyl)]-3-[(pyrid-3-yl)(méthylsulfonyl)méthyl]-azétidine,
(S)-1-[bis(4-chlorophényl)méthyl)]-3-[(pyrid-3-yl)(méthylsulfonyl)méthyl]-azétidine,
(RS)-1-[bis-(3-fluorophényl)méthyl]-3-[(3,5-difluorophényl)méthylsulfonylméthyl]azétidine,
(R)-1-[bis-(3-fluorophényl)méthyl]-3-[(3,5-difluorophényl)méthylsulfonylméthyl]azétidine,
(S)-1-[bis-(3-fluorophényl)méthyl]-3-[(3,5-difluorophényl)méthylsulfonyl-méthyl]azétidine,
1-[bis-(4-chlorophényl)méthyl]-3-(RS)-{[3-azétidin-1-yl-phényl]méthylsulfonylméthyl}azétidine,
1-[bis-(4-chlorophényl)méthyl]-3-(R)-{[3-azétidin-1-yl-phényl]méthylsulfonylméthyl}azétidine,
1-[bis-(4-chlorophényl)méthyl]-3-(S)-{[3-azétidin-1-yl-phényl]méthylsulfonylméthyl}azétidine,
(RS)-1-[3-({1-[bis-(4-chlorophényl)méthyl]azétidin-3-yl}méthylsulfonylméthyl)phényl]pyrrolidine,
(R)-1-[3-({1-[bis-(4-chlorophényl)méthyl]azétidin-3-yl}méthylsulfonylméthyl)phényl]pyrrolidine,
(S)-1-[3-({1-[bis-(4-chlorophényl)méthyl]azétidin-3-yl}méthylsulfonylméthyl)phényl]pyrrolidine,
(RS)-N-[3-({1-[bis-(4-chlorophényl)méthyl]azétidin-3-yl}méthylsulfonylméthyl)phényl]-N-méthyl-amine,
(R)-N-[3-({1-[bis-(4-chlorophényl)méthyl]azétidin-3-yl}méthylsulfonylméthyl)phényl]-N-méthyl-amine,
(S)-N-[3-({1-[bis-(4-chlorophényl)méthyl]azétidin-3-yl}méthylsulfonylméthyl)phényl]-N-méthyl-amine,
(RS)-1-[bis-(4-chlorophényl)méthyl]-3-[(3,5-bis-trifluorométhylphényl) méthylsulfonyl-méthyl]azétidine,
(R)-1-[bis-(4-chlorophényl)méthyl]-3-[(3,5-bis-trifluorométhylphényl)méthylsulfonyl-méthyl]azétidine,
(S)-1-[bis-(4-chlorophényl)méthyl]-3-[(3,5-bis-trifluorométhylphényl) méthylsulfonyl-méthyl]azétidine
1-[bis-(4-chlorophényl)méthyl]-3-(phénylsulfonylméthyl)-azétidine,
(RS)-1-[bis-(4-chlorophényl)-méthyl]-3-[(3,5-difluorophényl)-méthylsulfonylméthyl]-3-méthyl-azétidine,
(R)-1-[bis-(4-chlorophényl)-méthyl]-3-[(3,5-difluorophényl)-méthylsulfonylméthyl]-3-méthyl-azétidine,
(S)-1-[bis-(4-chlorophényl)-méthyl]-3-[(3,5-difluorophényl)-méthylsulfonylméthyl]-3-méthyl-azétidine,
(RS)-2-{1-[bis-(4-chlorophényl)méthyl]-azétidin-3-yl}-2-(3,5-difluorophényl)-N-cyclohexylacétamide,
(R)-2-{1-[bis-(4-chlorophényl)méthyl]-azétidin-3-yl}-2-(3,5-difluorophényl)-N-cyclohexylacétamide,
(S)-2-{1-[bis-(4-chlorophényl)méthyl]-azétidin-3-yl}-2-(3,5-difluorophényl)-N-cyclohexylacétamide,
(RS)-2-{1-[bis-(4-chlorophényl)méthyl]-azétidin-3-yl}-2-(3,5-difluorophényl)-N-isobutylacétamide,
(R)-2-{1-[bis-(4-chlorophényl)méthyl]-azétidin-3-yl}-2-(3,5-difluorophényl)-N-isobutylacétamide,
(S)-2-{1-[bis-(4-chlorophényl)méthyl]-azétidin-3-yl}-2-(3,5-difluorophényl)-N-isobutylacétamide,
(RS)-2-{1-[bis-(4-chlorophényl)méthyl]-azétidin-3-yl}-2-(3,5-difluorophényl)-N-cyclopropylméthyl acétamide,
(R)-2-{1-[bis-(4-chlorophényl)méthyl]-azétidin-3-yl}-2-(3,5-difluorophényl)-N-cyclopropylméthyl acétamide,
(S)-2-{1-[bis-(4-chlorophényl)méthyl]-azétidin-3-yl}-2-(3,5-difluorophényl)-N-cyclopropylméthyl acétamide,
(RS)-2-{1-[bis-(4-chlorophényl)méthyl]-azétidin-3-yl}-2-(3,5-difluorophényl)-N-isopropylacétamide,
(R)-2-{1-[bis-(4-chlorophényl)méthyl]-azétidin-3-yl}-2-(3,5-difluorophényl)-N-isopropylacétamide,
(S)-2-{1-[bis-(4-chlorophényl)méthyl]-azétidin-3-yl}-2-(3,5-difluorophényl)-N-isopropylacétamide,
(RS)-1-[bis-(4-chlorophényl)-méthyl]-3-[1-(3,5-difluorophényl)-1-méthylsulfonyl-éthyl]-azétidine,
(R)-1-[bis-(4-chlorophényl)-méthyl]-3-[1-(3,5-difluorophényl)-1-méthylsulfonyl-éthyl]-azétidine,
(S)-1-[bis-(4-chlorophényl)-méthyl]-3-[1-(3,5-difluorophényl)-1-méthylsulfonyl-éthyl]-azétidine,
(RS)-1-[bis-(4-fluoro-phényl)-méthyl]-3-[(3,5-difluorophényl)-méthylsulfonylméthyl]-azétidine,
(R)-1-[bis-(4-fluoro-phényl)-méthyl]-3-[(3,5-difluorophényl)-méthylsulfonylméthyl]-azétidine,
(S)-1-[bis-(4-fluoro-phényl)-méthyl]-3-[(3,5-difluorophényl)-méthylsulfonylméthyl]-azétidine,
(RS)-{1-[(3-pyridyl)-(4-chlorophényl)méthyl]-3-[(3,5-difluorophényl)-méthylsulfonyl-méthyl]-azétidine,
(SS)-{1-[(3-pyridyl)-(4-chlorophényl)méthyl]-3-[(3,5-difluorophényl)-méthylsulfonyl-méthyl]-azétidine,
(RR)-{1-[(3-pyridyl)-(4-chlorophényl)méthyl]-3-[(3,5-difluorophényl)-méthylsulfonyl-méthyl]-azétidine,
(SR)-{1-[(3-pyridyl)-(4-chlorophényl)méthyl]-3-[(3,5-difluorophényl)-méthylsulfonyl-méthyl]-azétidine,
(RS)-{1-[(4-pyridyl)-(4-chlorophényl)méthyl]-3-[(3,5-difluorophényl)-méthylsulfonyl-méthyl]-azétidine,
(SS)-{1-[(4-pyridyl)-(4-chlorophényl)méthyl]-3-[(3,5-difluorophényl)-méthylsulfonyl-méthyl]-azétidine,
(RR)-{1-[(4-pyridyl)-(4-chlorophényl)méthyl]-3-[(3,5-difluorophényl)-méthylsulfonyl-méthyl]-azétidine,
(SR)-{1-[(4-pyridyl)-(4-chlorophényl)méthyl]-3-[(3,5-difluorophényl)-méthylsulfonyl-méthyl]-azétidine,
(RS)-5-((4-chlorophényl)-{3-[(3,5-difluorophényl)-méthylsulfonyl-méthyl] azétidin-1-yl}-méthyl)-pyrimidine,
(SR)-5-((4-chlorophényl)-{3-[(3,5-difluorophényl)-méthylsulfonyl-méthyl]-azétidin-1-yl}-méthyl)-pyrimidine,
(RR)-5-((4-chlorophényl)-{3-[(3,5-difluorophényl)-méthylsulfonyl-méthyl]-azétidin-1-yl}-méthyl)-pyrimidine,
(SS)-5-((4-chlorophényl)-{3-[(3,5-difluorophényl)-méthylsulfonyl-méthyl]-azétidin-1-yl}-méthyl)-pyrimidine,
(SS)-{1-[(2-chloro-pyrid-5-yl)-(4-chlorophényl)méthyl]-3-[(3,5-difluorophényl)-méthylsulfonyl-méthyl]-azétidine,
(RR)-{1-[(2-chloro-pyrid-5-yl)-(4-chlorophényl)méthyl]-3-[(3,5-difluorophényl)-méthylsulfonyl-méthyl]-azétidine,
(RS)-{1-[(2-chloro-pyrid-5-yl)-(4-chlorophényl)méthyl]-3-[(3,5-difluorophényl)-méthylsulfonyl-méthyl]-azétidine,
(SR)-{1-[(2-chloro-pyrid-5-yl)-(4-chlorophényl)méthyl]-3-[(3,5-difluorophényl)-méthylsulfonyl-méthyl]-azétidine,
leurs isomères optiques et leurs sels pharmaceutiquement acceptables avec un acide minéral ou organique.

12. Procédé de préparation des composés de formule (I) selon la revendication 1 pour lesquels R représente un radical CR₁R₂ dans lequel R₁ représente un atome d'hydrogène et R₂ représente un radical C(R₈)(R₁₁)(R₁₂) dans lequel R₈ représente un atome d'hydrogène, R₁₁ représente un radical -SO₂-Ar, -SO₂-Het ou -SO₂alk et R₁₂ représente un atome d'hydrogène ou un radical Ar ou Het, **caractérisé en ce que** l'on réduit un dérivé de formule : 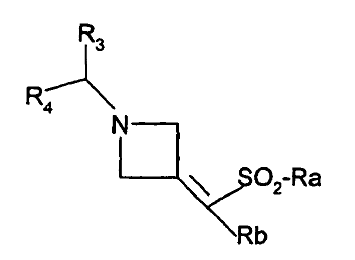 dans laquelle Ra représente un radical alkyle, Het ou Ar et Rb représente un atome d'hydrogène ou un radical Ar ou Het, alkyle, Ar et Het ayant les mêmes significations que dans la revendication 1, isole le produit et le transforme éventuellement en sel avec un acide minéral ou organique.

13. Procédé de préparation des composés de formule (I) selon la revendication 1 pour lesquels R représente un radical CR₁R₂ dans lequel R₁ représente un atome d'hydrogène et R₂ représente un radical C(R₈)(R₁₁)(R₁₂) dans lequel R₈ représente un atome d'hydrogène, R₁₁ représente un radical
- SO₂-Ar, -SO₂-Het ou -SO₂alk et R₁₂ représente un atome d'hydrogène ou un radical Ar ou Het, **caractérisé en ce que** l'on fait réagir un dérivé R₃CH(Br)R₄ avec un dérivé de formule : 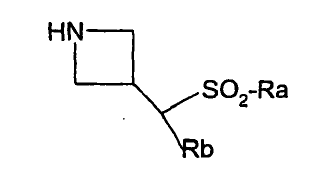
dans laquelle Ra représente un radical alkyle, Het ou Ar et Rb représente un atome d'hydrogène ou un radical Ar ou Het, alkyle, Ar et Het ayant les mêmes significations que dans la revendication 1, isole le produit et le transforme éventuellement en sel avec un acide minéral ou organique.

14. Procédé de préparation des composés de formule (I) selon la revendication 1 pour lesquels R représente un radical C=C(R₅)SO₂R₆ ou C=C(R₇)SO₂alk **caractérisé en ce que** l'on déshydrate un dérivé de formule : 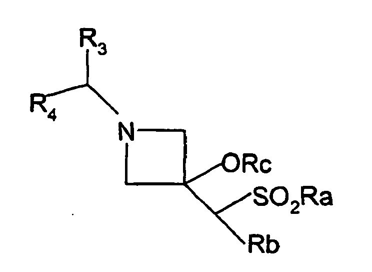 dans laquelle soit Ra représente un radical Ar ou Het et Rb représente un atome d'hydrogène ou un radical alkyle, soit Ra représente un radical alkyle et Rb représente un radical cycloalkyle, hétérocycloalkyle ou hétérocyclényle éventuellement substitué par un radical -CSO-phényle, Rc représente un atome d'hydrogène ou un radical acétyle, R₃, R₄, Ar et Het ayant les mêmes significations que dans la revendication 1, isole le produit et le transforme éventuellement en sel avec un acide minéral ou organique.

15. Procédé de préparation des composés de formule (I) selon la revendication 1 pour lesquels R représente un radical C=C(R₅)SO₂R₆ ou C=C(R₇)SO₂alk **caractérisé en ce que** l'on fait réagir R₃CH(Br)R₄ avec un dérivé de formule : 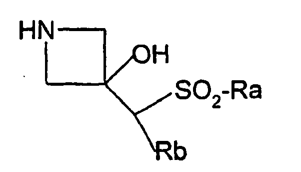 dans laquelle soit Ra représente un radical Ar ou Het et Rb représente un atome d'hydrogène ou un radical alkyle, soit Ra représente un radical alkyle et Rb représente un radical cycloalkyle, hétérocycloalkyle ou hétérocyclényle éventuellement substitué par un radical -CSO-phényle, Rc représente un atome d'hydrogène ou un radical acétyle, R₃, R₄, Ar et Het ayant les mêmes significations que dans la revendication 1, isole le produit et le transforme éventuellement en sel avec un acide minéral ou organique.

16. Procédé de préparation des composés de formule (I) selon la revendication 1 pour lesquels R représente un radical CR₁R₂ dans lequel R₁ représente un atome d'hydrogène et R₂ représente un radical C(R₈)(R₉)(R₁₀) dans lequel R₈ représente un atome d'hydrogène, R₉ représente un radical -CO-NR₂₆R₂₇ et R₁₀ représente un radical Ar ou Het **caractérisé en ce que** l'on fait réagir une amine HNR₂₆R₂₇ dans laquelle R₂₆ et R₂₇ ont les mêmes significations que dans la revendication 1, sur un acide de formule: 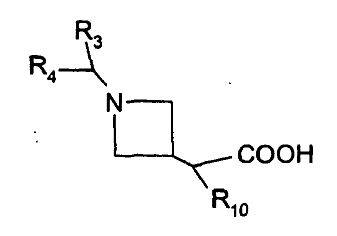 dans laquelle R₃, R₄ et R₁₀ ont les mêmes significations que dans la revendication 1, isole le produit et le transforme éventuellement en sel avec un acide minéral ou organique.

17. Procédé de préparation des composés de formule (I) selon la revendication 1 pour lesquels R représente un radical CR₁R₂ dans lequel R₁ représente un atome d'hydrogène et R₂ représente un radical C(R₈)(R₉)(R₁₀) dans lequel R₈ représente un atome d'hydrogène, R₉ représente un radical -COOH et R₁₀ représente un radical Ar ou Het **caractérisé en ce que** l'on hydrolyse l'ester correspondant de formule : 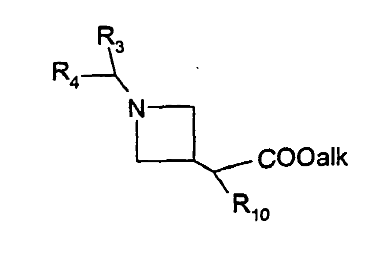 dans laquelle R₃, R₄ et R₁₀ ont les mêmes significations que dans la revendication 1 et alk représente un radical alkyle (1-6C en chaîne droite ou ramifiée, isole le produit et le transforme éventuellement en sel avec un acide minéral ou organique.

18. Procédé de préparation des composés de formule (I) selon la revendication 1 pour lesquels R représente un radical CR₁R₂ dans lequel R₁ représente un atome d'hydrogène et R₂ représente un radical C(R₈)(R₉)(R₁₀) dans lequel R₈ représente un atome d'hydrogène, R₉ représente un radical -COOalk ou -CH₂OH et R₁₀ représente un radical Ar ou Het **caractérisé en ce que** l'on réduit un composé de formule : 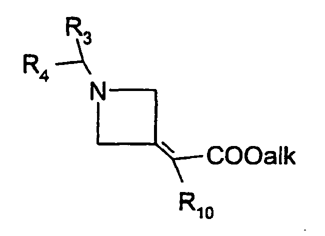 dans laquelle R₃, R₄ et R₁₀ ont les mêmes significations que dans la revendication 1 et alk représente un radical alkyle (1-6C en chaîne droite ou ramifiée), isole le produit et le transforme éventuellement en sel avec un acide minéral ou organique.

19. Procédé de préparation des composés de formule (I) selon la revendication 1 pour lesquels R représente un radical CR₁R₂ dans lequel R₁ représente un atome d'hydrogène et R₂ représente un radical C(R₈)(R₉)(R₁₀) dans lequel R₈ représente un atome d'hydrogène, R₉ représente un radical -NHCOOalk et R₁₀ représente un radical Ar ou Het **caractérisé en ce que** l'on fait réagir un alcool alkOH pour lequel alk représente un radical alkyle (1-6C en chaîne droite ou ramifiée) sur un dérivé de formule : 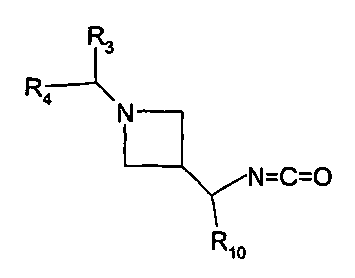 dans laquelle R₃, R₄ et R₁₀ ont les mêmes significations que dans la revendication 1, isole le produit et le transforme éventuellement en sel avec un acide minéral ou organique.

20. Procédé de préparation des composés de formule (I) selon la revendication 1 pour lesquels R représente un radical CR₁R₂ dans lequel R₁ représente un atome d'hydrogène et R₂ représente un radical C(R₈)(R₉)(R₁₀) dans lequel R₈ représente un atome d'hydrogène, R₉ représente un radical -NH-CO-NH-alk et R₁₀ représente un radical Ar ou Het **caractérisé en ce que** l'on fait réagir une amine alkNH₂ pour laquelle alk représente un radical alkyle (1-6C en chaîne droite ou ramifiée) avec un dérivé de formule : 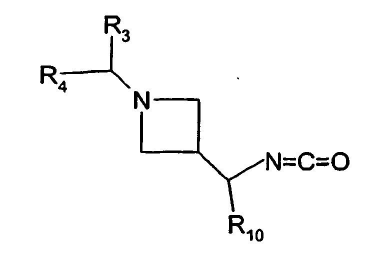 dans laquelle R₃, R₄ et R₁₀ ont les mêmes significations que dans la formule (I), isole le produit et le transforme éventuellement en sel avec un acide minéral ou organique.

21. Procédé de préparation des composés de formule (I) selon la revendication 1 pour lesquels R représente un radical CR₁R₂ dans lequel R₁ est un atome d'hydrogène et R₂ représente un radical -C(R₈)(R₉)(R₁₀), R₈ est un atome d'hydrogène, R₉ est un radical -CH₂-NHR₂₈, R₂₈ représente un radical -CH₂-alk ou benzyle et R₁₀ représente un radical Ar ou Het **caractérisé en ce que** l'on fait réagir un dérivé de formule : 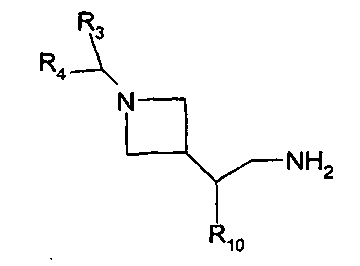 dans laquelle R₃, R₄ et R₁₀ ont les mêmes significations que dans la revendication 1, avec un aldéhyde RdCHO pour lequel Rd représente un radical -CH₂-alk ou benzyle et alk représente un radical alkyle (1-6C en chaîne droite ou ramifiée), isole le produit et le transforme éventuellement en sel avec un acide minéral ou organique.

22. Procédé de préparation des composés de formule (I) selon la revendication 1 pour lesquels R représente un radical CR₁R₂ dans lequel R₁ est un atome d'hydrogène et R₂ représente un radical -C(R₈)(R₉)(R₁₀), R₈ est un atome d'hydrogène, R₉ est un radical -CH₂-NHR₂₈, R₂₈ représente un radical -SO₂alk et R₁₀ représente un radical Ar ou Het **caractérisé en ce que** l'on fait réagir une amine de formule : 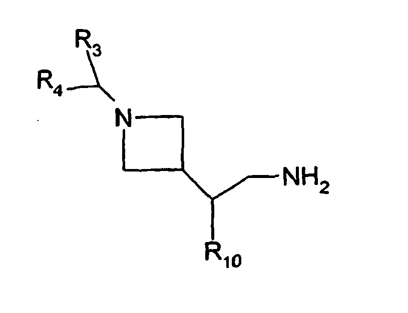 dans laquelle R₃, R₄ et R₁₀ ont les mêmes significations que dans la revendication 1 avec un dérivé CISO₂Re pour lequel Re représente un radical alkyle (1-6C en chaîne droite ou ramifiée), isole le produit et le transforme éventuellement en sel avec un acide minéral ou organique.

23. Procédé de préparation des composés de formule (I) selon la revendication 1 pour lesquels R représente un radical CR₁R₂ dans lequel R₁ est un atome d'hydrogène et R₂ représente un radical -C(R₈)(R₉)(R₁₀), R₈ est un atome d'hydrogène, R₉ est un radical -CH₂-NHR₂₈, R₂₈ représente un radical -CO-NHalk et R₁₀ représente un radical Ar ou Het **caractérisé en ce que** l'on fait réagir un dérivé de formule : 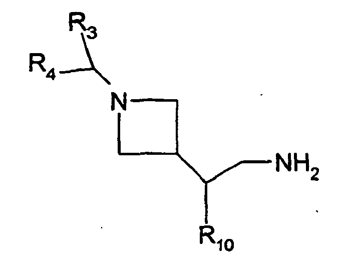 dans laquelle R₃, R₄ et R₁₀ ont les mêmes significations que dans la revendication 1 avec un dérivé RfNCO pour lequel Rf représente un radical alkyle (1-6C en chaîne droite ou ramifiée), isole le produit et le transforme éventuellement en sel avec un acide minéral ou organique.

24. Procédé de préparation des composés de formule (I) selon la revendication 1 pour lesquels R représente un radical CR₁R₂ dans lequel R₁ est un atome d'hydrogène et R₂ représente un radical -C(R₈)(R₉)(R₁₀), R₈ est un atome d'hydrogène, R₉ est un radical -CH₂-NHR₂₈, R₂₈ représente un radical -COalk, cycloalkylalkylcarbonyle, cycloalkylcarbonyle, -CO-(CH₂)ₙOH et R₁₀ représente un radical Ar ou Het **caractérisé en ce que** l'on fait réagir un dérivé de formule : 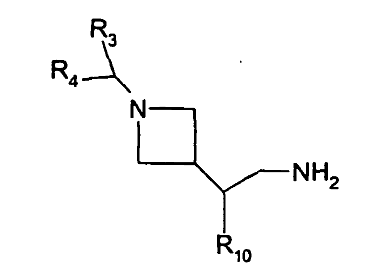 dans laquelle R₃, R₄ et R₁₀ ont les mêmes significations que dans la revendication 1 avec un acide HOOCRg pour lequel Rg représente un radical alkyle (1-6C en chaîne droite ou ramifiée), cycloalkyl(3-10C)alkyle(1-6C en chaîne droite ou ramifiée), cycloalkyle(3-10C), -(CH₂)ₙOH et n est égal à 1, 2, 3, isole le produit et le transforme éventuellement en sel avec un acide minéral ou organique.

25. Procédé de préparation des composés de formule (I) selon la revendication 1 pour lesquels R représente un radical CR₁R₂ dans lequel R₁ est un atome d'hydrogène et R₂ est un radical -CONR₁₃R₁₄ **caractérisé en ce que** l'on fait réagir une amine HNR₁₃R₁₄ dans laquelle R₁₃ et R₁₄ ont les mêmes significations que dans la revendication 1 avec un dérivé de formule : 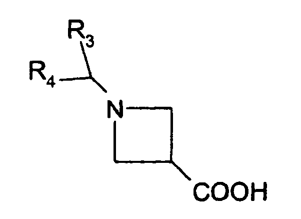 dans laquelle R₃ et R₄ ont les mêmes significations que dans la formule (I), isole le produit et le transforme éventuellement en sel avec un acide minéral ou organique.

26. Procédé de préparation des composés de formule (I) selon la revendication 1 pour lesquels R représente un radical CR₁R₂ dans lequel R₁ est un atome d'hydrogène et R₂ est un radical -CH₂-CONR₁₃R₁₄ **caractérisé en ce que** l'on fait réagir une amine HNR₁₃R₁₄ dans laquelle R₁₃ et R₁₄ ont les mêmes significations que dans la revendication 1 avec un dérivé de formule : 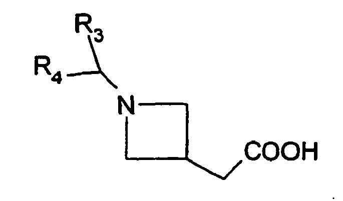 dans laquelle R₃ et R₄ ont les mêmes significations que dans la revendication 1, isole le produit et le transforme éventuellement en sel avec un acide minéral ou organique.

27. Procédé de préparation des composés de formule (I) selon la revendication 1 pour lesquels R représente un radical CR₁R₂ dans lequel R₁ est un atome d'hydrogène et R₂ est un radical -CH₂-CONR₁₃R₁₄ **caractérisé en ce que** l'on réduit un dérivé de formule : 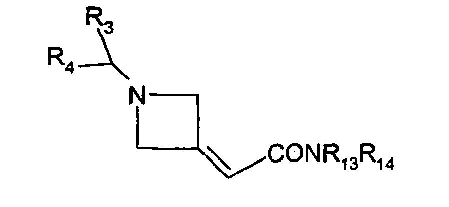 dans laquelle R₃, R₄, R₁₃ et R₁₄ ont les mêmes significations que dans la revendication 1, isole le produit et le transforme éventuellement en sel avec un acide minéral ou organique.

28. Procédé de préparation des composés de formule (I) selon la revendication 1 pour lesquels R représente un radical CR₁R₂ dans lequel R, est un atome d'hydrogène et R₂ représente un radical -SOR₆ **caractérisé en ce que** l'on oxyde un dérivé de formule : 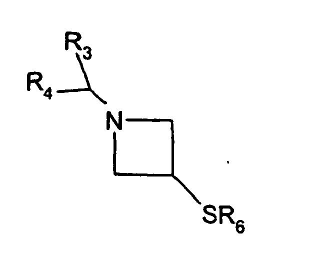 dans laquelle R₃, R₄ et R₆ ont les mêmes significations que dans la revendication 1, isole le produit et le transforme éventuellement en sel avec un acide minéral ou organique.

29. Procédé de préparation des composés de formule (I) selon la revendication 1 pour lesquels R représente un radical CR₁R₂ dans lequel R₁ est un atome d'hydrogène et R₂ représente un radical -SO₂R₆ **caractérisé en ce que** l'on oxyde un dérivé de formule : 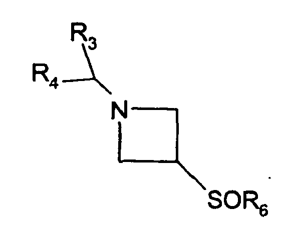 dans laquelle R₃, R₄ et R₆ ont les mêmes significations que dans la revendication 1, isole le produit et le transforme éventuellement en sel avec un acide minéral ou organique.

30. Procédé de préparation des composés de formule (I) selon la revendication 1 pour lesquels R représente un radical CR₁R₂ dans lequel R₁ est un atome d'hydrogène et R₂ représente un radical -COR₆ ou -CO-cycloalkyle, **caractérisé en ce que** l'on fait réagir un dérivé de formule : 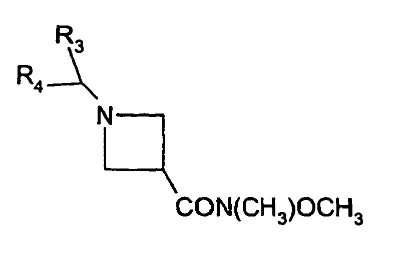 dans laquelle R₃, R₄ ont les mêmes significations que dans la revendication 1 avec un dérivé RhMgBr pour lequel Rh a les mêmes significations que R₆ dans la revendication 1 ou bien représente un radical cycloalkyle(3-10C), isole le produit et le transforme éventuellement en sel avec un acide minéral ou organique.

31. Procédé de préparation des composés de formule (I) selon la revendication 1 pour lesquels R représente un radical CR₁R₂ dans lequel R₁ est un atome d'hydrogène et R₂ représente un radical -C(OH)(R₆)(R₁₂) ou -C(OH)(R₆)(alkyle) **caractérisé en ce que** l'on fait réagir un dérivé de formule : 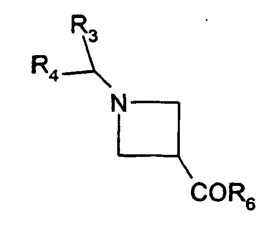 dans laquelle R₃, R₄ et R₆ ont les mêmes significations que dans la revendication 1 avec un dérivé RiMgBr pour lequel Ri a les mêmes significations que R₁₂ dans la revendication 1 ou bien représente un radical alkyle (1-6C en chaîne droite ou ramifiée), isole le produit et le transforme éventuellement en sel avec un acide minéral ou organique.

32. Procédé de préparation des composés de formule (I) selon la revendication 1 pour lesquels R représente un radical CR₁R₂ dans lequel R₁ est un atome d'hydrogène et R₂ représente un radical -C(=NOalk)R₆ ou -C(=NO-CH₂-CH=CH₂)R₆ **caractérisé en ce que** l'on fait réagir un dérivé de formule : 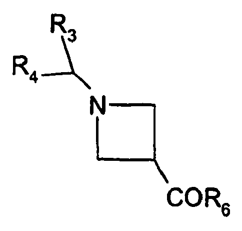 dans laquelle R₃, R₄ et R₆ ont les mêmes significations que dans la revendication 1 avec un dérivé RjONH₂ pour lequel Rj représente un radical alkyle (1-6C en chaîne droite ou ramifiée) ou -CH₂-CH=CH₂, , isole le produit et le transforme éventuellement en sel avec un acide minéral ou organique.

33. Procédé de préparation des composés de formule (I) selon la revendication 1 pour lesquels R représente un radical CR₁R₂ dans lequel R₁ est un atome d'hydrogène et R₂ représente un radical -CH(R₆)NR₃₁R₃₂, R₃₁ et R₃₂ représentent des atomes d'hydrogène **caractérisé en ce que** l'on fait réagir de l'ammoniaque sur un dérivé de formule : 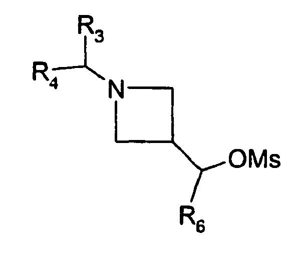 dans laquelle R₃, R₄, R₆ ont les mêmes significations que dans la revendication 1 et Ms représente un radical méthylsulfonyloxy, isole le produit et le transforme éventuellement en sel avec un acide minéral ou organique.

34. Procédé de préparation des composés de formule (I) selon la revendication 1 pour lesquels R représente un radical CR₁R₂ dans lequel R₁ est un atome d'hydrogène et R₂ représente un radical -CH(R₆)NR₃₁R₃₂ , R₃₁ est un atome d'hydrogène et R₃₂ est un radical alkyle, Ar ou -alk-Ar **caractérisé en ce que** l'on fait réagir un halogénure HalR₃₁ sur un composé de formule (I) correspondant pour lequel R représente un radical CR₁R₂ dans lequel R₁ est un atome d'hydrogène et R₂ représente un radical -CH(R₆)NR₃₁R₃₂, R₃₁ et R₃₂ sont des atomes d'hydrogène, isole le produit et le transforme éventuellement en sel avec un acide minéral ou organique.

35. Procédé de préparation des composés de formule (I) selon la revendication 1 pour lesquels R représente un radical CR₁R₂ dans lequel R₁ est un atome d'hydrogène et R₂ représente un radical -CH(R₆)NR₃₁R₃₂ , R₃₁ et R₃₂ sont des radicaux alkyle, Ar ou -alk-Ar **caractérisé en ce que** l'on fait réagir un halogénure HalR₃₂ sur un composé de formule (I) correspondant pour lequel R représente un radical CR₁R₂ dans lequel R₁ est un atome d'hydrogène et R₂ représente un radical -CH(R₆)NR₃₁R_{32 .} R₃₁ est un atome d'hydrogène et R₃₂ est un radical alkyle, Ar ou -alk-Ar, isole le produit et le transforme éventuellement en sel avec un acide minéral ou organique.

36. Procédé de préparation des composés de formule (I) selon la revendication 1 pour lesquels R représente un radical CR₁R₂ dans lequel R₁ est un atome d'hydrogène et R₂ représente un radical -CH(R₆)NR₃₁R₃₂, R₃₁ est un atome d'hydrogène et R₃₂ est un radical alkyle (2-6C) ou -alk(2-6C)-Ar **caractérisé en ce que** l'on fait réagir un aldéhyde RaCHO pour lequel Ra est un radical alkyle ou -alk-Ar sur un composé de formule (I) correspondant pour lequel R représente un radical CR₁R₂ dans lequel R₁ est un atome d'hydrogène et R₂ représente un radical -CH(R₆)NR₃₁R_{32,} R₃₁ et R₃₂ sont des atomes d'hydrogène, isole le produit et le transforme éventuellement en sel avec un acide minéral ou organique.

37. Procédé de préparation des composés de formule (I) selon la revendication 1 pour lesquels R représente un radical CR₁R₂ dans lequel R₁ est un atome d'hydrogène et R₂ représente un radical -CH(R₆)NR₃₁R₃₂ , R₃₁ est un radical alkyle, Ar ou -alk-Ar et R₃₂ est un radical alkyle (2-6C) ou -alk(2-6C)-Ar **caractérisé en ce que** l'on fait réagir un aldéhyde RaCHO pour lequel Ra est un radical alkyle ou -alk-Ar sur un composé de formule (I) correspondant pour lequel R représente un radical CR₁R₂ dans lequel R₁ est un atome d'hydrogène et R₂ représente un radical -CH(R₆)NR₃₁R₃₂, R₃₁ est un atome d'hydrogène et R₃₂ est un radical alkyle, Ar ou -alk-Ar, isole le produit et le transforme éventuellement en sel avec un acide minéral ou organique.

38. Procédé de préparation des composés de formule (I) selon la revendication 1 pour lesquels R représente un radical CR₁R₂ dans lequel R₁ est un atome d'hydrogène et R₂ représente un radical -CH(R₆)NR₃₁R₃₂, R₃₁ et R₃₂ forment avec l'atome d'azote auquel ils sont rattachés un hétérocycle choisi parmi aziridinyle, azétidinyle, pyrrolidinyle, pipéridinyle **caractérisé en ce que** l'on fait réagir un dihalogénure Hal-alk(2-5C)-Hal sur un composé de formule (I) correspondant pour lequel R représente un radical CR₁R₂ dans lequel R₁ est un atome d'hydrogène et R₂ représente un radical -CH(R₆)NR₃₁R_{32,} R₃₁ et R₃₂ sont des atomes d'hydrogène, isole le produit et le transforme éventuellement en sel avec un acide minéral ou organique.

39. Procédé de préparation des composés de formule (I) selon la revendication 1 pour lesquels R représente un radical CR₁R₂ dans lequel R₁ est un atome d'hydrogène et R₂ représente un radical -CH(R₆)NHSO₂alk **caractérisé en ce que** l'on fait réagir un dérivé de formule : 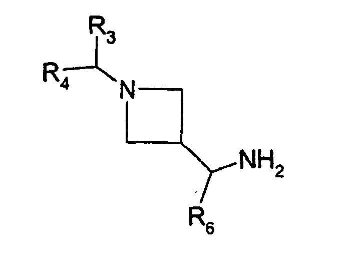 dans laquelle R₃, R₄ et R₆ ont les mêmes significations que dans la revendication 1 avec un dérivé ClSO₂alk pour lequel alk représente un radical alkyle (1-6C en chaîne droite ou ramifiée), isole le produit et le transforme éventuellement en sel avec un acide minéral ou organique.

40. Procédé de préparation des composés de formule (I) selon la revendication 1 pour lesquels R représente un radical CR₁R₂ dans lequel R₁ est un atome d'hydrogène et R₂ représente un radical -CH(R₆)NHCONHalk **caractérisé en ce que** l'on fait réagir un dérivé de formule : 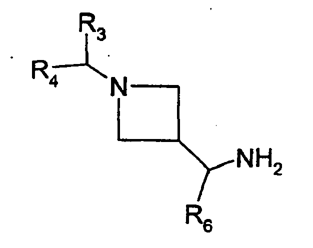 dans laquelle R₃, R₄ et R₆ ont les mêmes significations que dans la revendication 1 avec un dérivé alkNCO pour lequel alk représente un radical alkyle (1-6C en chaîne droite ou ramifiée), isole le produit et le transforme éventuellement en sel avec un acide minéral ou organique.

41. Procédé de préparation des composés de formule (I) selon la revendication 1 pour lesquels R représente un radical CR₁R₂ dans lequel R₁ est un atome d'hydrogène et R₂ représente un radical ou -CH(R₆)NHCOR₃₁ **caractérisé en ce que** l'on fait réagir un dérivé de formule : 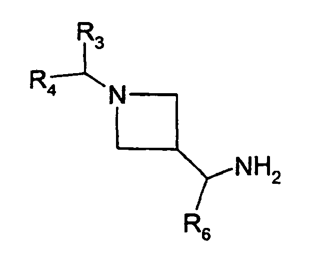 dans laquelle R₃, R₄ et R₆ ont les mêmes significations que dans la revendication 1 avec un dérivé R₃₁COOH pour lequel R₃₁ a les mêmes significations que dans la revendication 1, isole le produit et le transforme éventuellement en sel avec un acide minéral ou organique.

42. Procédé de préparation des composés de formule (I) selon la revendication 1 pour lesquels R représente un radical CR₁R₂ dans lequel R₁ est un atome d'hydrogène et R₂ représente un radical -CH₂-COR₆ **caractérisé en ce que** l'on fait réagir un dérivé de formule : 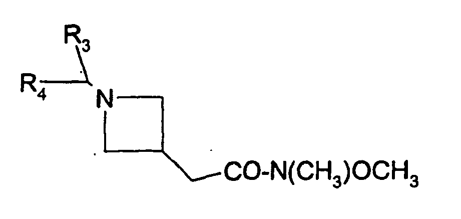 dans laquelle R₃ et R₄ ont les mêmes significations que dans la revendication 1 avec un dérivé de formule R₆MgBr pour lequel R₆ a les mêmes significations que dans la revendication 1, isole le produit et le transforme éventuellement en sel avec un acide minéral ou organique.

43. Procédé de préparation des composés de formule (I) selon la revendication 1 pour lesquels R représente un radical CR₁R₂ dans lequel R₁ est un atome d'hydrogène et R₂ représente un radical -CH₂-CH(R₆)-NR₃₁R₃₂ **caractérisé en ce que** l'on fait réagir une dérivé de formule : 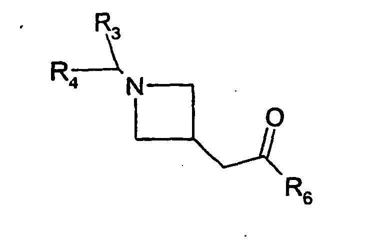 dans laquelle R₃ et R₄ ont les mêmes significations que dans la revendication 1 avec un dérivé de formule HNR₃₁R₃₂ pour lequel R₃₁ et R₃₂ ont les mêmes significations que dans la revendication 1, isole le produit et le transforme éventuellement en sel avec un acide minéral ou organique.

44. Procédé de préparation des composés de formule (I) selon la revendication 1 pour lesquels R représente un radical CR₁R₂ dans lequel R₁ est un atome d'hydrogène et R₂ représente un radical -CH₂-C(=NOalk)R₆ **caractérisé en ce que** l'on fait réagir un dérivé de formule : 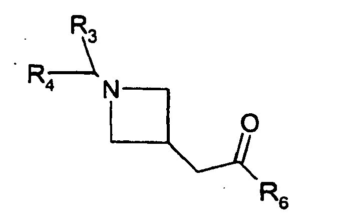 dans laquelle R₃ et R₄ ont les mêmes significations que dans la revendication 1 avec un dérivé de formule alkONH₂ pour lequel alk représente un radical alkyle (1-6C en chaîne droite ou ramifiée), isole le produit et le transforme éventuellement en sel avec un acide minéral ou organique.

45. Procédé de préparation des composés de formule (I) selon la revendication 1 pour lesquels R représente un radical CR₁R₂ dans lequel R₁ représente un radical cyano et R₂ représente un radical -C(R₈)(R₁₁)(R₁₂) dans lequel R₈ est un atome d'hydrogène **caractérisé en ce que** l'on fait réagir un dérivé de formule : 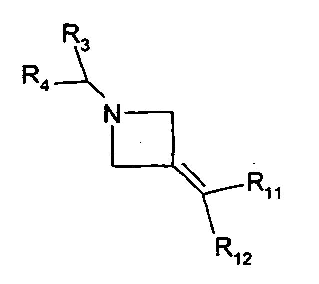 dans laquelle R₃, R₄, R₁₁ et R₁₂ ont les mêmes significations que dans la revendication 1 avec du cyanure de sodium, isole le produit et le transforme éventuellement en sel avec un acide minéral ou organique.

46. Procédé de préparation des composés de formule (I) selon la revendication 1 pour lesquels R représente un radical CR₁R₂ dans lequel R₁ représente un radical -S-alk-NR₁₆R₁₇ et R₂ représente un radical -C(R₈)(R₁₁)(R₁₂) dans lequel R₈ est un atome d'hydrogène **caractérisé en ce que** l'on fait réagir un dérivé de formule : 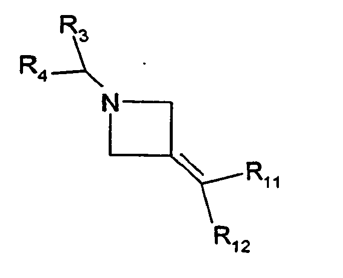 dans laquelle R₃, R₄, R₁₁ et R₁₂ ont les mêmes significations que dans la revendication 1 avec un dérivé HS-alk-NR₁₆R₁₇ pour lequel alk représente un radical alk représente un radical alkyle (1-6C en chaîne droite ou ramifiée) et R₁₆ et R₁₇ ont les mêmes significations que dans la revendication 1, isole le produit et le transforme éventuellement en sel avec un acide minéral ou organique.

47. Procédé de préparation des composés de formule (I) selon la revendication 1 pour lesquels R représente un radical CR₁R₂ dans lequel R₁ représente un radical -NHR₁₅ et R₂ représente un radical -C(R₈)(R₁₁)(R₁₂) dans lequel R₈ est un atome d'hydrogène **caractérisé en ce que** l'on fait réagir un dérivé de formule : 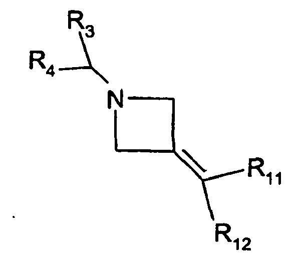 dans laquelle R₃, R₄, R₁₁ et R₁₂ ont les mêmes significations que dans la revendication 1 avec un dérivé H₂NR₁₅ pour lequel R₁₅ a les mêmes significations que dans la revendication 1, isole le produit et le transforme éventuellement en sel avec un acide minéral ou organique.

48. Procédé de préparation des composés de formule (I) selon la revendication 1 pour lesquels R représente un radical CR₁R₂ dans lequel R₁ représente un radical alkyle et R₂ représente un radical -C(R₈)(R₁₁)(R₁₂) dans lequel R₈ est un atome d'hydrogène **caractérisé en ce que** l'on fait réagir un dérivé de formule : 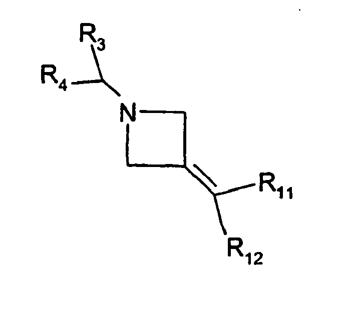 dans laquelle R₃, R₄, R₁₁ et R₁₂ ont les mêmes significations que dans la revendication 1 avec un dérivé alkMHal pour lequel alk représente un radical alkyle (1-6C en chaîne droite ou ramifiée) et M représente un métal, isole le produit et le transforme éventuellement en sel avec un acide minéral ou organique.

49. Procédé de préparation des composés de formule (I) selon la revendication 1 pour lesquels R représente un radical CR₁R₂ dans lequel R₁ représente un radical -NR₂₀R₂₁ et R₂ représente un radical -C(R₈)(R₁₁)(R₁₂) dans lequel R₈ est un atome d'hydrogène **caractérisé en ce que** l'on fait réagir un dérivé de formule : 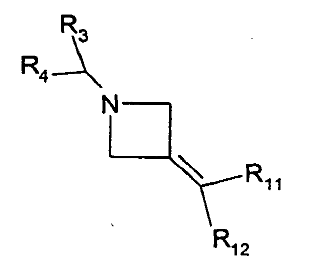 dans laquelle R₃, R₄, R₁₁ et R₁₂ ont les mêmes significations que dans la revendication 1 avec un dérivé HNR₂₀R₂₁ pour lequel NR₂₀R₂₁ les mêmes significations que dans la revendication 1, isole le produit et le transforme éventuellement en sel avec un acide minéral ou organique.

50. Procédé de préparation des composés de formule (I) selon la revendication 1 pour lesquels R représente un radical CR₁R₂ dans lequel R₁ représente un radical -alk-NR₁₈R₁₉, R₁₈ et R₁₉ représentent des atomes d'hydrogène **caractérisé en ce que** l'on réduit un composé de formule (I) correspondant pour lequel R₁ représente un radical cyano, isole le produit et le transforme éventuellement en sel avec un acide minéral ou organique.

51. Procédé de préparation des composés de formule (I) selon la revendication 1 pour lesquels R représente un radical CR₁R₂ dans lequel R₁ représente un radical -alk-NR₁₈R₁₉, R₁₈ représente un atome d'hydrogène et R₁₉ représente un radical alkyle, cycloalkyle, cycloalkylalkyle, cycloalkylcarbonyle, -SO₂alk, -CO-NHalk ou -COOalk **caractérisé en ce que** l'on fait réagir un halogénure HalR₁₉, Hal représente un halogène avec un composé de formule (I) correspondant pour lequel R représente un radical CR₁R₂ dans lequel R₁ représente un radical -alk-NR₁₈R₁₉, R₁₈ et R₁₉ représentent un atome d'hydrogène, isole le produit et le transforme éventuellement en sel avec un acide minéral ou organique.

52. Procédé de préparation des composés de formule (I) selon la revendication 1 pour lesquels R représente un radical CR₁R₂ dans lequel R₁ représente un radical -alk-NR₁₈R₁₉, R₁₈ représente un radical alkyle et R₁₉ représente un radical alkyle, cycloalkyle, cycloalkylalkyle, cycloalkylcarbonyle, -SO₂alk, -CO-NHalk ou -COOalk **caractérisé en ce que** l'on fait réagir un halogénure d'alkyle avec un composé de formule (I) correspondant pour lequel R représente un radical CR₁R₂ dans lequel R₁ représente un radical -alk-NR₁₈R₁₉, R₁₈ représente un atome d'hydrogène et R₁₉ représente un radical alkyle, cycloalkyle, cycloalkylalkyle, cycloalkylcarbonyle, -SO₂alk, -CO-NHalk ou -COOalk, isole le produit et le transforme éventuellement en sel avec un acide minéral ou organique.

53. Procédé de préparation des composés de formule (I) selon la revendication 1 pour lesquels R représente un radical CR₁R₂ dans lequel soit R₁ représente un atome d'hydrogène et R₂ représente un radical -C(R₈)(R₉)(R₁₀) ou -C(R₈)(R₁₁)(R₁₂), soit R₁ représente un radical alkyle, NH-R₁₅, cyano, -S-alk-NR₁₆R₁₇, -alk-NR₁₈R₁₉, ou -NR₂₀R₂₁ et R₂ représente un radical -C(R₈)(R₁₁)(R₁₂) et R₈ représente un radical alkyle **caractérisé en ce que** l'on alkyle un composé de formule (I) correspondant pour lequel R₈ est un atome d'hydrogène, isole le produit et le transforme éventuellement en sel avec un acide minéral ou organique.

54. Procédé de préparation des composés de formule (I) selon la revendication 1 pour lesquels R représente un radical C=C(R₇)SO₂alk **caractérisé en ce que** l'on oxyde un dérivé de formule: 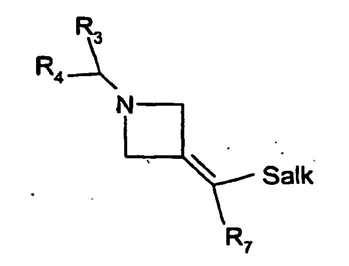 dans laquelle R₃, R₄ et R₇ ont les mêmes significations que dans la revendication 1 et alk représente un radical alkyle (1-6C en chaîne droite ou ramifiée), isole le produit et le transforme éventuellement en sel avec un acide minéral ou organique.

55. Procédé de préparation des composés de formule (I) selon la revendication 1 pour lesquels R représente un radical CR₁R₂ dans lequel R₁ est un atome d'hydrogène et R₂ représente un radical -CH(R₆)NR₃₁R₃₂, R₃₁ est un atome d'hydrogène et R₃₂ est un radical alkyle **caractérisé en ce que** l'on fait réagir un composé de formule (I) correspondant pour lequel R représente un radical CR₁R₂ dans lequel R₁ est un atome d'hydrogène et R₂ représente un radical -CO-R₆ avec une amine HNR₃₁R₃₂ pour laquelle R₃₁ est un atome d'hydrogène et R₃₂ est un radical alkyle, isole le produit et le transforme éventuellement en sel avec un acide minéral ou organique.

56. Composition pharmaceutique contenant en tant que principe actif au moins un composé de formule (I) selon l'une des revendications 1 à 11.

57. Médicament contenant tand que principe actif au moins un composé de formule (I) selon l'une des revendications 1 à 11

## Claims

1. Compound of formula 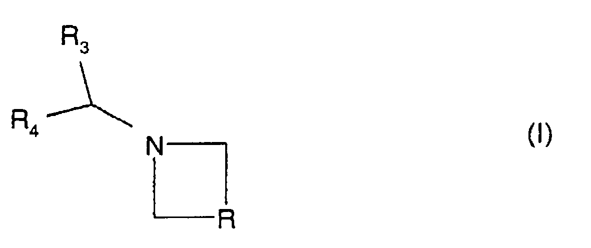 in which
R represents a radical CR₁R₂, C=C (R₅)SO₂R₆ or C=C(R₇)SO₂alk,
either R₁ represents a hydrogen atom and R₂ represents a radical -C(R₈)(R₉)(R₁₀), -C(R₈)(R₁₁)(R₁₂), -CO-NR₁₃R₁₄, -CH₂-CO-NR₁₃R₁₄, -CH₂-CO-R₆, -CO-R₆, -CO-cyclo-alkyl, -SO-R₆, -SO₂-R₆, -C(OH)(R₁₂)(R₆), -C(OH)(R₆)(alkyl), -C(=NOalk)R₆, -C(=NO-CH₂-CH=CH₂)R₆, -CH₂-CH(R₆)NR₃₁R₃₂, -CH₂-C(=NOalk)R₆, -CH(R₆)NR₃₁R₃₂, -CH(R₆)NHSO₂alk, -CH(R₆)NHCONHalk or -CH(R₆)NHCOalk,
or R₁ represents an alkyl, NH-R₁₅, cyano, -S-alk-NR₁₆R₁₇, -CH₂-NR₁₈R₁₉, or -NR₂₀R₂₁ radical and R₂ represents a radical -C(R₈)(R₁₁)(R₁₂),
R₃ and R₄, which are identical or different, represent either an alkyl or cycloalkyl radical, or an aromatic chosen from phenyl, naphthyl or indenyl, these aromatics being unsubstituted or substituted with one or more halogen atoms or alkyl, alkoxy, formyl, hydroxyl, trifluoromethyl, trifluoromethoxy, -CO-alk, cyano, -COOH, -COOalk, -CONR₂₂R₂₃, -CO-NH-NR₂₄R₂₅, alkylsulfanyl, alkylsulfinyl, alkylsulfonyl, alkylsulfanylalkyl, alkylsulfinylalkyl, alkylsulfonylalkyl, hydroxyalkyl or -alk-NR₂₄R₂₅ radicals; or a heteroaromatic chosen from the benzofuryl, benzothiazolyl, benzothienyl, benzoxazolyl, chromanyl, 2,3-dihydrobenzofuryl, 2,3₋dihydrobenzothienyl, furyl, imidazolyl, isochromanyl, isoquinolyl, pyrrolyl, pyridyl, pyrimidinyl, quinolyl, 1,2,3,4-tetrahydroisoquinolyl, thiazolyl and thienyl rings, it being possible for these heteroaromatics to be unsubstituted or substituted with one or more halogen atoms or alkyl, alkoxy, hydroxyl, trifluoromethyl, trifluoromethoxy, cyano, -COOH, -COOalk, -CO-NH-NR₂₄R₂₅, -CONR₂₂R₂₃, -alk-NR₂₄R₂₅, alkylsulfanyl, alkylsulfinyl, alkylsulfonyl, alkylsulfanylalkyl, alkylsulfinylalkyl, alkylsulfonylalkyl or hydroxyalkyl radicals,
R₅ represents a hydrogen atom or an alkyl radical,
R₆ represents a radical Ar or Het,
R₇ represents a cycloalkyl, heterocycloalkyl or heterocyclenyl radical optionally substituted by a -CSO-phenyl radical,
R₈ represents a hydrogen atom or an alkyl radical,
R₉ represents a radical -CO-NR₂₆R₂₇, -COOH, -COOalk, -CH₂OH, -NH-CO-NH-alk, -CH₂-NHR₂₈ or -NHCOOalk,
R₁₀ represents a radical Ar or Het,
R₁₁ represents a radical -SO₂-alk, -SO₂-Ar or -SO₂-Het,
R₁₂ represents a hydrogen atom or a radical Ar or Het,
R₁₃ represents a hydrogen atom or an alkyl radical,
R₁₄ represents a radical Ar, Het, -alk-Ar or -alk-Het,
R₁₅ represents an alkyl, cycloalkyl or -alk-NR₂₉R₃₀ radical,
R₁₆ and R₁₇, which are identical or different, represent a hydrogen atom or an alkyl radical or alternatively R₁₆ and R₁₇ together form with the nitrogen atom to which they are attached a 3- to 10-membered unsaturated or saturated mono- or bicyclic heterocycle optionally containing one or more other heteroatoms chosen from oxygen, sulfur and nitrogen and optionally substituted with one or more alkyl radicals,
R₁₈ represents a hydrogen atom or an alkyl radical,
R₁₉ represents a hydrogen atom or an alkyl, cycloalkyl, cycloalkylalkyl, cycloalkylcarbonyl, -SO₂alk, -CO-NHalk or -COOalk radical,
or alternatively R₁₈ and R₁₉ form with the nitrogen atom to which they are attached a 3- to 10-membered unsaturated or saturated mono- or bicyclic heterocycle optionally containing one or more heteroatoms chosen from oxygen, sulfur and nitrogen and optionally substituted with one or more alkyl radicals,
-NR₂₀R₂₁ represents a 3- to 8-membered saturated or unsaturated monocyclic heterocycle optionally containing another heteroatom chosen from oxygen, nitrogen and sulfur,
R₂₂ and R₂₃, which are identical or different, represent a hydrogen atom or an alkyl radical or alternatively R₂₂ and R₂₃ together form with the nitrogen atom to which they are attached a 3- to 10-membered saturated mono-or bicyclic heterocycle optionally containing another heteroatom chosen from oxygen, sulfur and nitrogen and being optionally substituted with one or more alkyl radicals,
R₂₄ and R₂₅, which are identical or different, represent a hydrogen atom or an alkyl, -COOalk, cycloalkyl, alkylcycloalkyl, -alk-O-alk or hydroxyalkyl radical or alternatively R₂₄ and R₂₅ together form with the nitrogen atom to which they are attached a 3- to 10-membered saturated or unsaturated mono- or bicyclic heterocycle, optionally containing another heteroatom chosen from oxygen, sulfur and nitrogen and being optionally substituted with one or more alkyl, -COalk, -COOalk, -CO-NHalk, -CS-NHalk, oxo, hydroxyalkyl, -alk-O-alk or -CO-NH₂ radicals,
R₂₆ and R₂₇, which are identical or different, represent a hydrogen atom or an alkyl, hydroxyalkyl, cycloalkyl, cycloalkylalkyl, -alk-COOalk, -alk-Ar, -alk-Het, Het or -alk-N(alk)₂ radical, R₂₆ and R₂₇ may also form with the nitrogen atom to which they are attached a 3- to 10-membered unsaturated or saturated mono- or bicyclic heterocycle optionally containing one or more other heteroatoms chosen from oxygen, sulfur and nitrogen and optionally substituted with one or more alkyl or alkoxy radicals or halogen atoms,
R₂₈ represents a -CH₂-alk, benzyl, -SO₂alk, -CONHalk, -COalk, cycloalkylalkylcarbonyl, cycloalkylcarbonyl or -CO-(CH₂)ₙOH radical,
n is equal to 1, 2 or 3,
R₂₉ and R₃₀, which are identical or different, represent a hydrogen atom or an alkyl radical or alternatively R₂₉ and R₃₀ together form with the nitrogen atom to which they are attached a 3- to 10-membered saturated mono-or bicyclic heterocycle optionally containing another heteroatom chosen from oxygen, sulfur and nitrogen and being optionally substituted with one or more alkyl radicals,
R₃₁ and R₃₂, which are identical or different, represent a hydrogen atom or an alkyl, Ar or -alk-Ar radical or alternatively R₃₁ and R₃₂ together form with the nitrogen atom to which they are attached a heterocycle chosen from aziridinyl, azetidinyl, pyrrolidinyl and piperidinyl,
alk represents an alkyl or alkylene radical,
Ar represents a phenyl or naphthyl radical optionally substituted with one or more substituents chosen from a halogen atom or an alkyl, alkoxy, -CO-alk, cyano, -COOH, -COOalk, -CONR₂₂R₂₃, -CO-NH-NR₂₄R₂₅, alkylsulfanyl, alkylsulfinyl, alkylsulfonyl, alkylsulfanylalkyl, alkylsulfinylalkyl, alkylsulfonylalkyl, hydroxyalkyl, -alk-NR₂₄R₂₅, -NR₂₄R₂₅, alkylthioalkyl, formyl, hydroxyl, CF₃, OCF₃, Het, -O-alk-NH-cycloalkyl or SO₂NH₂ radical,
Het represents a 3- to 10-membered unsaturated or saturated mono- or bicyclic heterocycle containing one or more heteroatoms chosen from oxygen, sulfur and nitrogen and optionally substituted with one or more halogen atoms or alkyl, alkoxy, alkoxycarbonyl, -CONR₂₂R₂₃, hydroxyl, hydroxyalkyl, oxo or SO₂NH₂ radicals,
the alkyl and alkylene radicals and portions and the alkoxy radicals and portions are in the form of a straight or branched chain and contain 1 to 6 carbon atoms, the cycloalkyl radicals contain 3 to 10 carbon atoms and the heterocycloalkyl and heterocyclenyl radicals contain 3 to 10 carbon atoms,
their optical isomers and their salts with an inorganic or organic acid.

2. Compound according to claim 1, for which Het is chosen from benzofuryl, benzothiazolyl, benzothienyl, benzoxazolyl, chromanyl, 2,3-dihydrobenzofuryl, 2,3-dihydrobenzothienyl, furyl, indolinyl, indolyl, isochromanyl, isoquinolyl, piperidyl, pyrrolyl, pyridyl, pyrimidinyl, quinolyl, 1,2,3,4-tetrahydroisoquinolyl, 1,2,3,4-tetrahydroquinolyl, thiazolyl and thienyl.

3. Compound of formula (I) according to claim 1, for which when R₁₆ and R₁₇ together form with the nitrogen atom to which they are attached a 3- to 10-membered saturated or unsaturated mono- or bicyclic heterocycle, the latter is an azetidinyl, pyrrolidinyl, piperidinyl, morpholinyl, thiamorpholinyl or piperazinyl ring.

4. Compound of formula (I) according to claim 1, for which when R₁₈ and R₁₉ together form with the nitrogen atom to which they are attached a 3- to 10-membered saturated or unsaturated mono- or bicyclic heterocycle, the latter is an azetidinyl, pyrrolidinyl, piperidinyl, morpholinyl, thiamorpholinyl or piperazinyl ring.

5. Compound of formula (I) according to claim 1, for which the heterocycle formed by NR₂₀R₂₁ is an azetidinyl, pyrrolidinyl, piperidinyl, morpholinyl, thiamorpholinyl, piperazinyl or imidazolyl ring.

6. Compound of formula (I) according to claim 1, for which when R₂₂ and R₂₃ together form with the nitrogen atom to which they are attached a 3- to 10-membered saturated or unsaturated mono- or bicyclic heterocycle, the latter is an azetidinyl, pyrrolidinyl, piperidinyl, morpholinyl, thiamorpholinyl or piperazinyl ring.

7. Compound of formula (I) according to claim 1, for which when R₂₄ and R₂₅ together form with the nitrogen atom to which they are attached a 3- to 10-membered saturated or unsaturated mono- or bicyclic heterocycle, the latter is an azetidinyl, pyrrolidinyl, piperidinyl, morpholinyl, thiamorpholinyl or piperazinyl ring.

8. Compound of formula (I) according to claim 1, for which when R₂₆ and R₂₇ together form with the nitrogen atom to which they are attached a 3- to 10-membered saturated or unsaturated mono- or bicyclic heterocycle, the latter is an azetidinyl, pyrrolidinyl, piperidinyl, morpholinyl, thiamorpholinyl or piperazinyl ring.

9. Compound of formula (I) according to claim 1, for which when R₂₉ and R₃₀ together form with the nitrogen atom to which they are attached a 3- to 10-membered saturated or unsaturated mono- or bicyclic heterocycle, the latter is an azetidinyl, pyrrolidinyl, piperidinyl, morpholinyl, thiamorpholinyl or piperazinyl ring.

10. Compound of formula (I) according to claim 1, for which:
R represents a radical CR₁R₂,
either R₁ represents a hydrogen atom and R₂ represents a radical -C(R₈)(R₁₁)(R₁₂) or C(R₈)(R₉)(R₁₀),
or R₁ represents an alkyl radical and R₂ represents a radical -C(R₈)(R₁₁)(R₁₂),
R₃ and R₄, which are identical or different, represent either a phenyl which is unsubstituted or substituted with one or more halogen atoms or alkyl, alkoxy, trifluoromethyl, trifluoromethoxy, cyano, -CONR₂₂R₂₃, hydroxyalkyl or -alk-NR₂₄R₂₅ radicals; or a heteroaromatic chosen from the pyridyl, pyrimidinyl, thiazolyl and thienyl rings, it being possible for these heteroaromatics to be unsubstituted or substituted with one or more halogen atoms or alkyl, alkoxy, hydroxyl, trifluoromethyl, trifluoromethoxy, cyano, -CONR₂₂R₂₃, -alk-NR₂₄R₂₅ or hydroxyalkyl radicals,
R₈ represents a hydrogen atom,
R₉ represents a -CO-NR₂₆R₂₇, -COOalk, -CH₂OH, -NH-CO-NH-alk, -CH₂-NHR₂₈ or -NHCOOalk radical,
R₁₀ represents a radical Ar or Het,
R₁₁ represents a radical -SO₂-alk, -SO₂-Ar or -SO₂-Het,
R₁₂ represents represents a hydrogen atom or a radical Ar or Het,
R₂₂ and R₂₃, which are identical or different, represent a hydrogen atom or an alkyl radical or alternatively R₂₂. and R₂₃ together form with the nitrogen atom to which they are attached a 3- to 10-membered saturated mono-or bicyclic heterocycle optionally containing another heteroatom chosen from oxygen, sulfur and nitrogen and being optionally substituted with one or more alkyl radicals,
R₂₄ and R₂₅, which are identical or different, represent a hydrogen atom or an alkyl, cycloalkyl, alkylcycloalkyl or hydroxyalkyl radical or alternatively R₂₄ and R₂₅ together form with the nitrogen atom to which they are attached a 3- to 10-membered saturated or unsaturated mono- or bicyclic heterocycle optionally containing another heteroatom chosen from oxygen, sulfur and nitrogen and being optionally substituted with one or more alkyl, -COalk, -COOalk, -CO-NHalk, -CS-NHalk, oxo or -CO-NH₂ radicals,
Ar represents a phenyl or naphthyl radical optionally substituted with 1 or 2 subtituents chosen from a halogen atom or an alkyl, alkoxy, -CO-alk, cyano, -COOalk, -CONR₂₂R₂₃, alkylsulfonyl, hydroxyalkyl, -alk-NR₂₄R₂₅, -NR₂₄R₂₅, hydroxyl, CF₃, OCF₃, -O-alk-NH-cycloalkyl or SO₂NH₂ radical, Het represents a benzofuryl, benzothiazolyl, benzothienyl, benzoxazolyl, furyl, isoquinolyl, pyrrolyl, pyridyl, quinolyl, 1,2,3,4-tetrahydroisoquinolyl, 1,2,3,4-tetrahydroquinolyl, thiazolyl or thienyl ring,
their optical isomers and their salts with an inorganic or organic acid.

11. Compound chosen from the following compounds:
(RS)-1-[bis(4-chlorophenyl)methyl)]-3-[(3,5-difluorophenyl)(methylsulfonyl)methyl]azetidine,
(R)-1-[bis(4-chlorophenyl)methyl)]-3-[(3,5-difluorophenyl)(methylsulfonyl)methyl]azetidine,
(S)-1-[bis(4-chlorophenyl)methyl)]-3-[(3,5-difluorophenyl)(methylsulfonyl)methyl]azetidine,
(RS)-1-[bis(4-chlorophenyl)methyl)]-3-[(pyrid-3-yl) (methylsulfonyl)methyl]azetidine,
(R)-1-[bis(4-chlorophenyl)methyl)]-3-[(pyrid-3-yl) (methylsulfonyl)methyl]azetidine,
(S)-1-[bis(4-chlorophenyl)methyl)]-3-[(pyrid-3-yl) (methylsulfonyl)methyl]azetidine,
(RS)-1-[bis(3-fluorophenyl)methyl]-3-[(3,5-difluorophenyl)methylsulfonylmethyl]azetidine,
(R)-1-[bis(3-fluorophenyl)methyl]-3-[(3,5-difluorophenyl)methylsulfonylmethyl]azetidine,
(S)-1-[bis(3-fluorophenyl)methyl]-3-[(3,5-difluorophenyl)methylsulfonylmethyl]azetidine,
1-[bis(4-chlorophenyl)methyl]-3-(RS)-{[3-azetidin-1-yl-phenyl]methylsulfonylmethyl}azetidine,
1-[bis(4-chlorophenyl)methyl]-3-(R)-{[3-azetidin-1-yl-phenyl]methylsulfonylmethyl}azetidine,
1-[bis(4-chlorophenyl)methyl]-3-(S)-{[3-azetidin-1-yl-phenyl]methylsulfonylmethyl}azetidine,
(RS)-1-[3({1-[bis(4-chlorophenyl)methyl]azetidin-3-yl}-methylsulfonylmethyl)phenyl]pyrrolidine,
(R)-1-[3-({1-[bis(4-chlorophenyl)methyl]azetidin-3-yl}-methylsulfonylmethyl)phenyl]pyrrolidine,
(S)-1-[3-({1-[bis(4-chlorophenyl)methyl]azetidin-3-yl}methylsulfonylmethyl)phenyl]pyrrolidine,
(RS)-N-[3-({1-[bis(4-chlorophenyl)methyl]azetidin-3-yl}methylsulfonylmethyl)phenyl]-N-methylamine,
(R)-N-[3-({1-[bis(4-chlorophenyl)methyl]azetidin-3-yl}-methylsulfonylmethyl)phenyl]-N-methylamine,
(S)-N-[3-({1-[bis(4-chlorophenyl)methyl]azetidin-3-yl}-methylsulfonylmethyl)phenyl]-N-methylamine,
(RS)-1-[bis(4-chlorophenyl)methyl]-3-[(3,5-bistrifluoromethylphenyl)methylsulfonylmethyl]azetidine,
(R)-1-[bis(4-chlorophenyl)methyl]-3-[(3,5-bistrifluoromethylphenyl)methylsulfonylmethyl]azetidine,
(S)-1-[bis(4-chlorophenyl)methyl]-3-[(3,5-bistrifluoromethylphenyl)methylsulfonylmethyl]azetidine,
1-[bis(4-chlorophenyl)methyl]-3-(phenylsulfonylmethyl)azetidine,
(RS)-1-[bis(4-chlorophenyl)methyl]-3-[(3,5-difluorophenyl)methylsulfonylmethyl]-3-methylazetidine,
(R)-1-[bis(4-chlorophenyl)methyl]-3-[(3,5-difluorophenyl)methylsulfonylmethyl]-3-methylazetidine,
(S)-1-[bis(4-chlorophenyl)methyl]-3-[(3,5-difluorophenyl)methylsulfonylmethyl]-3-methylazetidine,
(RS)-2-{1-[bis(4-chlorophenyl)methyl]azetidin-3-yl}-2-(3,5-difluorophenyl)-N-cyclohexylacetamide,
(R)-2-{1-[bis(4-chlorophenyl)methyl]azeditin-3-yl}-2-(3,5-difluorophenyl)-N-cyclohexylacetamide,
(S)-2-{1-[bis(4-chlorophenyl)methyl]azetidin-3-yl}-2-(3,5-difluorophenyl)-N-cyclohexylacetamide,
(RS)-2-{1-[bis(4-chlorophenyl)methyl]azetidin-3-yl}-2-(3,5-difluorophenyl)-N-isobutylacetamide,
(R)-2-{1-[bis(4-chlorophenyl)methyl]azetidin-3-yl}-2-(3,5-difluorophenyl)-N-isobutylacetamide,
(S)-2-{1-[bis(4-chlorophenyl)methyl]azetidin-3-yl}-2-(3,5-difluorophenyl)-N-isobutylacetamide,
(RS)-2-{1-[bis(4-chlorophenyl)methyl]azetidin-3-yl}-2-(3,5-difluorophenyl)-N-cyclopropylmethylacetamide,
(R)-2-{1-[bis(4-chlorophenyl)methyl]azetidin-3-yl}-2-(3,5-difluorophenyl)-N-cyclopropylmethylacetamide,
(S)-2-{1-bis(4-chlorophenyl)methyl]azetidin-3-yl}-2-(3,5-difluorophenyl)-N-cyclopropylmethylacetamide,
(RS)-2-{1-[bis(4-chlorophenyl)methyl]azetidin-3-yl}-2-(3,5-difluorophenyl)-N-isopropylacetamide,
(R)-2-{1-[bis(4-chlorophenyl)methyl]azetidin-3-yl}-2-(3,5-difluorophenyl)-N-isopropylacetamide,
(S)-2-{1-[bis(4-chlorophenyl)methyl]azetidin-3-yl}-2-(3,5-difluorophenyl)-N-isopropylacetamide,
(RS)-1-[bis(4-chlorophenyl)methyl]-3-[1-(3,5-difluorophenyl)-1-methylsulfonylethyl]azetidine,
(R)-1-[bis(4-chlorophenyl)methyl]-3-[1-(3,5-difluorophenyl)-1-methylsulfonylethyl]azetidine,
(S)-1-[bis(4-chlorophenyl)methyl]-3-[1-(3,5-difluorophenyl)-1-methylsulfonylethyl]azetidine,
(RS)-1-[bis(4-fluorophenyl)methyl]-3-[(3,5-difluorophenyl)methylsulfonylmethyl]azetidine,
(R)-1-[bis(4-fluorophenyl)methyl]-3-[(3,5-difluorophenyl)methylsulfonylmethyl]azetidine,
(S)-1-[bis(4-fluorophenyl)methyl]-3-[(3,5-difluorophenyl)methylsulfonylmethyl]azetidine,
(RS)-{1-[(3-pyridyl)-(4-chlorophenyl)methyl]-3-[(3,5-difluorophenyl)methylsulfonylmethyl]azetidine,
(SS)-{1-[(3-pyridyl)-(4-chlorophenyl)methyl]-3-[(3,5-difluorophenyl)methylsulfonylmethyl]azetidine,
(RR)-{1-[(3-pyridyl)-(4-chlorophenyl)methyl]-3-[(3,5-difluorophenyl)methylsulfonylmethyl]azetidine,
(SR)-{1-[(3-pyridyl)-(4-chlorophenyl)methyl]-3-[(3,5-difluorophenyl)methylsulfonylmethyl]azetidine,
(RS)-{1-[(4-pyridyl)-(4-chlorophenyl)methyl]-3-[(3,5-difluorophenyl)methylsulfonylmethyl]azetidine,
(SS)-{1-[(4-pyridyl)-(4-chlorophenyl)methyl]-3-[(3,5-difluorophenyl)methylsulfonylmethyl]azetidine,
(RR)-{1-[(4-pyridyl)-(4-chlorophenyl)methyl]-3-[(3,5-difluorophenyl)methylsulfonylmethyl]azetidine,
(SR)-{1-[(4-pyridyl)-(4-chlorophenyl)methyl]-3-[(3,5-difluorophenyl)methylsulfonylmethyl]azetidine,
(RS)-5-((4-chlorophenyl)-{3-[(3,5-difluorophenyl)-methylsulfonylmethyl]azetidin-1-yl}methyl)pyrimidine,
(SR)-5-((4-chlorophenyl)-{3-[(3,5-difluorophenyl)-methylsulfonylmethyl]azetidin-1-yl}methyl)pyrimidine,
(RR)-5-((4-chlorophenyl)-{3-[(3,5-difluorophenyl)-methylsulfonylmethyl]azetidin-1-yl}methyl)pyrimidine,
(SS)-5-((4-chlorophenyl)-{3-[(3,5-difluorophenyl)-methylsulfonylmethyl]azetidin-1-yl}methyl)pyrimidine,
(SS)-{1-[(2-chloropyrid-5-yl)-(4-chlorophenyl)methyl]-3-[(3,5-difluorophenyl)methylsulfonylmethyl]azetidine,
(RR)-{1-[(2-chloropyrid-5-yl)-(4-chlorophenyl)methyl]-3-[(3,5-difluorophenyl)methylsulfonylmethyl]azetidine,
(RS)-{1-[(2-chloropyrid-5-yl)-(4-chlorophenyl)methyl]-3-[(3,5-difluorophenyl)methylsulfonylmethyl]azetidine,
(SR)-{1-[(2-chloropyrid-5-yl)-(4-chlorophenyl)methyl]-3-[(3,5-difluorophenyl)methylsulfonylmethyl]azetidine,
their optical isomers and their pharmaceutically acceptable salts with an inorganic or organic acid.

12. Process for preparing the compounds of formula (I) according to claim 1 for which R represents a radical CR₁R₂, in which R₁ represents a hydrogen atom and R₂ represents a radical C(R₈)(R₁₁)(R₁₂) in which R₈ represents a hydrogen atom, R₁₁ represents a radical -SO₂-Ar, -SO₂-Het or -SO₂alk and R₁₂ represents a hydrogen atom or a radical Ar or Het, **characterized in that** a derivative of formula: 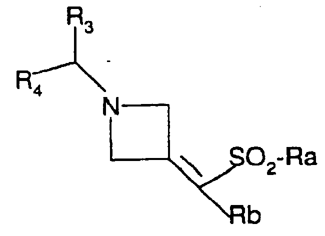 in which Ra represents an alkyl, Het or Ar radical and Rb represents a hydrogen atom or an Ar, Het or alkyl radical, Ar and Het having the same meanings as in claim 1, is reduced, the product isolated and optionally converted to a salt with an inorganic or organic acid.

13. Process for preparing the compounds of formula (I) according to claim 1 for which R represents a radical CR₁R₂, in which R₁ represents a hydrogen atom and R₂ represents a radical C(R₈)(R₁₁)(R₁₂) in which R₈ represents a hydrogen atom, R₁₁ represents a radical -SO₂-Ar, -SO₂-Het or -SO₂alk and R₁₂ represents a hydrogen atom or a radical Ar or Het, **characterized in that** a derivative R₃CH(Br)R₄ is reacted with a derivative of formula: 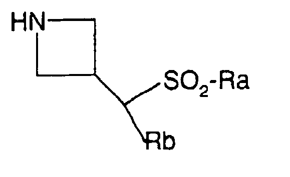 in which Ra represents an alkyl, Het or Ar radical and Rb represents a hydrogen atom or an Ar, Het or alkyl radical, Ar and Het having the same meanings as in claim 1, the product isolated and optionally converted to a salt with an inorganic or organic acid.

14. Process for preparing the compounds of formula (I) according to claim 1 for which R represents a radical C=C(R₅)SO₂R₆ or C=C(R₇)SO₂alk, **characterized in that** a derivative of formula: 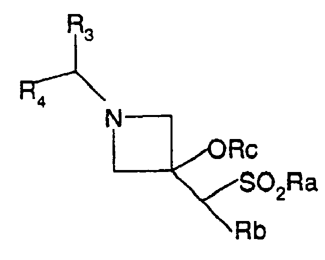 in which either Ra represents a radical Ar or Het and Rb represents a hydrogen atom or an alkyl radical, or Ra represents an alkyl radical and Rb represents a cycloalkyl, heterocycloalkyl or heterocyclenyl radical optionally substituted with a radical -CSO-phenyl, Rc represents a hydrogen atom or an acetyl radical, R₃, R₄, Ar and Het having the same meanings as in claim 1, is dehydrated, the product isolated and optionally converted to a salt with an inorganic or organic acid.

15. Process for preparing the compounds of formula (I) according to claim 1 for which R represents a radical C=C(R₅)SO₂R₆ or C=C(R₇)SO₂alk, **characterized in that** R₃CH(Br)R₄ is reacted with a derivative of formula: 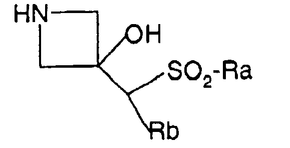 in which either Ra represents a radical Ar or Het and Rb represents a hydrogen atom or an alkyl radical, or Ra represents an alkyl radical and Rb represents a cycloalkyl, heterocycloalkyl or heterocyclenyl radical optionally substituted with a radical -CSO-phenyl, Rc represents a hydrogen atom or an acetyl radial, R₃, R₄, Ar and Het having the same meanings as in claim 1, the product isolated and optionally converted to a salt with an inorganic or organic acid.

16. Process for preparing the compounds of formula (I) according to claim 1 for which R represents a radical CR₁R₂ in which R₁ represents a hydrogen atom and R₂ represents a radical C(R₈)(R₉)(R₁₀) in which R₈ represents a hydrogen atom, R₉ represents a radical -CO-NR₂₆R₂₇ and R₁₀ represents a radical Ar or Het, **characterized in that** an amine HNR₂₆R₂₇ in which R₂₆ and R₂₇ have the same meanings as in claim 1, is reacted with an acid of formula: 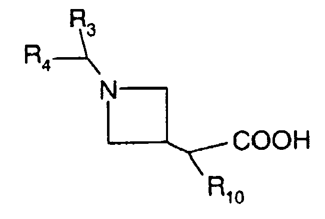 in which R₃, R₄ and R₁₀ have the same meanings as in claim 1, the product isolated and optionally converted to a salt with an inorganic or organic acid.

17. Process for preparing the compounds of formula (I) according to claim 1 for which R represents a radical CR₁R₂ in which R₁ represents a hydrogen atom and R₂ represents a radical C(R₈)(R₉)(R₁₀) in which R₈ represents a hydrogen atom, R₉ represents a radical -COOH and R₁₀ represents a radical Ar or Het, **characterized in that** the corresponding ester of formula: 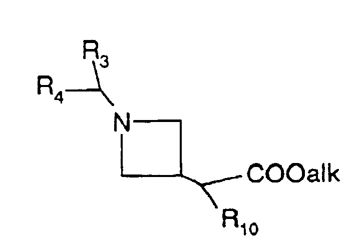 in which R₃, R₄ and R₁₀ have the same meanings as in claim 1 and alk represents an alkyl radical (1-6C in the form of a straight or branched chain, is hydrolyzed, the product isolated and optionally converted to a salt with an inorganic or organic acid.

18. Process for preparing the compounds of formula (I) according to claim 1 for which R represents a radical CR₁R₂ in which R₁ represents a hydrogen atom and R₂ represents a radical C(R₈)(R₉)(R₁₀) in which R₈ represents a hydrogen atom, R₉ represents a radical -COOalk or -CH₂OH and R₁₀ represents a radical Ar or Het, **characterized in that** a compound of formula: 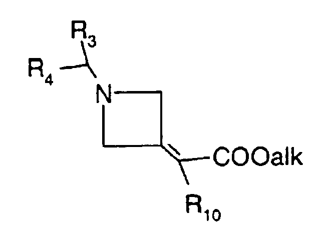 in which R₃, R₄ and R₁₀ have the same meanings as in claim 1 and alk represents an alkyl radical (1-6C in the form of a straight or branched chain), is reduced, the product isolated and optionally converted to a salt with an inorganic or organic acid.

19. Process for preparing the compounds of formula (I) according to claim 1 for which R represents a radical CR₁R₂ in which R₁ represents a hydrogen atom and R₂ represents a radical C(R₈)(R₉)(R₁₀) in which R₈ represents a hydrogen atom, R₉ represents a radical -NHCOOalk and R₁₀ represents a radical Ar or Het, **characterized in that** an alcohol alkOH for which alk represents an alkyl radical (1-6C in the form of a straight or branched chain) is reacted with a derivative of formula: 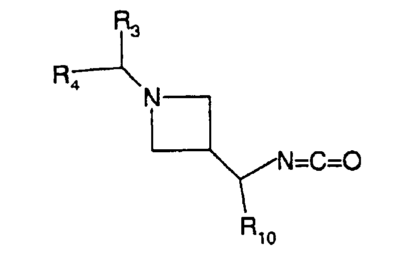 in which R₃, R₄ and R₁₀ have the same meanings as in claim 1, the product isolated and optionally converted to a salt with an inorganic or organic acid.

20. Process for preparing the compounds of formula (I) according to claim 1 for which R represents a radical CR₁R₂ in which R₁ represents a hydrogen atom and R₂ represents a radical C(R₈)(R₉)(R₁₀) in which R₈ represents a hydrogen atom, R₉ represents a radical -NH-CO-NH-alk and R₁₀ represents a radical Ar or Het, **characterized in that** an amine alkNH₂ for which alk represents an alkyl radical (1-6C in the form of a straight or branched chain) is reacted with a derivative of formula: 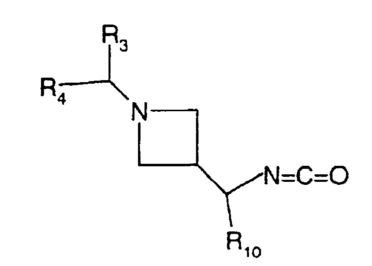 in which R₃, R₄ and R₁₀ have the same meanings as in formula (I), the product isolated and optionally converted to a salt with an inorganic or organic acid.

21. Process for preparing the compounds of formula (I) according to claim 1 for which R represents a radical CR₁R₂ in which R₁ is a hydrogen atom and R₂ represents a radical -C(R₈)(R₉)(R₁₀), R₈ is a hydrogen atom, R₉ is a radical -CH₂-NHR₂₈, R₂₈ represents a -CH₂-alk or benzyl radical and R₁₀ represents a radical Ar or Het, **characterized in that** a derivative of formula: 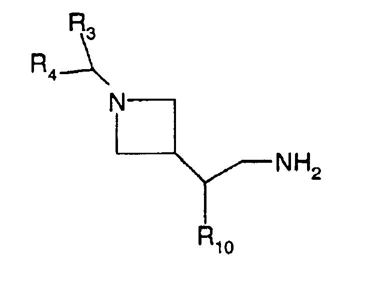 in which R₃, R₄ and R₁₀ have the same meanings as in claim 1, is reacted with an aldehyde RdCHO for which Rd represents a -CH₂-alk or benzyl radical and alk represents an alkyl radical (1-6C in the form of a straight or branched chain), the product isolated and optionally converted to a salt with an inorganic or organic acid.

22. Process for preparing the compounds of formula (I) according to claim 1 for which R represents a radical CR₁R₂ in which R₁ is a hydrogen atom and R₂ represents a radical -C(R₈)(R₉)(R₁₀), R₈ is a hydrogen atom, R₉ is a radical -CH₂-NHR₂₈, R₂₈ represents a radical -SO₂alk and R₁₀ represents a radical Ar or Het, **characterized in that** an amine of formula: 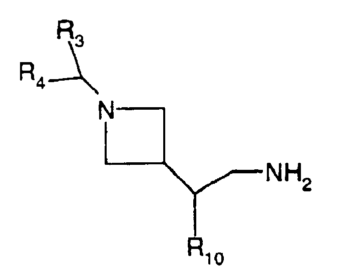 in which R₃, R₄ and R₁₀ have the same meanings as in claim 1, is reacted with a derivative ClSO₂Re for which Re represents an alkyl radical (1-6C in the form of a straight or branched chain), the product isolated and optionally converted to a salt with an inorganic or organic acid.

23. Process for preparing the compounds of formula (I) according to claim 1 for which R represents a radical CR₁R₂ in which R₁ is a hydrogen atom and R₂ represents a radical -C(R₈)(R₉)(R₁₀), R₈ is a hydrogen atom, R₉ is a radical -CH₂-NHR₂₈, R₂₈ represents a radical -CO-NHalk and R₁₀ represents a radical Ar or Het, **characterized in that** a derivative of formula: 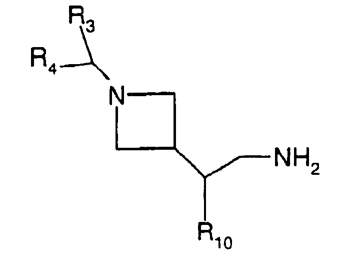 in which R₃, R₄ and R₁₀ have the same meanings as in claim 1, is reacted with a derivative RfNCO for which Rf represents an alkyl radical (1-6C in the form of a straight or branched chain), the product isolated and optionally converted to a salt with an inorganic or organic acid.

24. Process for preparing the compounds of formula (I) according to claim 1 for which R represents a radical CR₁R₂ in which R₁ is a hydrogen atom and R₂ represents a radical -C(R₈)(R₉)(R₁₀), R₈ is a hydrogen atom, R₉ is a radical -CH₂-NHR₂₈, R₂₈ represents a -COalk, cycloalkylalkylcarbonyl, cycloalkylcarbonyl or -CO-(CH₂)ₙOH radical and R₁₀ represents a radical Ar or Het, **characterized in that** a derivative of formula: 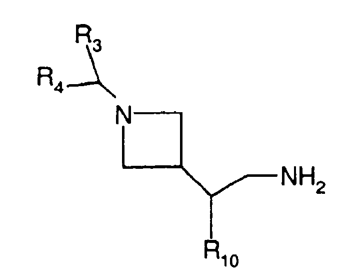 in which R₃, R₄ and R₁₀ have the same meanings as in claim 1, is reacted with an acid HOOCRg for which Rg represents an alkyl (1-6C in the form of a straight or branched chain), (3-10C)cycloalkyl(1-6C in the form of a straight or branched chain)alkyl, (3-10C)cycloalkyl or -(CH₂)ₙOH radical and n is equal to 1, 2 or 3, the product isolated and optionally converted to a salt with an inorganic or organic acid.

25. Process for preparing the compounds of formula (I) according to claim 1 for which R represents a radical CR₁R₂ in which R₁ is a hydrogen atom and R₂ is a radical -CONR₁₃R₁₄, **characterized in that** an amine HNR₁₃R₁₄ in which R₁₃ and R₁₄ have the same meanings as in claim 1, is reacted with a derivative of formula: 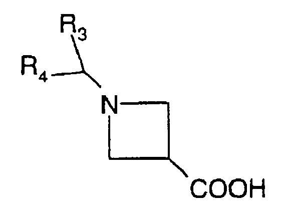 in which R₃ and R₄ have the same meanings as in formula (I), the product isolated and optionally converted to a salt with an inorganic or organic acid.

26. Process for preparing the compounds of formula (I) according to claim 1 for which R represents a radical CR₁R₂ in which R₁ is a hydrogen atom and R₂ is a radical -CH₂-CONR₁₃R₁₄, **characterized in that** an amine HNR₁₃R₁₄ in which R₁₃ and R₁₄ have the same meanings as in claim 1, is reacted with a derivative of formula: 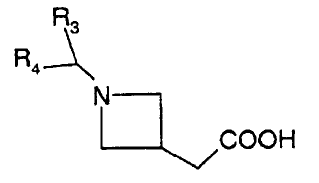 in which R₃ and R₄ have the same meanings as in claim 1, the product isolated and optionally converted to a salt with an inorganic or organic acid.

27. Process for preparing the compounds of formula (I) according to claim 1 for which R represents a radical CR₁R₂ in which R₁ is a hydrogen atom and R₂ is a radical -CH₂-CONR₁₃R₁₄, **characterized in that** a derivative of formula: 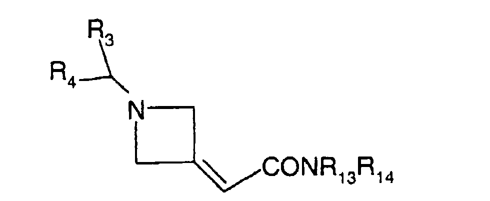 in which R₃, R₄, R₁₃ and R₁₄ have the same meanings as in claim 1, is reduced, the product isolated and optionally converted to a salt with an inorganic or organic acid.

28. Process for preparing the compounds of formula (I) according to claim 1 for which R represents a radical CR₁R₂ in which R₁ is a hydrogen atom and R₂ represents a radical -SOR₆, **characterized in that** a derivative of formula: 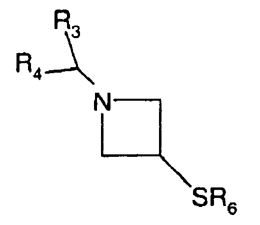 in which R₃, R₄ and R₆ have the same meanings as in claim 1, is oxidized, the product isolated and optionally converted to a salt with an inorganic or organic acid.

29. Process for preparing the compounds of formula (I) according to claim 1 for which R represents a radical CR₁R₂ in which R₁ is a hydrogen atom and R₂ represents a radical -SO₂R₆, **characterized in that** a derivative of formula: 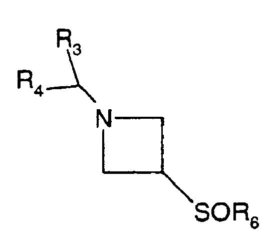 in which R₃, R₄ and R₆ have the same meanings as in claim 1, is oxidized, the product isolated and optionally converted to a salt with an inorganic or organic acid.

30. Process for preparing compounds of formula (I) according to claim 1 for which R represents a radical CR₁R₂ in which R₁ is a hydrogen atom and R₂ represents a radical -COR₆ or a -CO-cycloalkyl radical, **characterized in that** a derivative of formula: 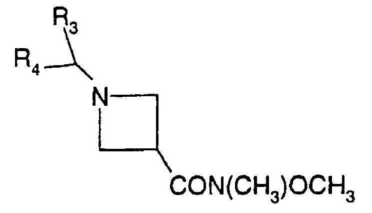 in which R₃ and R₄ have the same meanings as in claim 1, is reacted with a derivative RhMgBr for which Rh has the same meanings as R₆ in claim 1 or else represents a (3-10C)cycloalkyl radical, the product isolated and optionally converted to a salt with an inorganic or organic acid.

31. Process for preparing the compounds of formula (I) according to claim 1 for which R represents a radical CR₁R₂ in which R₁ is a hydrogen atom and R₂ represents a radical -C(OH)(R₆)(R₁₂) or -C (OH)-(R₆)(alkyl) **characterized in that** a derivative of formula: 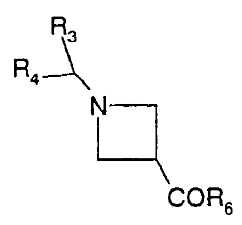 in which R₃, R₄ and R₆ have the same meanings as in claim 1, is reacted with a derivative RiMgBr for which Ri has the same meanings as R₁₂ in claim 1 or else represents an alkyl radical (1-6C in the form of a straight or branched chain), the product isolated and optionally converted to a salt with an inorganic or organic acid.

32. Process for preparing the compounds of formula (I) according to claim 1 for which R represents a radical CR₁R₂ in which R₁ is a hydrogen atom and R₂ represents a radical -C(=NOalk)R₆ or -C(=NO-CH₂-CH=CH₂)R₆, **characterized in that** a derivative of formula: 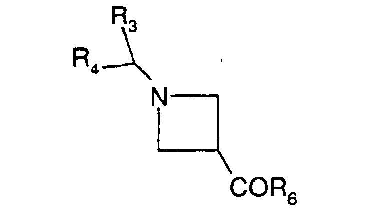 in which R₃, R₄ and R₆ have the same meanings as in claim 1, is reacted with a derivative RjONH₂ for which Rj represents an alkyl radical (1-6C in the form of a straight or branched chain) or a radical -CH₂-CH=CH₂, the product isolated and optionally converted to a salt with an inorganic or organic acid.

33. Process for preparing the compounds of formula (I) according to claim 1 for which R represents a radical CR₁R₂ in which R₁ is a hydrogen atom and R₂ represents a radical -CH(R₆)NR₃₁R₃₂, R₃₁ and R₃₂ represent hydrogen atoms, **characterized in that** aqueous ammonia is reacted with a derivative of formula: 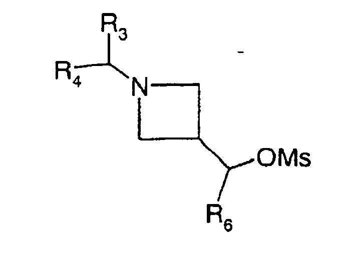 in which R₃, R₄ and R₆ have the same meanings as in claim 1 and Ms represents a methylsulfonyloxy radical, the product isolated and optionally converted to a salt with an inorganic or organic acid.

34. Process for preparing the compounds of formula (I) according to claim 1 for which R represents a radical CR₁R₂ in which R₁ is a hydrogen atom and R₂ represents a radical -CH(R₆)NR₃₁R₃₂, R₃₁ is a hydrogen atom and R₃₂ is an alkyl, Ar or -alk-Ar radical, **characterized in that** a halide HalR₃₁ is reacted with a corresponding compound of formula (I) for which R represents a radical CR₁R₂ in which R₁ is a hydrogen atom and R₂ represents a radical -CH(R₆)NR₃₁R₃₂, R₃₁ and R₃₂ are hydrogen atoms, the product isolated and optionally converted to a salt with an inorganic or organic acid.

35. Process for preparing the compounds of formula (I) according to claim 1 for which R represents a radical CR₁R₂ in which R₁ is a hydrogen atom and R₂ represents a radical -CH(R₆)NR₃₁R₃₂, R₃₁ and R₃₂ are alkyl, Ar or -alk-Ar radicals, **characterized in that** a halide HalR₃₂ is reacted with a corresponding compound of formula (I) for which R represents a radical CR₁R₂ in which R₁ is a hydrogen atom and R₂ represents a radical -CH(R₆)NR₃₁R₃₂, R₃₁ is a hydrogen atom and R₃₂ is an alkyl, Ar or -alk-Ar radical, the product isolated and optionally converted to a salt with an inorganic or organic acid.

36. Process for preparing the compounds of formula (I) according to claim 1 for which R represents a radical CR₁R₂ in which R₁ is a hydrogen atom and R₂ represents a radical -CH(R₆)NR₃₁R₃₂, R₃₁ is a hydrogen atom and R₃₂ is a (2-6C) alkyl or -(2-6C)alk-Ar radical, **characterized in that** an aldehyde RaCHO for which Ra is an alkyl or -alk-Ar radical is reacted with a corresponding compound of formula (I) for which R represents a radical CR₁R₂ in which R₁ is a hydrogen atom and R₂ represents a radical -CH(R₆)NR₃₁R₃₂, R₃₁ and R₃₂ are hydrogen atoms, the product isolated and optionally converted to a salt with an inorganic or organic acid.

37. Process for preparing the compounds of formula (I) according to claim 1 for which R represents a radical CR₁R₂ in which R₁ is a hydrogen atom and R₂ represents a radical -CH(R₆)NR₃₁R₃₂, R₃₁ is an alkyl, Ar or -alk-Ar radical and R₃₂ is a (2-6C) alkyl or -(2-6C)alk-Ar radical **characterized in that** an aldehyde RaCHO for which Ra is an alkyl or -alk-Ar radical is reacted with a corresponding compound of formula (I) for which R represents a radical CR₁R₂ in which R₁ is a hydrogen atom and R₂ represents a radical -CH(R₆)NR₃₁R₃₂, R₃₁ is a hydrogen atom and R₃₂ is an alkyl, Ar or -alk-Ar radical, the product isolated and optionally converted to a salt with an inorganic or organic acid.

38. Process for preparing the compounds of formula (I) according to claim 1 for which R represents a radical CR₁R₂ in which R₁ is a hydrogen atom and R₂ represents a radical -CH(R₆)NR₃₁R₃₂, R₃₁ and R₃₂ form with the nitrogen atom to which they are attached a heterocycle chosen from aziridinyl, azetidinyl, pyrrolidinyl and piperidinyl, **characterized in that** a dihalide Hal-(2-5C)alk-Hal is reacted with a corresponding compound of formula (I) for which R represents a radical CR₁R₂ in which R₁ is a hydrogen atom and R₂ represents a radical -CH(R₆)NR₃₁R₃₂, R₃₁ and R₃₂ are hydrogen atoms, the product isolated and optionally converted to a salt with an inorganic or organic acid.

39. Process for preparing the compounds of formula (I) according to claim 1 for which R represents a radical CR₁R₂ in which R₁ is a hydrogen atom and R₂ represents a radical -CH(R₆)NHSO₂alk, **characterized in that** a derivative of formula: 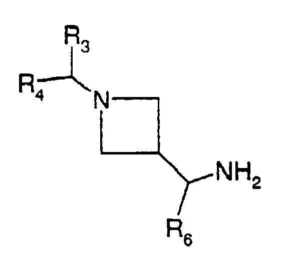 in which R₃, R₄ and R₆ have the same meanings as in claim 1, is reacted with a derivative ClSO₂alk for which alk represents an alkyl radical (1-6C in the form of a straight or branched chain), the product isolated and optionally converted to a salt with an inorganic or organic acid.

40. Process for preparing the compounds of formula (I) according to claim 1 for which R represents a radical CR₁R₂ in which R₁ is a hydrogen atom and R₂ represents a radical -CH(R₆)NHCONHalk, **characterized in that** a derivative of formula: 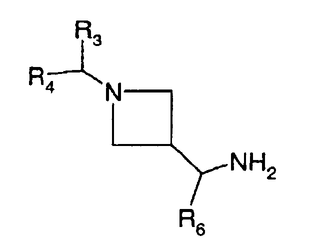 in which R₃, R₄ and R₆ have the same meanings as in claim 1, is reacted with a derivative alkNCO for which alk represents an alkyl radical (1-6C in the form of a straight or branched chain), the product isolated and optionally converted to a salt with an inorganic or organic acid.

41. Process for preparing the compounds of formula (I) according to claim 1 for which R represents a radical CR₁R₂ in which R₁ is a hydrogen atom and R₂ represents a radical or -CH(R₆)NHCOR₃₁, **characterized in that** a derivative of formula: 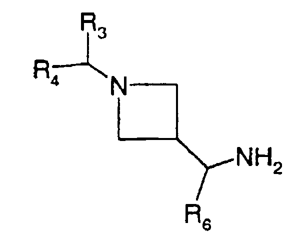 in which R₃, R₄ and R₆ have the same meanings as in claim 1, is reacted with a derivative R₃₁COOH for which R₃₁ has the same meanings as in claim 1, the product isolated and optionally converted to a salt with an inorganic or organic acid.

42. Process for preparing the compounds of formula (I) according to claim 1 for which R represents a radical CR₁R₂ in which R₁ is a hydrogen atom and R₂ represents a radical -CH₂-COR₆, **characterized in that** a derivative of formula: 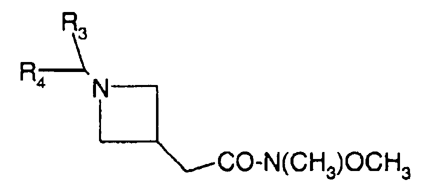 in which R₃ and R₄ have the same meanings as in claim 1, is reacted with a derivative of formula R₆MgBr for which R₆ has the same meanings as in claim 1, the product isolated and optionally converted to a salt with an inorganic or organic acid.

43. Process for preparing the compounds of formula (I) according to claim 1 for which R represents a radical CR₁R₂ in which R₁ is a hydrogen atom and R₂ represents a radical -CH₂-CH(R₆)-NR₃₁R₃₂, **characterized in that** a derivative of formula: 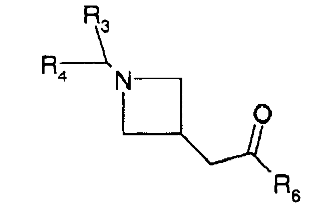 in which R₃ and R₄ have the same meanings as in claim 1, is reacted with a derivative of formula HNR₃₁R₃₂ for which R₃₁ and R₃₂ have the same meanings as in claim 1, the product isolated and optionally converted to a salt with an inorganic or organic acid.

44. Process for preparing the compounds of formula (I) according to claim 1 for which R represents a radical CR₁R₂ in which R₁ is a hydrogen atom and R₂ represents a radical -CH₂-C(=NOalk)R₆, **characterized in that** a derivative of formula: 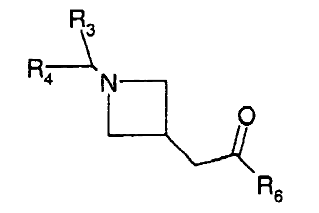 in which R₃ and R₄ have the same meanings as in claim 1, is reacted with a derivative of formula alkONH₂ for which alk represents an alkyl radical (1-6C in the form of a straight or branched chain), the product isolated and optionally converted to a salt with an inorganic or organic acid.

45. Process for preparing the compounds of formula (I) according to claim 1 for which R represents a radical CR₁R₂ in which R₁ represents a cyano radical and R₂ represents a radical -C(R₈)(R₁₁)(R₁₂) in which R₈ is a hydrogen atom, **characterized in that** a derivative of formula: 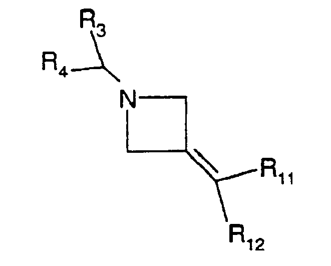 in which R₃, R₄, R₁₁ and R₁₂ have the same meanings as in claim 1, is reacted with sodium cyanide, the product isolated and optionally converted to a salt with an inorganic or organic acid.

46. Process for preparing the compounds of formula (I) according to claim 1 for which R represents a radical CR₁R₂ in which R₁ represents a radical -S-alk-NR₁₆R₁₇ and R₂ represents a radical -C(R₈)(R₁₁)(R₁₂) in which R₈ is a hydrogen atom, **characterized in that** a derivative of formula: 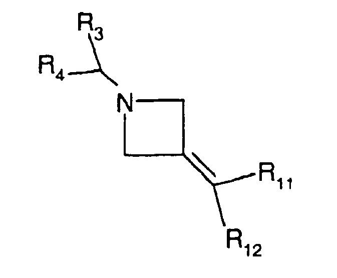 in which R₃, R₄, R₁₁ and R₁₂ have the same meanings as in claim 1, is reacted with a derivative HS-alk-NR₁₆R₁₇ for which alk represents an alk radical represents an alkyl radical (1-6C in the form of a straight or branched chain) and R₁₆ and R₁₇ have the same meanings as in claim 1, the product isolated and optionally converted to a salt with an inorganic or organic acid.

47. Process for preparing the compounds of formula (I) according to claim 1 for which R represents a radical CR₁R₂ in which R₁ represents a radical -NHR₁₅ and R₂ represents a radical -C(R₈)(R₁₁)(R₁₂) in which R₈ is a hydrogen atom, **characterized in that** a derivative of formula: 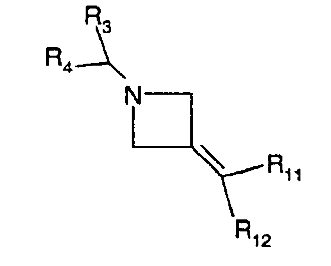 in which R₃, R₄, R₁₁ and R₁₂ have the same meanings as in claim 1, is reacted with a derivative H₂NR₁₅ for which R₁₅ has the same meanings as in claim 1, the product isolated and optionally converted to a salt with an inorganic or organic acid.

48. Process for preparing the compounds of formula (I) according to claim 1 for which R represents a radical CR₁R₂ in which R₁ represents an alkyl radical and R₂ represents a radical -C(R₈)(R₁₁)(R₁₂) in which R₈ is a hydrogen atom, **characterized in that** a derivative of formula: 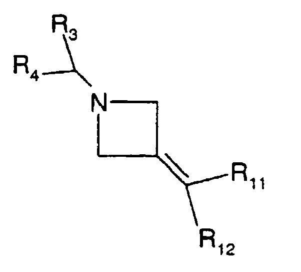 in which R₃, R₄, R₁₁ and R₁₂ have the same meanings as in claim 1, is reacted with a derivative alkMHal for which alk represents an alkyl radical (1-6C in the form of a straight or branched chain) and M represents a metal, the product isolated and optionally converted to a salt with an inorganic or organic acid.

49. Process for preparing the compounds of formula (I) according to claim 1, for which R represents a radical CR₁R₂ in which R₁ represents a radical -NR₂₀R₂₁ and R₂ represents a radical -C(R₈)(R₁₁)(R₁₂) in which R₈ is a hydrogen atom, **characterized in that** a derivative of formula: 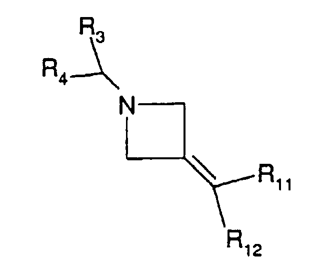 in which R₃, R₄, R₁₁ and R₁₂ have the same meanings as in claim 1, is reacted with a derivative HNR₂₀R₂₁ for which NR₂₀R₂₁ has the same meanings as in claim 1, the product isolated and optionally converted to a salt with an inorganic or organic acid

50. Process for preparing the compounds of formula (I) according to claim 1 for which R represents a radical CR₁R₂ in which R₁ represents a radical -alk-NR₁₈R₁₉, R₁₈ and R₁₉ represent hydrogen atoms,
**characterized in that** a corresponding compound of formula (I) for which R₁ represents a cyano radical is reduced, the product isolated and optionally converted to a salt with an inorganic or organic acid.

51. Process for preparing the compounds of formula (I) according to claim 1 for which R represents a radical CR₁R₂ in which R₁ represents a radical -alk-NR₁₈R₁₉, R₁₈ represents a hydrogen atom and R₁₉ represents an alkyl, cycloalkyl, cycloalkylalkyl, cycloalkylcarbonyl, -SO₂alk, -CO-NHalk or -COOalk radical, **characterized in that** a halide HalR₁₉, Hal represents a halogen, is reacted with a corresponding compound of formula (I) for which R represents a radical CR₁R₂ in which R₁ represents a radical -alk-NR₁₈R₁₉, R₁₈ and R₁₉ represent a hydrogen atom, the product isolated and optionally converted to a salt with an inorganic or organic acid.

52. Process for preparing the compounds of formula (I) according to claim 1 for which R represents a radical CR₁R₂ in which R₁ represents a radical -alk-NR₁₈R₁₉, R₁₈ represents an alkyl radical and R₁₉ represents an alkyl, cycloalkyl, cycloalkylalkyl, cycloalkylcarbonyl, -SO₂alk, -CO-NHalk or -COOalk radical, **characterized in that** an alkylhalide is reacted with a corresponding compound of formula (I) for which R represents a radical CR₁R₂ in which R₁ represents a radical -alk-NR₁₈R₁₉, R₁₈ represents a hydrogen atom, and R₁₉ represents an alkyl, cycloalkyl, cycloalkylalkyl, cycloalkylcarbonyl, -SO₂alk, -CO-NHalk or -COOalk radical, the product isolated and optionally converted to a salt with an inorganic or organic acid.

53. Process for preparing the compounds of formula (I) according to claim 1 for which R represents a radical CR₁R₂ in which either R₁ represents a hydrogen atom and R₂ represents a radical -C(R₈)(R₉)(R₁₀) or -C(R₈)(R₁₁)(R₁₂), or R₁ represents an alkyl, NH-R₁₅, cyano, -S-alk-NR₁₆R₁₇, -alk-NR₁₈R₁₉ or -NR₂₀R₂₁ radical and R₂ represents a radical -C(R₈)(R₁₁)(R₁₂) and R₈ represents an alkyl radical, **characterized in that** a corresponding compound of formula (I) for which R₈ is a hydrogen atom is alkylated, the product isolated and optionally converted to a salt with an inorganic or organic acid.

54. Process for preparing the compounds of formula (I) according to claim 1 for which R represents a radical C=C(R₇)SO₂alk, **characterized in that** a derivative of formula: 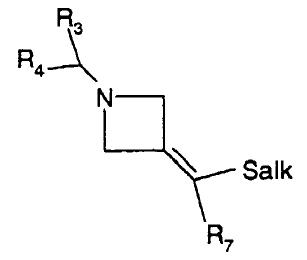 in which R₃, R₄ and R₇ have the same meanings as in claim 1 and alk represents an alkyl radical (1-6C in the form of a straight or branched chain), is oxidized, the product isolated and optionally converted to a salt with an inorganic or organic acid.

55. Process for preparing the compounds of formula (I) according to claim 1 for which R represents a radical CR₁R₂ in which R₁ is a hydrogen atom and R₂ represents a radical -CH(R₆)NR₃₁R₃₂, R₃₁ is a hydrogen atom and R₃₂ is an alkyl radical, **characterized in that** a corresponding compound of formula (I) for which R represents a radical CR₁R₂ in which R₁ is a hydrogen atom and R₂ represents a radical -CO-R₆ is reacted with an amine HNR₃₁R₃₂ for which R₃₁ is a hydrogen atom and R₃₂ is an alkyl radical, the product isolated and optionally converted to a salt with an inorganic or organic acid.

56. Pharmaceutical composition containing, as active ingredient, at least one compound of formula (I) according to one of claims 1 to 11.

57. Medicament containing, as active ingredient, at least one compound of formula (I) according to one of claims 1 to 11.

## Patentansprüche

1. Verbindung der Formel 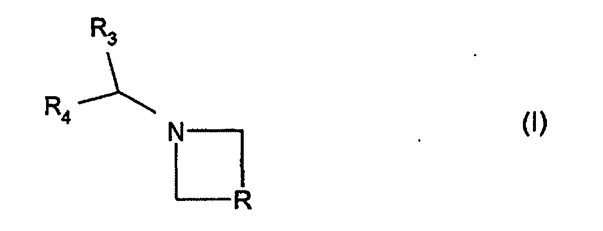 worin
R für einen CR₁R₂-, C=C(R₅)SO₂R₆- oder C=C(R₇)SO₂alk-Rest steht,
R₁ für ein Wasserstoffatom steht und R₂ für einen -C(R₈)(R₉)(R₁₀)-, -C(R₈)(R₁₁)(R₁₂)-, -CO-NR₁₃R₁₄-, -CH₂-CO-NR₁₃R₁₄-, -CH₂-CO-R₆-, -CO-R₆-, -CO-Cycloalkyl-, -SO-R₆-, -SO₂-R₆-, -C(OH)(R₁₂)(R₆)-, -C(OH)(R₆)(Alkyl)-, -C(=NOalk)R₆-, -C(=NO-CH₂-CH=CH₂)R₆-, -CH₂-CH(R₆)NR₃₁R₃₂-, -CH₂-C(=NOalk)R₆-, -CH(R₆)NR₃₁R₃₂-, -CH(R₆)NHSO₂alk-, -CH(R₆)NHCONHalk- oder -CH(R₆)NHCOalk-Rest steht
oder R₁ für einen Alkyl-, NH-R₁₅-, Cyano-, -S-alk-NR₁₆R₁₇-, -CH₂-NR₁₈R₁₉- oder -NR₂₀R₂₁-Rest steht und R₂ für einen -C(R₈)(R₁₁)(R₁₂)-Rest steht, R₃ und R₄ gleich oder verschieden sind und für einen Alkyl- oder Cycloalkylrest oder einen Aromaten, der unter Phenyl, Naphthyl oder Indenyl ausgewählt ist, wobei diese Aromaten unsubstituiert oder durch einen oder mehere Halogen-, Alkyl-, Alkoxy-, Formyl-, Hydroxyl-, Trifluormethyl-, Trifluormethoxy-, -CO-alk-, Cyano-, -COOH-, -COOalk-, -CONR₂₂R₂₃-, -CO-NH-NR₂₄R₂₅-Alkylsulfanyl-, Alkylsulfinyl-, Alkylsulfonyl-, Alkylsulfanylalkyl-, Alkylsulfinylalkyl-, Alkylsulfonylalkyl-, Hydroxyalkyl- oder -alk-NR₂₄R₂₅-Reste substituiert ist; oder einen Heteroaromaten, der unter Benzofuryl-, Benzothiazolyl-, Benzothienyl-, Benzoxazolyl-, Chromanyl-, 2,3-Dihydrobenzofuryl-, 2,3-Dihydrobenzothienyl-, Furyl-, Imidazolyl-, Isochromanyl-, Isochinolyl-, Pyrrolyl-, Pyridyl-, Pyrimidinyl-, Chinolyl-, 1,2,3,4-Tetrahydroisochinolyl-, Thiazolyl- und Thienylringen ausgewählt ist, wobei diese Heteroaromaten unsubstituiert oder durch einen oder mehrere Halogen-, Alkyl-, Alkoxy-, Hydroxyl-, Trifluormethyl-, Trifluormethoxy-, Cyano-, -COOH-, -COOalk-, -CO-NH-NR₂₄R₂₅-, -CONR₂₂R₂₃-, -alk-NR₂₄R₂₅-, Alkylsulfanyl-, Alkylsulfinyl-, Alkylsulfonyl-, Alkylsulfanylalkyl-, Alkylsulfinylalkyl-, Alkylsulfonylalkyl- oder Hydroxyalkylreste substituiert sein können, stehen,
R₅ für ein Wasserstoffatom oder einen Alkylrest steht,
R₆ für einen Ar- oder Het-Rest steht,
R₇ für einen gegebenenfalls durch einen CSO-PhenylRest substituierten Cycloalkyl-, Heterocycloalkyl-oder Heterocyclenylrest steht,
R₈ für ein Wasserstoffatom oder einen Alkylrest steht,
R₉ für einen -CO-NR₂₆R₂₇-, -COOH-, -COOalk-, -CH₂OH-, -NH-CO-NH-alk-, -CH₂-NHR₂₈- oder -NHCOOalk-Rest steht,
R₁₀ für einen Ar- oder Het-Rest steht,
R₁₁ für einen -SO₂-alk-, -SO₂-Ar- oder -SO₂-Het-Rest steht,
R₁₂ für ein Wasserstoffatom oder einen Ar- oder Het-Rest steht,
R₁₃ für ein Wasserstoffatom oder einen Alkylrest steht,
R₁₄ für einen Ar-, Het-, -alk-Ar- oder -alk-Het-Rest steht,
R₁₅ für einen Alkyl-, Cycloalkyl- oder -alk-NR₂₉R₃₀-Rest steht,
R₁₆ und R₁₇ gleich oder verschieden sind und für ein Wasserstoffatom oder einen Alkylrest stehen oder gemeinsam mit dem Stickstoffatom, an das sie gebunden sind, einen 3- bis 10-gliedrigen gesättigten oder ungesättigten mono- oder bicyclischen Heterocyclus bilden, der gegebenenfalls ein oder mehrere andere unter Sauerstoff, Schwefel und Stickstoff ausgewählte Heteroatome enthält und gegebenenfalls durch einen oder mehrere Alkylreste substituiert ist,
R₁₈ für ein Wasserstoffatom oder einen Alkylrest steht,
R₁₉ für ein Wasserstoffatom oder einen Alkyl-, Cycloalkyl-, Cycloalkylalkyl-, Cycloalkylcarbonyl-, -SO₂alk-, -CO-NHalk- oder -COOalk-Rest steht,
oder R₁₈ und R₁₉ gemeinsam mit dem Stickstoffatom, an das sie gebunden sind, einen 3- bis 10-gliedrigen gesättigten oder ungesättigten mono-oder bicyclischen Heterocyclus bilden, der gegebenenfalls ein oder mehrere andere unter Sauerstoff, Schwefel und Stickstoff ausgewählte Heteroatome enthält und gegebenenfalls durch einen oder mehrere Alkylreste substituiert ist,
-NR₂₀R₂₁ für einen 3- bis 8-gliedrigen gesättigten oder ungesättigten monocyclischen Heterocyclus steht, der gegebenenfalls ein anderes unter Sauerstoff, Stickstoff und Schwefel ausgewähltes Heteroatom enthält,
R₂₂ und R₂₃ gleich oder verschieden sind und für ein Wasserstoffatom oder einen Alkylrest stehen oder gemeinsam mit dem Stickstoffatom, an das sie gebunden sind, einen 3- bis 10-gliedrigen gesättigten mono- oder bicyclischen Heterocyclus bilden, der gegebenenfalls ein anderes unter Sauerstoff, Schwefel und Stickstoff ausgewähltes Heteroatom enthält und gegebenenfalls durch einen oder mehrere Alkylreste substituiert ist,
R₂₄ und R₂₅ gleich oder verschieden sind und für ein Wasserstoffatom oder einen Alkyl-, -COOalk-, Cycloalkyl-, Alkylcycloalkyl-, -alk-O-alk- oder Hydroxyalkylrest stehen oder gemeinsam mit dem Stickstoffatom, an das sie gebunden sind, einen 3-bis 10-gliedrigen gesättigten oder ungesättigten mono- oder bicyclischen Heterocyclus bilden, der gegebenenfalls ein anderes unter Sauerstoff, Schwefel und Stickstoff ausgewähltes Heteroatom enthält und gegebenenfalls durch einen oder mehrere Alkyl-, -COalk-, -COOalk-, CO-NHalk-, CS-NH-alk-, Oxo-, Hydroxyalkyl-, -alk-O-alk- oder -CO-NH₂-Reste substituiert ist,
R₂₆ und R₂₇ gleich oder verschieden sind und für ein Wasserstoffatom oder einen Alkyl-, Hydroxyalkyl-, Cycloalkyl-, Cycloalkylalkyl-, -alk-COOalk-, -alk-Ar-, -alk-Het-, Het- oder -alk-N(alk)₂-Rest stehen oder mit dem Stickstoffatom, an das sie gebunden sind, einen 3- bis 10-gliedrigen gesättigten oder ungesättigten mono- oder bicyclischen Heterocyclus bilden können, der gegebenenfalls ein oder mehrere andere unter Sauerstoff, Schwefel und Stickstoff ausgewählte Heteroatome enthält und gegebenenfalls durch einen oder mehrere Alkyl-, Alkoxy- oder Halogenreste substituiert ist,
R₂₈ für einen -CH₂-alk-, Benzyl-, -SO₂alk-, -CONHalk-, -COalk-, Cycloalkylalkylcarbonyl-, Cycloalkylcarbonyl- oder -CO-(CH₂)ₙOH-Rest steht,
n gleich 1, 2 oder 3 ist,
R₂₉ und R₃₀ gleich oder verschieden sind und für ein Wasserstoffatom oder einen Alkylrest stehen oder gemeinsam mit dem Stickstoffatom, an das sie gebunden sind, einen 3- bis 10-gliedrigen gesättigten mono- oder bicyclischen Heterocyclus bilden, der gegebenenfalls ein anderes unter Sauerstoff, Schwefel und Stickstoff ausgewähltes Heteroatom enthält und gegebenenfalls durch einen oder mehrere Alkylreste substituiert ist,
R₃₁ und R₃₂ gleich oder verschieden sind und für ein Wasserstoffatom oder einen Alkyl-, Ar- oder -alk-Ar-Rest stehen oder gemeinsam mit dem Stickstoffatom, an das sie gebunden sind, einen unter Aziridinyl, Azetidinyl, Pyrrolidinyl und Piperidinyl ausgewählten Heterocyclus bilden, alk für einen Alkyl- oder Alkylenrest steht,
Ar für einen Phenyl- oder Naphthylrest steht, der gegebenfalls durch einen oder mehrere unter Halogen, Alkyl, Alkoxy, -CO-alk, Cyano, -COOH, -COOalk, -CONR₂₂R₂₃, -CO-NH-NR₂₄R₂₅, Alkylsulfanyl, Alkylsulfinyl, Alkylsulfonyl, Alkylsulfanylalkyl, Alkylsulfinylalkyl, Alkylsulfonylalkyl, Hydroxyalkyl, -alk-NR₂₄R₂₅, -NR₂₄R₂₅, Alkylthioalkyl, Formyl, Hydroxyl, CF₃, OCF₃, Het, -O-alk-NH-Cycloalkyl oder SO₂NH₂ ausgewählte Substituenten substituiert ist,
Het für einen 3- bis 10-gliedrigen gesättigten oder ungesättigten mono- oder bicyclischen Ring steht, der ein oder mehrere unter Sauerstoff, Schwefel und Stickstoff ausgewählte Heteroatome enthält und gegebenenfalls durch einen oder mehrere Halogen-, Alkyl-, Alkoxy-, Alkoxycarbonyl-, -CONR₂₂R₂₃-, Hydroxyl-, Hydroxyalkyl-, Oxo- oder SO₂NH₂-Reste substituiert ist,
wobei die Alkyl- und Alkylenreste oder -teile und die Alkoxyreste und -teile gerad-oder verzweigtkettig sind und 1 bis 6 Kohlenstoffatome enthalten, die Cycloalkylreste 3 bis 10 Kohlenstoffatome enthalten und die Heterocycloalkyl- und Heterocyclenylreste 3 bis 10 Kohlenstoffatome enthalten,
ihre optischen Isomere und ihre Salze mit einer anorganischen oder organischen Säure.

2. Verbindung nach Anspruch 1, für die Het unter Benzofuryl, Benzothiazolyl, Benzothienyl, Benzoxazolyl, Chromanyl, 2,3-Dihydrobenzofuryl, 2,3-Dihydrobenzothienyl, Furyl, Indolinyl, Indolyl, Isochromanyl, Isochinolyl, Piperidyl, Pyrrolyl, Pyridyl, Pyrimidinyl, Chinolyl, 1,2,3,4-Tetrahydroisochinolyl, 1,2,3,4-Tetrahydrochinolyl, Thiazolyl und Thienyl ausgewählt ist.

3. Verbindung der Formel (I) nach Anspruch 1, für die dann, wenn R₁₆ und R₁₇ gemeinsam mit dem Stickstoffatom, an das sie gebunden sind, einen 3-bis 10-gliedrigen gesättigten oder ungesättigten mono- oder bicyclischen Heterocyclus bilden, dieser ein Azetidinyl-, Pyrrolidinyl-, Piperidinyl-, Morpholinyl-, Thiamorpholinyl- oder Piperazinylring ist.

4. Verbindung der Formel (I) nach Anspruch 1, für die dann, wenn R₁₈ und R₁₉ gemeinsam mit dem Stickstoffatom, an das sie gebunden sind, einen 3-bis 10-gliedrigen gesättigten oder ungesättigten mono- oder bicyclischen Heterocyclus bilden, dieser ein Azetidinyl-, Pyrrolidinyl-, Piperidinyl-, Morpholinyl-, Thiamorpholinyl- oder Piperazinylring ist.

5. Verbindung der Formel (I) nach Anspruch 1, für die der durch -NR₂₀R₂₁ gebildete Heterocyclus ein Azetidinyl-, Pyrrolidinyl-, Piperidinyl-, Morpholinyl-, Thiamorpholinyl-, Piperazinyl- oder Imidazolylring ist.

6. Verbindung der Formel (I) nach Anspruch 1, für die dann, wenn R₂₂ und R₂₃ gemeinsam mit dem Stickstoffatom, an das sie gebunden sind, einen 3-bis 10-gliedrigen gesättigten oder ungesättigten mono- oder bicyclischen Heterocyclus bilden, dieser ein Azetidinyl-, Pyrrolidinyl-, Piperidinyl-, Morpholinyl-, Thiamorpholinyl- oder Piperazinylring ist.

7. Verbindung der Formel (I) nach Anspruch 1, für die dann, wenn R₂₄ und R₂₅ gemeinsam mit dem Stickstoffatom, an das sie gebunden sind, einen 3-bis 10-gliedrigen gesättigten oder ungesättigten mono- oder bicyclischen Heterocyclus bilden, dieser ein Azetidinyl-, Pyrrolidinyl-, Piperidinyl-, Morpholinyl-, Thiamorpholinyl- oder Piperazinylring ist.

8. Verbindung der Formel (I) nach Anspruch 1, für die dann, wenn R₂₆ und R₂₇ gemeinsam mit dem Stickstoffatom, an das sie gebunden sind, einen 3-bis 10-gliedrigen gesättigten oder ungesättigten mono- oder bicyclischen Heterocyclus bilden, dieser ein Azetidinyl-, Pyrrolidinyl-, Piperidinyl-, Morpholinyl-, Thiamorpholinyl- oder Piperazinylring ist.

9. Verbindung der Formel (I) nach Anspruch 1, für die dann, wenn R₂₉ und R₃₀ gemeinsam mit dem Stickstoffatom, an das sie gebunden sind, einen 3-bis 10-gliedrigen gesättigten oder ungesättigten mono- oder bicyclischen Heterocyclus bilden, dieser ein Azetidinyl-, Pyrrolidinyl-, Piperidinyl-, Morpholinyl-, Thiamorpholinyl- oder Piperazinylring ist.

10. Verbindung der Formel (I) nach Anspruch 1, für die
R für einen CR₁R₂-Rest steht,
R₁ für ein Wasserstoffatom steht und R₂ für einen -C(R₈)(R₁₁)(R₁₂)- oder C(R₈)(R₉)(R₁₀)-Rest steht
oder R₁ für einen Alkyrest steht und R₂ für einen -C(R₈)(R₁₁)(R₁₂) -Rest steht,
R₃ und R₄ gleich oder verschieden sind und für einen Phenylrest, der unsubstituiert oder durch einen oder mehere Halogen-, Alkyl-, Alkoxy-, Trifluormethyl-, Trifluormethoxy-, Cyano-, -CONR₂₂R₂₃-, Hydroxyalkyl- oder -alk-NR₂₄R₂₅-Reste substituiert ist; oder einen Heteroaromaten, der unter Pyridyl-, Pyrimidinyl-, Thiazolyl- und Thienylringen ausgewählt ist, wobei diese Heteroaromaten unsubstituiert oder durch einen oder mehrere Halogen-, Alkyl-, Alkoxy-, Hydroxy-, Trifluormethyl-, Trifluormethoxy-, Cyano-, -CONR₂₂R₂₃-, -alk-NR₂₄R₂₅- oder Hydroxyalkylreste substituiert sein können, stehen,
R₈ für ein Wasserstoffatom steht,
R₉ für einen -CO-NR₂₆R₂₇-, -COOalk-, -CH₂OH-, -NH-CO-NH-alk-, CH₂-NHR₂₈- oder -NHCOOalk-Rest steht,
R₁₀ für einen Ar- oder Het-Rest steht,
R₁₁ für einen -SO₂-alk-, -SO₂Ar- oder -SO₂Het-Rest steht,
R₁₂ für ein Wasserstoffatom oder einen Ar- oder Het-Rest steht,
R₂₂ und R₂₃ gleich oder verschieden sind und für ein Wasserstoffatom oder einen Alkylrest stehen oder gemeinsam mit dem Stickstoffatom, an das sie gebunden sind, einen 3- bis 10-gliedrigen gesättigten mono- oder bicyclischen Heterocyclus bilden, der gegebenenfalls ein anderes unter Sauerstoff, Schwefel und Stickstoff ausgewähltes Heteroatom enthält und gegebenenfalls durch einen oder mehrere Alkylreste substituiert ist,
R₂₄ und R₂₅ gleich oder verschieden sind und für ein Wasserstoffatom oder einen Alkyl-, Cycloalkyl-, Alkylcycloalkyl- oder Hydroxyalkylrest stehen oder gemeinsam mit dem Stickstoffatom, an das sie gebunden sind, einen 3- bis 10-gliedrigen gesättigten oder ungesättigten mono- oder bicyclischen Heterocyclus bilden, der gegebenenfalls ein anderes unter Sauerstoff, Schwefel und Stickstoff ausgewähltes Heteroatom enthält und gegebenenfalls durch einen oder mehrere Alkyl-, -COalk-, -COOalk-, CO-NHalk-, CS-NH-alk-, Oxo-, oder -CO-NH₂-Reste substituiert ist,
Ar für einen Phenyl- oder Naphthylrest steht, der gegebenfalls durch 1 oder 2 unter Halogen, Alkyl, Alkoxy, -CO-alk, Cyano, -COOalk, -CONR₂₂R₂₃, Alkylsulfonyl, Hydroxyalkyl, -alk-NR₂₄R₂₅, -NR₂₄R₂₅, Hydroxy, CF₃, OCF₃, -O-alk-NH-Cycloalkyl oder SO₂NH₂ ausgewählte Substituenten substituiert ist,
Het für einen Benzofuryl-, Benzothiazolyl-, Benzothienyl-, Benzoxazolyl-, Furyl-, Isochinolyl-, Pyrrolyl-, Pyridyl-, Chinolyl-, 1,2,3,4-Tetrahydroisochinolyl-, 1,2,3,4-Tetrahydrochinolyl-, Thiazolyl- oder Thienylring steht,
ihre optischen Isomere und ihre Salze mit einer anorganischen oder organischen Säure.

11. Verbindung, ausgewählt unter den folgenden Verbindungen:
(RS)-1-[Bis(4-chlorphenyl)methyl)]-3-[(3,5-difluorphenyl)(methylsulfonyl)methyl]azetidin,
(R)-1-[Bis(4-chlorphenyl)methyl)]-3-[(3,5-difluorphenyl)(methylsulfonyl)methyl]azetidin,
(S)-1-[Bis(4-chlorphenyl)methyl)]-3-[(3,5-difluorphenyl)(methylsulfonyl)methyl]azetidin,
(RS)-1-[Bis(4-chlorphenyl)methyl)]-3-[(pyrid-3-yl)(methylsulfonyl)methyl]azetidin,
(R)-1-[Bis(4-chlorphenyl)methyl)]-3-[(pyrid-3-yl)-(methylsulfonyl)methyl]azetidin,
(S)-1-[Bis(4-chlorphenyl)methyl)]-3-[(pyrid-3-yl)-(methylsulfonyl)methyl]azetidin,
(RS)-1-[Bis(3-fluorphenyl)methyl]-3-[(3,5-difluorphenyl)methylsulfonylmethyl]azetidin,
(R)-1-[Bis(3-fluorphenyl)methyl]-3-[(3,5-difluorphenyl)methylsulfonylmethyl]azetidin,
(S)-1-[Bis(3-fluorphenyl)methyl]-3-[(3,5-difluorphenyl)methylsulfonylmethyl]azetidin,
1-[Bis(4-chlorphenyl)methyl]-3-(RS)-{[3-azetidin-1-ylphenyl]methylsulfonylmethyl}azetidin,
1-[Bis(4-chlorphenyl)methyl]-3-(R)-{[3-azetidin-1-ylphenyl]methylsulfonylmethyl}azetidin,
1-[Bis(4-chlorphenyl)methyl]-3-(S)-{[3-azetidin-1-ylphenyl]methylsulfonylmethyl}azetidin,
(RS)-1-[3({1-[Bis(4-chlorphenyl)methyl]azetidin-3-yl}methylsulfonylmethyl)phenyl]pyrrolidin,
(R)-1-[3-({1-[Bis(4-chlorphenyl)methyl]azetidin-3-yl}methylsulfonylmethyl)phenyl]pyrrolidin,
(S)-1-[3-({1-[Bis(4-chlorphenyl)methyl]azetidin-3-yl}methylsulfonylmethyl)phenyl]pyrrolidin,
(RS)-N-[3-({1-[Bis(4-chlorphenyl)methyl]azetidin-3-yl}methylsulfonylmethyl)phenyl]-N-methylamin,
(R)-N-[3-({1-[Bis(4-chlorphenyl)methyl]azetidin-3-yl}methylsulfonylmethyl)phenyl]-N-methylamin,
(S)-N-[3-({1-[Bis(4-chlorphenyl)methyl]azetidin-3-yl}methylsulfonylmethyl)phenyl]-N-methylamin,
(RS)-1-[Bis(4-chlorphenyl)methyl]-3-[(3,5-bistri-fluormethylphenyl)methylsulfonylmethyl]azetidin,
(R)-1-[Bis(4-chlorphenyl)methyl]-3-[(3,5-bistri-fluormethylphenyl)methylsulfonylmethyl]azetidin,
(S)-1-[Bis(4-chlorphenyl)methyl]-3-[(3,5-bistri-fluormethylphenyl)methylsulfonylmethyl]azetidin,
1-[Bis(4-chlorphenyl)methyl]-3-(phenylsulfonyl-methyl)azetidin,
(RS)-1-[Bis(4-chlorphenyl)methyl]-3-[(3,5-difluorphenyl)methylsulfonylmethyl]-3-methylazetidin,
(R)-1-[Bis(4-chlorphenyl)methyl]-3-[(3,5-difluorphenyl)methylsulfonylmethyl]-3-methylazetidin,
(S)-1-[Bis(4-chlorphenyl)methyl]-3-[(3,5-difluorphenyl)methylsulfonylmethyl]-3-methylazetidin,
(RS)-2-{1-[Bis(4-chlorphenyl)methyl]azetidin-3-yl}-2-(3,5-difluorphenyl)-N-cyclohexylacetamide,
(R)-2-{1-[Bis(4-chlorphenyl)methyl]azeditin-3-yl}-2-(3,5-difluorphenyl)-N-cyclohexylacetamid,
(S)-2-{1-[Bis(4-chlorphenyl)methyl]azetidin-3-yl}-2-(3,5-difluorphenyl)-N-cyclohexylacetamid,
(RS)-2-{1-[Bis(4-chlorphenyl)methyl]azetidin-3-yl}-2-(3,5-difluorphenyl)-N-isobutylacetamid,
(R)-2-{1-[Bis(4-chlorphenyl)methyl]azetidin-3-yl}-2-(3,5-difluorphenyl)-N-isobutylacetamid,
(S)-2-{1-[Bis(4-chlorphenyl)methyl]azetidin-3-yl}-2-(3,5-difluorphenyl)-N-isobutylacetamid,
(RS)-2-{1-[Bis(4-chlorphenyl)methyl]azetidin-3-yl}-2-(3,5-difluorphenyl)-N-cyclopropylmethylacetamid,
(R)-2-{1-[Bis(4-chlorphenyl)methyl]azetidin-3-yl}-2-(3,5-difluorphenyl)-N-cyclopropylmethylacetamid,
(S)-2-{1-[Bis(4-chlorphenyl)methyl]azetidin-3-yl}-2-(3,5-difluorphenyl)-N-cyclopropylmethylacetamid,
(RS)-2-{1-[Bis(4-chlorphenyl)methyl]azetidin-3-yl} -2-(3,5-difluorphenyl)-N-isopropylacetamid,
(R)-2-{1-[Bis(4-chlorphenyl)methyl]azetidin-3-yl}-2-(3,5-difluorphenyl)-N-isopropylacetamid,
(S)-2-{1-[Bis(4-chlorphenyl)methyl]azetidin-3-yl}-2-(3,5-difluorphenyl)-N-isopropylacetamid,
(RS)-1-[Bis(4-chlorphenyl)methyl]-3-[1-(3,5-difluorphenyl)-1-methylsulfonylethyl]azetidin,
(R)-1-[Bis(4-chlorphenyl)methyl]-3-[1-(3,5-difluorphenyl)-1-methylsulfonylethyl]azetidin,
(S)-1-[Bis(4-chlorphenyl)methyl]-3-[1-(3,5-difluorphenyl)-1-methylsulfonylethyl]azetidin,
(RS)-1-[Bis(4-fluorphenyl)methyl]-3-[(3,5-difluorphenyl)methylsulfonylmethyl]azetidin,
(R)-1-[Bis(4-fluorphenyl)methyl]-3-[(3,5-difluorphenyl)methylsulfonylmethyl]azetidin,
(S)-1-[Bis(4-fluorphenyl)methyl]-3-[(3,5-difluorphenyl)methylsulfonylmethyl]azetidin,
(RS)-{1-[(3-Pyridyl)-(4-chlorphenyl)methyl]-3-[(3,5-difluorphenyl)methylsulfonylmethyl]azetidin,
(SS)-{1-[(3-Pyridyl)-(4-chlorphenyl)methyl]-3-[(3,5-difluorphenyl)methylsulfonylmethyl]azetidin,
(RR)-{1-[(3-Pyridyl)-(4-chlorphenyl)methyl]-3-[(3,5-difluorphenyl)methylsulfonylmethyl]azetidin,
(SR)-{1-[(3-Pyridyl)-(4-chlorphenyl)methyl]-3-[(3,5-difluorphenyl)methylsulfonylmethyl]azetidin,
(RS)-{1-[(4-Pyridyl)-(4-chlorphenyl)methyl]-3-[(3,5-difluorphenyl)methylsulfonylmethyl]azetidin,
(SS)-{1-[(4-Pyridyl)-(4-chlorphenyl)methyl]-3-[(3,5-difluorphenyl)methylsulfonylmethyl]azetidin,
(RR)-{1-[(4-Pyridyl)-(4-chlorphenyl)methyl]-3-[(3,5-difluorphenyl)methylsulfonylmethyl]azetidin,
(SR)-{1-[(4-Pyridyl)-(4-chlorphenyl)methyl]-3-[(3,5-difluorphenyl)methylsulfonylmethyl]azetidin,
(RS)-5-((4-Chlorphenyl)-{3-[(3,5-difluorphenyl)-methylsulfonylmethyl]azetidin-1-yl}methyl)-pyrimidin,
(SR)-5-((4-Chlorphenyl)-{3-[(3,5-difluorphenyl)-methylsulfonylmethyl]azetidin-1-yl}methyl)-pyrimidin,
(RR)-5-((4-Chlorphenyl)-{3-[(3,5-difluorphenyl)-methylsulfonylmethyl]azetidin-1-yl}methyl)-pyrimidin,
(SS)-5-((4-Chlorphenyl)-{3-[(3,5-difluorphenyl)-methylsulfonylmethyl]azetidin-1-yl}methyl)-pyrimidin,
(SS)-{1-[(2-Chlorpyrid-5-yl)-(4-chlorphenyl)-methyl]-3-[(3,5-difluorphenyl)methylsulfonyl-methyl]azetidin,
(RR)-{1-[(2-Chlorpyrid-5-yl)-(4-chlorphenyl)-methyl]-3-[(3,5-difluorphenyl)methylsulfonyl-methyl]azetidin,
(RS)-{1-[(2-Chlorpyrid-5-yl)-(4-chlorphenyl)-methyl]-3-[(3,5-difluorphenyl)methylsulfonyl-methyl]azetidin,
(SR)-{1-[(2-Chlorpyrid-5-yl)-(4-chlorphenyl)-methyl]-3-[(3,5-difluorphenyl)methylsulfonyl-methyl]azetidin,
ihre optischen Isomere und ihre Salze mit einer anorganischen oder organischen Säure.

12. Verfahren zur Herstellung von Verbindungen der Formel (I) nach Anspruch 1, für die R für einen CR₁R₂-Rest steht, worin R₁ für ein Wasserstoffatom steht und R₂ für einen C(R₈)(R₁₁)(R₁₂)-Rest steht, worin R₈ für ein Wasserstoffatom steht, R₁₁ für einen -SO₂-Ar-, -SO₂-Het- oder -SO₂alk-Rest steht und R₁₂ für ein Wasserstoffatom oder einen Ar- oder Het-Rest steht, **dadurch gekennzeichnet, daß** man ein Derivat der Formel: 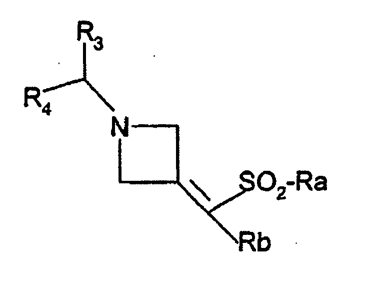 worin Ra für einen Alkyl-, Het- oder Ar-Rest steht und Rb für ein Wasserstoffatom oder einen Ar- oder Het-Rest steht, wobei Alkyl, Ar und Het die gleichen Bedeutungen wie in Anspruch 1 besitzen, reduziert und das Produkt isoliert und gegebenenfalls in ein Salz mit einer anorganischen oder organischen Säure umwandelt.

13. Verfahren zur Herstellung von Verbindungen der Formel (I) nach Anspruch 1, für die R für einen CR₁R₂-Rest steht, worin R₁ für ein Wasserstoffatom steht und R₂ für einen C(R₈)(R₁₁)(R₁₂)-Rest steht, worin R₈ für ein Wasserstoffatom steht, R₁₁ für einen -SO₂-Ar-, -SO₂-Het- oder -SO₂alk-Rest steht und R₁₂ für ein Wasserstoffatom oder einen Ar- oder Het-Rest steht, **dadurch gekennzeichnet, daß** man ein Derivat R₃CH(Br)R₄ mit einem Derivat der Formel: 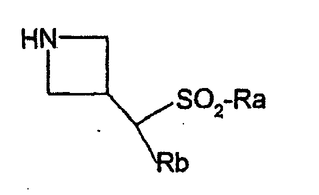 worin Ra für einen Alkyl-, Het- oder Ar-Rest steht und Rb für ein Wasserstoffatom oder einen Ar- oder Het-Rest steht, wobei Alkyl, Ar und Het die gleichen Bedeutungen wie in Anspruch 1 besitzen, umsetzt und das Produkt isoliert und gegebenenfalls in ein Salz mit einer anorganischen oder organischen Säure umwandelt.

14. Verfahren zur Herstellung von Verbindungen der Formel (I) nach Anspruch 1, für die R für einen C=C(R₅)SO₂R₆- oder C=C(R₇)SO₂alk-Rest steht, **dadurch gekennzeichnet, daß** man ein Derivat der Formel: 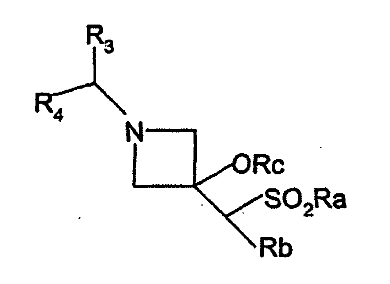 worin Ra für einen Ar- oder Het-Rest steht und Rb für ein Wasserstoffatom oder einen Alkyrest steht oder Ra für einen Alkylrest steht und Rb für einen Cycloalkyl-, Heterocycloalkyl- oder Heterocyclenylrest steht, der gegebenenfalls durch einen -CSO-Phenylrest substituiert ist, Rc für ein Wasserstoffatom oder einen Acetylrest steht, wobei R₃, R₄, Ar und Het die gleichen Bedeutungen wie in Anspruch 1 besitzen, dehydratisiert und das Produkt isoliert und gegebenenfalls in ein Salz mit einer anorganischen oder organischen Säure umwandelt.

15. Verfahren zur Herstellung von Verbindungen der Formel (I) nach Anspruch 1, für die R für einen C=C(R₅)SO₂R₆- oder C=C(R₇)SO₂alk-Rest steht, **dadurch gekennzeichnet, daß** man R₃CH(Br)R₄ mit einem Derivat der Formel: 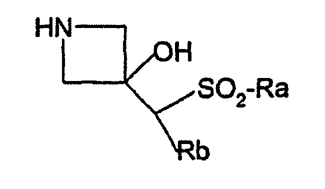 worin Ra für einen Ar- oder Het-Rest steht und Rb für ein Wasserstoffatom oder einen Alkyrest steht oder Ra für einen Alkylrest steht und Rb für einen Cycloalkyl-, Heterocycloalkyl- oder Heterocyclenylrest steht, der gegebenenfalls durch einen -CSO-Phenylrest substituiert ist, Rc für ein Wasserstoffatom oder einen Acetylrest steht, wobei R₃, R₄, Ar und Het die gleichen Bedeutungen wie in Anspruch 1 besitzen, umsetzt und das Produkt isoliert und gegebenenfalls in ein Salz mit einer anorganischen oder organischen Säure umwandelt.

16. Verfahren zur Herstellung von Verbindungen der Formel (I) nach Anspruch 1, für die R für einen CR₁R₂-Rest steht, worin R₁ für ein Wasserstoffatom steht und R₂ für einen C(R₈)(R₉)(R₁₀)-Rest steht, worin R₈ für ein Wasserstoffatom steht, R₉ für einen -CO-NR₂₆R₂₇-Rest steht und R₁₀ für einen Ar-oder Het-Rest steht, **dadurch gekennzeichnet, daß** man ein Amin HNR₂₆R₂₇, worin R₂₆ und R₂₇ die gleichen Bedeutungen wie in Anspruch 1 besitzen, mit einer Säure der Formel: 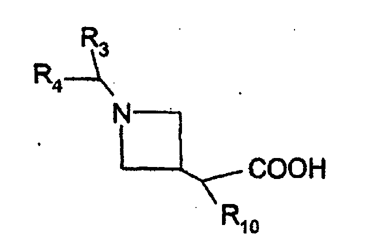 worin R₃, R₄ und R₁₀ die gleichen Bedeutungen wie in Anspruch 1 besitzen, umsetzt und das Produkt isoliert und gegebenenfalls in ein Salz mit einer anorganischen oder organischen Säure umwandelt.

17. Verfahren zur Herstellung von Verbindungen der Formel (I) nach Anspruch 1, für die R für einen CR₁R₂-Rest steht, worin R₁ für ein Wasserstoffatom steht und R₂ für einen C(R₈)(R₉)(R₁₀)-Rest steht, worin R₈ für ein Wasserstoffatom steht, R₉ für einen -COOH-Rest steht und R₁₀ für einen Ar- oder Het-Rest steht, **dadurch gekennzeichnet, daß** man einen Ester der Formel: 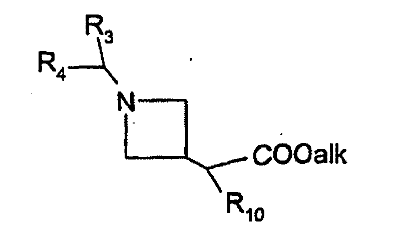 worin R₃, R₄ und R₁₀ die gleichen Bedeutungen wie in Anspruch 1 besitzen und alk für einen gerad- oder verzweigtkettigen C₁₋₆-Alkylrest steht, hydrolysiert und das Produkt isoliert und gegebenenfalls in ein Salz mit einer anorganischen oder organischen Säure umwandelt.

18. Verfahren zur Herstellung von Verbindungen der Formel (I) nach Anspruch 1, für die R für einen CR₁R₂-Rest steht, worin R₁ für ein Wasserstoffatom steht und R₂ für einen C(R₈)(R₉)(R₁₀)-Rest steht, worin R₈ für ein Wasserstoffatom steht, R₉ für einen -COOalk- oder -CH₂OH-Rest steht und R₁₀ für einen Ar- oder Het-Rest steht, **dadurch gekennzeichnet, daß** man eine Verbindung der Formel: 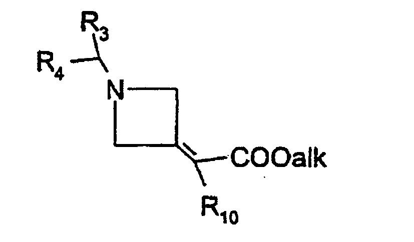 worin R₃, R₄ und R₁₀ die gleichen Bedeutungen wie in Anspruch 1 besitzen und alk für einen gerad- oder verzweigtkettigen C₁₋₆-Alkylrest steht, reduziert und das Produkt isoliert und gegebenenfalls in ein Salz mit einer anorganischen oder organischen Säure umwandelt.

19. Verfahren zur Herstellung von Verbindungen der Formel (I) nach Anspruch 1, für die R für einen CR₁R₂-Rest steht, worin R₁ für ein Wasserstoffatom steht und R₂ für einen C(R₈)(R₉)(R₁₀)-Rest steht, worin R₈ für ein Wasserstoffatom steht, R₉ für einen -NHCOOalk-Rest steht und R₁₀ für einen Ar-oder Het-Rest steht, **dadurch gekennzeichnet, daß** man einen Alkohol der Formel alkOH, für den alk für einen gerad- oder verzweigtkettigen C₁₋₆-Alkylrest steht, mit einem Derivat der Formel: 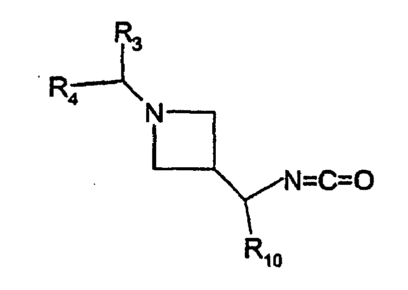 worin R₃, R₄ und R₁₀ die gleichen Bedeutungen wie in Anspruch 1 besitzen, umsetzt und das Produkt isoliert und gegebenenfalls in ein Salz mit einer anorganischen oder organischen Säure umwandelt.

20. Verfahren zur Herstellung von Verbindungen der Formel (I) nach Anspruch 1, für die R für einen CR₁R₂-Rest steht, worin R₁ für ein Wasserstoffatom steht und R₂ für einen C(R₈)(R₉)(R₁₀)-Rest steht, worin R₈ für ein Wasserstoffatom steht, R₉ für einen -NH-CO-NH-alk-Rest steht und R₁₀ für einen Ar- oder Het-Rest steht, **dadurch gekennzeichnet, daß** man ein Amin der Formel alkNH₂, für das alk für einen gerad- oder verzweigtkettigen C₁₋₆-Alkylrest steht, mit einem Derivat der Formel: 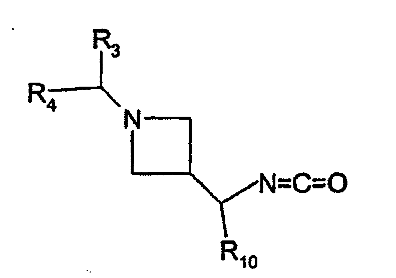 worin R₃, R₄ und R₁₀ die gleichen Bedeutungen wie in Formel (I) besitzen, umsetzt und das Produkt isoliert und gegebenenfalls in ein Salz mit einer anorganischen oder organischen Säure umwandelt.

21. Verfahren zur Herstellung von Verbindungen der Formel (I) nach Anspruch 1, für die R für einen CR₁R₂-Rest steht, worin R₁ für ein Wasserstoffatom steht und R₂ für einen -C(R₈)(R₉)(R₁₀)-Rest steht, worin R₈ für ein Wasserstoffatom steht, R₉ für einen -CH₂-NHR₂₈-Rest steht, R₂₈ für einen -CH₂-alk-oder Benzylrest steht und R₁₀ für einen Ar- oder Het-Rest steht, **dadurch gekennzeichnet, daß** man ein Derivat der Formel: 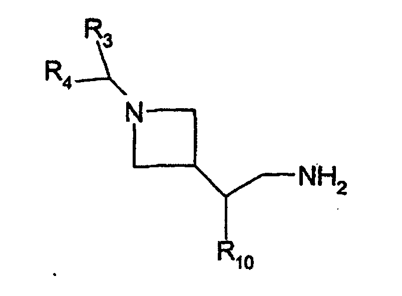 worin R₃, R₄ und R₁₀ die gleichen Bedeutungen wie in Anspruch 1 besitzen, mit einem Aldehyd RdCHO, für den Rd für einen -CH₂-alk- oder Benzylrest steht und alk für einen gerad- oder verzweigtkettigen C₁₋₆-Alkylrest steht, umsetzt und das Produkt isoliert und gegebenenfalls in ein Salz mit einer anorganischen oder organischen Säure umwandelt.

22. Verfahren zur Herstellung von Verbindungen der Formel (I) nach Anspruch 1, für die R für einen CR₁R₂-Rest steht, worin R₁ für ein Wasserstoffatom steht und R₂ für einen -C(R₈)(R₉)(R₁₀)-Rest steht, worin R₈ für ein Wasserstoffatom steht, R₉ für einen -CH₂-NHR₂₈-Rest steht, R₂₈ für einen -SO₂-alk-Rest steht und R₁₀ für einen Ar- oder Het-Rest steht, **dadurch gekennzeichnet, daß** man ein Derivat der Formel: 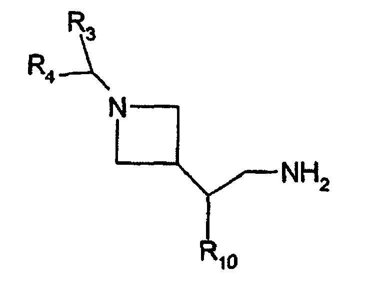 worin R₃, R₄ und R₁₀ die gleichen Bedeutungen wie in Anspruch 1 besitzen, mit einem Derivat ClSO₂Re, für das Re für einen gerad- oder verzweigtkettigen C₁₋₆-Alkylrest steht, umsetzt und das Produkt isoliert und gegebenenfalls in ein Salz mit einer anorganischen oder organischen Säure umwandelt.

23. Verfahren zur Herstellung von Verbindungen der Formel (I) nach Anspruch 1, für die R für einen CR₁R₂-Rest steht, worin R₁ für ein Wasserstoffatom steht und R₂ für einen -C(R₈)(R₉)(R₁₀)-Rest steht, worin R₈ für ein Wasserstoffatom steht, R₉ für einen -CH₂-NHR₂₈-Rest steht, R₂₈ für einen -CO-NH-alk-Rest steht und R₁₀ für einen Ar- oder Het-Rest steht, **dadurch gekennzeichnet, daß** man ein Derivat der Formel: 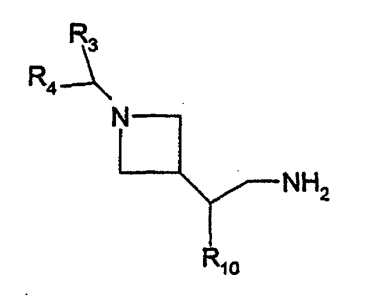 worin R₃, R₄ und R₁₀ die gleichen Bedeutungen wie in Anspruch 1 besitzen, mit einem Derivat RfNCO, für das Rf für einen gerad- oder verzweigtkettigen C₁₋₆-Alkylrest steht, umsetzt und das Produkt isoliert und gegebenenfalls in ein Salz mit einer anorganischen oder organischen Säure umwandelt.

24. Verfahren zur Herstellung von Verbindungen der Formel (I) nach Anspruch 1, für die R für einen CR₁R₂-Rest steht, worin R₁ für ein Wasserstoffatom steht und R₂ für einen -C(R₈)(R₉)(R₁₀)-Rest steht, worin R₈ für ein Wasserstoffatom steht, R₉ für einen -CH₂-NHR₂₈-Rest steht, R₂₈ für einen -COalk-, Cycloalkylalkylcarbonyl-, Cyclolalkylcarbonyl-oder -CO-(CH₂)ₙOH- Rest steht und R₁₀ für einen Ar-oder Het-Rest steht, **dadurch gekennzeichnet, daß** man ein Derivat der Formel: 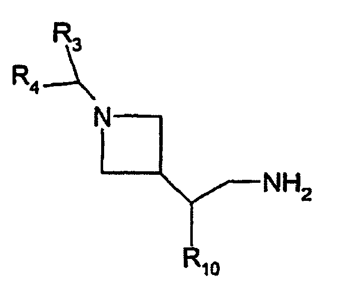 worin R₃, R₄ und R₁₀ die gleichen Bedeutungen wie in Anspruch 1 besitzen, mit einer Säure HOOCRg, für die Rg für einen gerad- oder verzweigtkettigen C₁₋₆-Alkylrest, einen C₃₋₁₀-Cycloalkyl-C₁₋₆-alkylrest mit gerad- oder verzweigtkettigem C₁₋₆-Alkylrest, einen C₃₋₁₀-Cycloalkylrest oder einen -(CH₂)ₙOH-Rest steht und n gleich 1, 2 oder 3 ist, umsetzt und das Produkt isoliert und gegebenenfalls in ein Salz mit einer anorganischen oder organischen Säure umwandelt.

25. Verfahren zur Herstellung von Verbindungen der Formel (I) nach Anspruch 1, für die R für einen CR₁R₂-Rest steht, worin R₁ für ein Wasserstoffatom steht und R₂ für einen CONR₁₃R₁₄-Rest steht, **dadurch gekennzeichnet, daß** man ein Amin HNR₁₃R₁₄, worin R₁₃ und R₁₄ die gleichen Bedeutungen wie in Anspruch 1 besitzen, mit einem Derivat der Formel: 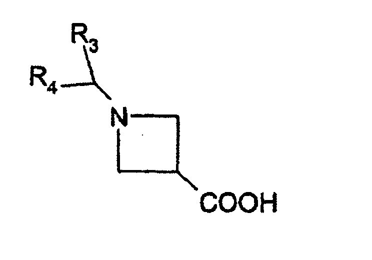 worin R₃ und R₄ die gleichen Bedeutungen wie in Formel (I) besitzen, umsetzt und das Produkt isoliert und gegebenenfalls in ein Salz mit einer anorganischen oder organischen Säure umwandelt.

26. Verfahren zur Herstellung von Verbindungen der Formel (I) nach Anspruch 1, für die R für einen CR₁R₂-Rest steht, worin R₁ für ein Wasserstoffatom steht und R₂ für einen -CH₂-CONR₁₃R₁₄-Rest steht, **dadurch gekennzeichnet, daß** man ein Amin HNR₁₃R₁₄, worin R₁₃ und R₁₄ die gleichen Bedeutungen wie in Anspruch 1 besitzen, mit einem Derivat der Formel: 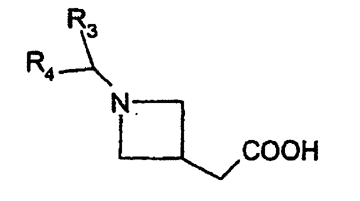 worin R₃ und R₄ die gleichen Bedeutungen wie in Anspruch 1 besitzen, umsetzt und das Produkt isoliert und gegebenenfalls in ein Salz mit einer anorganischen oder organischen Säure umwandelt.

27. Verfahren zur Herstellung von Verbindungen der Formel (I) nach Anspruch 1, für die R für einen CR₁R₂-Rest steht, worin R₁ für ein Wasserstoffatom steht und R₂ für einen -CH₂-CONR₁₃R₁₄-Rest steht, **dadurch gekennzeichnet, daß** man ein Derivat der Formel: 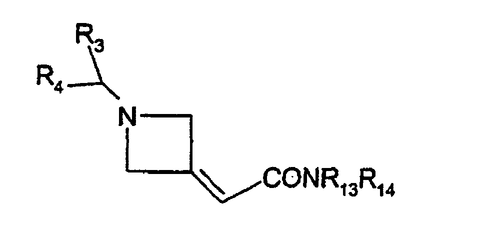 worin R₃, R₄, R₁₃ und R₁₄ die gleichen Bedeutungen wie in Anspruch 1 besitzen, reduziert und das Produkt isoliert und gegebenenfalls in ein Salz mit einer anorganischen oder organischen Säure umwandelt.

28. Verfahren zur Herstellung von Verbindungen der Formel (I) nach Anspruch 1, für die R für einen CR₁R₂-Rest steht, worin R₁ für ein Wasserstoffatom steht und R₂ für einen -SOR₆-Rest steht, **dadurch gekennzeichnet, daß** man ein Derivat der Formel: 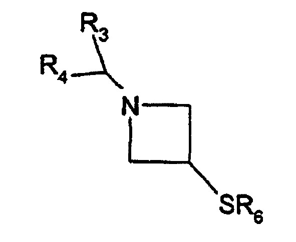 worin R₃, R₄ und R₆ die gleichen Bedeutungen wie in Anspruch 1 besitzen, oxidiert und das Produkt isoliert und gegebenenfalls in ein Salz mit einer anorganischen oder organischen Säure umwandelt.

29. Verfahren zur Herstellung von Verbindungen der Formel (I) nach Anspruch 1, für die R für einen CR₁R₂-Rest steht, worin R₁ für ein Wasserstoffatom steht und R₂ für einen -SOR₆-Rest steht, **dadurch gekennzeichnet, daß** man ein Derivat der Formel: 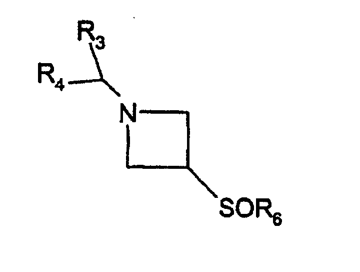 worin R₃, R₄ und R₆ die gleichen Bedeutungen wie in Anspruch 1 besitzen, oxidiert und das Produkt isoliert und gegebenenfalls in ein Salz mit einer anorganischen oder organischen Säure umwandelt.

30. Verfahren zur Herstellung von Verbindungen der Formel (I) nach Anspruch 1, für die R für einen CR₁R₂-Rest steht, worin R₁ für ein Wasserstoffatom steht und R₂ für einen -COR₆- oder -CO-CycloalkylRest steht, **dadurch gekennzeichnet, daß** man ein Derivat der Formel: 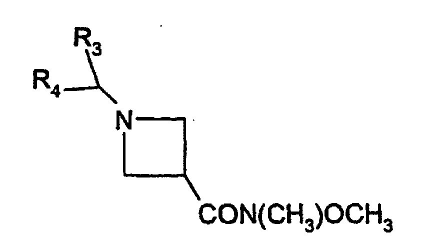 worin R₃ und R₄ die gleichen Bedeutungen wie in Anspruch 1 besitzen, mit einem Derivat RhMgBr, für das Rh die gleichen Bedeutungen wie R₆ in Anspruch 1 besitzt oder für einen C₃₋₁₀-Cycloalkylrest steht, umsetzt und das Produkt isoliert und gegebenenfalls in ein Salz mit einer anorganischen oder organischen Säure umwandelt.

31. Verfahren zur Herstellung von Verbindungen der Formel (I) nach Anspruch 1, für die R für einen CR₁R₂-Rest steht, worin R₁ für ein Wasserstoffatom steht und R₂ für einen -C(OH)(R₆)(R₁₂)- oder -C(OH)(R₆)(Alkyl)-Rest steht, **dadurch gekennzeichnet, daß** man ein Derivat der Formel: 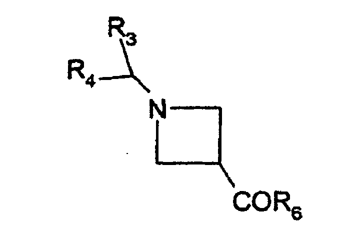 worin R₃, R₄ und R₆ die gleichen Bedeutungen wie in Anspruch 1 besitzen, mit einem Derivat RiMgBr, für das Ri die gleichen Bedeutungen wie R₁₂ in Anspruch 1 besitzt oder für einen gerad- oder verzweigtkettigen C₁₋₆-Alkylrest steht, umsetzt und das Produkt isoliert und gegebenenfalls in ein Salz mit einer anorganischen oder organischen Säure umwandelt.

32. Verfahren zur Herstellung von Verbindungen der Formel (I) nach Anspruch 1, für die R für einen CR₁R₂-Rest steht, worin R₁ für ein Wasserstoffatom steht und R₂ für einen -C(=NOalk)R₆- oder-C (=NOCH₂-CH=CH₂)R₆-Rest steht, **dadurch gekennzeichnet, daß** man ein Derivat der Formel: 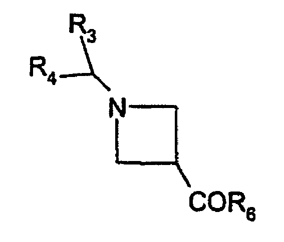 worin R₃, R₄ und R₆ die gleichen Bedeutungen wie in Anspruch 1 besitzen, mit einem Derivat RjONH₂, für das Rj für einen gerad- oder verzweigtkettigen C₁₋₆-Alkyl- oder -CH₂-CH=CH₂-Rest steht, umsetzt und das Produkt isoliert und gegebenenfalls in ein Salz mit einer anorganischen oder organischen Säure umwandelt.

33. Verfahren zur Herstellung von Verbindungen der Formel (I) nach Anspruch 1, für die R für einen CR₁R₂-Rest steht, worin R₁ für ein Wasserstoffatom steht und R₂ für einen -CH(R₆)NR₃₁R₃₂-Rest steht, wobei R₃₁ und R₃₂ für Wasserstoffatome stehen, **dadurch gekennzeichnet, daß** man Ammoniak mit einem Derivat der Formel: 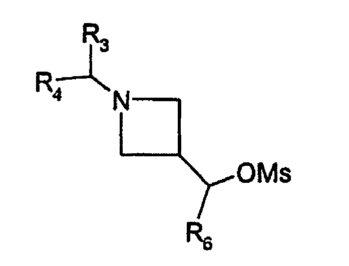 worin R₃, R₄ und R₆ die gleichen Bedeutungen wie in Anspruch 1 besitzen und Ms für einen Methylsulfonyloxyrest steht, umsetzt und das Produkt isoliert und gegebenenfalls in ein Salz mit einer anorganischen oder organischen Säure umwandelt.

34. Verfahren zur Herstellung von Verbindungen der Formel (I) nach Anspruch 1, für die R für einen CR₁R₂-Rest steht, worin R₁ für ein Wasserstoffatom steht und R₂ für einen -CH(R₆)NR₃₁R₃₂-Rest steht, wobei R₃₁ für ein Wasserstoffatom steht und R₃₂ für einen Alkyl-, Ar- oder -alk-Ar-Rest steht, **dadurch gekennzeichnet, daß** man ein Halogenid HalR₃₁ mit einer entsprechenden Verbindung der Formel (I), für die R für einen CR₁R₂-Rest steht, worin R₁ für ein Wasserstoffatom steht und R₂ für einen -CH (R₆)NR₃₁R₃₂-Rest steht und R₃₁ und R₃₂ für Wasserstoffatome stehen, umsetzt und das Produkt isoliert und gegebenenfalls in ein Salz mit einer anorganischen oder organischen Säure umwandelt.

35. Verfahren zur Herstellung von Verbindungen der Formel (I) nach Anspruch 1, für die R für einen CR₁R₂-Rest steht, worin R₁ für ein Wasserstoffatom steht und R₂ für einen -CH(R₆)NR₃₁R₃₂-Rest steht und R₃₁ und R₃₂ für Alkyl-, Ar- oder -alk-Ar-Reste stehen, **dadurch gekennzeichnet, daß** man ein Halogenid HalR₃₂ mit einer entsprechenden Verbindung der Formel (I), für die R für einen CR₁R₂-Rest steht, worin R₁ für ein Wasserstoffatom steht und R₂ für einen -CH(R₆)NR₃₁R₃₂-Rest steht und R₃₁ für ein Wasserstoffatom steht und R₃₂ für einen Alkyl-, Ar- oder -alk-Ar-Rest steht, umsetzt und das Produkt isoliert und gegebenenfalls in ein Salz mit einer anorganischen oder organischen Säure umwandelt.

36. Verfahren zur Herstellung von Verbindungen der Formel (I) nach Anspruch 1, für die R für einen CR₁R₂-Rest steht, worin R₁ für ein Wasserstoffatom steht und R₂ für einen -CH(R₆)NR₃₁R₃₂-Rest steht, R₃₁ für ein Wasserstoffatom steht und R₃₂ für einen C₂₋₆-Alkyl- oder -C₂₋₆-alk-Ar-Rest steht, **dadurch gekennzeichnet, daß** man einen Aldehyd RaCHO, für den Ra für einen Alkyl- oder -alk-Ar-Rest steht, mit einer entsprechenden Verbindung der Formel (I), für die R für einen CR₁R₂-Rest steht, worin R₁ für ein Wasserstoffatom steht und R₂ für einen -CH(R₆)NR₃₁R₃₂-Rest steht und R₃₁ und R₃₂ für Wasserstoffatome stehen, umsetzt und das Produkt isoliert und gegebenenfalls in ein Salz mit einer anorganischen oder organischen Säure umwandelt.

37. Verfahren zur Herstellung von Verbindungen der Formel (I) nach Anspruch 1, für die R für einen CR₁R₂-Rest steht, worin R₁ für ein Wasserstoffatom steht und R₂ für einen -CH(R₆)NR₃₁R₃₂-Rest steht, R₃₁ für einen Alkyl-, Ar- oder -alk-Ar-Rest steht und R₃₂ für einen C₂₋₆-Alkyl- oder -C₂₋₆-alk-Ar-Rest steht, **dadurch gekennzeichnet, daß** man einen Aldehyd RaCHO, für den Ra für einen Alkyl- oder -alk-Ar-Rest steht, mit einer entsprechenden Verbindung der Formel (I), für die R für einen CR₁R₂-Rest steht, worin R₁ für ein Wasserstoffatom steht und R₂ für einen -CH(R₆)NR₃₁R₃₂-Rest steht, R₃₁ für ein Wasserstoffatom steht und R₃₂ für einen Alkyl-, Ar- oder -alk-Ar-Rest steht, umsetzt und das Produkt isoliert und gegebenenfalls in ein Salz mit einer anorganischen oder organischen Säure umwandelt.

38. Verfahren zur Herstellung von Verbindungen der Formel (I) nach Anspruch 1, für die R für einen CR₁R₂-Rest steht, worin R₁ für ein Wasserstoffatom steht und R₂ für einen -CH(R₆)NR₃₁R₃₂-Rest steht und R₃₁ und R₃₂ mit dem Stickstoffatom, an das sie gebunden sind, einen unter Aziridinyl, Azetidinyl, Pyrrolidinyl und Piperidinyl ausgewählten Heterocyclus bilden, **dadurch gekennzeichnet, daß** man ein Dihalogenid Hal-C₂₋₅-alk-Hal mit einer entsprechenden Verbindung der Formel (I), für die R für einen CR₁R₂-Rest steht, worin R₁ für ein Wasserstoffatom steht und R₂ für einen -CH(R₆)NR₃₁R₃₂-Rest steht und R₃₁ und R₃₂ für Wasserstoffatome stehen, umsetzt und das Produkt isoliert und gegebenenfalls in ein Salz mit einer anorganischen oder organischen Säure umwandelt.

39. Verfahren zur Herstellung von Verbindungen der Formel (I) nach Anspruch 1, für die R für einen CR₁R₂-Rest steht, worin R₁ für ein Wasserstoffatom steht und R₂ für einen -CH(R₆)NHSO₂alk-Rest steht, **dadurch gekennzeichnet, daß** man ein Derivat der Formel: 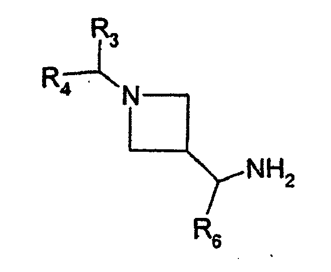 worin R₃, R₄ und R₆ die gleichen Bedeutungen wie in Anspruch 1 besitzen, mit einem Derivat ClSO₂alk, für das alk für einen gerad- oder verzweigtkettigen C₁₋₆-Alkylrest steht, umsetzt und das Produkt isoliert und gegebenenfalls in ein Salz mit einer anorganischen oder organischen Säure umwandelt.

40. Verfahren zur Herstellung von Verbindungen der Formel (I) nach Anspruch 1, für die R für einen CR₁R₂-Rest steht, worin R₁ für ein Wasserstoffatom steht und R₂ für einen -CH(R₆)NHCONHalk-Rest steht, **dadurch gekennzeichnet, daß** man ein Derivat der Formel: 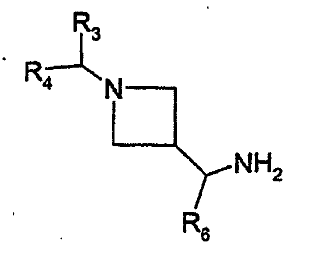 worin R₃, R₄ und R₆ die gleichen Bedeutungen wie in Anspruch 1 besitzen, mit einem Derivat alkNCO, für das alk für einen gerad- oder verzweigtkettigen C₁₋₆-Alkylrest steht, umsetzt und das Produkt isoliert und gegebenenfalls in ein Salz mit einer anorganischen oder organischen Säure umwandelt.

41. Verfahren zur Herstellung von Verbindungen der Formel (I) nach Anspruch 1, für die R für einen CR₁R₂-Rest steht, worin R₁ für ein Wasserstoffatom steht und R₂ für einen -CH(R₆)NHCOR₃₁-Rest steht, **dadurch gekennzeichnet, daß** man ein Derivat der Formel: 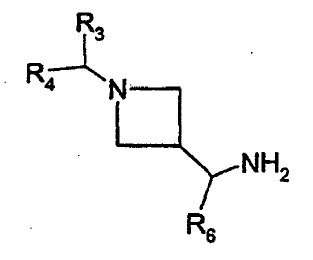 worin R₃, R₄ und R₆ die gleichen Bedeutungen wie in Anspruch 1 besitzen, mit einem Derivat R₃₁COOH, für das R₃₁ die gleichen Bedeutungen wie in Anspruch 1 besitzt, umsetzt und das Produkt isoliert und gegebenenfalls in ein Salz mit einer anorganischen oder organischen Säure umwandelt.

42. Verfahren zur Herstellung von Verbindungen der Formel (I) nach Anspruch 1, für die R für einen CR₁R₂-Rest steht, worin R₁ für ein Wasserstoffatom steht und R₂ für einen -CH₂-COR₆-Rest steht, **dadurch gekennzeichnet, daß** man ein Derivat der Formel: 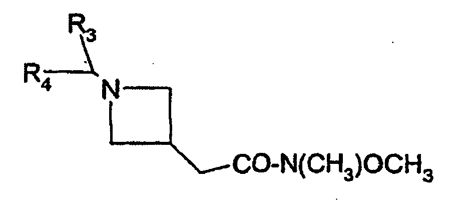 worin R₃ und R₄ die gleichen Bedeutungen wie in Anspruch 1 besitzen, mit einem Derivat der Formel R₆MgBr, für das R₆ die gleichen Bedeutungen wie in Anspruch 1 besitzt, umsetzt und das Produkt isoliert und gegebenenfalls in ein Salz mit einer anorganischen oder organischen Säure umwandelt.

43. Verfahren zur Herstellung von Verbindungen der Formel (I) nach Anspruch 1, für die R für einen CR₁R₂-Rest steht, worin R₁ für ein Wasserstoffatom steht und R₂ für einen -CH₂-CH(R₆)-NR₃₁R₃₂-Rest steht, **dadurch gekennzeichnet, daß** man ein Derivat der Formel: 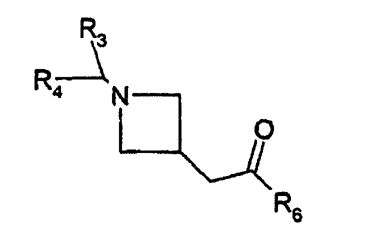 worin R₃ und R₄ die gleichen Bedeutungen wie in Anspruch 1 besitzen, mit einem Derivat der Formel NHR₃₁R₃₂, für das R₃₁ und R₃₂ die gleichen Bedeutungen wie in Anspruch 1 besitzen, umsetzt und das Produkt isoliert und gegebenenfalls in ein Salz mit einer anorganischen oder organischen Säure umwandelt.

44. Verfahren zur Herstellung von Verbindungen der Formel (I) nach Anspruch 1, für die R für einen CR₁R₂-Rest steht, worin R₁ für ein Wasserstoffatom steht und R₂ für einen -CH₂-C(=NOalk)R₆-Rest steht, **dadurch gekennzeichnet, daß** man ein Derivat der Formel: 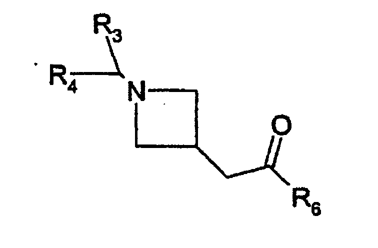 worin R₃ und R₄ die gleichen Bedeutungen wie in Anspruch 1 besitzen, mit einem Derivat der Formel alkONH₂, für das alk für einen gerad- oder verzweigtkettigen C₁₋₆-Alkylrest steht, umsetzt und das Produkt isoliert und gegebenenfalls in ein Salz mit einer anorganischen oder organischen Säure umwandelt.

45. Verfahren zur Herstellung von Verbindungen der Formel (I) nach Anspruch 1, für die R für einen CR₁R₂-Rest steht, worin R₁ für einen Cyanorest steht und R₂ für einen -C(R₈)(R₁₁)(R₁₂)-Rest steht, worin R₈ für ein Wasserstoffatom steht, **dadurch gekennzeichnet, daß** man ein Derivat der Formel: 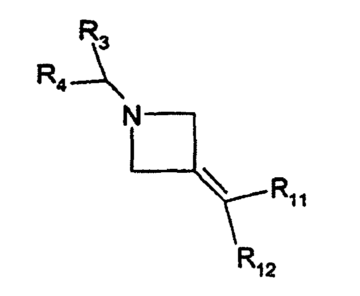 worin R₃, R₄, R₁₁ und R₁₂ die gleichen Bedeutungen wie in Anspruch 1 besitzen, mit Natriumcyanid umsetzt und das Produkt isoliert und gegebenenfalls in ein Salz mit einer anorganischen oder organischen Säure umwandelt.

46. Verfahren zur Herstellung von Verbindungen der Formel (I) nach Anspruch 1, für die R für einen CR₁R₂-Rest steht, worin R₁ für einen -S-alk-NR₁₆R₁₇-Rest steht und R₂ für einen -C(R₈)(R₁₁)(R₁₂)-Rest steht, worin R₈ für ein Wasserstoffatom steht, **dadurch gekennzeichnet, daß** man ein Derivat der Formel: 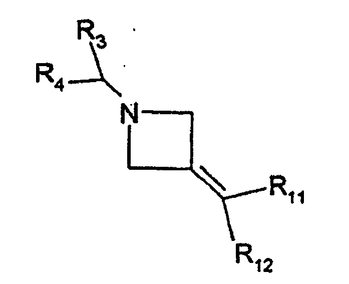 worin R₃, R₄, R₁₁ und R₁₂ die gleichen Bedeutungen wie in Anspruch 1 besitzen, mit einem Derivat HS-alk-NR₁₆R₁₇, für das alk für einen alk-Rest steht für einen gerad- oder verzweigtkettigen C₁₋₆-Alkylrest steht und R₁₆ und R₁₇ die gleichen Bedeutungen wie in Anspruch 1 besitzen, umsetzt und das Produkt isoliert und gegebenenfalls in ein Salz mit einer anorganischen oder organischen Säure umwandelt.

47. Verfahren zur Herstellung von Verbindungen der Formel (I) nach Anspruch 1, für die R für einen CR₁R₂-Rest steht, worin R₁ für einen -NHR₁₅-Rest steht und R₂ für einen -C(R₈)(R₁₁)(R₁₂)-Rest steht, worin R₈ für ein Wasserstoffatom steht, **dadurch gekennzeichnet, daß** man ein Derivat der Formel: 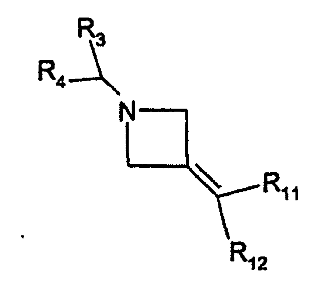 worin R₃, R₄, R₁₁ und R₁₂ die gleichen Bedeutungen wie in Anspruch 1 besitzen, mit einem Derivat H₂NR₁₅, für das R₁₅ die gleichen Bedeutungen wie in Anspruch 1 besitzt, umsetzt und das Produkt isoliert und gegebenenfalls in ein Salz mit einer anorganischen oder organischen Säure umwandelt.

48. Verfahren zur Herstellung von Verbindungen der Formel (I) nach Anspruch 1, für die R für einen CR₁R₂-Rest steht, worin R₁ für einen Alkylrest steht und R₂ für einen -C(R₈)(R₁₁)(R₁₂) -Rest steht, worin R₈ für ein Wasserstoffatom steht, **dadurch gekennzeichnet, daß** man ein Derivat der Formel: 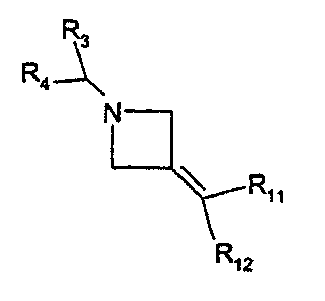 worin R₃, R₄, R₁₁ und R₁₂ die gleichen Bedeutungen wie in Anspruch 1 besitzen, mit einem Derivat alkMHal, für das alk für einen gerad- oder verzweigtkettigen C₁₋₆-Alkylrest steht und M für ein Metall steht, umsetzt und das Produkt isoliert und gegebenenfalls in ein Salz mit einer anorganischen oder organischen Säure umwandelt.

49. Verfahren zur Herstellung von Verbindungen der Formel (I) nach Anspruch 1, für die R für einen CR₁R₂-Rest steht, worin R₁ für einen -NR₂₀R₂₁-Rest steht und R₂ für einen -C(R₈)(R₁₁)(R₁₂) -Rest steht, worin R₈ für ein Wasserstoffatom steht, **dadurch gekennzeichnet, daß** man ein Derivat der Formel: 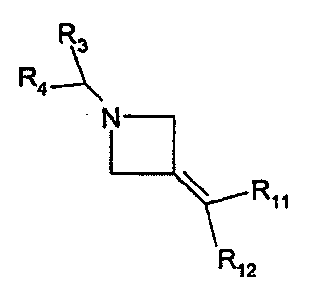 worin R₃, R₄, R₁₁ und R₁₂ die gleichen Bedeutungen wie in Anspruch 1 besitzen, mit einem Derivat HNR₂₀R₂₁, für das NR₂₀R₂₁ die gleichen Bedeutungen wie in Anspruch 1 besitzt, umsetzt und das Produkt isoliert und gegebenenfalls in ein Salz mit einer anorganischen oder organischen Säure umwandelt.

50. Verfahren zur Herstellung von Verbindungen der Formel (I) nach Anspruch 1, für die R für einen CR₁R₂-Rest steht, worin R₁ für einen -alk-NR₁₈R₁₉-Rest und R₁₈ und R₁₉ für Wasserstoffatome stehen, **dadurch gekennzeichnet, daß** man eine entsprechende Verbindung der Formel (I), für die R₁ für einen Cyanorest steht, reduziert und das Produkt isoliert und gegebenenfalls in ein Salz mit einer anorganischen oder organischen Säure umwandelt.

51. Verfahren zur Herstellung von Verbindungen der Formel (I) nach Anspruch 1, für die R für einen CR₁R₂-Rest steht, worin R₁ für einen -alk-NR₁₈R₁₉-Rest steht, R₁₈ für ein Wasserstoffatom steht und R₁₉ für einen Alkyl-, Cycloalkyl-, Cycloalkylalkyl-, Cycloalkylcarbonyl-, -SO₂alk-, -CO-NHalk-oder -COOalk-Rest steht, **dadurch gekennzeichnet, daß** man ein Halogenid HalR₁₉, worin Hal für ein Halogen steht, mit einer entsprechenden Verbindung der Formel (I), für die R für einen CR₁R₂-Rest steht, worin R₁ für einen -alk-NR₁₈R₁₉-Rest steht und R₁₈ und R₁₉ für ein Wasserstoffatom stehen, umsetzt und das Produkt isoliert und gegebenenfalls in ein Salz mit einer anorganischen oder organischen Säure umwandelt.

52. Verfahren zur Herstellung von Verbindungen der Formel (I) nach Anspruch 1, für die R für einen CR₁R₂-Rest steht, worin R₁ für einen -alk-NR₁₈R₁₉-Rest steht, R₁₈ für einen Alkylrest steht und R₁₉ für einen Alkyl-, Cycloalkyl-, Cycloalkylalkyl-, Cycloalkylcarbonyl-, -SO₂alk-, -CO-NHalk- oder -COOalk-Rest steht, **dadurch gekennzeichnet, daß** man ein Alkylhalogenid mit einer entsprechenden Verbindung der Formel (I), für die R für einen CR₁R₂-Rest steht, worin R₁ für einen -alk-NR₁₈R₁₉-Rest steht, R₁₈ für ein Wasserstoffatom steht und R₁₉ für einen Alkyl-, Cycloalkyl-, Cycloalkylalkyl-, Cycloalkylcarbonyl-, -SO₂alk-, -CO-NHalk-oder -COOalk-Rest steht, umsetzt und das Produkt isoliert und gegebenenfalls in ein Salz mit einer anorganischen oder organischen Säure umwandelt.

53. Verfahren zur Herstellung von Verbindungen der Formel (I) nach Anspruch 1, für die R für einen CR₁R₂-Rest steht, worin R₁ für ein Wasserstoffatom steht und R₂ für einen -C(R₈)(R₉)(R₁₀) - oder -C(R₈)(R₁₁)(R₁₂) -Rest steht oder R₁ für einen Alkyl-, NH-R₁₅-, Cyano-, -S-alk-NR₁₆R₁₇, -alkNR₁₈R₁₉-oder -NR₂₀R₂₁ steht und R₂ für einen -C(R₈)(R₁₁)(R₁₂) steht und R₈ für einen Alkylrest steht, **dadurch gekennzeichnet, daß** man eine entsprechende Verbindung der Formel (I), für die R₈ für ein Wasserstoffatom steht, alkyliert und das Produkt isoliert und gegebenenfalls in ein Salz mit einer anorganischen oder organischen Säure umwandelt.

54. Verfahren zur Herstellung von Verbindungen der Formel (I) nach Anspruch 1, für die R für einen C=C(R₇)SO₂alk-Rest steht, **dadurch gekennzeichnet, daß** man ein Derivat der Formel: 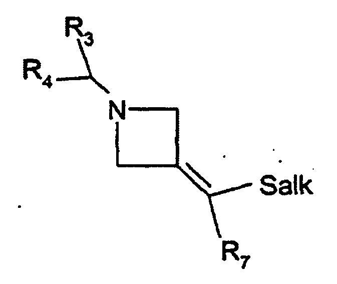 worin R₃, R₄ und R₇ die gleichen Bedeutungen wie in Anspruch 1 besitzen und alk für einen gerad- oder verzweigtkettigen C₁₋₆-Alkylrest steht, oxidiert und das Produkt isoliert und gegebenenfalls in ein Salz mit einer anorganischen oder organischen Säure umwandelt.

55. Verfahren zur Herstellung von Verbindungen der Formel (I) nach Anspruch 1, für die R für einen CR₁R₂-Rest steht, worin R₁ für ein Wasserstoffatom steht und R₂ für einen -CH(R₆)NR₃₁R₃₂-Rest steht, R₃₁ für ein Wasserstoffatom steht und R₃₂ für einen Alkylrest steht, **dadurch gekennzeichnet, daß** man eine entsprechende Verbindung der Formel (I), für die R für einen CR₁R₂-Rest steht, worin R₁ für ein Wasserstoffatom steht und R₂ für einen -CO-R₆-Rest steht, mit einem Amin HNR₃₁R₃₂, für das R₃₁ für ein Wasserstoffatom steht und R₃₂ für einen Alkylrest steht, umsetzt und das Produkt isoliert und gegebenenfalls in ein Salz mit einer anorganischen oder organischen Säure umwandelt.

56. Pharmazeutische Zusammensetzung, die als Wirkstoff mindestens eine Verbindung der Formel (I) nach einem der Ansprüche 1 bis 11 enthält.

57. Arzneimittel, das als Wirkstoff mindestens eine Verbindung der Formel (I) nach einem der Ansprüche 1 bis 11 enthält.
